# EUROPEAN PATENT APPLICATION

(11) **EP 4 527 857 A1**
(43) Date of publication of application: **26.03.2025**
(21) Application number: 23806888.6
(22) Date of filing: 15.05.2023
(51) Int. Cl.: C07K 19/00, C07K 16/28, A61P 35/00, A61K 39/395, C07K 14/71

(54) **BIFUNCTIONAL PROTEIN, AND PREPARATION THEREOF AND USE THEREOF**

(30) Priority: 17.05.2022 CN 202210541662
(71) Applicant: Suzhou Transcenta Therapeutics Co., Ltd., Suzhou, Jiangsu 215123 (CN)
(72) Inventor: ZHANG, Fan, Suzhou, Jiangsu 215123 (CN); MENG, Pan, Suzhou, Jiangsu 215123 (CN); QIAN, Xueming, Suzhou, Jiangsu 215123 (CN); LI, Hongjun, Suzhou, Jiangsu 215123 (CN); TENG, Fei, Suzhou, Jiangsu 215123 (CN); GUO, Huanhuan, Suzhou, Jiangsu 215123 (CN); GU, Yi, Suzhou, Jiangsu 215123 (CN)
(74) Representative: Bianchetti & Minoja with Trevisan & Cuonzo IPS SRL
(86) International application number: PCT/CN2023/094267
(87) International publication number: WO 2023/221936

(57) **Abstract**

Provided are a protein comprising a PD-L1 binding moiety linked to a TGF β binding moiety, an IL-1 binding moiety, an immunostimulating polypeptide (such as soluble LAG3 or soluble CD4) or a CD47 binding moiety, and a pharmaceutical preparation thereof, a preparation method thereof and the use thereof.

## Description

### CROSS REFERENCE TO RELATED APPLICATIONS

The present application claims priority from patent application CN202210541662.4 filed on May 17, 2022, the disclosure of which is incorporated herein by reference.

### FIELD OF THE INVENTION

The present disclosure relates to bi-functional protein molecules that target immune checkpoint molecules (e.g., PD-L1) and block the activity of anti-tumor immune suppression (ATIS) cytokines (e.g., IL-1 or TGFβ) or stimulate immunity, pharmaceutical formulations thereof, methods of preparing the same, and uses thereof.

### BACKGROUND

PD-L1 is often overexpressed in different tumors, and its interaction with PD-1 on T cells enables cancer cells to evade T-cell-mediated immune responses (Okazaki T, et al, Nature immunology, 2013, 14(12): 1212-1218). Thus, blocking the PD-1/PD-L1 interaction can restore T-cell activation and antitumor responses (Callahan M K, et al, Immunity, 2016, 44(5): 1069-1078). The success of antibody-based PD-1/PD-L1 blockade therapy, such as atezolizumab (Tecentriq^{®}) (Rittmeyer A, et al, The Lancet, 2017, 389(10066): 255-265), avelumab (Bavencio^{®}) (Hamilton G, et al, Expert Opinion on Biological Therapy, 2017, 17(4): 515-523) and durvalumab (Imfinzi^{®}) (Brower V, The Lancet Oncology, 2016, 17(7): e275), has provided a breakthrough in the fight against human cancers, especially for solid tumors. Although an association between PD-L1 expression by tumor cells and/or infiltrating immune cells and clinical response to PD-1/PD-L1-targeted therapies has been shown, this association is not flawless (Herbst, R., et al, Nature 515, 563-567 (2014); Taube J M, et al, Clinical cancer research, 2014, 20(19): 5064-5074). Only a minority of PD-L1-positive tumors respond to these treatments, and certain PD-L1-negative tumors are nevertheless responsive to treatment. This raises the possibility that additional factors govern patient response to PD-1/PD-L1-targeted therapies, and that additional predictive biomarkers must be identified to improve the clinical use of these agents.

Mariathasan S et al. found that lack of response was associated with a signature of transforming growth factor β (TGF-β, TGFβ) signaling in fibroblasts (Mariathasan S, et al, Nature, 2018, 554(7693): 544-548). David JM et al also found that as a pleiotropic cytokine known to induce epithelial mesenchymal transition (EMT) and suppress antitumor immunity, TGF-β could upregulate tumor PD-L1 expression in several epithelial NSCLC cell lines and the upregulation is associated with phosphorylation of Smad2, which is a key downstream effector of TGF-β signaling (David J M, et al, Oncoimmunology, 2017, 6(10): e1349589). In mouse, therapeutic administration of a TGF-β blocking antibody together with anti-PD-L1 reduced TGF-β signaling in stromal cells, facilitated T cell penetration into the center of the tumor, and provoked vigorous anti-tumor immunity and tumor regression (Mariathasan S, et al, Nature, 2018, 554(7693): 544-548).

However, low affinity of anti-PD-L1 antibodies still poses a challenge to achieve high treatment efficacy and low toxic side effect.

Therefore, there is a need for therapeutic molecules with high binding affinity to PD-L1, improved therapeutic efficacy and reduced toxic side effect, as well as pharmaceutical preparations thereof.

### SUMMARY OF THE INVENTION

The present disclosure provides proteins comprising a PD-L1 binding moiety linked to a TGFβ-binding moiety, an IL-1-binding moiety, an immunostimulatory polypeptide (e.g., soluble LAG3 or soluble CD 4), or a CD47 binding moiety, pharmaceutical formulations thereof, methods of preparing the same, and uses thereof.

The present disclosure provides a protein formulation comprising:
(1) a bi-functional molecule, comprising:
   a first moiety that binds to an immune checkpoint molecule, the first moiety preferably is an antibody against PD-L1 or an antigen-binding fragment thereof, and
   a second moiety that i) blocks activity of an immunosuppressive cytokine or ii) stimulates immunity, the second moiety preferably is TGFβ-binding moiety, IL-1-binding moiety, LAG-3-binding moiety or Sirpa-binding moiety, wherein the TGFβ-binding moiety preferably is a soluble TGFβ receptor (TGFβR) or a TGFβ-binding fragment or variant thereof, or an antibody against TGFβ and an antigen-binding fragment thereof;
(2) a buffer; and
(3) a surfactant.

More specifically, the present disclosure also provides a protein formulation, comprising:
(1) a bi-functional molecule, comprising a first moiety that binds to PD-L1 and a second moiety that i) blocks activity of an immunosuppressive cytokine or b) stimulates immunity; wherein the first moiety comprises the antibody against PD-L1 or an antigen-binding fragment thereof;
(2) a buffer; and
(3) a surfactant.

In this regard, the present disclosure also provides the following more specific embodiments:
**Embodiment 1.** A protein formulation, comprising:
   (1) a bi-functional molecule comprising a first moiety that binds to an immune checkpoint molecule and a second moiety that blocks activity of Interleukin-1 (IL-1);
   (2) a buffer; and
   (3) a surfactant.
**Embodiment 2.** The protein formulation according to Embodiment 1, wherein the first moiety comprises an agonist of immunostimulatory check point molecule, optionally selected from the group consisting of: CD27, CD70, CD28, CD80 (B7-1), CD86 (B7-2), CD40, CD40L (CD154), CD122, CD137, CD137L, OX40 (CD134), OX40L (CD252), GITR, ICOS (CD278) and ICOSLG (CD275), CD2, ICAM-1, LFA-1 (CD11a/CD18), CD30, BAFFR, HVEM, CD7, LIGHT, NKG2C, SLAMF7, NKp80, CD160 and CD83.
**Embodiment 3.** The protein formulation according to Embodiment 1, wherein the first moiety comprises an antagonist of immunoinhibitory check point molecule, optionally selected from the group consisting of: A2AR, B7-H3 (CD276), B7-H4 (VTCN1), BTLA (CD272), CTLA-4 (CD152), IDO1, IDO2, TDO, KIR, LAG3, NOX2, PD-1, PD-L1, PD-L2, TIM-3, VISTA, SIGLEC7 (CD328), TIGIT, PVR (CD155), SIGLEC9 (CD329), CD160, LAIR1, 2B4 (CD244), CD47, B7-H5.
**Embodiment 4.** The protein formulation according to Embodiment 3, wherein the immune checkpoint molecule is PD-L1.
**Embodiment 5.** The protein formulation according to any of the preceding embodiments, wherein the first moiety comprises the antibody against PD-L1 or an antigen-binding fragment thereof, and the second moiety comprises an IL-1-binding moiety or an IL-1 Receptor (IL-1R)-binding moiety.
**Embodiment 6.** The protein formulation according to Embodiment 5, wherein the IL-1-binding moiety comprises an IL-1R or a fragment or variant thereof, or an antibody against IL-1 or an antigen-binding fragment thereof.
**Embodiment 7.** The protein formulation according to Embodiment 6, wherein the antibody against IL-1 or an antigen-binding fragment thereof comprises a heavy chain variable region and/or a light variable region from an anti-IL-1α antibody or an anti-IL-1β antibody, the anti-IL-1α antibody is selected from the group consisting of: XB2001, lutikizumab, LY2189102 and bermekimab, or the anti-IL-1β antibody is selected from the group consisting of: SSGJ-613, CDP484, canakinumab and gevokizumab.
**Embodiment 8.** The protein formulation according to Embodiment 6, wherein the antibody against IL-1 or an antigen-binding fragment thereof comprises: a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.
**Embodiment 9.** The protein formulation according to Embodiment 6, wherein the antibody against IL-1 or an antigen-binding fragment thereof comprises: a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.
**Embodiment 10.** The protein formulation according to Embodiment 6, wherein the antibody against IL-1 or an antigen-binding fragment thereof comprises: a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.
**Embodiment 11.** The protein formulation according to Embodiment 6, wherein the antibody against IL-1 or an antigen-binding fragment thereof comprises: a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.
**Embodiment 12.** The protein formulation according to Embodiment 6, wherein the antibody against IL-1 or an antigen-binding fragment thereof comprises: a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.
**Embodiment 13.** The protein formulation according to Embodiment 6, wherein the antibody against IL-1 or an antigen-binding fragment thereof comprises: a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.
**Embodiment 14.** The protein formulation according to Embodiment 5, wherein the IL-1R-binding moiety comprises Interleukin-1 receptor antagonist or a fragment or variant thereof, or an antibody against IL-1R or an antigen-binding fragment thereof.
**Embodiment 15.** The protein formulation according to Embodiment 14, wherein the antibody against IL-1R or an antigen-binding fragment thereof comprises a heavy chain variable region and/or a light variable region from an antibody selected from the group consisting of: spesolimab, astegolimab, imsidolimab, AMG 108, melrilimab, nidanilimab, MEDI8968, REGN6490, HB0034 and CSC012.
**Embodiment 16.** A protein formulation, comprising:
   (1) a bi-functional molecule comprising a first moiety that binds to PD-L1 and a second moiety that a) blocks activity of an immunosuppressive cytokine or b) stimulates immunity, wherein the first moiety comprises an antibody against PD-L1 or an antigen-binding fragment thereof comprising a heavy chain variable (VH) region and/or a light chain variable (VL) region, wherein the heavy chain variable region comprises:
      a) a HCDR1 comprising **DYYMN** (SEQ ID NO: 1) or a homologous sequence of at least 80% sequence identity thereof,
      b) a HCDR2 comprising **DINPNNX₁X₂TX₃YNHKFKG** (SEQ ID NO: 19) or a homologous sequence of at least 80% sequence identity thereof, and
      c) a HCDR3 comprising **WGDGPFAY** (SEQ ID NO: 3) or a homologous sequence of at least 80% sequence identity thereof, and/or
      wherein the light chain variable region comprises:
      d) a LCDR1 comprising a sequence selected from the group consisting of **KASQNVX₄X₅X₆VA** (SEQ ID NO: 20) or a homologous sequence of at least 80% sequence identity thereof,
      e) a LCDR2 comprising a sequence selected from the group consisting of **SX₇SX₈RYT** (SEQ ID NO: 21) or a homologous sequence of at least 80% sequence identity thereof, and
      f) a LCDR3 comprising a sequence selected from the group consisting of **QQYSNYPT** (SEQ ID NO: 6) or a homologous sequence of at least 80% sequence identity thereof;
      wherein X₁ is G or A, X₂ is G or D or Q or E or L, X₃ is S or M or Q or L or V, X₄ is G or P or K, X₅ is A or G, X₆ is A or I, X₇ is A or N or R or V, and X₈ is N or H or V or D;
   (2) a buffer; and
   (3) a surfactant.
**Embodiment 17.** The protein formulation according to Embodiment 16, wherein the heavy chain variable region comprises:
   a) a HCDR1 comprising a sequence of SEQ ID NO: 1,
   b) a HCDR2 comprising a sequence selected from group consisting of SEQ ID NO: 2, SEQ ID NO: 13, SEQ ID NO: 14, SEQ ID NO: 15, SEQ ID NO: 17 and SEQ ID NO: 18, and
   c) a HCDR3 comprising a sequence of SEQ ID NO: 3,
   and/or
   a light chain variable region comprising:
   d) a LCDR1 comprising a sequence selected from the group consisting of SEQ ID NO: 4, SEQ ID NO: 7, SEQ ID NO: 8 and SEQ ID NO: 9,
   e) a LCDR2 comprising a sequence selected from the group consisting of SEQ ID NO: 5, SEQ ID NO: 10, SEQ ID NO: 11 and SEQ ID NO: 12, and
   f) a LCDR3 comprising a sequence of SEQ ID NO: 6.
**Embodiment 18.** The protein formulation according to Embodiment 17, wherein the heavy chain variable region is selected from the group consisting of:
   a) a heavy chain variable region comprising a HCDR1 comprising the sequence of SEQ ID NO: 1, a HCDR2 comprising the sequence of SEQ ID NO: 2 and a HCDR3 comprising the sequence of SEQ ID NO: 3;
   b) a heavy chain variable region comprising a HCDR1 comprising the sequence of SEQ ID NO: 1, a HCDR2 comprising the sequence of SEQ ID NO: 13 and a HCDR3 comprising the sequence of SEQ ID NO: 3;
   c) a heavy chain variable region comprising a HCDR1 comprising the sequence of SEQ ID NO: 1, a HCDR2 comprising the sequence of SEQ ID NO: 14 and a HCDR3 comprising the sequence of SEQ ID NO: 3;
   d) a heavy chain variable region comprising a HCDR1 comprising the sequence of SEQ ID NO: 1, a HCDR2 comprising the sequence of SEQ ID NO: 15 and a HCDR3 comprising the sequence of SEQ ID NO: 3; and
   e) a heavy chain variable region comprising a HCDR1 comprising the sequence of SEQ ID NO: 1, a HCDR2 comprising the sequence of SEQ ID NO: 17 and a HCDR3 comprising the sequence of SEQ ID NO: 3.
**Embodiment 19.** The protein formulation according to Embodiment 17 or 18, wherein the light variable region is selected from the group consisting of:
   a) a light chain variable region comprising a LCDR1 comprising the sequence of SEQ ID NO: 4, a LCDR2 comprising the sequence of SEQ ID NO: 5 and a LCDR3 comprising the sequence of SEQ ID NO: 6;
   b) a light chain variable region comprising a LCDR1 comprising the sequence of SEQ ID NO: 9, a LCDR2 comprising the sequence of SEQ ID NO: 5 and a LCDR3 comprising the sequence of SEQ ID NO: 6;
   c) a light chain variable region comprising a LCDR1 comprising the sequence of SEQ ID NO: 8, a LCDR2 comprising the sequence of SEQ ID NO: 5 and a LCDR3 comprising the sequence of SEQ ID NO: 6;
   d) a light chain variable region comprising a LCDR1 comprising the sequence of SEQ ID NO: 4, a LCDR2 comprising the sequence of SEQ ID NO: 12 and a LCDR3 comprising the sequence of SEQ ID NO: 6; and
   e) a light chain variable region comprising a LCDR1 comprising the sequence of SEQ ID NO: 4, a LCDR2 comprising the sequence of SEQ ID NO: 11 and a LCDR3 comprising the sequence of SEQ ID NO: 6.
**Embodiment 20.** The protein formulation according to any of Embodiments 16 to 19, wherein the antibody against PD-L1 or an antigen-binding fragment thereof further comprises one or more of heavy chain HFR1, HFR2, HFR3 and HFR4, and/or one or more of light chain LFR1, LFR2, LFR3 and LFR4, wherein:
   a) the HFR1 comprises **QVQLVQSGAEVKKPGASVKVSCKASGYX₉FT** (SEQ ID NO: 40) or a homologous sequence of at least 80% sequence identity thereof,
   b) the HFR2 comprises **WVRQAPGQX₁₀LEWMG** (SEQ ID NO: 41) or a homologous sequence of at least 80% sequence identity thereof,
   c) the HFR3 sequence comprises **RVTX₁₆TVDX₁₁SISTAYMELSRLRSDDTAVYYCX₁₂X₁₃** (SEQ ID NO: 42) or a homologous sequence of at least 80% sequence identity thereof,
   d) the HFR4 comprises **WGQGTLVTVSS** (SEQ ID NO: 25) or a homologous sequence of at least 80% sequence identity thereof,
   e) the LFR1 comprises **DIQMTQSPSSLSASVGDRVTITC** (SEQ ID NO: 26) or a homologous sequence of at least 80% sequence identity thereof,
   f) the LFR2 comprises **WYQQKPGKX₁₄PKLLIY** (SEQ ID NO: 43) or a homologous sequence of at least 80% sequence identity thereof,
   g) the LFR3 comprises **GVPX₁₅RFSGSGSGTDFTX₁₇TISSLQPEDIATYYC** (SEQ ID NO: 44) or a homologous sequence of at least 80% sequence identity thereof
   h) the LFR4 comprises **FGQGTKLEIK** (SEQ ID NO: 29) or a homologous sequence of at least 80% sequence identity thereof,
   wherein X₉ is T or V, X₁₀ is G or S, X₁₁ is T or K, X₁₂ is A or V, X₁₃ is R or K, X₁₄ is A or S, X₁₅ is S or D, X₁₆ is M or V, and X₁₇ is F or L.
**Embodiment 21.** The protein formulation according to Embodiment 20, wherein:
   the HFR1 comprises a sequence selected from the group consisting of SEQ ID NOs: 22 and 30,
   the HFR2 comprises a sequence selected from the group consisting of SEQ ID NOs: 23 and 31,
   the HFR3 comprises a sequence selected from the group consisting of SEQ ID NOs: 24 and 32-35,
   the HFR4 comprises a sequence of SEQ ID NOs: 25,
   the LFR1 comprises a sequence from the group consisting of SEQ ID NO: 26,
   the LFR2 comprises a sequence selected from the group consisting of SEQ ID NOs: 27 and 36,
   the LFR3 comprises a sequence selected from the group consisting of SEQ ID NOs: 28, and 37-38, 39, 45, and
   the LFR4 comprises a sequence of SEQ ID NO: 29.
**Embodiment 22.** The protein formulation according to any of Embodiments 16 to 21, wherein the heavy chain variable region comprises a sequence selected from the group consisting of SEQ ID NO: 46, SEQ ID NO: 49, SEQ ID NO: 50, SEQ ID NO: 51, SEQ ID NO: 52, SEQ ID NO: 56, SEQ ID NO: 57, SEQ ID NO: 58, SEQ ID NO: 59, SEQ ID NO: 60 and a homologous sequence thereof having at least 80% sequence identity thereof.
**Embodiment 23.** The protein formulation according to any of Embodiments 16 to 22, wherein the light variable region comprises a sequence selected from group consisting of SEQ ID NO: 47, SEQ ID NO: 54, SEQ ID NO: 55, SEQ ID NO: 61, SEQ ID NO: 62, SEQ ID NO: 63, SEQ ID NO: 64, SEQ ID NO: 65 and a homologous sequence thereof having at least 80% sequence identity thereof.
**Embodiment 24.** The protein formulation according to Embodiment 16, wherein the antibody against PD-L1 or an antigen-binding fragment thereof comprises a pair of heavy chain variable region and light chain variable region sequences selected from the group consisting of: SEQ ID NO: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64 and 60/65 and a homologous sequence thereof having at least 80% sequence identity thereof.
**Embodiment 25.** according to Embodiments 16 to 24 any of The protein formulation, wherein the antibody against PD-L1 or an antigen-binding fragment thereof further comprises one or more amino acid residue substitutions or modifications yet retains specific binding specificity and/or affinity to PD-L1.
**Embodiment 26.** The protein formulation according to Embodiment 25, wherein at least one of the substitutions or modifications is in one or more of the CDR sequences, and/or in one or more of the non-CDR regions of the VH or VL sequences.
**Embodiment 27.** The protein formulation according to any of Embodiments 16 to 26, wherein the antibody against PD-L1 or antigen-binding fragment thereof further comprises an immunoglobulin constant region, optionally a constant region of human Ig, or optionally a constant region of human IgG.
**Embodiment 28.** The protein formulation according to Embodiment 27, wherein the constant region comprises an Fc region of human IgG1, IgG2, IgG3, or IgG4.
**Embodiment 29.** The protein formulation according to Embodiment 28, wherein the constant region comprises Fc variant having reduced effector function relative to the corresponding wildtype Fc region.
**Embodiment 30.** The protein formulation according to Embodiment 29, wherein the Fc variant comprises one or more amino acid residue substitutions selected from the group consisting of: 220S, 226S, 228P, 229S, 233P, 234V, 234G, 234A, 234F, 234A, 235A, 235G, 235E, 236E, 236R, 237A, 237K, 238S, 267R, 268A, 268Q, 269R, 297A, 297Q, 297G, 309L, 318A, 322A, 325L, 328R, 330S, 331S and any combination thereof, wherein the numbering of the residues in the Fc region is that of the EU index as in Kabat.
**Embodiment 31.** The protein formulation according to any of Embodiments 29 to 30, wherein the Fc variant comprises a combination of mutations selected from the group consisting of: a) K322A, L234A and L235A; b) P331S, L234F and L235E; c) L234A and L235A; c) N297A; d) N297Q; e) N297G; f) L235E; g) L234A and L235A (IgG1); h) F234A and L235A (IgG4); i) H268Q, V309L, A330S and P331S (IgG2); j) V234A, G237A, P238S, H268A, V309L, A330S and P331S (IgG2), wherein the numbering of the residues in the Fc region is that of the EU index as in Kabat.
**Embodiment 32.** The protein formulation according to any of Embodiments 29 to 31, wherein the Fc variant comprises an amino acid sequence of SEQ ID NO: 81.
**Embodiment 33.** The protein formulation according to Embodiments any of 16 to 32, wherein the antibody against PD-L1 or an antigen-binding fragment thereof is humanized.
**Embodiment 34.** The protein formulation according to any of Embodiments 16 to 33, wherein the antigen-binding fragment is a diabody, a Fab, a Fab', a F(ab')₂, a Fd, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a multispecific antibody, a camelized single domain antibody, a nanobody, a domain antibody, and a bivalent domain antibody.
**Embodiment 35.** The protein formulation according to any of Embodiments 16 to 34, wherein the antibody or an antigen-binding fragment thereof is capable of binding to both human PD-L1 and cyno PD-L1.
**Embodiment 36.** The protein formulation according to any of Embodiments 1 to 15, wherein the first moiety comprises an antibody or an antigen-binding fragment thereof that competes for binding to PD-L1 with the antibody or antigen-binding fragment thereof of any of Embodiments 16-35.
**Embodiment 37.** The protein formulation according to any of embodiments 16 to 36, wherein the immunosuppressive cytokine comprises a cytokine in transforming growth factor beta (TGF-β) superfamily, IL-1, or Vascular endothelial growth factor (VEGF).
**Embodiment 38.** The protein formulation according to Embodiment 37, wherein the immunosuppressive cytokine in TGF-β superfamily includes TGF-β, bone morphogenetic proteins (BMPs), activins, NODAL, and growth and differentiation factors (GDFs).
**Embodiment 39.** The protein formulation according to any of embodiments 16 to 38, wherein the immunosuppressive cytokine is TGF-β.
**Embodiment 40.** The protein formulation according to embodiments 16 to 39, wherein the second moiety comprises TGFβ-binding moiety.
**Embodiment 41.** The protein formulation according to Embodiment 40, wherein the TGFβ-binding moiety comprises a soluble TGFβ receptor (TGFβR) or a TGFβ-binding fragment or variant thereof, or an antibody against TGFβ and an antigen-binding fragment thereof.
**Embodiment 42.** The protein formulation according to Embodiment 41, wherein the soluble TGFβR comprises an extracellular domain (ECD) of the TGFβR or a TGFβ-binding fragment or variant thereof.
**Embodiment 43.** The protein formulation according to Embodiment 42, wherein the TGFβR is selected from the group consisting of: TGFβ Receptor I (TGFβRI), TGFβ Receptor II (TGFβRII), TGFβ Receptor III (TGFβRIII) and any combination thereof.
**Embodiment 44.** The protein formulation according to Embodiment 42, wherein the TGFβR is TGFβRII.
**Embodiment 45.** The protein formulation according to Embodiment 44, wherein the TGFβRII selectively binds to TGFβ1 over TGFβ2 and TGFβ3.
**Embodiment 46.** The protein formulation according to Embodiment 45, wherein the TGFβ1 is human TGFβ1 or mouse TGFβ1.
**Embodiment 47.** The protein formulation according to any of Embodiments 42 to 46, wherein the ECD of TGFβR comprises an amino acid sequence of SEQ ID NO: 66, 79, 78, 77 or a sequence having at least 80% sequence identity thereof yet retains specific binding specificity and/or affinity to TGF-β.
**Embodiment 48.** The protein formulation according to Embodiment 36, wherein the second moiety comprises an IL-1-binding moiety or an IL-1 Receptor (IL-1R)-binding moiety.
**Embodiment 49.** The protein formulation according to Embodiment 48, wherein the IL-1-binding moiety comprises a soluble IL-1R, an IL-1-binding fragment or variant of an IL-1R, or an antibody against IL-1 or an antigen-binding fragment thereof.
**Embodiment 50.** The protein formulation according to Embodiment 49, wherein the antibody against IL-1 or an antigen-binding fragment thereof comprises a heavy chain variable region and/or a light variable region from an anti-IL-1α antibody or an anti-IL-1β antibody, the anti-IL-1α antibody is selected from the group consisting of: XB2001, lutikizumab, LY2189102 and bermekimab, or the anti-IL-1β antibody is selected from the group consisting of: SSGJ-613, CDP484, canakinumab and gevokizumab.
**Embodiment 51.** The protein formulation according to Embodiment 49, wherein the antibody against IL-1 or an antigen-binding fragment thereof comprises: a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.
**Embodiment 52.** The protein formulation according to Embodiment 49, wherein the antibody against IL-1 or an antigen-binding fragment thereof comprises: a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.
**Embodiment 53.** The protein formulation according to Embodiment 49, wherein the antibody against IL-1 or an antigen-binding fragment thereof comprises: a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.
**Embodiment 54.** The protein formulation according to Embodiment 49, wherein the antibody against IL-1 or an antigen-binding fragment thereof comprises: a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.
**Embodiment 55.** The protein formulation according to Embodiment 49, wherein the antibody against IL-1 or an antigen-binding fragment thereof comprises: a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.
**Embodiment 56.** The protein formulation according to Embodiment 49, wherein the antibody against IL-1 or an antigen-binding fragment thereof comprises: a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.
**Embodiment 57.** The protein formulation according to Embodiment 49, wherein the bi-functional molecule comprises a heavy chain comprising an amino acid sequence of SEQ ID NO: 118 or SEQ ID NO: 120, and/or a light chain comprising an amino acid sequence of SEQ ID NO: 119 or SEQ ID NO: 121.
**Embodiment 58.** The protein formulation according to Embodiment 49, wherein the IL-1-binding moiety comprises an extracellular domain (ECD) of the IL-1RI, an IL-1-binding fragment or variant of any of IL-1RI, ECD of IL-1RI, IL-1RII, or ECD of IL-1RII, or IL-1RAP, or ECD of IL-1RAP, IL-1sRI or IL-1sRII.
**Embodiment 59.** The protein formulation according to Embodiment 48, wherein the IL-1R-binding moiety comprises an IL-1Ra or an IL-1-binding fragment or variant thereof, or an antibody against IL-1R or an antigen-binding fragment thereof.
**Embodiment 60.** The protein formulation ccording to Embodiment 59, wherein the antibody against IL-1R or an antigen-binding fragment thereof comprises a heavy chain variable region and/or a light variable region form an antibody selected from the group consisting of: spesolimab, astegolimab, imsidolimab, AMG 108, melrilimab, nidanilimab, MEDI8968, REGN6490, HB0034 and CSC012.
**Embodiment 61.** The protein formulation according to Embodiment 60, wherein the IL-1R-binding moiety comprises an amino acid sequence of SEQ ID NO: 67 or 76, or an amino acid sequence having at least 80% sequence identity to SEQ ID NO: 67 or 76, or an IL-1 binding fragment or variant thereof.
**Embodiment 62.** The protein formulation according to any of Embodiments 48 to 49, wherein the IL-1 is IL-1α or IL-1β.
**Embodiment 63.** The protein formulation according to Embodiment 62, wherein the IL-1β is human IL-1β.
**Embodiment 64.** The protein formulation according to any of Embodiments 16 to 36, wherein the second moiety stimulates anti-tumor immunity and comprises an immunostimulatory polypeptide.
**Embodiment 65.** The protein formulation according to Embodiment 64, wherein the immunostimulatory polypeptide comprises Interleukin (IL) -2 (IL-2), IL-15, IL-21, IL-10, IL-12, IL-23, IL-27, IL-35, granulocyte-macrophage colony-stimulating factor (GM-CSF), soluble CD4, soluble LAG-3, IFN-α or a functional equivalent thereof.
**Embodiment 66.** The protein formulation according to Embodiment 65, wherein the soluble LAG-3 comprises the LAG-3 an extracellular domain (ECD) of or a MHCII-binding variant thereof.
**Embodiment 67.** The protein formulation according to any of Embodiments 16 to 36, wherein the second moiety stimulates anti-tumor immunity and comprises an antagonist of an immunoinhibitory receptor signaling.
**Embodiment 68.** The protein formulation according to Embodiment 67, wherein the immunoinhibitory receptor is Signal-regulatory protein alpha (SIRPα).
**Embodiment 69.** The protein formulation according to Embodiment 68, wherein the second moiety blocks interaction between CD47 and SIRPα.
**Embodiment 70.** The protein formulation according to Embodiment 69, wherein the second moiety comprises CD47 binding domain or SIRPα binding domain.
**Embodiment 71.** The protein formulation according to Embodiment 70, wherein the CD47 binding domain comprises a soluble SIRPα or a CD47 binding fragment or variant thereof, or an anti-CD47 antibody or an antigen-binding fragment thereof.
**Embodiment 72.** The protein formulation according to Embodiment 71, wherein the soluble SIRPα comprises an extracellular domain (ECD) of the SIRPα or a CD47-binding variant thereof.
**Embodiment 73.** The protein formulation according to Embodiment 70, wherein the CD47 binding domain comprises an amino acid sequence of SEQ ID NO: 84 or an amino acid sequence having at least 80% sequence identity thereof yet retaining binding specificity to CD47.
**Embodiment 74.** The protein formulation according to Embodiment 70, wherein the SIRPα binding domain comprises a soluble CD47 or a SIRPα binding fragment or variant thereof, or an anti-SIRPα antibody or an antigen-binding fragment thereof.
**Embodiment 75.** The protein formulation according to Embodiment 74, wherein the soluble CD47 comprises an extracellular domain (ECD) of the CD47 or a SIRPα binding fragment or variant thereof, an anti-SIRPα antibody or an antigen-binding fragment thereof.
**Embodiment 76.** The protein formulation according to any of the preceding embodiments, further comprising a linker connecting the first moiety and the second moiety.
**Embodiment 77.** The protein formulation according to Embodiment 76, wherein the linker is selected from the group consisting of a cleavable linker, a non-cleavable linker, a peptide linker, a flexible linker, a rigid linker, a helical linker, and a non-helical linker.
**Embodiment 78.** The protein formulation according to Embodiment 77, wherein the linker comprising an amino acid sequence of ((G)nS)m, wherein m and n are independently an integer selected from 0 to 30.
**Embodiment 79.** The protein formulation according to any of Embodiments 16 to 78, wherein the bi-functional molecule comprises one or more of the second moieties.
**Embodiment 80.** The protein formulation according to Embodiment 79, wherein at least one of the second moieties is linked to an N terminus or a C terminus of a polypeptide chain of the first moiety.
**Embodiment 81.** The protein formulation according to Embodiment 79, wherein at least one of the second moieties is linked to: a) an N terminus or a C terminus of a heavy chain of the first moiety, or b) an N terminus or a C terminus of a light chain of the first moiety.
**Embodiment 82.** The protein formulation according to Embodiment 79, wherein at least one of the second moieties is linked to a C terminus of a heavy chain constant region of the first moiety.
**Embodiment 83.** The protein formulation according to Embodiment 79, wherein each of the second moieties is linked respectively to the C terminus of each heavy chain constant region of the first moiety.
**Embodiment 84.** The protein formulation according to Embodiment 79, wherein the bi-functional molecule comprises more than one of the second moieties that are linked respectively to: an N terminus of a heavy chain of the first moiety, a C terminus of a heavy chain of the first moiety, an N terminus of a light chain of the first moiety, a C terminus of a light chain of the first moiety, or any combination thereof.
**Embodiment 85.** The protein formulation according to any of Embodiments 79 to 84, wherein the bi-functional molecule comprises homodimeric or heterodimeric heavy chains.
**Embodiment 86.** The protein formulation according to any of Embodiments 79 to 84, wherein the heavy chains are heterodimeric with respect to presence or position of the second moiety.
**Embodiment 87.** The protein formulation according to Embodiment 86, wherein the heterodimeric heavy chains comprise one heavy chain having the second moiety but the other heavy chain having not.
**Embodiment 88.** The protein formulation according to Embodiment 86, wherein the heterodimeric heavy chains further comprise heterodimeric Fc regions that associate in a way that discourages homodimerization and/or favors heterodimerization.
**Embodiment 89.** The protein formulation according to Embodiment 86 T, wherein the heterodimeric Fc regions are capable of associating into heterodimers via knobs-into-holes, hydrophobic interaction, electrostatic interaction, hydrophilic interaction, or increased flexibility.
**Embodiment 90.** The protein formulation according to any of Embodiments 86 to 89, wherein the heterodimeric Fc regions comprise Y349C, T366S, L368A or Y407V or any combination thereof in one Fc polypeptide chain, and S354C, or T366W or combination thereof in another Fc polypeptide chain, wherein the numbering of the residues in the Fc polypeptide chain is that of the EU index as in Kabat.
**Embodiment 91.** The protein formulation according to any of the preceding embodiments, further linked to one or more conjugate moieties.
**Embodiment 92.** The protein formulation according to Embodiment 91, wherein the conjugate moiety comprises a clearance-modifying agent, a chemotherapeutic agent, a toxin, a radioactive isotope, a lanthanide, a luminescent label, a fluorescent label, an enzyme-substrate label, a DNA-alkylator, a topoisomerase inhibitor, a tubulin-binders, or other anticancer drugs such as androgen receptor inhibitor.
**Embodiment 93.** The protein formulation according to any of Embodiments 16 to 35, 37 to 47 and 76 to 92, wherein the first moiety of the bi-functional molecule comprises the antibody against PD-L1 or an antigen-binding fragment thereof, and the second moiety comprises a TGFβ-binding moiety,
   wherein the antibody against PD-L1 or an antigen-binding fragment thereof comprises a pair of heavy chain variable region and light chain variable region sequences selected from the group consisting of: SEQ ID NO: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64 and 60/65 and a homologous sequence thereof having at least 80% sequence identity thereof; and/or
   wherein the TGFβ-binding moiety comprises a soluble TGFβ receptor (TGFβR) or a TGFβ-binding fragment or variant thereof, wherein the soluble TGFβR comprises an extracellular domain (ECD) of the TGFβR or a TGFβ-binding fragment or variant thereof, and the ECD of TGFβR comprises an amino acid sequence of SEQ ID NO: 66, 79, 78, 77 or a sequence having at least 80% sequence identity thereof yet retains specific binding specificity and/or affinity to TGF-β.
**Embodiment 94.** The protein formulation according to Embodiment 93, wherein the first moiety of the bi-functional molecule comprises the antibody against PD-L1 or an antigen-binding fragment thereof, and the second moiety comprises TGFβ-binding moiety,
   wherein the antibody against PD-L1 or an antigen-binding fragment thereof comprises a pair of heavy chain variable region and light chain variable region sequences selected from the group consisting of: SEQ ID NO: 59/64 and a homologous sequence thereof having at least 80% sequence identity thereof; and/or
   wherein the TGFβ-binding moiety comprises a soluble TGFβ receptor (TGFβR) or a TGFβ-binding fragment or variant thereof, wherein the soluble TGFβR comprises an extracellular domain (ECD) of the TGFβR or a TGFβ-binding fragment or variant thereof, and the ECD of TGFβR comprises an amino acid sequence of SEQ ID NO: 66 or a sequence having at least 80% sequence identity thereof yet retains specific binding specificity and/or affinity to TGF-β.
**Embodiment 95.** The protein formulation according to any of the preceding embodiments, wherein the concentration of the bi-functional molecule is about 1-200 mg/mL, for example about 1-150 mg/mL, about 100-200 mg/mL, about 1-100 mg/mL, about 10-100 mg/mL, about 10-50 mg/mL, about 20-30 mg/mL, about 20 mg/mL or about 30 mg/mL.
**Embodiment 96.** The protein formulation according to any of the preceding embodiments, wherein the buffer is acetate, histidine, citrate, succinate, malate, glutamate, phosphate, or lactate buffer, for example acetate buffer or histidine buffer; the acetate buffer for example, is acetic acid-sodium acetate buffer; the histidine buffer for example, is histidine-hydrochloric acid buffer.
**Embodiment 97.** The protein formulation according to any of the preceding embodiments, wherein the concentration of the buffer is about 5-100 mM, for example about 10-50 mM, about 15-30 mM, about 10-20 mM, about 25 mM or about 20 mM.
**Embodiment 98.** The protein formulation according to any of the preceding embodiments, wherein the buffer is acetate buffer, for example acetic acid-sodium acetate buffer at a concentrated of about 10-50 mM, about 15-30 mM or about 20 mM.
**Embodiment 99.** The protein formulation according to any of the preceding embodiments, wherein the buffer is histidine buffer, for example histidine-hydrochloric acid buffer at a concentration of about 10-50 mM, about 15-30 mM or about 20 mM.
**Embodiment 100.** The protein formulation according to any of the preceding embodiments, wherein the pH of said formulation is in the range of 4.5-6.0, for example about 5.0-5.6, about 5.0-5.5, about 5.3, or 5.3±5%.
**Embodiment 101.** The protein formulation according to any of the preceding embodiments, wherein the surfactant is a nonionic surfactant, for example polysorbate, poloxamer, Brij, or Triton X, for example, polysorbate 80 or polysorbate 20.
**Embodiment 102.** The protein formulation according to any of the preceding embodiments, wherein the concentration of the surfactant is about 0.01-0.5% (w/v), for example about 0.01-0.1% (w/v), 0.02-0.1% (w/v), about 0.025%-0.1% (w/v) or about 0.05% (w/v).
**Embodiment 103.** The protein formulation according to any of the preceding embodiments, wherein the surfactant is polysorbate, for example, polysorbate 80 or polysorbate 20 at a concentration of about 0.01-0.5% (w/v), about 0.01-0.1% (w/v), 0.02-0.1% (w/v), about 0.025%-0.1% (w/v), or about 0.05% (w/v).
**Embodiment 104.** The protein formulation according to any of the preceding embodiments, wherein the surfactant is polysorbate 80 at a concentration of about 0.01-0.5% (w/v), about 0.01-0.1% (w/v), 0.02-0.1% (w/v), about 0.025%-0.1% (w/v), or about 0.05% (w/v).
**Embodiment 105.** The protein formulation according to any of the preceding embodiments, wherein the formulation comprises a stabilizer.
**Embodiment 106.** The protein formulation according to any of the preceding embodiments, wherein the formulation comprises a stabilizer selected from one or more of an amino acid, an inorganic salt, a sugar, a polyol and a chelating agent, preferably arginine hydrochloride, arginine or NaCl.
**Embodiment 107.** The protein formulation according to any of the preceding embodiments, wherein the formulation comprises a stabilizer at a concentration of about 10-300 mM, for example about 100-300 mM, about 120-300 mM, about 100-200 mM, about 130-170 mM, about 140-160 mM or about 150 mM.
**Embodiment 108.** The protein formulation according to any of the preceding embodiments, wherein the formulation comprises arginine hydrochloride or arginine.
**Embodiment 109.** The protein formulation according to any of the preceding embodiments, wherein the formulation comprises arginine hydrochloride or arginine at a concentration of about 100-200 mM, about 130-170 mM, about 140-160 mM or about 150 mM.
**Embodiment 110.** The protein formulation according to any of the preceding embodiments, wherein the formulation comprises arginine hydrochloride at a concentration of about 100-200 mM, about 130-170 mM, about 140-160 mM or about 150 mM.
**Embodiment 111.** The protein formulation according to any of the preceding embodiments, wherein the formulation comprises arginine hydrochloride at a concentration of about 130-170 mM, about 140-160 mM or about 150 mM_{∘}
**Embodiment 112.** The protein formulation according to any of the preceding embodiments, wherein the formulation comprises NaCl.
**Embodiment 113.** The protein formulation according to any of the preceding embodiments, wherein the formulation comprises NaCl at a concentration of about 100-200 mM, about 130-170 mM or about 135 mM.
**Embodiment 114.** The protein formulation according to any of the preceding embodiments, wherein the formulation optionally comprises chelating agents, for example EDTA•2Na, at a concentration of for example about 30-350 µM, for example 75-300 µM.
**Embodiment 115.** The protein formulation according to any of the preceding embodiments, wherein the formulation comprises polysorbate, for example polysorbate 80 or polysorbate 20 at a concentration of about 0.01-0.5% (w/v), about 0.01-0.1% (w/v), 0.02-0.1% (w/v), about 0.025%-0.1% (w/v), or about 0.05% (w/v), and EDTA•2Na at a concentration of about 30-350 µM, for example 75-300 µM.
**Embodiment 116.** The protein formulation according to any of the preceding embodiments, wherein the formulation comprises about 0.025%-0.1% (w/v) (e.g., about 0.05% (w/v)) of polysorbate (e.g., polysorbate 80) and about 30-350 µM of EDTA•2Na.
**Embodiment 117.** The protein formulation according to any of the preceding embodiments, comprising:
   10-50 mg/mL of the bi-functional molecule;
   10-50 mM of a buffer;
   0.025%-0.1% (w/v) of polysorbate 80 or polysorbate 20;
   100-200 mM of arginine hydrochloride; and
   optionally 30-350 µM of EDTA•2Na;
   the pH is 5.0-5.6.
**Embodiment 118.** The protein formulation according to any of the preceding embodiments, comprising:
   10-50 mg/mL of the bi-functional molecule;
   10-50 mM of an acetate buffer or histidine buffer;
   0.025%-0.1% (w/v) of polysorbate 80 or polysorbate 20;
   100-200 mM of arginine hydrochloride; and
   optionally 30-350 µM of EDTA•2Na;
   the pH is 5.0-5.6.
**Embodiment 119.** The protein formulation according to any of the preceding embodiments, comprising:
   10-50 mg/mL of the bi-functional molecule;
   10-50 mM of an acetate buffer or histidine buffer;
   0.025%-0.1% (w/v) of polysorbate 80 or polysorbate 20;
   130-170 mM of arginine hydrochloride; and
   optionally 30-350 µM of EDTA•2Na;
   the pH is 5.0-5.6.
**Embodiment 120.** The protein formulation according to any of the preceding embodiments, comprising:
   10-50 mg/mL of the bi-functional molecule;
   10-50 mM of an acetate buffer or histidine buffer;
   0.025%-0.1% (w/v) of polysorbate 80 or polysorbate 20;
   140-160 mM of arginine hydrochloride; and
   optionally 30-350 µM of EDTA•2Na;
   the pH is 5.3±5%.
**Embodiment 121.** The protein formulation according to any of the preceding embodiments, comprising:
   10-50 mg/mL of the bi-functional molecule;
   10-50 mM of an acetate buffer or histidine buffer;
   0.025%-0.1% (w/v) of polysorbate 80 or polysorbate 20; and
      140-160 mM of arginine hydrochloride;
   the pH is 5.3±5%.
**Embodiment 122.** The protein formulation according to any of the preceding embodiments, comprising:
   20-30 mg/mL of the bi-functional molecule;
   20-30 mM of an acetate buffer or histidine buffer;
   0.025%-0.1% (w/v) of polysorbate 80; and
      140-160 mM of arginine hydrochloride;
   the pH is 5.3±5%.
**Embodiment 123.** The protein formulation according to any of the preceding embodiments, comprising:
   30 mg/mL of the bi-functional molecule;
   20 mM of an acetate buffer or histidine buffer;
   0.05% (w/v) of polysorbate 80; and
      150 mM of arginine hydrochloride;
   the pH is 5.3.
**Embodiment 124.** The protein formulation according to any of the preceding embodiments, wherein the bi-functional molecule is treated with a chelating agent, for example EDTA•2Na (e.g., about 0.3-10 mM, about 0.1-20 mM, about 0.3-15 mM, about 0.3-10 mM or about 0.5-5 mM of EDTA•2Na) during the preparation process, and then the chelating agent is removed; for example, said treatment is carried out in a buffer at room temperature; for example, the chelating agent is removed by dialysis, ultrafiltration or diafiltration (e.g. dialysis with a buffer); optionally, other excipients are added after removing the chelating agent to give the formulation.
**Embodiment 125.** The protein formulation according to any of the preceding embodiments, wherein the bifunctional molecule is treated with a chelating agent, for example, EDTA•2Na (e.g., about 0.3-10 mM, about 0.1-20 mM, about 0.3-15 mM, about 0.3-10 mM or about 0.5-5 mM 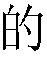 EDTA•2Na) in a buffer at room temperature (for example, overnight) during the preparation process, and then the chelating agent is removed by dialysis with a buffer, ultrafiltration, or diafiltration.
**Embodiment 126.** The protein formulation according to any of the preceding embodiments, wherein said bifunctional molecule is treated with a chelating agent, for example EDTA•2Na (e.g., about 0.3-10 mM, about 0.1-20 mM, about 0.3-15 mM, about 0.3-10 mM or about 0.5-5 mM 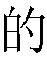 EDTA•2Na) in a buffer at room temperature (for example, overnight) during the preparation process, and then the chelating agent is removed by dialysis with a buffer, ultrafiltration, or diafiltration, and then other pharmaceutically acceptable excipients are added to give the formulation.
**Embodiment 127.** The protein formulation according to any of the preceding embodiments, which is in a liquid form, a lyophilized form or a liquid form reconstituted from a lyophilized form.
**Embodiment 128.** The protein formulation according to any of the preceding embodiments, which is an injection, for example an intravenous injection, an intramuscular injection or a subcutaneous injection.
**Embodiment 129.** The protein formulation according to any of the preceding embodiments, which is a liquid injection.
**Embodiment 130.** The protein formulation according to any of Embodiments 1 to 129, which is used for treating, preventing, or alleviating PD-L1-related diseases in a subject.
**Embodiment 131.** The protein formulation used according to Embodiment 130, wherein the disease is an immune-related disease or condition, cancer, or an infectious disease.
**Embodiment 132.** The protein formulation used according to Embodiment 131, wherein the cancer is selected from the group consisting of: lung cancer (e.g., non-small cell lung cancer), liver cancer, pancreatic cancer, breast cancer, bronchial cancer, bone cancer, liver and bile duct cancer, ovarian cancer, testicle cancer, kidney cancer, bladder cancer, head and neck cancer, spine cancer, brain cancer, cervix cancer, uterine cancer, endometrial cancer, colon cancer, colorectal cancer, prostate cancer, gastric-esophageal cancer, rectal cancer, anal cancer, gastrointestinal cancer, skin cancer, pituitary cancer, stomach cancer, vagina cancer, thyroid cancer, glioblastoma, astrocytoma, melanoma, myelodysplastic syndrome, sarcoma, teratoma, glioma, and adenocarcinoma.
**Embodiment 133.** The protein formulation used according to any of Embodiments 130 to 132, wherein the subject has been identified as having a PD-L1-expressing cancer cell.
**Embodiment 134.** The protein formulation used according to any of Embodiments 130 to 133, wherein the PD-L1-related disease is resistant to PD-L1/PD-1 mono therapy.
**Embodiment 135.** The protein formulation used according to any of Embodiments 130 to 134, wherein the subject is human.
**Embodiment 136.** The protein formulation according to any of Embodiments 1 to 129, which is used for treating, preventing, or alleviating a disease or condition that would benefit from suppression of an immunosuppressive cytokine, from induction of sustained immune responses, or from stimulation of anti-tumor immunity;
   wherein, for example, the immunosuppressive cytokine is TGFβ; for example, said disease or condition is a TGFβ-related disease or condition; for example, said TGFβ-related disease is cancer, fibrotic disease, or kidney disease;
   wherein, for example, the immunosuppressive cytokine is IL-1; for example, said disease or condition is an IL-1-related disease or condition;
   wherein, for example, said disease or condition would benefit from induction of sustained immune responses by stimulating MHCII signaling with an immunostimulatory polypeptide; for example, said immunostimulatory polypeptide is a soluble LAG-3; or
   wherein, for example, said disease or condition would benefit from stimulation of anti-tumor immunity by inhibiting an immunoinhibitory receptor signaling; for example, the immunoinhibitory receptor is SIRPα.
**Embodiment 137.** The protein formulation used according to any of Embodiments 130 to 136, wherein protein formulation is used in combination with a second therapeutic agent.
**Embodiment 138.** The protein formulation used according to Embodiment 137, wherein the second therapeutic agent is selected from a chemotherapeutic agent, an anti-cancer drug, radiation therapy, an immunotherapy agent, anti-angiogenesis agent, a targeted therapy agent, a cellular therapy agent, a gene therapy agent, a hormonal therapy agent, or cytokines.
**Embodiment 139.** Pharmaceutical combination, comprising the protein formulation according to any of Embodiments 1 to 129, and a second therapeutic agent selected from the group consisting of a chemotherapeutic agent, an anti-cancer drug, radiation therapy, an immunotherapy agent, anti-angiogenesis agent, a targeted therapy agent, a cellular therapy agent, a gene therapy agent, a hormonal therapy agent, or cytokines.
**Embodiment 140.** Use of the protein formulation according to any of Embodiments 1 to 129 in the manufacture of a medicament for treating, preventing, or alleviating PD-L1-related diseases in a subject.
**Embodiment 141.** Use according to Embodiment 140, wherein the disease is an immune-related disease or condition, cancer, or an infectious disease.
**Embodiment 142.** Use according to Embodiment 141, wherein the cancer is selected from the group consisting of: lung cancer (e.g., non-small cell lung cancer), liver cancer, pancreatic cancer, breast cancer, bronchial cancer, bone cancer, liver and bile duct cancer, ovarian cancer, testicle cancer, kidney cancer, bladder cancer, head and neck cancer, spine cancer, brain cancer, cervix cancer, uterine cancer, endometrial cancer, colon cancer, colorectal cancer, prostate cancer, gastric-esophageal cancer, rectal cancer, anal cancer, gastrointestinal cancer, skin cancer, pituitary cancer, stomach cancer, vagina cancer, thyroid cancer, glioblastoma, astrocytoma, melanoma, myelodysplastic syndrome, sarcoma, teratoma, glioma, and adenocarcinoma.
**Embodiment 143.** Use according to any of Embodiments 140 to 142, wherein the subject has been identified as having a PD-L1-expressing cancer cell.
**Embodiment 144.** Use according to any of Embodiments 140 to 143, wherein the PD-L1-related disease is resistant to PD-L1/PD-1 mono therapy.
**Embodiment 145.** Use according to any of Embodiments 140 to 144, wherein the subject is human.
**Embodiment 146.** Use of the protein formulation according to any of Embodiments 1 to 129 in the manufacture of a medicament for treating, preventing, or alleviating a disease or condition that would benefit from suppression of an immunosuppressive cytokine, from induction of sustained immune responses, or from stimulation of anti-tumor immunity;
   wherein, for example, the immunosuppressive cytokine is TGFβ; for example, said disease or condition is a TGFβ-related disease or condition; for example, said TGFβ-related disease is cancer, fibrotic disease, or kidney disease;
   wherein, for example, the immunosuppressive cytokine is IL-1; for example, said disease or condition is an IL-1-related disease or condition;
   wherein, for example, said disease or condition would benefit from induction of sustained immune responses by stimulating MHCII signaling with an immunostimulatory polypeptide; for example said immunostimulatory polypeptide is a soluble LAG-3; or
   wherein, for example, said disease or condition would benefit from stimulation of anti-tumor immunity by inhibiting an immunoinhibitory receptor signaling; for example, said immunoinhibitory receptor is SIRPα.
**Embodiment 147.** Use according to any of Embodiments 140 to 146, wherein the medicament is administered in combination with a second therapeutic agent.
**Embodiment 148.** Use according to Embodiment 147, wherein the second therapeutic agent is selected from the group consisting of a chemotherapeutic agent, an anti-cancer drug, radiation therapy, an immunotherapy agent, anti-angiogenesis agent, a targeted therapy agent, a cellular therapy agent, a gene therapy agent, a hormonal therapy agent, or cytokines.
**Embodiment 149.** The method for treating, preventing, or alleviating PD-L1-related diseases in a subject, comprising administering to the subject a therapeutically effective amount of the protein formulation according to any of Embodiments 1 to 129.
**Embodiment 150.** The method according to Embodiment 149, wherein the disease is an immune-related disease or condition, cancer, or an infectious disease.
**Embodiment 151.** The method according to Embodiment 150, wherein the cancer is selected from the group consisting of: lung cancer (e.g., non-small cell lung cancer), liver cancer, pancreatic cancer, breast cancer, bronchial cancer, bone cancer, liver and bile duct cancer, ovarian cancer, testicle cancer, kidney cancer, bladder cancer, head and neck cancer, spine cancer, brain cancer, cervix cancer, uterine cancer, endometrial cancer, colon cancer, colorectal cancer, prostate cancer, gastric-esophageal cancer, rectal cancer, anal cancer, gastrointestinal cancer, skin cancer, pituitary cancer, stomach cancer, vagina cancer, thyroid cancer, glioblastoma, astrocytoma, melanoma, myelodysplastic syndrome, sarcoma, teratoma, glioma, and adenocarcinoma.
**Embodiment 152.** The method according to any of Embodiments 149-151, wherein the subject has been identified as having a PD-L1-expressing cancer cell.
**Embodiment 153.** The method according to any of Embodiments 149-152, wherein the PD-L1-related disease is resistant to PD-L1/PD-1 mono therapy.
**Embodiment 154.** The method according to any of Embodiments 149-153, wherein the subject is human.
**Embodiment 155.** The method for treating, preventing, or alleviating a disease or condition in a subject, the disease or condition would benefit from suppression of an immunosuppressive cytokine, from induction of sustained immune responses, or from stimulation of anti-tumor immunity, said method comprising administering an effective amount of the protein formulation according to any of Embodiments 1 to 129;
   wherein, for example, the immunosuppressive cytokine is TGFβ; for example, said disease or condition is a TGFβ-related disease or condition; for example, said TGFβ-related disease is cancer, fibrotic disease, or kidney disease;
   wherein, for example, the immunosuppressive cytokine is IL-1; for example, said disease or condition is an IL-1-related disease or condition;
   wherein, for example, said disease or condition would benefit from induction of sustained immune responses by stimulating MHCII signaling with an immunostimulatory polypeptide; for example said immunostimulatory polypeptide is a soluble LAG-3; or
   wherein, for example, said disease or condition would benefit from stimulation of anti-tumor immunity by inhibiting an immunoinhibitory receptor signaling; for example, said immunoinhibitory receptor is SIRPα.
**Embodiment 156.** The method according to any of Embodiments 149-155, further comprising administering a therapeutically effective amount of a second therapeutic agent.
**Embodiment 157.** The method according to Embodiment 156, wherein the second therapeutic agent is selected from the group consisting of a chemotherapeutic agent, an anti-cancer drug, radiation therapy, an immunotherapy agent, anti-angiogenesis agent, a targeted therapy agent, a cellular therapy agent, a gene therapy agent, a hormonal therapy agent, or cytokines.
**Embodiment 158.** The method for preparing the protein formulation according to any of Embodiments 1 to 129, comprising the following steps:
   a. providing the bi-functional molecule according to any of Embodiments 1 to 129 in a buffer;
   b. adding a surfactant according to any of Embodiments 1 to 129, optionally a stabilizer, and optionally other pharmaceutically acceptable excipients;
   c. optionally sterile filtered.
**Embodiment 159.** The preparation method according to Embodiment 158, wherein the bifunctional molecule in step a is treated with a chelating agent, for example EDTA•2Na (e.g., about 0.3-10 mM, about 0.1-20 mM, about 0.3-15 mM, about 0.3-10 mM or about 0.5-5 mM of EDTA•2Na), and then the chelating agent is removed; for example, said treatment is carried out in a buffer at room temperature; for example said chelating agent is removed by dialysis, ultrafiltration, or diafiltration (for example, dialysis with the buffer, ultrafiltration, or diafiltration).
**Embodiment 160.** The preparation method according to Embodiment 158, wherein the bifunctional molecule in step a is treated with a chelating agent, for example EDTA•2Na (e.g., about 0.3-10 mM, about 0.1-20 mM, about 0.3-15 mM, about 0.3-10 mM or about 0.5-5 mM of EDTA•2Na) in a buffer at room temperature (for example, overnight), and then the chelating agent is removed by dialysis with the buffer, ultrafiltration, or diafiltration.

With respect to the bifunctional molecules described above, in particular in the embodiments above, the present disclosure also provides the following embodiments:
In one aspect, the present disclosure provides a bi-functional molecule comprising a first moiety that binds to an immune checkpoint molecule, and a second moiety that blocks activity of Interleukin-1 (IL-1).

In some embodiments, the first moiety comprises an agonist of immunostimulatory check point molecule, optionally selected from the group consisting of: CD27, CD70, CD28, CD80 (B7-1), CD86 (B7-2), CD40, CD40L (CD154), CD122, CD137, CD137L, OX40 (CD134), OX40L (CD252), GITR, ICOS (CD278) and ICOSLG (CD275), CD2, ICAM-1, LFA-1 (CD11a/CD18), CD30, BAFFR, HVEM, CD7, LIGHT, NKG2C, SLAMF7, NKp80, CD160 and CD83.

In some embodiments, the first moiety comprises an antagonist of immunoinhibitory check point molecule, optionally selected from the group consisting of: A2AR, B7-H3 (CD276), B7-H4 (VTCN1), BTLA (CD272), CTLA-4 (CD152), IDO1, IDO2, TDO, KIR, LAG3, NOX2, PD-1, PD-L1, PD-L2, TIM-3, VISTA, SIGLEC7 (CD328), TIGIT, PVR (CD155), SIGLEC9 (CD329), CD160, LAIR1, 2B4 (CD244), CD47 and B7-H5.

In some embodiments, the immune checkpoint molecule is PD-L1.

In some embodiments, the first moiety comprises the antibody against PD-L1 or an antigen-binding fragment thereof, and the second moiety comprises an IL-1-binding moiety or an IL-1 Receptor (IL-1R)-binding moiety.

In some embodiments, the IL-1-binding moiety comprises an IL-1R or an IL-1-binding fragment or variant thereof, or an antibody against IL-1 or an antigen-binding fragment thereof.

In some embodiments, the antibody against IL-1 or an antigen-binding fragment thereof comprises a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.

In some embodiments, the antibody against IL-1 or an antigen-binding fragment thereof comprises a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.

In some embodiments, the antibody against IL-1 or an antigen-binding fragment thereof comprises a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.

In some embodiments, the antibody against IL-1 or an antigen-binding fragment thereof comprises a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.

In some embodiments, the IL-1R-binding moiety comprises Interleukin-1 receptor antagonist or a fragment or variant thereof, or an antibody against IL-1R or an antigen-binding fragment thereof.

In another aspect, a bi-functional molecule comprises a first moiety that binds to PD-L1 and a second moiety that a) blocks activity of an immunosuppressive cytokine or b) stimulates immunity, wherein the first moiety comprises an antibody against PD-L1 or an antigen-binding fragment thereof comprising a heavy chain variable (VH) region and/or a light chain variable (VL) region, wherein the heavy chain variable region comprises:
a) a HCDR1 comprising **DYYMN** (SEQ ID NO: 1) or a homologous sequence of at least 80% sequence identity thereof,
b) a HCDR2 comprising **DINPNNX₁X₂TX₃YNHKFKG** (SEQ ID NO: 19) or a homologous sequence of at least 80% sequence identity thereof, and
c) a HCDR3 comprising **WGDGPFAY** (SEQ ID NO: 3) or a homologous sequence of at least 80% sequence identity thereof, and/or
wherein the light chain variable region comprises:
d) a LCDR1 comprising a sequence selected from the group consisting of **KASQNVX₄X₅X₆VA** (SEQ ID NO: 20) or a homologous sequence of at least 80% sequence identity thereof,
e) a LCDR2 comprising a sequence selected from the group consisting of **SX₇SX₈RYT** (SEQ ID NO: 21) or a homologous sequence of at least 80% sequence identity thereof, and
f) a LCDR3 comprising a sequence selected from the group consisting of **QQYSNYPT** (SEQ ID NO: 6) or a homologous sequence of at least 80% sequence identity thereof;
wherein X₁ is G or A, X₂ is G or D or Q or E or L, X₃ is S or M or Q or L or V, X₄ is G or P or K, X₅ is A or G, X₆ is A or I, X₇ is A or N or R or V, and X₈ is N or H or V or D.

In some embodiments, the heavy chain variable region comprises:
a) a HCDR1 comprising a sequence of SEQ ID NO: 1,
b) a HCDR2 comprising a sequence selected from group consisting of SEQ ID NO: 2, SEQ ID NO: 13, SEQ ID NO: 14, SEQ ID NO: 15, SEQ ID NO: 17 and SEQ ID NO: 18, and
c) a HCDR3 comprising a sequence of SEQ ID NO: 3,
and/or
a light chain variable region comprising:
d) a LCDR1 comprising a sequence selected from the group consisting of SEQ ID NO: 4, SEQ ID NO: 7, SEQ ID NO: 8 and SEQ ID NO: 9,
e) a LCDR2 comprising a sequence selected from the group consisting of SEQ ID NO: 5, SEQ ID NO: 10, SEQ ID NO: 11 and SEQ ID NO: 12, and
f) a LCDR3 comprising a sequence of SEQ ID NO: 6.

In some embodiments, the heavy chain variable region is selected from the group consisting of:
a) a heavy chain variable region comprising a HCDR1 comprising the sequence of SEQ ID NO: 1, a HCDR2 comprising the sequence of SEQ ID NO: 2 and a HCDR3 comprising the sequence of SEQ ID NO: 3;
b) a heavy chain variable region comprising a HCDR1 comprising the sequence of SEQ ID NO: 1, a HCDR2 comprising the sequence of SEQ ID NO: 13 and a HCDR3 comprising the sequence of SEQ ID NO: 3;
c) a heavy chain variable region comprising a HCDR1 comprising the sequence of SEQ ID NO: 1, a HCDR2 comprising the sequence of SEQ ID NO: 14 and a HCDR3 comprising the sequence of SEQ ID NO: 3;
d) a heavy chain variable region comprising a HCDR1 comprising the sequence of SEQ ID NO: 1, a HCDR2 comprising the sequence of SEQ ID NO: 15 and a HCDR3 comprising the sequence of SEQ ID NO: 3;
e) a heavy chain variable region comprising a HCDR1 comprising the sequence of SEQ ID NO: 1, a HCDR2 comprising the sequence of SEQ ID NO: 17 and a HCDR3 comprising the sequence of SEQ ID NO: 3; and
f) a heavy chain variable region comprising a HCDR1 comprising the sequence of SEQ ID NO: 1, a HCDR2 comprising the sequence of SEQ ID NO: 18 and a HCDR3 comprising the sequence of SEQ ID NO: 3.

In some embodiments, the light variable region is selected from the group consisting of:
a) a light chain variable region comprising a LCDR1 comprising the sequence of SEQ ID NO: 4, a LCDR2 comprising the sequence of SEQ ID NO: 5 and a LCDR3 comprising the sequence of SEQ ID NO: 6;
b) a light chain variable region comprising a LCDR1 comprising the sequence of SEQ ID NO: 9, a LCDR2 comprising the sequence of SEQ ID NO: 5 and a LCDR3 comprising the sequence of SEQ ID NO: 6;
c) a light chain variable region comprising a LCDR1 comprising the sequence of SEQ ID NO: 8, a LCDR2 comprising the sequence of SEQ ID NO: 5 and a LCDR3 comprising the sequence of SEQ ID NO: 6;
d) a light chain variable region comprising a LCDR1 comprising the sequence of SEQ ID NO: 4, a LCDR2 comprising the sequence of SEQ ID NO: 12 and a LCDR3 comprising the sequence of SEQ ID NO: 6; and
e) a light chain variable region comprising a LCDR1 comprising the sequence of SEQ ID NO: 4, a LCDR2 comprising the sequence of SEQ ID NO: 11 and a LCDR3 comprising the sequence of SEQ ID NO: 6.

In some embodiments, the antibody against PD-L1 or an antigen-binding fragment thereof further comprises one or more of heavy chain HFR1, HFR2, HFR3 and HFR4, and/or one or more of light chain LFR1, LFR2, LFR3 and LFR4, wherein:
a) the HFR1 comprises amino acid sequence **QVQLVQSGAEVKKPGASVKVSCKASGYX₉FT** (SEQ ID NO: 40) or a homologous sequence of at least 80% sequence identity thereof,
b) the HFR2 comprises amino acid sequence **WVRQAPGQX₁₀LEWMG** (SEQ ID NO: 41) or a homologous sequence of at least 80% sequence identity thereof,
c) the HFR3 sequence comprises amino acid sequence **RVTX₁₆TVDX₁₁SISTAYMELSRLRSDDTAVYYCX₁₂X₁₃** (SEQ ID NO: 42) or a homologous sequence of at least 80% sequence identity thereof,
d) the HFR4 comprises amino acid sequence **WGQGTLVTVSS** (SEQ ID NO: 25) or a homologous sequence of at least 80% sequence identity thereof,
e) the LFR1 comprises amino acid sequence **DIQMTQSPSSLSASVGDRVTITC** (SEQ ID NO: 26) or a homologous sequence of at least 80% sequence identity thereof,
f) the LFR2 comprises amino acid sequence **WYQQKPGKX₁₄PKLLIY** (SEQ ID NO: 43) or a homologous sequence of at least 80% sequence identity thereof,
g) the LFR3 comprises amino acid sequence **GVPX₁₅RFSGSGSGTDFTX₁₇TISSLQPEDIATYYC** (SEQ ID NO: 44) or a homologous sequence of at least 80% sequence identity thereof, and
h) the LFR4 comprises amino acid sequence FGQGTKLEIK (SEQ ID NO: 29) or a homologous sequence of at least 80% sequence identity thereof,
wherein X₉ is T or V, X₁₀ is G or S, X₁₁ is T or K, X₁₂ is A or V, X₁₃ is R or K, X₁₄ is A or S, X₁₅ is S or D, X₁₆ is M or V, and X₁₇ is F or L.

In some embodiments,
the HFR1 comprises a sequence selected from the group consisting of SEQ ID NOs: 22 and 30,
the HFR2 comprises a sequence selected from the group consisting of SEQ ID NOs: 23 and 31,
the HFR3 comprises a sequence selected from the group consisting of SEQ ID NOs: 24 and 32-35,
the HFR4 comprises a sequence of SEQ ID NOs: 25,
the LFR1 comprises a sequence from the group consisting of SEQ ID NO: 26,
the LFR2 comprises a sequence selected from the group consisting of SEQ ID NOs: 27 and 36,
the LFR3 comprises a sequence selected from the group consisting of SEQ ID NOs: 28, and 37-38, 39, 45, and
the LFR4 comprises a sequence of SEQ ID NO: 29.

In some embodiments, the heavy chain variable region comprises a sequence selected from group consisting of SEQ ID NO: 46, SEQ ID NO: 49, SEQ ID NO: 50, SEQ ID NO: 51, SEQ ID NO: 52, SEQ ID NO: 56, SEQ ID NO: 57, SEQ ID NO: 58, SEQ ID NO: 59, SEQ ID NO: 60 and a homologous sequence thereof having at least 80% sequence identity thereof.

In some embodiments, the light variable region comprises a sequence selected from group consisting of SEQ ID NO: 47, SEQ ID NO: 54, SEQ ID NO: 55, SEQ ID NO: 61, SEQ ID NO: 62, SEQ ID NO: 63, SEQ ID NO: 64, SEQ ID NO: 65 and a homologous sequence thereof having at least 80% sequence identity thereof.

In some embodiments, the antibody against PD-L1 or an antigen-binding fragment thereof comprises a pair of heavy chain variable region and light chain variable region sequences selected from the group consisting of: SEQ ID NO: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64 and 60/65.

In some embodiments, the antibody against PD-L1 or an antigen-binding fragment thereof further comprises one or more amino acid residue substitutions or modifications yet retains specific binding specificity and/or affinity to PD-L1.

In some embodiments, at least one of the substitutions or modifications is in one or more of the CDR sequences, and/or in one or more of the non-CDR regions of the VH or VL sequences.

In some embodiments, the antibody against PD-L1 or antigen-binding fragment thereof further comprises an immunoglobulin constant region, optionally a constant region of human Ig, or optionally a constant region of human IgG.

In some embodiments, the constant region comprises an Fc region of human IgG1, IgG2, IgG3, or IgG4.

In some embodiments, the Fc region of human IgG1 comprises SEQ ID NO: 80, or a variant thereof having at least 80% (e.g. at least 85%, 90%, 95%, 96%, 97%, 98%, or 99%) sequence identity thereof.

In some embodiments, the constant region comprises an Fc variant having reduced effector function relative to the corresponding wildtype Fc region. In some embodiments, the Fc region comprises one or more amino acid residue modifications or substitutions resulting in reduced effector functions relative to SEQ ID NO: 80.

In some embodiments, the Fc region comprises one or more amino acid residue substitutions selected from the group consisting of: 220S, 226S, 228P, 229S, 233P, 234V, 234G, 234A, 234F, 234A, 235A, 235G, 235E, 236E, 236R, 237A, 237K, 238S, 267R, 268A, 268Q, 269R, 297A, 297Q, 297G, 309L, 318A, 322A, 325L, 328R, 330S, 331S and any combination thereof, wherein the numbering of the residues in the Fc region is that of the EU index as in Kabat.

In some embodiments, the Fc region comprises a combination of mutations selected from the group consisting of: a) K322A, L234A and L235A; b) P331S, L234F and L235E; c) L234A and L235A; c) N297A; d) N297Q; e) N297G; f) L235E; g) L234A and L235A (IgG1); h) F234A and L235A (IgG4); i) H268Q, V309L, A330S and P331S (IgG2); j) V234A, G237A, P238S, H268A, V309L, A330S and P331S (IgG2), wherein the numbering of the residues in the Fc region is that of the EU index as in Kabat.

In some embodiments, the Fc variant comprises an amino acid sequence of SEQ ID NO: 81.

In some embodiments, the antibody against PD-L1 or an antigen-binding fragment thereof is humanized.

In some embodiments, the antigen-binding fragment is a diabody, a Fab, a Fab', a F(ab')₂, a Fd, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a multispecific antibody, a camelized single domain antibody, a nanobody, a domain antibody, and a bivalent domain antibody.

In some embodiments, the antibody or an antigen-binding fragment thereof is capable of binding to both human PD-L1 and cyno PD-L1.

In some embodiments, the first moiety comprises an antibody or an antigen-binding fragment thereof that competes for binding to PD-L1 with the antibody or antigen-binding fragment thereof provided herein.

In some embodiments, the immunosuppressive cytokine comprises a cytokine in transforming growth factor beta (TGF-β) superfamily, IL-1, or Vascular endothelial growth factor (VEGF).

In some embodiments, the immunosuppressive cytokine in TGF-β superfamily includes TGF-β, bone morphogenetic proteins (BMPs), activins, NODAL, and growth and differentiation factors (GDFs).

In some embodiments, the immunosuppressive cytokine is TGF-β.

In some embodiments, the second moiety comprises TGFβ-binding moiety.

In some embodiments, the TGFβ-binding moiety comprises a soluble TGFβ receptor (TGFβR) or a TGFβ-binding fragment or variant thereof, or an antibody against TGFβ and an antigen-binding fragment thereof.

In some embodiments, the soluble TGFβ comprises an extracellular domain (ECD) of the TGFβR or a TGFβ-binding fragment or variant thereof.

In some embodiments, the TGFβR is selected from the group consisting of: TGFβ Receptor I (TGFβRI), TGFβ Receptor II (TGFβRII), TGFβ Receptor III (TGFβRIII) and any combination thereof.

In some embodiments, the TGFβR is TGFβRII.

In some embodiments, the TGFβRII selectively binds to TGFβ1 over TGFβ2 and TGFβ3.

In some embodiments, the TGFβ1 is human TGFβ1 or mouse TGFβ1.

In some embodiments, the ECD of TGFβR comprises an amino acid sequence of SEQ ID NO: 66, 79, 78, 77 or a sequence having at least 80% sequence identity thereof yet retains specific binding specificity and/or affinity to TGF-β.

In some embodiments, the second moiety comprises an IL-1-binding moiety or an IL-1 Receptor (IL-1R)-binding moiety.

In some embodiments, the IL-1-binding moiety comprises a soluble IL-1R, IL-1R 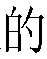 IL-1-binding fragment or variant, or an antibody against IL-1 or an antigen-binding fragment thereof.

In some embodiments, the IL-1-binding moiety comprises an extracellular domain (ECD) of the IL-1RI, an IL-1-binding fragment or variant of any of IL-1RI, ECD of IL-1RI, IL-1RII, or ECD of IL-1RII, or IL-1RAP, or ECD of IL-1RAP, IL-1sRI or IL-1sRII.

In some embodiments, the IL-1R-binding moiety comprises an IL-1Ra or an IL-1-binding fragment or variant thereof, or an antibody against IL-1R or an antigen-binding fragment thereof.

In some embodiments, the IL-1R-binding moiety comprises an amino acid sequence of SEQ ID NO: 67 or 76, or an amino acid sequence having at least 80% sequence identity to SEQ ID NO: 67 or 76, or an IL-1 binding fragment or variant thereof.

In some embodiments, the IL-1 is IL-1α or IL-1β.

In some embodiments, the IL-1β is human IL-1β.

In some embodiments, the antibody against IL-1 or an antigen-binding fragment thereof comprises a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.

In some embodiments, the antibody against IL-1 or an antigen-binding fragment thereof comprises a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.

In some embodiments, the antibody against IL-1 or an antigen-binding fragment thereof comprises a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.

In some embodiments, the antibody against IL-1 or an antigen-binding fragment thereof comprises a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.

In some embodiments, the bi-functional molecule comprises a heavy chain comprising an amino acid sequence of SEQ ID NO: 118 or SEQ ID NO: 120, and/or a light chain comprising an amino acid sequence of SEQ ID NO: 119 or SEQ ID NO: 121.

In some embodiments, the second moiety stimulates anti-tumor immunity and comprises an immunostimulatory polypeptide.

In some embodiments, the immunostimulatory polypeptide comprises Interleukin (IL) -2 (IL-2), IL-15, IL-21, IL-10, IL-12, IL-23, IL-27, IL-35, granulocyte-macrophage colony-stimulating factor (GM-CSF), soluble CD4, soluble LAG-3 or IFN-α, or a functional equivalent thereof.

In some embodiments, the soluble LAG-3 comprises an extracellular domain (ECD) of the LAG-3 or a MHCII-binding fragment or variant thereof.

In some embodiments, the second moiety stimulates anti-tumor immunity and comprises an antagonist of an immunoinhibitory receptor signaling.

In some embodiments, the immunoinhibitory receptor is Signal-regulatory protein alpha (SIRPα).

In some embodiments, the second moiety blocks interaction between CD47 and SIRPα.

In some embodiments, the second moiety comprises CD47 binding domain or SIRPα binding domain.

In some embodiments, the CD47 binding domain comprises a soluble SIRPα or a CD47 binding fragment or variant thereof, or an anti-CD47 antibody or an antigen-binding fragment thereof.

In some embodiments, the soluble SIRPα comprises an extracellular domain (ECD) of the SIRPα, or a CD47-binding fragment or variant thereof.

In some embodiments, the soluble SIRPα comprises an amino acid sequence of SEQ ID NO: 84 or an amino acid sequence having at least 80% sequence identity thereof yet retaining binding specificity to CD47.

In some embodiments, the SIRPα binding domain comprises a soluble CD47 or a SIRPα binding fragment or variant thereof, or an anti-SIRPα antibody or an antigen-binding fragment thereof.

In some embodiments, the soluble CD47 comprises an extracellular domain (ECD) of the CD47 or a SIRPα binding fragment or variant thereof, an anti-SIRPα antibody or an antigen-binding fragment thereof.

In some embodiments, the bi-functional molecule further comprises a linker connecting the first moiety and the second moiety.

In some embodiments, the linker is selected from the group consisting of a cleavable linker, a non-cleavable linker, a peptide linker, a flexible linker, a rigid linker, a helical linker, and a non-helical linker.

In some embodiments, the linker comprises an amino acid sequence of ((G)nS)m, wherein m and n are independently an integer selected from 0 to 30 (e.g. 1, 2, 3, 4, 5, 6, 7, 8, 9, 10). In some embodiments, n is 2, 3, 4 or 5, and m is 1, 2, 3, 4, 5, 6, 7, 8, 9, or 10. In some embodiments, the linker comprises an amino acid sequence of SEQ ID NO: 68.

In some embodiments, the bi-functional molecule comprises one or more of the second moiety.

In some embodiments, at least one of the second moieties is linked to an N terminus or a C terminus of a polypeptide chain of the first moiety.

In some embodiments, at least one of the second moieties is linked to: a) an N terminus or a C terminus of a heavy chain of the first moiety, or b) an N terminus or a C terminus of a light chain of the first moiety.

In some embodiments, at least one of the second moieties is linked to a C terminus of a heavy chain constant region of the first moiety.

In some embodiments, each of the second moieties is linked respectively to the C terminus of each heavy chain constant region of the first moiety.

In some embodiments, the bi-functional molecule comprises more than one of the second moieties that are linked respectively to: an N terminus of a heavy chain of the first moiety, a C terminus of a heavy chain of the first moiety, an N terminus of a light chain of the first moiety, a C terminus of a light chain of the first moiety, or any combination thereof.

In some embodiments, the bi-functional molecule comprises homodimeric or heterodimeric heavy chains.

In some embodiments, the heavy chains are heterodimeric with respect to presence or position of the second moiety.

In some embodiments, the heterodimeric heavy chains comprise one heavy chain having the second moiety but the other heavy chain having not.

In some embodiments, the heterodimeric heavy chains further comprise heterodimeric Fc regions that associate in a way that discourages homodimerization and/or favors heterodimerization.

In some embodiments, the first and the heterodimeric Fc regions are capable of associating into heterodimers via knobs-into-holes, hydrophobic interaction, electrostatic interaction, hydrophilic interaction, or increased flexibility.

In some embodiments, the heterodimeric Fc regions comprises Y349C, T366S, L368A or Y407V or any combination thereof in one Fc region, and S354C, or T366W or combination thereof in another Fc region, wherein the numbering of the residues in the Fc region is that of the EU index as in Kabat.

In some embodiments, the bi-functional molecule is further linked to one or more conjugate moieties.

In some embodiments, the conjugate moiety comprises a clearance-modifying agent, a chemotherapeutic agent, a toxin, a radioactive isotope, a lanthanide, a luminescent label, a fluorescent label, an enzyme-substrate label, a DNA-alkylator, a topoisomerase inhibitor, a tubulin-binders, or other anticancer drugs such as androgen receptor inhibitor.

In another aspect, the present disclosure further provides a pharmaceutical composition or kit comprising the bi-functional molecule provided herein and a pharmaceutically acceptable carrier.

In another aspect, the present disclosure further provides an isolated polynucleotide encoding the bi-functional molecule provided herein.

In another aspect, the present disclosure further provides a vector comprising the isolated polynucleotide provided herein.

In another aspect, the present disclosure further provides a host cell comprising the vector provided herein.

In another aspect, the present disclosure further provides a method of expressing the bi-functional molecule provided herein, comprising culturing the host cell provided herein under the condition at which the vector is expressed.

In another aspect, the present disclosure further provides a method of treating, preventing or alleviating a PD-L1 related disease in a subject, comprising administering to the subject a therapeutically effective amount of the bi-functional molecule provided herein and/or the pharmaceutical composition or kit provided herein.

In some embodiments, the disease is immune related disease or disorder, cancers, autoimmune diseases, or infectious disease.

In some embodiments, the cancer is selected from the group consisting of: lung cancer (e.g., non-small cell lung cancer), liver cancer, pancreatic cancer, breast cancer, bronchial cancer, bone cancer, liver and bile duct cancer, ovarian cancer, testicle cancer, kidney cancer, bladder cancer, head and neck cancer, spine cancer, brain cancer, cervix cancer, uterine cancer, endometrial cancer, colon cancer, colorectal cancer, prostate cancer, gastric-esophageal cancer, rectal cancer, anal cancer, gastrointestinal cancer, skin cancer, pituitary cancer, stomach cancer, vagina cancer, thyroid cancer, glioblastoma, astrocytoma, melanoma, myelodysplastic syndrome, sarcoma, teratoma, glioma, and adenocarcinoma.

In some embodiments, the subject has been identified as having a PD-L1-expressing cancer cell.

In some embodiments, the subject is human.

In some embodiments, the method further comprises administering an effective amount of a second therapeutic agent.

In some embodiments, the second therapeutic agent is selected from the group consisting of a chemotherapeutic agent, an anti-cancer drug, radiation therapy, an immunotherapy agent, anti-angiogenesis agent, a targeted therapy agent, a cellular therapy agent, a gene therapy agent, a hormonal therapy agent, or cytokines.

In another aspect, the present disclosure provides use of the bi-functional molecule provided herein in the manufacture of a medicament for treating a PD-L1 related disease or condition in a subject.

In another aspect, the present disclosure provides a method of treating, preventing or alleviating in a subject a disease or condition that would benefit from suppression of an immunosuppressive cytokine, from induction of sustained immune responses, or from stimulation of anti-tumor immunity, comprising administering an effective amount of the bi-functional molecule provided herein.

In some embodiments, the immunosuppressive cytokine is TGFβ.

In some embodiments, the disease or condition is a TGFβ-related disease or condition.

In some embodiments, the TGFβ-related disease is cancer, fibrotic disease, or kidney disease.

In some embodiments, the immunosuppressive cytokine is IL-1.

In some embodiments, the disease or condition is an IL-1-related disease or condition.

In some embodiments, the disease or condition would benefit from stimulation of anti-tumor immunity by inhibiting an immunoinhibitory receptor signaling, e.g., SIRPα signaling.

### DESCRIPTION OF THE DRAWINGS

Figure 1 shows humanized 4B6 antibodies binding to human PD-L1 by ELISA.
Figure 2 shows Hu4B6_HgLa binding to human PD-L1 by ELISA.
Figure 3A - Figure 3C show AM-4B6-IgG1-TGFβRII variants binding to PD-L1 by ELISA.
Figure 4 shows affinity ranking of AM-4B6-IgG1-TGFβRII variants using flow cytometry.
Figure 5A and 5B show blockade of PD-L1/PD-1 or PD-L1B7-1 by AM-4B6-IgG1-TGFβRII variants.
Figure 6 shows blockade of PD-L1/PD-1 by AM-4B6-IgG1-TGFβRII variants using cell based assay.
Figure 7 shows SDS-PAGE of AM4B6_hIgG1_TBRII (20-136) expressed with stable cell line.
Figure 8A and Figure 8B show binding to human PD-L1 or cyno PD-L1 by ELISA analysis.
Figure 9A - Figure 9C show binding to human PD-L1 and B7 family other members and other members of TGFβ superfamily by ELISA analysis.
Figure 10A - Figure 10F show binding to PD-L1 expressing cells by FACS analysis.
Figure 11 shows binding to human PD-L1 on activated human T cells by FACS analysis.
Figure 12A - Figure 12B show blockade of human PD-L1 binding to human PD-1 or cyno PD-L1 binding to cyno PD-1 by ELISA analysis.
Figure 13 shows simultaneously binding to hPD-L1 and TGFb1 by ELISA analysis.
Figure 14 shows blocking hPD-L1/hPD-1 using a reporter assay.
Figure 15 shows blocking TGFβ1 signaling using a TGF-β reporter HEK-293 cell line.
Figure 16 shows effect of AM4B6-hIgG1-TGFβRII' on IFNγ release of PBMC stimulated by tuberculin (TB).
Figure 17A - Figure 17B show anti-tumor activity in MC38-hPD-L1 tumor model.
Figure 18A - Figure 18B show anti-tumor activity in H460 tumor model.
Figure 19A - Figure 19B show anti-tumor activity in EMT6-hPD-L1 tumor model.
Figure 20A - Figure 20C show pharmacokinetics and pharmacodynamics study of AM4B6-hIgG1-TGFβRII in vivo.
Figure 21 shows binding activity of AM4B6-hIgG1-IL-1RA to human PD-L1 by ELISA.
Figure 22 shows binding activity of AM4B6-hIgG1-IL-1RA to human PD-L1 by FACS analysis.
Figure 23 shows blockade of PD-L1/PD-1 by AM4B6-hIgG1-IL-1RA using cell based assay.
Figure 24 shows blocking activity of AM4B6-IgG1-IL-1RA to human IL-1β by ELISA.
Figure 25 shows blocking activity of AM4B6-hIgG1-IL-1RA to human IL-1β on reporter cells.
Figure 26 shows SEC-HPLC purity of asymmetric bifunctional antibodies.
Figure 27 shows binding of the bi-functional molecule to human PD-L1 as measured by ELISA.
Figure 28 shows binding of the bi-functional molecule to human CD47 as measured by ELISA.
Figure 29 shows ELISA binding activities of IgG-scFv-ACZ885-AM4B6 and IgG-scFv-XOMA052-AM4B6 bispecific antibody to hIL-1β protein.
Figure 30 shows ELISA binding activities of IgG-scFv-ACZ885-AM4B6 and IgG-scFv-XOMA052-AM4B6 bispecific antibody to hPD-L1 protein.
Figure 31 shows binding of IgG-scFv-ACZ885-AM4B6 and IgG-scFv-XOMA052-AM4B6 bispecific antibody to PD-L1 expressing 293T cells by FACS.
Figure 32 shows cell based PD1/PD-L1 blockade activity of IgG-scFv-ACZ885-AM4B6 and IgG-scFv-XOMA052-AM4B6.
Figure 33 shows blocking activity of IgG-scFv-XOMA052-AM4B6 to human IL-1β on HDF cells.
Figure 34 shows blocking activity of IgG-scFv-ACZ885-AM4B6 to hIL-1β on reporter cell.
Figure 35 (analysis of results at 40°C) shows the analysis results of ΔHMW% and ΔLMW% in JMP software by SEC and NR CE-SDS assays for samples of formulas F1-F12 left at 40°C for 4 weeks, indicating the effect of pH, PS80 and other excipients on protein stability.
Figure 36 (analysis of results under stirring) shows analysis results of ΔHMW% and ΔLMW% in JMP software by SEC and NR CE-SDS assays for samples of formulas F1-F12 stirred for 3 hours at 25°C, indicating the effect of pH, PS80 and other excipients on protein stability.
Figures 37 and 38 (Figure 37: change in polymer at 40°C; Figure 38: change in small molecule fragment at 40°C) show the changes in % HMW and% LMW measured by SEC and NR CE-SDS for samples of formulas F11 and F13, left at 40°C for 4 weeks, indicating the effect of buffer on protein stability.
Figures 39 and 40 (Figure 39: change in polymer at 40°C; Figure 40: change in small molecule fragment at 40°C) show the changes in % HMW and% LMW measured by SEC and NR CE-SDS for samples of formulas F14 and F15, left at 40°C for 6 weeks, indicating the effect of buffer on protein stability.
Figure 41 - Figure 43 (Figure 41: change in polymer under light exposure conditions; Figure 42: change in polymer at 40°C; Figure 43: change in polymer at 25°C) show the changes in % HMW measured by SEC for samples of formulas F16 and F17, respectively, after being exposed to light for 7 days, and left at 40°C for 6 weeks or left at 25°C for 6 weeks, indicating the effects of the excipients on protein stability.
Figure 44 - Figure 45 (Figure 44: change in PS80 concentration at 40°C; Figure 45: change in PS80 concentration at 25°C) show the degradation results of PS80 for the samples of formulas F18-F21, respectively, left at 40°C for one month or at 25°C for 6 months, indicating the protective effect of disodium edetate on PS80.
Figure 46 - Figure 49 (Figure 46: change in polymer at 25°C; Figure 47: change in small molecule fragment at 25°C; Figure 48: change in polymer at 5°C; Figure 49: change in small molecule fragment at 5°C) show the change in % HMW and% LMW measured by SEC and NR CE-SDS, respectively, for samples of formulas F18-F21 when left at 25°C for 4 weeks or at 5°C for 6 months, indicating the effect of the excipients on protein stability.
Figure 50 (change in PS80 concentration at 40°C) shows the comparative results of PS80 degradation at 40°C for 4 weeks of the obtained formulations with or without the addition of disodium edetate at various concentrations during the formulation process.

### Definition

Terms used in the present disclosure have the definitions listed below, and if no definitions are given herein, the terms used have meanings that are commonly understood in the art

Throughout the present disclosure, the articles "a," "an," and "the" are used herein to refer to one or to more than one (i.e., to at least one) of the grammatical object of the article. By way of example, "an antibody" means one antibody or more than one antibody. It is to be understood that the terms used herein are for the purpose of describing particular embodiments only, and is not intended to be limiting.

As used herein, the term "and/or" means any one of the options or two or more of the options.

As used herein, the terms "comprise", "comprising" or "containing" are intended to include the stated elements, values, or steps, but not to exclude any other elements, values, or steps. When the terms "comprise", "comprising" or "containing" are used herein, unless otherwise indicated, they also encompass the combination of the stated elements, values, or steps. For example, when referring to an antibody variable region "comprising" a particular sequence, it is also intended to encompass the antibody variable region consisting of that particular sequence.

As used herein, the terms "formulation" and "pharmaceutical formulation" are used interchangeably and refer to a composition or preparation suitable for administration to an animal, preferably a mammal (including a human), comprising at least one active ingredient and at least one inactive ingredient. The liquid formulation of the present disclosure is for example in in a liquid form, a lyophilized form or a liquid form reconstituted from a lyophilized form. The "liquid formulation" means a formulation in liquid form. The formulation of the present disclosure is preferably a liquid formulation, such as an injection, an intravenous injection, an intramuscular injection, or a subcutaneous injection.

As used herein, "buffer" or "buffer solution" refers to a pharmaceutically acceptable excipient that stabilizes the pH of the pharmaceutical formulation, e.g., acetate, histidine, glutamate, citrate, succinate, malate, phosphate, or lactate, and/or their respective free acids or bases, and mixtures of various salts and/or their acids and bases. Preferred pharmaceutically acceptable buffers include, but are not limited to, acetate buffers and histidine buffers, "acetate buffers" or "acetate buffer solutions" include acetic acid-sodium acetate, histidine-acetate, acetic acid-potassium acetate, acetic acid-calcium acetate, acetic acid-magnesium acetate, and the like. The "histidine buffers" or "histidine buffer solutions" include histidine-hydrochloride, histidine-acetate, histidine-phosphate, histidine-sulfate, and the like. Such buffers are typically used at a concentration of about 1-100 mM, such as about 5-100 mM, about 10-50 mM, about 15-30 mM, or about 20 mM. The buffer is capable of maintaining the pH of the liquid formulations of the present disclosure in the range of about 4.5-7.0, such as about 4.5-6.0, about 5.0-5.6 or about 5.0-5.5, such as about 5.3 or 5.3±5%.

As used herein, "surfactant" means a pharmaceutically acceptable excipient having surface activity. Preferably, a nonionic surfactant is used. Pharmaceutically acceptable surfactants include, but are not limited to, polyoxyethylene sorbitan fatty acid esters (Tween), polyoxyethylene alkyl ethers (Brij), alkylphenylpolyoxyethylene ethers (Triton X), polyoxyethylene-polyoxypropylene copolymers (poloxamers, Pluronic), and sodium dodecyl sulfate (SDS), such as polysorbate 20 (PS20), polysorbate 80 (PS80), poloxamer 188, the Brij series, Triton X. When polysorbates, such as PS80 and PS20, are used, the concentration is typically about 0.001-1%, such as about 0.01-0.1%, about 0.02-0.1%, about 0.025-0.1%, or about 0.05%. In the formulations of the present disclosure, the concentration of the surfactant is described as a percentage expressed as weight/volume (w/v, g/100 mL).

As used herein, "stabilizer" refers to a stabilizer other than the surfactants described above which is a pharmaceutically acceptable excipient that protects the active pharmaceutical ingredient and/or formulation during manufacture, storage, and use, in a stable or unaltered state, e.g., to help prevent aggregation, oxidation, color change, and the like. The stabilizers include, but are not limited to, saccharides (including monosaccharides such as glucose, fructose, galactose, mannose, sorbose, ribose, deoxyribose, neuraminic acid, and oligosaccharides such as sucrose, trehalose, lactose, maltose and raffinose), amino acids (including, but not limited to, arginine, glycine, ornithine, lysine, histidine, glutamic acid, aspartic acid, isoleucine, leucine, alanine, phenylalanine, tyrosine, tryptophan, methionine, serine, proline. In each case, the amino acids employed are preferably in the L-form, for example in the form of their inorganic salts, for example in the form of hydrochloride salts, such as arginine hydrochloride, histidine hydrochloride or lysine hydrochloride, at a concentration of, for example, from about 20 to about 250 mM, from about 100 to about 200 mM, for example about 150 mM), salts (for example, inorganic salts, for example sodium chloride, magnesium chloride, calcium chloride), chelating agents (for example EDTA (edetic acid), EDTA salts, for example EDTA•2Na), polyols (for example sorbitol, mannitol, xylitol, dextran, glycerol, arabitol, propylene glycol, polyethylene glycol), cyclodextrins (for example hydroxypropyl-β-cyclodextrin, sulfobutylethyl-β-cyclodextrin, β-cyclodextrin), polyethylene glycol (e.g., PEG 3000, PEG 3350, PEG 4000, PEG 6000), albumin (e.g., human serum albumin (HSA), bovine serum albumin (BSA)). The stabilizer is e.g., arginine hydrochloride, NaCl, sucrose or sorbitol, preferably arginine hydrochloride or NaCl, e.g., about 150 mM arginine hydrochloride or about 135 mM NaCl. The stabilizer may be present in the formulation at a concentration of about 1-500 mM, e.g., about 10-300 mM, about 100-300 mM, about 120-300 mM, about 100-200 mM, about 135-280 mM, about 135-200 mM, about 130-170 mM, about 140-160 mM, 135, 150, 260 or 280 mM. The concentration of the amino acid or inorganic salt stabilizer, e.g., arginine hydrochloride or NaCl is about 120-300 mM, about 100-200 mM, about 135-200 mM, about 130-170 mM, about 140-160 mM, about 135 mM or about 150 mM. For example, the concentration of arginine hydrochloride is about 120-300 mM, about 100-200 mM, about 135-200 mM, about 130-170 mM, about 140-160 mM or about 150 mM. The concentration of chelating agent such as EDTA•2Na is about 30-350 µM, such as about 75-350 µM, such as about 75, about 150 or about 300 µM. More than one stabilizer, selected from the same or different groups, may be present in the formulations of the present disclosure. The stabilizer in the formulations of the present disclosure may also function as an osmotic pressure regulator at the same time.

As used herein, "osmotic pressure regulator" refers to pharmaceutically acceptable excipients for regulating the osmotic pressure of a solution. Examples of the osmotic pressure regulator include, but are not limited to, saccharides (including monosaccharides, disaccharides, trisaccharides, polysaccharides, sugar alcohols, reducing sugars, non-reducing sugars such as glucose, sucrose, trehalose), amino acids (including arginine, glycine, cysteine, histidine), salts (e.g., inorganic salts such as sodium chloride, magnesium chloride, calcium chloride), and polyols (e.g., sorbitol, mannitol, xylitol, dextran, glycerol, arabitol, propylene glycol, polyethylene glycol).

One subset of stabilizers are lyoprotectants, which include, but are not limited to, saccharides, polyols (such as, for example, sugar alcohols), and amino acids. A preferred lyoprotectant may be selected from saccharides such as sucrose, trehalose, lactose, glucose, mannose, maltose, galactose, fructose, sorbose, raffinose, neuraminic acid, amino sugars such as glucosamine, galactosamine, meglumine, polyols such as mannitol and sorbitol, and amino acids such as arginine and glycine or mixtures thereof. Lyoprotectants are generally used in an amount of about 10 to 500 mM, preferably in an amount of about 10 to about 300 mM and more preferably in an amount of about 100 to about 300 mM.

One subset of stabilizers are antioxidants, which include, but are not limited to, ascorbic acid, glutathione, cysteine, methionine, citric acid, EDTA. Stabilizers may be used in an amount of about 0.01 to about 100 mM, preferably in an amount of about 5 to about 50 mM and more preferably in an amount of about 5 to about 25 mM.

As used herein, the term "about" when used in conjunction with numerical values is intended to encompass values within a range having a lower limit of 10% less than the specified numerical value and an upper limit of 10% greater than the specified numerical value, i.e., a range of ± 10%, e.g., ± 5%, e.g., ± 3%.

As used herein, "w/v" means "weight/volume" in a unit of g/100 mL.

All numerical ranges herein should be understood to disclose each and every value and subset of values within the range, regardless of whether specifically otherwise disclosed. For example, when referring to any one numerical range, it is intended to refer to each numerical value within that range, e.g., each integer within that numerical range. The disclosure relates to all values falling within these ranges, all smaller ranges and the upper or lower limit of the range of values.

The term "antibody" as used herein includes any immunoglobulin, monoclonal antibody, polyclonal antibody, multivalent antibody, bivalent antibody, monovalent antibody, multispecific antibody, or bispecific antibody that binds to a specific antigen. A native intact antibody comprises two heavy (H) chains and two light (L) chains. Mammalian heavy chains are classified as alpha, delta, epsilon, gamma, and mu, each heavy chain consists of a variable region (VH) and a first, second, third, and optionally fourth constant region (CH1, CH2, CH3, CH4 respectively); mammalian light chains are classified as λ or κ, while each light chain consists of a variable region (VL) and a constant region. The antibody has a "Y" shape, with the stem of the Y consisting of the second and third constant regions of two heavy chains bound together via disulfide bonding. Each arm of the Y includes the variable region and first constant region of a single heavy chain bound to the variable and constant regions of a single light chain. The variable regions of the light and heavy chains are responsible for antigen binding. The variable regions in both chains generally contain three highly variable loops called the complementarity determining regions (CDRs) (light chain CDRs including LCDR1, LCDR2, and LCDR3, heavy chain CDRs including HCDR1, HCDR2, HCDR3). CDR boundaries for the antibodies and antigen-binding fragments disclosed herein may be defined or identified by the conventions of Kabat, IMGT, Chothia, or Al-Lazikani (Al-Lazikani, B., Chothia, C., Lesk, A. M., J. Mol. Biol., 273(4), 927 (1997); Chothia, C. et al., J Mol Biol. Dec 5;186(3):651-63 (1985); Chothia, C. and Lesk, A.M., J. Mol. Biol., 196,901 (1987); Chothia, C. et al., Nature. Dec 21-28;342(6252):877-83 (1989); Kabat E.A. et al., Sequences of Proteins of immunological Interest, 5th Ed. Public Health Service, National Institutes of Health, Bethesda, Md. (1991); Marie-Paule Lefranc et al., Developmental and Comparative Immunology, 27: 55-77 (2003); Marie-Paule Lefranc et al., Immunome Research, 1(3), (2005); Marie-Paule Lefranc, Molecular Biology of B cells (second edition), chapter 26, 481-514, (2015)). The three CDRs are interposed between flanking stretches known as framework regions (FRs) (light chain FRs including LFR1, LFR2, LFR3, and LFR4, heavy chain FRs including HFR1, HFR2, HFR3, and HFR4), which are more highly conserved than the CDRs and form a scaffold to support the highly variable loops. The constant regions of the heavy and light chains are not involved in antigen-binding, but exhibit various effector functions. The term "effector function" as used herein refers to cell-mediated or complement-mediated cytotoxic effects brought about by interactions between the Fc region of an antibody and C1q complement protein or Fc receptors (FcRs) on immune cells. Exemplary effector functions include, without limitation, antibody-dependent cellular cytotoxicity (ADCC), antibody-dependent cell-mediated phagocytosis (ADCP) and complement-dependent cytotoxicity (CDC) effects. Antibodies are assigned to classes based on the amino acid sequences of the constant regions of their heavy chains. The five major classes or isotypes of antibodies are IgA, IgD, IgE, IgG, and IgM, which are characterized by the presence of alpha, delta, epsilon, gamma, and mu heavy chains, respectively. Several of the major antibody classes are divided into subclasses such as IgG1 (gamma1 heavy chain), IgG2 (gamma2 heavy chain), IgG3 (gamma3 heavy chain), IgG4 (gamma4 heavy chain), IgA1 (alpha1 heavy chain), or IgA2 (alpha2 heavy chain).

In some embodiments, the antibody provided herein encompasses any antigen-binding fragments thereof. The term "antigen-binding fragment" as used herein refers to an antibody fragment formed from a portion of an antibody comprising one or more CDRs, or any other antibody fragment that binds to an antigen but does not comprise an intact native antibody structure. Examples of antigen-binding fragments include, without limitation, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a bispecific antibody, a multispecific antibody, a camelized single domain antibody, a nanobody, a domain antibody, and a bivalent domain antibody. An antigen-binding fragment is capable of binding to the same antigen to which the parent antibody binds.

"Fab" with regard to an antibody refers to that portion of the antibody consisting of a single light chain (both variable and constant regions) bound to the variable region and first constant region of a single heavy chain by a disulfide bond.

"Fab'" refers to a Fab fragment that includes a portion of the hinge region.

"F(ab')₂" refers to a dimer of Fab'.

"Fc" with regard to an antibody (e.g. of IgG, IgA, or IgD isotype) refers to that portion of the antibody consisting of the second and third constant domains of a first heavy chain bound to the second and third constant domains of a second heavy chain via disulfide bonding. Fc with regard to antibody of IgM and IgE isotype further comprises a fourth constant domain. The Fc portion of the antibody is responsible for various effector functions such as antibody-dependent cell-mediated cytotoxicity (ADCC), and complement dependent cytotoxicity (CDC), but does not function in antigen binding.

"Fv" with regard to an antibody refers to the smallest fragment of the antibody to bear the complete antigen binding site. An Fv fragment consists of the variable region of a single light chain bound to the variable region of a single heavy chain.

"Single-chain Fv antibody" or "scFv" refers to an engineered antibody consisting of a light chain variable region and a heavy chain variable region connected to one another directly or via a peptide linker sequence (Huston JS et al. Proc Natl Acad Sci USA, 85:5879(1988)).

"Single-chain Fv-Fc antibody" or "scFv-Fc" refers to an engineered antibody consisting of a scFv connected to the Fc region of an antibody.

"Camelized single domain antibody," "heavy chain antibody," or "HCAb" refers to an antibody that contains two V_{H} domains and no light chains (Riechmann L. and Muyldermans S., J Immunol Methods. Dec 10; 231(1-2):25-38 (1999); Muyldermans S., J Biotechnol. Jun; 74(4):277-302 (2001); WO94/04678; WO94/25591; U.S. Patent No. 6,005,079). Heavy chain antibodies were originally derived from Camelidae (camels, dromedaries, and llamas). Although devoid of light chains, camelized antibodies have an authentic antigen-binding repertoire (Hamers-Casterman C. et al., Nature. Jun 3; 363(6428):446-8 (1993); Nguyen VK. et al. Immunogenetics. Apr; 54(1):39-47 (2002); Nguyen VK. et al. Immunology. May; 109(1): 93-101 (2003)). The variable domain of a heavy chain antibody (VHH domain) represents the smallest known antigen-binding unit generated by adaptive immune responses (Koch-Nolte F. et al., FASEB J. Nov; 21(13): 3490-8. Epub 2007 Jun 15 (2007)).

A "nanobody" refers to an antibody fragment that consists of a VHH domain from a heavy chain antibody and two constant domains, CH2 and CH3.

A "diabody" or "dAb" includes small antibody fragments with two antigen-binding sites, wherein the fragments comprise a VH domain connected to a VL domain in the same polypeptide chain (VH-VL or VL-VH) (see, e.g. Holliger P. et al., Proc Natl Acad Sci USA. Jul 15;90(14):6444-8 (1993); EP404097; WO93/11161). By using a linker that is too short to allow pairing between the two domains on the same chain, the domains are forced to pair with the complementary domains of another chain, thereby creating two antigen-binding sites. The antigen-binding sites may target the same or different antigens (or epitopes). In some embodiments, a "bispecific ds diabody" is a diabody target two different antigens (or epitopes).

A "domain antibody" refers to an antibody fragment containing only the variable region of a heavy chain or the variable region of a light chain. In some instances, two or more VH domains are covalently joined with a peptide linker to create a bivalent or multivalent domain antibody. The two VH domains of a bivalent domain antibody may target the same or different antigens.

The term "valent" as used herein refers to the presence of a specified number of antigen binding sites in a given molecule. The term "monovalent" refers to an antibody or an antigen-binding fragment having only one single antigen-binding site; and the term "multivalent" refers to an antibody or antigen-binding fragment having multiple antigen-binding sites. As such, the terms "bivalent", "tetravalent", and "hexavalent" denote the presence of two binding sites, four binding sites, and six binding sites, respectively, in an antigen-binding molecule. In some embodiments, the antibody or an antigen-binding fragment thereof is bivalent.

As used herein, a "bispecific" antibody refers to an artificial antibody which has fragments derived from two different monoclonal antibodies and is capable of binding to two different epitopes. The two epitopes may present on the same antigen, or they may present on two different antigens.

In some embodiments, an "scFv dimer" is a bivalent diabody or bispecific scFv (BsFv) comprising VH-VL (linked by a peptide linker) dimerized with another VH-VL moiety such that VH's of one moiety coordinate with the VL's of the other moiety and form two binding sites which can target the same antigens (or epitopes) or different antigens (or epitopes). In other embodiments, an "scFv dimer" is a bispecific diabody comprising VH1-VL2 (linked by a peptide linker) associated with VL1-VH2 (also linked by a peptide linker) such that VH1 and VL1 coordinate and VH2 and VL2 coordinate and each coordinated pair has a different antigen specificity.

A "dsFv" refers to a disulfide-stabilized Fv fragment that the linkage between the variable region of a single light chain and the variable region of a single heavy chain is a disulfide bond. In some embodiments, a "(dsFv)2" or "(dsFv-dsFv')" comprises three peptide chains: two VH moieties linked by a peptide linker (e.g. a long flexible linker) and bound to two VL moieties, respectively, via disulfide bridges. In some embodiments, dsFv-dsFv' is bispecific in which each disulfide paired heavy and light chain has a different antigen specificity.

The term "chimeric" as used herein, means an antibody or antigen-binding fragment, having a portion of heavy and/or a light chain derived from one species, and the rest of the heavy and/or a light chain derived from a different species. In an illustrative example, a chimeric antibody may comprise a constant region derived from human and a variable region from a non-human animal, such as from mouse. In some embodiments, the non-human animal is a mammal, for example, a mouse, a rat, a rabbit, a goat, a sheep, a guinea pig, or a hamster.

The term "humanized" as used herein means that the antibody or antigen-binding fragment comprises CDRs derived from non-human animals, FR regions derived from human, and when applicable, the constant regions derived from human.

The term "affinity" as used herein refers to the strength of non-covalent interaction between an immunoglobulin molecule (i.e. antibody) or fragment thereof and an antigen.

The term "specific binding" or "specifically binds" as used herein refers to a non-random binding reaction between two molecules, such as for example between an antibody and an antigen. Specific binding can be characterized in binding affinity, for example, represented by K_{D} value, i.e., the ratio of dissociation rate to association rate (k_{off}/kₒₙ) when the binding between the antigen and antigen-binding molecule reaches equilibrium. K_{D} may be determined by using any conventional method known in the art, including but are not limited to surface plasmon resonance method, Octet method, microscale thermophoresis method, HPLC-MS method and FACS assay method. A K_{D} value of ≤10⁻⁶ M (e.g. ≤5×10⁻⁷ M, ≤2×10⁻⁷ M, ≤10⁻⁷ M, ≤5×10⁻⁸ M, ≤2×10⁻⁸ M, ≤10⁻⁸ M, ≤5×10⁻⁹ M, ≤4×10⁻⁹ M, ≤3×10⁻⁹ M, ≤2×10⁻⁹ M, or ≤10⁻⁹ M) can indicate specific binding between an antibody or antigen binding fragments thereof and PD-L1 (e.g. human PD-L1 or cynomolgus PD-L1).

The ability to "compete for binding to PD-L1" as used herein refers to the ability of a first antibody or antigen-binding fragment to inhibit the binding interaction between PD-L1 and a second anti-PD-L1 antibody to any detectable degree. In some embodiments, an antibody or antigen-binding fragment that compete for binding to PD-L1 inhibits the binding interaction between PD-L1 and a second anti- PD-L1 antibody by at least 85%, or at least 90%. In some embodiments, this inhibition may be greater than 95%, or greater than 99%.

The term "amino acid" as used herein refers to an organic compound containing amine (-NH₂) and carboxyl (-COOH) functional groups, along with a side chain specific to each amino acid. The names of amino acids are also represented as standard single letter or three-letter codes in the present disclosure, which are summarized as follows.

| **Names** | **Three-letter Code** | **Single-letter Code** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The terms "polypeptide", "peptide" and "protein" are used interchangeably herein to refer to a polymer of amino acid residues. The terms also apply to amino acid polymers in which one or more amino acid residue is an artificial chemical mimetic of a corresponding naturally occurring amino acid, as well as to naturally occurring amino acid polymers and non-naturally occurring amino acid polymers.

A "conservative substitution" with reference to amino acid sequence refers to replacing an amino acid residue with a different amino acid residue having a side chain with similar physiochemical properties. For example, conservative substitutions can be made among amino acid residues with hydrophobic side chains (e.g. Met, Ala, Val, Leu, and Ile), among amino acid residues with neutral hydrophilic side chains (e.g. Cys, Ser, Thr, Asn and Gln), among amino acid residues with acidic side chains (e.g. Asp, Glu), among amino acid residues with basic side chains (e.g. His, Lys, and Arg), or among amino acid residues with aromatic side chains (e.g. Trp, Tyr, and Phe). As known in the art, conservative substitution usually does not cause significant change in the protein conformational structure, and therefore could retain the biological activity of a protein.

"Percent (%) sequence identity" with respect to amino acid sequence (or nucleic acid sequence) is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum correspondence. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al, J. Mol. Biol., 215:403-410 (1990); Stephen F. et al, Nucleic Acids Res., 25:3389-3402 (1997)), ClustalW2 (available on the website of European Bioinformatics Institute, see also, Higgins D.G. et al, Methods in Enzymology, 266:383-402 (1996); Larkin M.A. et al, Bioinformatics (Oxford, England), 23(21): 2947-8 (2007)), and ALIGN or Megalign (DNASTAR) software. Those skilled in the art may use the default parameters provided by the tool, or may customize the parameters as appropriate for the alignment, such as for example, by selecting a suitable algorithm. In some embodiments, the non-identical residue positions may differ by conservative amino acid substitutions. A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art. See, e.g., Pearson (1994) Methods Mol. Biol. 24: 307-331, which is herein incorporated by reference.

As used herein, a "homologous sequence" refers to a polynucleotide sequence (or its complementary strand) or an amino acid sequence that has sequence identity of at least 80% (e.g. at least 85%, 88%, 90%, 91%, 92%, 93%, 94%, 95%, 96%, 97%, 98%, 99%) to another sequence when optionally aligned.

An "isolated" substance has been altered by the hand of man from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide is "isolated" if it has been sufficiently separated from the coexisting materials of its natural state so as to exist in a substantially pure state. An isolated "nucleic acid" or "polynucleotide" are used interchangeably and refer to the sequence of an isolated nucleic acid molecule. In some embodiments, an "isolated antibody or an antigen-binding fragment thereof" refers to the antibody or antigen-binding fragments having a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, 99% as determined by electrophoretic methods (such as SDS-PAGE, isoelectric focusing, capillary electrophoresis), or chromatographic methods (such as ion exchange chromatography or reverse phase HPLC).

The term "subject" includes human and non-human animals. Non-human animals include all vertebrates, e.g., mammals and non-mammals, such as non-human primates, mouse, rat, cat, rabbit, sheep, dog, cow, chickens, amphibians, and reptiles. Except when noted, the terms "patient" or "subject" are used herein interchangeably.

"Treating" or "treatment" of a condition as used herein includes preventing or alleviating a condition, slowing the onset or rate of development of a condition, reducing the risk of developing a condition, preventing or delaying the development of symptoms associated with a condition, reducing or ending symptoms associated with a condition, generating a complete or partial regression of a condition, curing a condition, or some combination thereof.

The term "vector" as used herein refers to a vehicle into which a genetic element may be operably inserted so as to bring about the expression of that genetic element, such as to produce the protein, RNA or DNA encoded by the genetic element, or to replicate the genetic element. A vector may be used to transform, transduce, or transfect a host cell so as to bring about expression of the genetic element it carries within the host cell. Examples of vectors include plasmids, phagemids, cosmids, artificial chromosomes such as yeast artificial chromosome (YAC), bacterial artificial chromosome (BAC), or P1-derived artificial chromosome (PAC), bacteriophages such as lambda phage or M13 phage, and animal viruses. A vector may contain a variety of elements for controlling expression, including promoter sequences, transcription initiation sequences, enhancer sequences, selectable elements, and reporter genes. In addition, the vector may contain an origin of replication. A vector may also include materials to aid in its entry into the cell, including but not limited to a viral particle, a liposome, or a protein coating. A vector can be an expression vector or a cloning vector. The present disclosure provides vectors (e.g. expression vectors) containing the nucleic acid sequence provided herein encoding the antibody or an antigen-binding fragment thereof, at least one promoter (e.g. SV40, CMV, EF-1α) operably linked to the nucleic acid sequence, and at least one selection marker.

The "host cell" as used herein refers to a cell into which an exogenous polynucleotide and/or a vector has been introduced.

The term "soluble" as used herein refers to the capability of a molecule (e.g., protein) of being dissolved in a solvent, such as a liquid and an aqueous environment.

The terms "transforming growth factor beta" and "TGFβ" as used herein refer to any of the TGFβ family proteins that have either the full-length, native amino acid sequence of any of the TGF-betas from subjects (e.g. human), including the latent forms and associated or unassociated complex of precursor and mature TGFβ ("latent TGFβ"). Reference to such TGFβ herein will be understood to be a reference to any one of the currently identified forms, including TGFβ1, TGFβ2, TGFβ3 isoforms and latent versions thereof, as well as to human TGFβ species identified in the future, including polypeptides derived from the sequence of any known TGFβ and being at least about 75%, preferably at least about 80%, more preferably at least about 85%, still more preferably at least about 90%, and even more preferably at least about 95% homologous with the sequence. The specific terms "TGFβ1," "TGFβ2," and "TGFβ3" refer to the TGF-betas defined in the literature, e.g., Derynck et al., Nature, Cancer Res., 47: 707 (1987); Seyedin et al., J. Biol. Chem., 261: 5693-5695 (1986); deMartin et al., EMBO J., 6: 3673 (1987); Kuppner et al., Int. J. Cancer, 42: 562 (1988). The terms "transforming growth factor beta", "TGFβ", "TGFbeta", "TGF-β", and "TGF-beta" are used interchangeably in the present disclosure.

As used herein, the term "human TGFβ1" refers to a TGFβ1 protein encoded by a human TGFB1 gene (e.g., a wild-type human TGFB1 gene). An exemplary wild-type human TGFβ1 protein is provided by GenBank Accession No. NP_000651.3. As used herein, the term "human TGFβ2" refers to a TGFβ2 protein encoded by a human TGFB2 gene (e.g., a wild-type human TGFB2 gene). Exemplary wild-type human TGFβ2 proteins are provided by GenBank Accession Nos. NP_001129071.1 and NP_003229.1. As used herein, the term "human TGFβ3" refers to a TGFβ3 protein encoded by a human TGFB3 gene (e.g., a wild-type human TGFB3 gene). Exemplary wild-type human TGFβ3 proteins are provided by GenBank Accession Nos. NP_003230.1, NP_001316868.1, and NP_001316867.1.

As used herein, the terms "mouse TGFβ1", "mouse TGFβ2", and "mouse TGFβ3" refer to a TGFβ1 protein, TGFβ2 protein, and TGFβ3 protein encoded by a mouse TGFB1 gene (e.g., a wild-type mouse TGFB1 gene), mouse TGFB2 gene (e.g., a wild-type mouse TGFB2 gene), and mouse TGFB3 gene (e.g., a wild-type mouse TGFB3 gene), respectively. Exemplary wild-type mouse (Mus musculus) TGFβ1 protein are provided by GenBank Accession Nos. NP_035707.1 and CAA08900.1. An exemplary wild-type mouse TGFβ2 protein is provided by GenBank Accession No. NP_033393.2. An exemplary wild-type mouse TGFβ3 protein is provided by GenBank Accession No. AAA40422.1.

The term "TGFβ receptor" as used herein refers to any receptor that binds at least one TGFβ isoform. Generally, the TGFβ receptor includes TGFβ Receptor I (TGFβRI), TGFβ Receptor II (TGFβRII), or TGFβ Receptor III (TGFβRIII).

With regard to human, the term "TGFβ Receptor I" or "TGFβRI" refers to human TGFβ Receptor Type 1 sequence, including the wild type TGFβRI as well as all isoforms and variants thereof known to be capable of binding to at least one TGFβ isoform. Exemplary amino acid sequence of wild type TGFβRI is available under GenBank Accession No. ABD46753.1 or under UniProtKB-P36897, also included herein as SEQ ID NO: 69. A variant TGFβRI may have a sequence of at least 80%, 85%, 90%, 91%, 92%, 93%, 94%, 95%, 96%, 97%, 98%, 99% sequence identity to the amino acid sequence of SEQ ID NO: 69 and retain at least 25%, 35%, 50%, 75%, 90%, 95%, or 99% of the TGFβ-binding activity of the wild-type sequence (e.g. SEQ ID NO: 69).

With regard to human, the term "TGFβ Receptor II" or "TGFβRII" refers to human TGFβ Receptor Type 2 Isoform A sequence, including the wild type TGFβRII as well as all isoforms and variants thereof known to be capable of binding to at least one TGFβ isoform. Exemplary amino acid sequence of wild type TGFβRII isoform A or isoform 1 is available under GenBank Accession No. NP_001020018.1 or under UniProtKB - P37173-1, also included herein as SEQ ID NO: 70, and wild type TGFβRII isoform B is available under GenBank Accession No. NP_003233.4 or UniProtKB - P37173-2, also included herein as SEQ ID NO: 71. A variant TGFβRII may have a sequence of at least 80%, 85%, 90%, 91%, 92%, 93%, 94%, 95%, 96%, 97%, 98%, 99% sequence identity to SEQ ID NO: 70 or 71, and retains at least 25%, 35%, 50%, 75%, 90%, 95%, or 99% of the TGFβ-binding activity of the wild-type sequence (e.g. SEQ ID NO: 70 or 71).

With regard to human, the term "TGFβ Receptor III" or "TGFβRIII" refers to human TGFβ Receptor Type 3 sequence, including the wild type TGFβRII as well as all isoforms and variants. Exemplary amino acid sequence of wild type TGFβRIII is available under GenBank Accession No. NP_003234.2 or under UniProtKB - Q03167, also included herein as SEQ ID NO: 72.

As used herein, the term "variant" with respect to a some reference protein or peptide means a modified version of the reference protein or peptide, e.g., functional equivalents, fragments, fusions, derivatives, mimetics, or any combination thereof, that has an amino acid sequence of at least 70% (e.g. 80%, 85%, 90%, 91%, 92%, 93%, 94%, 95%, 96%, 97%, 98%, 99%) sequence identity to the reference sequence, and retains at least 25% (e.g. 35%, 50%, 75%, 90%, 95%, or 99%) of the biological activity or binding activity of the reference sequence (e.g. the wild-type sequence). The variant can be a fragment, mutant, a fusion, a truncation, or any combination thereof, of the reference protein or peptide.

The term "Interleukin-1" or "IL-1" as used herein include IL-1α□and IL-1β, their precursors (e.g. pro-IL-1α□and pro-IL-1β), isoforms, and variants.

As used herein, the term "human IL-1α" refers to an IL-1α□protein encoded by a human IL1A gene (e.g., a wild-type human IL1A gene), and the isoforms, and variants. An exemplary wild-type human IL1α□protein is provided by UniProtKB - P01583.

As used herein, the term "human IL-1β" refers to an IL-1β□protein encoded by a human IL1B gene (e.g., a wild-type human IL1B gene). An exemplary wild-type human IL1β protein is provided by GenBank Accession No. NP_000567.1, or under UniProtKB - C9JVK0.

The term "IL-1 receptor" or "IL-1R" as used herein refers to a receptor that can bind to IL-1, including all wild type receptors, isoforms, and variants thereof capable of binding to IL-1. Generally, there are two types of IL-1 receptors, i.e., IL-1 Receptor I (IL-1RI), and IL-1 Receptor II (IL-1RII). IL-1RII acts as a decoy receptor that binds to ligand without transducing a signal. Proteolytical cleavage of IL-1RII results in formation of soluble receptors, e.g., IL-1sRI and IL-1sRII, which bind to ligand without transducing signal (see, details in Thomas G. Kennedy, Chapter V.B.2., in Encyclopedia of Hormones, 2003). IL-1sRI and IL-1sRII are proteolytic cleavage products of IL-1RII and can be a group of extracellular domain fragments of IL-1RII. The term IL-1R is also intended to encompass the coreceptor IL-1RAP, which can associate with IL-1RI bound to IL-1β□ □to form the high affinity interleukin-1 receptor complex which mediates interleukin-1-dependent activation of NF-kappa-B and other pathways.

As used herein, the term "IL-1RI" includes the wild type IL-1RI as well as all isoforms and variants thereof capable of binding to IL-1α and/or IL-1β. Exemplary amino acid sequence of wild type IL-1RI is available under UniProtKB - P14778, also included herein as SEQ ID NO: 73.

As used herein, the term "IL-1RII" includes the wild type IL-1RII as well as all isoforms and variants thereof capable of binding to IL-1α and/or IL-1β. Exemplary amino acid sequence of wild type IL-1RII is available under UniProtKB - P27930, also included herein as SEQ ID NO: 75.

As used herein, the term "IL-1RAP" includes the wild type IL-1RAP as well as all isoforms and variants thereof capable of binding to IL-1R bound to IL-1β. Exemplary amino acid sequence of wild type IL-1RAP is available under UniProtKB - Q9NPH3, also included herein as SEQ ID NO: 74.

As used herein, the term "IL-1sRI" includes all soluble forms of IL-1RI that may be produced by proteolytic cleavage involving metalloproteinase. Naturally occurring IL-1sRI may have a molecular weight ranging from about 45 kDa to 60 Kda. This term also encompasses all isoforms and variants of IL-1sRI, capable of binding to IL-1α and/or IL-1β.

As used herein, the term "IL-1sRII" includes all soluble forms of IL-1RII that may be produced by proteolytic cleavage involving metalloproteinase. Naturally occurring IL-1sRII may have a molecular weight ranging from about 45 kDa to 60 Kda. This term also encompasses all isoforms and variants of IL-1sRII, capable of binding to IL-1α and/or IL-1β.

The term "IL-1 receptor antagonist" as used herein generally include any protein that can compete with IL-1α or IL-1β□for binding to IL-1 receptor, and inhibits activity of IL-1α or IL-1β. □ □IL-1 receptor antagonist can include naturally-occurring antagonists such as IL-1Ra, IL-1sRI and IL-1sRII, as well as other artificial antagonists that can block binding of IL-1α or IL-1β□for binding to IL-1 receptor, in particular IL-1RI.

The term "IL-1Ra" as used herein include the wild type IL-1Ra as well as all isoforms and variants thereof capable of binding to IL-1α□and/or IL-1β. Exemplary amino acid sequence of wild type IL-1Ra is available under UniProtKB - P18510, also included herein as SEQ ID NO: 76.

"Cancer" as used herein refers to any medical condition characterized by malignant cell growth or neoplasm, abnormal proliferation, infiltration or metastasis, and can be benign or malignant, and includes both solid tumors and non-solid cancers (e.g. hematologic malignancies) such as leukemia. As used herein "solid tumor" refers to a solid mass of neoplastic and/or malignant cells.

The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof.

The term "fusion" or "fused" when used with respect to amino acid sequences (e.g. peptide, polypeptide or protein) refers to combination of two or more amino acid sequences, for example by chemical bonding or recombinant means, into a single amino acid sequence which does not exist naturally. A fusion amino acid sequence may be produced by genetic recombination of two encoding polynucleotide sequences, and can be expressed by a method of introducing a construct containing the recombinant polynucleotides into a host cell.

### Further detailed description of embodiments of the disclosure

Each embodiment and feature of each embodiment described in the context of this disclosure should be understood as being combinable with each other in any manner and each embodiment obtained such combination is included within the scope of the present disclosure as if it were specifically and individually set forth herein unless the context clearly indicates otherwise.

The following description of the present disclosure is intended only to illustrate various embodiments of the present disclosure. As such, the specific variations discussed should not be construed as limiting the scope of the disclosure. It will be apparent to those skilled in the art that various equivalents, changes, and modifications may be made without departing from the scope of the disclosure, and it is to be understood that such equivalent embodiments are to be included herein. All documents, including publications, patents, and patent applications, cited herein are hereby incorporated by reference in their entirety.

### I. Bi-functional Molecules Targeting an Immune Checkpoint Molecule and Blocking IL-1 Activity

The present disclosure provides a bi-functional molecule comprising a first moiety that binds to an immune checkpoint molecule, and a second moiety that blocks activity of Interleukin-1 (IL-1). The bi-functional molecule provided herein allows blockade and/or reduction in IL-1 activity in a tumor microenvironment by blocking the interaction between IL-1 and the IL-1 Receptor with either an IL-1-binding moiety or an IL-1 Receptor (IL-1R)-binding moiety (i.e., the second moiety of the bi-functional molecule). The IL-1-binding moiety and/or the IL-1R-binding moiety can be linked to a moiety targeting an immune checkpoint molecule which can be found on the surface of certain tumor cells or immune cells (i.e., the first moiety of the bi-functional molecule).

IL-1 is an inflammatory cytokine. Inflammation is an important component of the tumor microenvironment, and IL-1 plays a key role in carcinogenesis and tumor progression (A. Mantovani et al, Immunol Rev. 2018 Jan; 281(1): 57-61.). IL-1 acts at different levels in tumor initiation and progression, including driving chronic non-resolving inflammation, tumor angiogenesis, activation of the IL-17 pathway, induction of myeloid-derived suppressor cells (MDSC) and macrophage recruitment, invasion and metastasis (Id.).

Immune checkpoint molecules are expressed on certain immune cells such as T cells, Natural Killer cells, and so on. Some cancer cells may also express certain immune checkpoint molecules, which can block activation of the immune check point, thereby enabling the cancer cells to evade surveillance of the immune system.

By reducing IL-1 in the tumor microenvironment, and reducing check point blockade, the present disclosure provides a novel bi-functional molecule that could be useful for treating immune check point related diseases such as cancer, autoimmune diseases, infectious diseases, and so on.

In some embodiments, the first moiety comprises an agonist of a check point molecule that has immunostimulatory or costimulatory activity. Such immunostimulatory check point molecules can include, without limitation, CD27, CD70, CD28, CD80 (B7-1), CD86 (B7-2), CD40, CD40L (CD154), CD122, CD137, CD137L, OX40 (CD134), OX40L (CD252), GITR, ICOS (CD278), and ICOSLG (CD275), CD2,, ICAM-1, LFA-1 (CD11a/CD18), CD30, BAFFR, HVEM, CD7, LIGHT, NKG2C, SLAMF7, NKp80, CD160, and CD83.

In some embodiments, the first moiety comprises an inhibitor of a check point molecule that has immunoinhibitory or co-inhibitory activity. Such immune inhibitory check point molecules can include, without limitation, A2AR, B7-H3 (CD276), B7-H4 (VTCN1), BTLA (CD272), CTLA-4 (CD152), IDO1, IDO2, TDO, KIR, LAG3, NOX2, PD-1, PD-L1, PD-L2, TIM-3, VISTA, SIGLEC7 (CD328), TIGIT, PVR(CD155), SIGLEC9 (CD329), CD160, LAIR1, 2B4 (CD244), CD47, and B7-H5.

In some embodiments, the immune checkpoint molecule is PD-L1. In some embodiments, the first moiety comprises an antibody moiety against PD-L1 or an antigen-binding fragment thereof. In some embodiments, the first moiety comprises an antagonist antibody moiety against PD-L1 or an antigen-binding fragment thereof.

In some embodiments, the second moiety comprises an IL-1-binding moiety or an IL-1 Receptor (IL-1R)-binding moiety.

Both IL-1α and IL-1β are proinflammatory and bind to IL-1R. Upon binding to IL-1α or IL-1β, IL-1R can recruit both the IL-1R accessory protein and the adaptor protein MyD88 to the receptor complex, resulting in activation of the downstream signaling cascade and ultimately in the activation of a myriad of immune and inflammatory genes. It is found by the present inventors that, blocking the activity of IL-1 or its binding to IL-1R would be useful in combination with modulation of immune check point molecules.

In some embodiments, the IL-1 is IL-1α or IL-1β. In some embodiments, the IL-1β is human IL-1β.

In some embodiments, the second moiety comprises an IL-1-binding moiety. In some embodiments, the IL-1-binding moiety specifically binds to IL-1α or IL-1β. In some embodiments, the IL-1-binding moiety comprises a soluble IL-1R, an IL-1-binding fragment or variant of an IL-1R, or an antibody against IL-1 or an antigen-binding fragment thereof.

A soluble IL-1R can be a domain or fragment of the IL-1R, for example, the extracellular domain (ECD) of the IL-1R. Alternatively, a soluble IL-1R can also be IL-1sRI or IL-1sRII, which are isoforms that are naturally soluble and capable of binding to IL-1.

A skilled person would understand that it could be sufficient to for a shortened fragment of IL-1RI, or ECD of IL-1RI, or IL-1RII, or ECD of IL-1RII, or IL-1RAP, or ECD of IL-1RAP, or IL-1sRI or IL-1sRII, to bind to IL-1 (e.g. IL-1α or IL-1β), as long as such a fragment contains the IL-1 binding domain. Therefore, the present disclosure also encompasses all the IL-1-binding fragments and variants of any of IL-1RI, ECD of IL-1RI, or IL-1RII, or ECD of IL-1RII, or IL-1RAP, or ECD of IL-1RAP IL-1sRI and IL-1sRII. In some embodiments, the IL-1-binding moiety comprises an amino acid sequence of SEQ ID NOs: 73, 74, or 75, or an IL-1 binding fragment or variant thereof. In some embodiments, the IL-1-binding moiety comprises an amino acid sequence having at least 80% sequence identity to any of SEQ ID NOs: 73, 74, and 75, or an IL-1 binding fragment or variant thereof.

In some embodiments, IL-1-binding moiety comprises an antibody against IL-1 or an antigen-binding fragment thereof. Antibodies against IL-1 or its antigen-binding fragment may also be used, as long as such antibodies or antigen-binding fragment can block the binding of IL-1 (e.g., IL-1α or IL-1β) to IL-1R.

In some embodiments, the antibody against IL-1 or an antigen-binding fragment thereof comprises a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.

In some embodiments, the antibody against IL-1 or an antigen-binding fragment thereof comprises a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.

In some embodiments, the antibody against IL-1 or an antigen-binding fragment thereof comprises a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.

In some embodiments, the antibody against IL-1 or an antigen-binding fragment thereof comprises a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.

In some embodiments, the second moiety comprises an IL-1R-binding moiety.

In some embodiments, the IL-1R-binding moiety comprises IL-1Ra or an IL-1R-binding fragment or variant thereof. IL-1Ra is an antagonist of IL-1R and can compete with IL-1α or IL-1β for binding to IL-1R. Similarly, a skilled person would understand that it could be sufficient for a shortened fragment of IL-1Ra to be useful in binding to IL-1R and/or compete with IL-1α or IL-1β. In some embodiments, the IL-1R-binding moiety comprises a truncated form of IL-1Ra. In some embodiments, the IL-1R-binding moiety comprises an amino acid sequence of SEQ ID NO: 67 or 76, or any IL-1 binding fragment or variant thereof. In some embodiments, the IL-1R-binding moiety comprises an amino acid sequence having at least 80% sequence identity to SEQ ID NO: 67 or 76, or any IL-1 binding fragment or variant thereof. A skilled person would understand that, a variant of a wild-type IL-1Ra could also be useful in the present disclosure, as long as such a variant is capable of compete with IL-1α or IL-1β for binding with IL-1R.

In some embodiments, the IL-1R-binding moiety comprises an antibody against IL-1R or an antigen-binding fragment thereof. Antibodies against IL-1R or its antigen-binding fragment may also be used, as long as such antibodies or antigen-binding fragment can compete with IL-1α or IL-1β for binding with IL-1R.

### II. Bi-functional Molecules Targeting PD-L1 and a second moiety

Therapeutic efficacy of PD-1/PD-L1 axis checkpoint inhibitors (e.g. PD-L1 antibodies) could be limited when a tumor microenvironment ("TME") is enriched with immunosuppressive cytokines. Signaling of such immunosuppressive cytokines in the localized microenvironment can reduce tumor-infiltrating T cells, and skew them toward Tregs and attenuate the activation of immune effector cells.

In one aspect, the present disclosure provides a novel bi-functional molecule, comprising a first moiety that binds to PD-L1, and a second moiety that a) blocks activity of an immunosuppressive cytokine or b) stimulates anti-tumor immunity. The molecule may be a compound, a peptide, a polypeptide, a protein, or any combination thereof. The second moiety can restore the immune response in the tumor microenvironment, by either blocking an immunosuppressive activity or cytokine, or increasing or stimulating immunity.

In some embodiments, the bi-functional molecule provided herein comprises first moiety that binds to PD-L1 (i.e., a PD-L1-binding moiety), and a second moiety that blocks activity of an immunosuppressive cytokine.

In some embodiments, an immunosuppressive cytokine comprises a cytokine in transforming growth factor beta (TGF-β) superfamily IL-1, or Vascular endothelial growth factor (VEGF). In some embodiments, the immunosuppressive cytokine in TGF-β superfamily includes bone morphogenetic proteins (BMPs), activins, NODAL, and growth and differentiation factors (GDFs).

In some embodiments, the immunosuppressive cytokine is TGF-β. In some embodiments, the immunosuppressive cytokine is IL-1.

In some embodiments, the second moiety comprises a TGFβ-binding moiety. In some embodiments, the second moiety comprises an IL-1-binding moiety. As used herein, the term "binding moiety", "binding fragment" refers to a moiety or fragment that has an ability to specifically bind to a target molecule or complex. The term "TGFβ-binding moiety" refers to a moiety that has an ability to specifically bind to one or more family members or isoforms of the TGFβ family (e.g. TGFβ1, TGFβ2, or TGFβ3). Similarly, the term "IL-1-binding moiety" refers to a moiety that has an ability to specifically bind to one or more family members of the IL-1 family (e.g., IL-1α, IL-1β).

In some embodiments, the bi-functional molecule provided herein comprises first moiety that binds to PD-L1 (i.e., a PD-L1-binding moiety), and a second moiety that stimulates anti-tumor immunity. In some embodiments, the second moiety comprises an immunostimulatory polypeptide or a functional equivalent thereof or a variant thereof. In some embodiments, the immunostimulatory polypeptide is Interleukin(IL)-2 (IL-2), IL-15, IL-21, IL-10, IL-12, IL-23, IL-27, IL-35, granulocyte-macrophage colony-stimulating factor (GM-CSF), soluble CD4, soluble LAG-3, or IFN-α, or a functional equivalent thereof.

In some embodiments, the second moiety comprises an antagonist of an immunoinhibitory receptor signaling. In some embodiments, the immunoinhibitory receptor is SIRPα. □ □

In some embodiments, the bi-functional molecule comprises one or more of the second moieties. In some embodiments, the one or more of the second moieties may be of the same type, for example, each of them may block activity of an immunosuppressive cytokine, or each of them may stimulate anti-tumor immunity. In some embodiments, the one or more of the second moieties may be of different types. In some embodiments, each of the second moieties may have the same sequence, or may have different in amino acid sequences.

### i. TGFβ-Binding Moiety

In some embodiments, the TGFβ-binding moiety comprises a soluble TGFβ Receptor (TGFβR) or a TGFβ-binding fragment or variant thereof, or an antibody against TGFβ and an antigen-binding fragment thereof.

The "TGFβ-binding moiety" may also be referred to as "TGFβ Trap" in the present disclosure. Accordingly, a protein targeting both PD-L1 and TGFβ may also be referred to as "anti-PD-L1/TGFβ Trap" in the present disclosure.

In some embodiments, the TGFβ-binding moiety binds to human and/or mouse TGFβ. In some embodiments, the TGFβ-binding moiety is capable of antagonizing and/or inhibiting TGFβ signaling pathway. In some embodiments, the TGFβ-binding moiety is capable of antagonizing and/or inhibiting TGFβ.

In the present disclosure, the TGFβ-binding moiety can comprise any moiety that specifically binds to one or more family members or isoforms of TGFβ family. In some embodiments, the TGFβ-binding moiety comprises a moiety that binds to TGFβ1 (e.g. human TGFβ1), TGFβ2 (e.g. human TGFβ2), and/or TGFβ3 (e.g. human TGFβ3), or a variant thereof that has similar or improved TGFβ binding affinity. In some embodiments, the TGFβ-binding moiety comprises a moiety that binds to TGFβ1 (e.g. human TGFβ1). In some embodiments, the TGFβ-binding moiety comprises a moiety that binds to TGFβ2 (e.g. human TGFβ2). In some embodiments, the TGFβ-binding moiety comprises a moiety that binds to TGFβ3 (e.g. human TGFβ3). In some embodiments, the TGFβ-binding moiety comprises a moiety that specifically binds to both TGFβ1 (e.g. human TGFβ1) and TGFβ2 (e.g. human TGFβ2). In some embodiments, the TGFβ-binding moiety comprises a moiety that specifically binds to both TGFβ1 (e.g. human TGFβ1) and TGFβ3 (e.g. human TGFβ3). In some embodiments, the TGFβ-binding moiety comprises a moiety that specifically binds to both TGFβ2 (e.g. human TGFβ2) and TGFβ3 (e.g. human TGFβ3). In some embodiments, the TGFβ-binding moiety comprises a moiety that specifically binds to each of TGFβ1 (e.g. human TGFβ1), TGFβ2 (e.g. human TGFβ2), and TGFβ3 (e.g. human TGFβ3). A person skilled in the art would appreciate that a TGFβ-binding moiety that binds to one family member or isoform of TGFβ family may be capable of binding to one or more other family members or isoforms of TGFβ family with similar or higher affinity.

In some embodiments, the TGFβ-binding moiety comprises a moiety that selectively binds to TGFβ1 over TGFβ2, and/or over TGFβ3.

In some embodiments, the TGFβ-binding moiety comprises a moiety that specifically binds to human TGFβ1 and mouse TGFβ1 with similar affinity.

In some embodiments, the TGFβ-binding moiety of the present disclosure comprises a soluble TGFβ Receptor (TGFβR) or a TGFβ-binding fragment or a variant thereof.

Exemplary TGFβ Receptors include TGFβRI, TGFβRII and TGFβRIII. In some embodiments, the TGFβ Receptor is selected from the group consisting of TGFβ Receptor I (TGFβRI), TGFβ Receptor II (TGFβRII), TGFβ Receptor III (TGFβRIII), and any combination thereof. In some embodiments, the TGFβ receptor is TGFβRI (e.g. human TGFβRI). In some embodiments, the TGFβ receptor is TGFβRII (e.g. human TGFβRII). In some embodiments, the TGFβ receptor is TGFβRIII (e.g. human TGFβRIII).

In some embodiments, the TGFβ-binding moiety comprises an extracellular domain (ECD) of a TGFβ receptor (e.g. a human TGFβ receptor), or a TGFβ-binding fragment or a variant thereof. In some embodiments, the ECD of a TGFβ receptor comprises an ECD of TGFβRI (e.g. human TGFβRI), an ECD of TGFβRII (e.g. human TGFβRII), an ECD of TGFβRIII (e.g. human TGFβRIII), or any combination thereof. In some embodiments, the ECD of the TGFβRII comprises an amino acid sequence of SEQ ID NO: 66, 79, or an amino acid sequence having at least 80% (e.g. at least 85%, 86%, 87%, 88%, 89%, 90%, 91%, 92%, 93%, 94%, 95%, 96%, 97%, 98%, or 99%) sequence identity thereof yet retaining binding specificity to TGFβ. In some embodiments, the ECD of the TGFβRI comprises an amino acid sequence of SEQ ID NO: 77, or an amino acid sequence having at least 80% (e.g. at least 85%, 86%, 87%, 88%, 89%, 90%, 91%, 92%, 93%, 94%, 95%, 96%, 97%, 98%, or 99%) sequence identity thereof yet retaining binding specificity to TGFβ. In some embodiments, the ECD of the TGFβRIII comprises an amino acid sequence of SEQ ID NO: 78, or an amino acid sequence having at least 80% (e.g. at least 85%, 86%, 87%, 88%, 89%, 90%, 91%, 92%, 93%, 94%, 95%, 96%, 97%, 98%, or 99%) sequence identity thereof yet retaining binding specificity to TGFβ.

In some embodiments, the TGFβ-binding moiety comprises an antibody against TGFβ□and an antigen-binding fragment thereof. Exemplary anti-TGFβ antibodies include fresolimumab and metelimumab, as well as the anti-TGFβ antibodies or antigen-binding fragments thereof described in, for example, US7494651B2, US8383780B2, US8012482B2, WO2017141208A1, each of which is incorporated herein by reference in its entirety.

In some embodiments, the TGFβ-binding moiety comprises a combination of one or more ECDs of one or more TGFβ receptors and/or one or more anti-TGFβ antibodies or antigen-binding fragments thereof.

The one or more ECDs may be the same or different. For example, the TGFβ-binding moiety may comprise identical repeats of an ECD of a TGFβ receptor, or alternatively may comprise a combination of different ECD sequences of the same TGFβ receptor, or alternatively may comprise a combination of different ECDs from different TGFβ receptors. Similarly, the one or more anti-TGFβ antibodies may be the same of different.

In some embodiments, the TGFβ-binding moiety comprises a combination (or fusion) of ECDs selected from the group consisting of: ECD of TGFβRI (e.g. human TGFβRI), ECD of TGFβRII (e.g. human TGFβRII), ECD of TGFβRIII (e.g. human TGFβRIII), or any combination thereof.

In some embodiments, the TGFβ-binding moiety comprises a combination (or fusion) of one or more anti-TGFβ antibodies or antigen-binding fragments thereof.

In some embodiments, the TGFβ-binding moiety comprises a combination (or fusion) of ECDs selected from the group consisting of: ECD of TGFβRI (e.g. human TGFβRI), ECD of TGFβRII (e.g. human TGFβRII), ECD of TGFβRIII (e.g. human TGFβRIII), one or more anti-TGFβ antibodies or antigen-binding fragments thereof, or any combination thereof.

### ii. IL-1-Binding Moiety

In some embodiments, the second moiety comprises an IL-1-binding moiety. In some embodiments, the IL-1 is IL-1α or IL-1β. □In some embodiments, the IL-1β is human IL-1β.

In some embodiments, the IL-1-binding moiety specifically binds to IL-1α or IL-1β. In some embodiments, the IL-1-binding moiety comprises a moiety that selectively binds to IL-1β over IL-1α, or selectively binds to IL-1α over IL-1β.

In some embodiments, the IL-1-binding moiety comprises a soluble IL-1R, an IL-1-binding fragment or variant of an IL-1R, or an antibody against IL-1 or an antigen-binding fragment thereof.

A soluble IL-1R can comprise a domain or fragment or variant of the IL-1R, for example, the extracellular domain (ECD) of the IL-1R. Alternatively, a soluble IL-1R can also comprise IL-1sRI or IL-1sRII, which are isoforms that are naturally soluble and capable of binding to IL-1.

A skilled person would understand that it could be sufficient to for a shortened fragment of IL-1R, or ECD of IL-1R, or IL-1sRI or IL-1sRII, to bind to IL-1 (e.g. IL-1α or IL-1β), as long as such a fragment contains the IL-1 binding domain. Therefore, the IL-1-binding moiety provided herein can also comprise an IL-1-binding fragment of any of IL-1R, ECD of IL-1R, IL-1sRI and IL-1sRII. In some embodiments, the IL-1-binding moiety comprises an amino acid sequence of SEQ ID NOs: 73, 74, or 75, or an IL-1 binding fragment or variant thereof. In some embodiments, the IL-1-binding moiety comprises an amino acid sequence having at least 80% sequence identity to any of SEQ ID NOs: 73, 74, and 75, or an IL-1 binding fragment or variant thereof.

In some embodiments, IL-1-binding moiety comprises an antibody against IL-1 or an antigen-binding fragment thereof. Antibodies against IL-1 or its antigen-binding fragment may also be used, as long as such antibodies or antigen-binding fragment can block the binding of IL-1 (e.g., IL-1α or IL-1β) to IL-1R.

In some embodiments, the antibody against IL-1 or an antigen-binding fragment thereof comprises a heavy chain variable region comprising a HCDR1 comprising a sequence of SEQ ID NO: 104 or SEQ ID NO: 112, a HCDR2 comprising a sequence of SEQ ID NO: 105 or SEQ ID NO: 113, and a HCDR3 comprising a sequence of SEQ ID NO: 106 or SEQ ID NO: 114, and/or a light chain variable region comprising a LCDR1 comprising a sequence of SEQ ID NO: 107 or SEQ ID NO: 115, a LCDR2 comprising a sequence of SEQ ID NO: 108 or SEQ ID NO: 116, and a LCDR3 comprising a sequence of SEQ ID NO: 109 or SEQ ID NO: 117.

In some embodiments, the antibody against IL-1 or an antigen-binding fragment thereof comprises a heavy chain variable region comprising a HCDR1 comprising a sequence of SEQ ID NO: 104, a HCDR2 comprising a sequence of SEQ ID NO: 105, and a HCDR3 comprising a sequence of SEQ ID NO: 106, and/or a light chain variable region comprising a LCDR1 comprising a sequence of SEQ ID NO: 107, a LCDR2 comprising a sequence of SEQ ID NO: 108, and a LCDR3 comprising a sequence of SEQ ID NO: 109.

In some embodiments, the antibody against IL-1 or an antigen-binding fragment thereof comprises a heavy chain variable region comprising a HCDR1 comprising a sequence of SEQ ID NO: 112, a HCDR2 comprising a sequence of SEQ ID NO: 113, and a HCDR3 comprising a sequence of SEQ ID NO: 114, and/or a light chain variable region comprising a LCDR1 comprising a sequence of SEQ ID NO: 115, a LCDR2 comprising a sequence of SEQ ID NO: 116, and a LCDR3 comprising a sequence of SEQ ID NO: 117.

In some embodiments, the antibody against IL-1 or an antigen-binding fragment thereof comprises a heavy chain variable region comprising a sequence selected from the group consisting of SEQ ID NO: 102, SEQ ID NO: 110, and a homologous sequence thereof having at least 80% sequence identity thereof, and/or a light chain variable region comprising a sequence selected from the group consisting of SEQ ID NO: 103, SEQ ID NO: 111, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 102, and a homologous sequence thereof having at least 80% sequence identity thereof, and/or a light chain variable region comprising a sequence selected from the group consisting of SEQ ID NO: 103, and a homologous sequence thereof having at least 80% sequence identity thereof.

In some embodiments, the antibody against IL-1 or an antigen-binding fragment thereof comprises a heavy chain variable region comprising a sequence selected from the group consisting of SEQ ID NO: 110, and a homologous sequence thereof having at least 80% sequence identity thereof, and/or a light chain variable region comprising a sequence selected from the group consisting of SEQ ID NO: 111, and a homologous sequence thereof having at least 80% sequence identity thereof.

In some embodiments, the IL-1-binding moiety comprises a combination of one or more moieties selected from the group consisting of: IL-1R, the ECD of IL-1R, IL-1sRI, IL-1sRII, antibody against IL-1, any IL-1-binding fragment thereof and any combination thereof. Such one or more moieties may be linked by a direct bond or may be linked by a suitable linker.

In some embodiments, the IL-1R-binding moiety comprises IL-1Ra or an IL-1R-binding fragment or variant thereof. IL-1Ra is an antagonist of IL-1R and can compete with IL-1α or IL-1β for binding to IL-1R. Similarly, a skilled person would understand that it could be sufficient for a shortened fragment of IL-1Ra to be useful in binding to IL-1R and/or compete with IL-1α or IL-1β. In some embodiments, the IL-1R-binding moiety comprises a truncated form of IL-1Ra. In some embodiments, the IL-1R-binding moiety comprises an amino acid sequence of SEQ ID NO: 67, or any IL-1 binding fragment or variant thereof. In some embodiments, the IL-1R-binding moiety comprises an amino acid sequence having at least 80% sequence identity to SEQ ID NO: 67, or any IL-1 binding fragment or variant thereof. A skilled person would understand that, a variant of a wild-type IL-1Ra could also be useful in the present disclosure, as long as such a variant is capable of compete with IL-1α or IL-1β for binding with IL-1R.

In some embodiments, the IL-1R-binding moiety comprises an antibody against IL-1R or an antigen-binding fragment thereof. Antibodies against IL-1R or its antigen-binding fragment may also be used, as long as such antibodies or antigen-binding fragment can compete with IL-1α or IL-1β for binding with IL-1R.

In some embodiments, the IL-1R-binding moiety comprises a combination of one or more moieties selected from the group consisting of: IL-1Ra, antibody against IL-1R, any IL-1R-binding fragment or variant thereof, and any combination thereof. Such one or more moieties may be linked by a direct bond or may be linked by a suitable linker.

### iii. Immunostimulatory polypeptide

In some embodiments, the second moiety comprises an immunostimulatory polypeptide or a functional equivalent thereof or a variant thereof. In some embodiments, the immunostimulatory polypeptide is soluble CD4, soluble LAG-3, or a functional equivalent thereof.

In some embodiments, the soluble LAG-3 comprises an extracellular domain (ECD) of the LAG-3 or a MHC class II (MHCII)-binding fragment or variant thereof.

LAG-3 (Uniprot number: Q61790) belongs to immunoglobulin (Ig) superfamily, which is a type I transmembrane protein comprising 503 amino acid. Lag-3 comprises an intracellular domain (ICD), a transmembrane domain (TMD), and an extracellular domain (ECD). The ECD comprises four Ig-like domains, i.e., D1 to D4, wherein D1 comprises 9□β-chains: A, B, C, C', C", D, E, F and G chains. Between the C and C' chains, there is an additional sequence having about 30 amino acids that forms an "extra loop". Such "extra loop" has been reported to be involved in the interaction between LAG-3 and MHCII. In some embodiments, the soluble LAG-3 comprises the amino acid sequence of the extra loop, the D1 domain, D1 plus D2 domains, or any MHC II-binding fragment or variant thereof. In some embodiments, the soluble LAG-3 comprises the amino acid sequence of SEQ ID NO: 99, SEQ ID NO: 100, SEQ ID NO: 101, or any MHC II-binding fragment or variant thereof.

LAG-3 is expressed on activated T cells, natural killer cells, B cells and plasmacytoid dendritic cells. Its principal ligand is MHC class II, to which it binds with higher affinity than CD4. A connecting peptide (CP) exists between D4 and the TMD of LAG-3, where cleavage occurs in presence of metalloproteinase ADAM10 and/or ADAM17 to produce cleaved soluble LAG-3. See, e.g., Huard et al., Proc Natl Acad Sci U S A 1997;94:5744-9.; Workman et al., J Immunol 2002;169:5392-5.doi:10.4049/jimmunol.169.10.5392; and Lawrence et al., J Immunother Cancer. 2015; 3(Suppl 2): P216, which have herein incorporated by reference.

LAG-3 also encodes an alternative splice variant that is translated to a soluble form of LAG-3. Soluble LAG-3 activates antigen-presenting cells (APCs) through MHCII signaling, leading to increased antigen-specific T-cell responses in vivo. For example, soluble LAG-3 activates dendritic cells (DC) and has been reported to be involved in the proinflammatory activity of cytokine-activated (such as TNF-α and/or IL-12-activated) bystander T cells and it may directly activate DC. See, e.g., Triebel, Trends Immunol., 2003, 24: 619-622, which is herein incorporated by reference.

In some embodiments, the soluble LAG-3 comprises Eftilagimod alpha (IMP321) or a MHC II-binding fragment or variant thereof. IMP321 is a soluble dimeric recombinant form of LAG-3. IMP321 induces sustained immune responses by stimulating dendritic cells through MHCII molecules. Combinatory therapy of MP321 and an anti-PD-1 antibody or an anti-PD-L1 antibody has been shown to synergistically activate T cells (in particular, CD8+ T cells). See, e.g., Luc et al., Future Oncol Actions Search in PubMed Search in NLM Catalog Add to Search. 2019 Jun;15(17):1963-1973. doi: 10.2217/fon-2018-0807. Epub 2019 Apr 12.; Julio et al., Journal of Clinical Oncology, Volume 37, Issue 15; and US10874713 B, which is herein incorporated by reference.

### iv. Antagonist of an Immunoinhibitory Receptor Signaling

In some embodiments, the second moiety comprises an antagonist of an immunoinhibitory receptor signaling. In some embodiments, the immunoinhibitory receptor is SIRPα. □ □

As used herein, the term "SIRPα", interchangeably with the term "Signal-regulatory protein alpha" refers to an inhibitory receptor expressed primarily on myeloid cells and dendritic cells. SIRPα belongs to the SIRPs family that also includes several other transmembrane glycoproteins, including, SIRPβ and SIRPγ. Each member of the SIRPs family contains 3 similar extracellular Ig-like domains with distinct transmembrane and cytoplasmic domains.

SIRPα can bind to CD47, which delivers a "don't eat me" signal to suppress phagocytosis, and blocking the CD47 mediated engagement of SIRPα on a phagocyte can cause removal of live cells bearing "eat me" signals. CD47 is a broadly expressed transmembrane glycoprotein with an extracellular N-terminal IgV domain, five transmembrane domains, and a short C-terminal intracellular tail. CD47 functions as a cellular ligand for SIRPα. Tumor cells frequently overexpress CD47 to evade macrophage-mediated destruction. The interaction of CD47 and SIRPα has been shown to be involved in the regulation of macrophage-mediated phagocytosis (Takenaka et al., Nature Immunol., 8(12): 1313-1323, 2007).

In some embodiments, the second moiety blocks interaction between CD47 and SIRPα. In a diverse range of preclinical models, therapies that block the interaction of CD47 and SIRPα stimulate phagocytosis of cancer cells in vitro and anti-tumor immune responses in vivo.

The second moiety can comprise a CD47 binding domain or a SIRPα binding domain. In some embodiments, the immunoinhibitory receptor is signal-regulatory protein alpha (SIRPα). In some embodiments, the second moiety blocks interaction between CD47 and SIRPα. In some embodiments, the second moiety comprises a CD47 binding domain or a SIRPα binding domain. In some embodiments, the CD47 binding domain comprises a soluble SIRPα or a CD47 binding fragment thereof, or an anti-CD47 antibody or an antigen-binding fragment thereof.

In some embodiments, the soluble SIRPα comprises an extracellular domain (ECD) of the SIRPα or a CD47-binding fragment or a variant thereof. In some embodiments, the soluble SIRPα comprises an amino acid sequence of SEQ ID NO: 84 or an amino acid sequence having at least 80% (e.g. at least 85%, 86%, 87%, 88%, 89%, 90%, 91%, 92%, 93%, 94%, 95%, 96%, 97%, 98%, or 99%) sequence identity thereof yet retaining binding specificity to CD47. Optionally, the soluble SIRPα is an engineered high-affinity SIRPα variant, which potently antagonized CD47 on cancer cells but does not induce macrophage phagocytosis on its own. In some embodiments, the SIRPα variant comprises one or more mutations selected from the group consisting of: L4V, L4I, V6I, V6L, A21V, V27I, V27L, I31T, I31S, I31F, E47V, E47L, K53R, E54Q, H56P, H56R, V63I, S66T, S66G, K68R, V92I, F94L, F94V and F103V, relative to SEQ ID NO: 98. In some embodiments, the SIRPα variant comprises a combination of mutations selected from the group consisting of: 1) V27I, K53R, S66T, K68R, F103V; 2) L4V, V27L, E47V, K53R, E54Q, S66G, K68R, V92I; 3) L4V, V6I, A21V, V27I, I31T, E47L, K53R, H56P, S66T, K68R, F94L; 4) V6I, V27I, I31S, E47V, K53R, E54Q, H56P, S66G, V92I, F94L; 5) L4I, A21V, V27I, I31F, E47V, K53R, E54Q, H56R, S66G, F94V, F103V; 6) L4V, V6I, V27I, I31F, E47V, K53R, H56R, S66G, K68R, V92I, F94L; 7) L4V, V6L, I31F, E47V, K53R, H56P, S66G, V92I, F103V; 8) V6I, V27I, I31F, E47L, K53R, E54Q, H56P, S66T; 9) L4V, V6I, V27I, I31F, E47V, K53R, E54Q, H56P, V63, S66T, K68R, V92I; 10) V6I, V27I, I31T, E47V, K53R, E54Q, H56P, S66G, K68R, V92I, F103V; and 11) V6I, V27I, I31F, E47V, K53R, E54Q, H56P, S66T,V92I. See, e.g., Kipp Weiskopf et al. Science 341, 88 (2013), which is herein incorporated by reference.

In some embodiments, the SIRPα binding domain comprises a soluble CD47 or a SIRPα binding fragment thereof, or an anti-SIRPα antibody or an antigen-binding fragment thereof. In some embodiments, the soluble CD47 comprises an extracellular domain (ECD) of the CD47 or a SIRPα binding fragment thereof, an anti-SIRPα antibody or an antigen-binding fragment thereof.

In some embodiments, the CD47-binding domain comprises an anti-CD47 antibody □ and an antigen-binding fragment thereof. Exemplary anti-CD47 antibodies include, without limitation, humanized 5F9 antibody, B6H12 antibody and ZF1 antibody. See, Lu et al., OncoTargets and Therapy, Volume 13, DOI https://doi.org/10.2147/OTT.S249822, which is herein incorporated by reference.. In some embodiments, the SIRPα binding domain comprises an anti-SIRPα antibody or an antigen-binding fragment thereof. Exemplary anti-SIRPα antibodies include, without limitation, BI765064 and AL008. See, e.g., WO2019073080A1, WO2019175218A1 and WO2018107058A1, which are herein incorporated by reference.

In some embodiments, the CD47-binding domain comprises a combination of one or more ECDs of one or more SIRPα, SIRPβ or SIRPγ and/or one or more anti-CD47 antibodies or antigen-binding fragments thereof.

The one or more ECDs may be the same or different. For example, the CD47-binding domain may comprise identical repeats of an ECD of a SIRPα, SIRPβ or SIRPγ or alternatively may comprise a combination of different ECD sequences of the same SIRPα, SIRPβ or SIRPγ, or alternatively may comprise a combination of different ECDs from different SIRPα, SIRPβ or SIRPγ. Similarly, the one or more anti-CD47 antibodies may be the same of different.

In some embodiments, the CD47-binding domain comprises a combination (or fusion) of ECDs selected from the group consisting of: ECD of SIRPα, ECD of SIRPβ, ECD of SIRPγ, or any combination thereof.

In some embodiments, the CD47-binding domain comprises a combination (or fusion) of one or more anti-CD47 antibodies or antigen-binding fragments thereof.

In some embodiments, the CD47-binding domain comprises a combination (or fusion) of ECDs selected from the group consisting of: ECD of SIRPα, ECD of SIRPβ, ECD of SIRPγ, one or more anti-CD47 antibodies or antigen-binding fragments thereof, or any combination thereof.

### v. PD-L1-Binding Moiety

In some embodiments, the bi-functional molecule provided herein comprises a first moiety which is a PD-L1-binding moiety.

In some embodiments, the PD-L1-binding moiety of the present disclosure binds to PD-L1 (e.g. human PD-L1, or cynomolgus PD-L1). In some embodiments, the PD-L1-binding moiety of the present disclosure binds to human PD-L1. In some embodiments, the PD-L1-binding moiety of the present disclosure binds to cynomolgus PD-L1.

In some embodiments, the PD-L1-binding moiety of the present disclosure comprises an anti-PD-L1 antibody moiety. In some embodiments, exemplary anti-PD-L1 antibodies are disclosed in Section Anti-PD-L1 Antibodies and Section Illustrative Anti-PD-L1 Antibodies of the present disclosure.

In some embodiments, the anti-PD-L1 antibody moiety comprises one or more CDRs. In some embodiments, the anti-PD-L1 antibody moiety comprises one or more CDRs described in Section Illustrative Anti-PD-L1 Antibodies of the present disclosure. In some embodiments, the anti-PD-L1 antibody moiety comprises a heavy chain variable region (VH) and a light chain variable region (VL). In some embodiments, the anti-PD-L1 antibody moiety comprises a VH and a VL of an anti-PD-L1 antibody as disclosed in Section Illustrative Anti-PD-L1 Antibodies of the present disclosure.

In some embodiments, the anti-PD-L1 antibody moiety further comprises a heavy chain constant domain appended to a carboxyl terminus of the heavy chain variable region. In some embodiments, the heavy chain constant region is derived from the group consisting of IgA, IgG, and IgM. In some embodiments, the heavy chain constant region is derived from human IgG1, IgG2, IgG3, IgG4, IgA1, IgA2 or IgM. In some embodiments, the anti-PD-L1 antibody moiety further comprises a light chain constant domain appended to a carboxyl terminus of the light chain variable region. In some embodiments, the light chain constant region is derived from Kappa light chain or Lamda light chain. In some embodiments, the heavy chain constant region comprises an amino acid sequence of SEQ ID NO: 80 or 81. In some embodiments, the light chain constant region comprises an amino acid sequence of SEQ ID NO: 82.

### vi. Linkage Between the First moiety and the Second Moiety

In the present disclosure, the second moiety can be linked to any portion of the first moiety. For example, the second moiety such as the TGFβ-binding moiety or the IL-1 - binding moiety can be linked to any suitable portion of the first moiety such as the PD-L1-binding moiety (e.g. the anti-PD-L1 antibody moiety).

In some embodiments, the PD-L1-binding moiety comprises one or more polypeptide chains, such as antibody heavy chain and light chain.

In some embodiments, the bi-functional molecule comprises one or more of the second moieties. In some embodiments, at least one of the second moieties is linked to an amino terminus (N terminus) or a carboxyl (C terminus) of a polypeptide chain of the first moiety. In some embodiments, the at least one of the second moieties is linked to an N terminus or a C terminus of a heavy chain of the first moiety, or linked to an N terminus or a C terminus of a light chain of the first moiety.

In some embodiments, the at least one of the second moieties is linked to a C terminus of a heavy chain constant region of the first moiety. In some embodiments, each of the second moieties is linked respectively to the C terminus of each heavy chain constant region of the first moiety.

In some embodiments, the bi-functional molecule comprises at least two of the second moieties, each of which is linked respectively to the C terminus of each heavy chain of the first moiety, or each of which is linked respectively to the C terminus of each light chain of the first moiety. In some embodiments, the bi-functional molecule comprises at least two of the second moieties, each of which is linked respectively to the N terminus of each heavy chain of the first moiety, or each of which is linked respectively to the N terminus of each light chain of the first moiety.

In some embodiments, the bi-functional molecule comprises more than one of the second moieties that are linked respectively to: an N terminus of a heavy chain of the first moiety, a C terminus of a heavy chain of the first moiety, an N terminus of a light chain of the first moiety, a C terminus of a light chain of the first moiety, or any combination thereof. For example, the bi-functional molecule can comprise at least two of the second moieties, one of which is linked to C terminus of a heavy chain of the first moiety and the other is linked to C terminus of a light chain of the first moiety. For example, the bi-functional molecule can comprise at least two of the second moieties, one of which is linked to N terminus of a heavy chain of the first moiety and the other is linked to N terminus of a light chain of the first moiety.

In some embodiments, the one or more TGFβ-binding moiety, the one or more IL-1 - binding moiety, the one or more immunostimulatory polypeptide (e.g., soluble LAG3 or soluble CD4) or the one or more CD47-binding moiety is linked to the anti-PD-L1 antibody moiety at one or more positions selected from the group consisting of: 1) N terminus of the heavy chain variable region, 2) N terminus of the light chain variable region, 3) C terminus of the heavy chain variable region; 4) C terminus of the light chain variable region; 5) C terminus of the heavy chain constant region; 6) C terminus of the light chain constant region, and 7) any combination thereof, of the anti-PD-L1 antibody moiety.

In some embodiments, the bi-functional molecule comprises homodimeric heavy chains. In some embodiments, the bi-functional molecule comprises heterodimeric heavy chains. The heavy chains are heterodimeric with respect to presence or position of the second moiety. In some embodiments, the heterodimeric heavy chains comprise one heavy chain having the second moiety but the other heavy chain having not.

### vii. Linker

The second moiety can be linked to the first moiety directly or via a linker. The direct linkage can be a chemical linkage (such as a covalent bond).

In some embodiments, the bi-functional molecule further comprises a linker connecting the first moiety and the second moiety. The term "linker" as used herein can be any suitable bifunctional moiety capable of reacting with at least two entities to be linked, thereby bonding the entities to form one molecule or maintaining association of the entities in sufficiently close proximity. The linker can be integrated in the resulting linked molecule or structure, with or without its reacted functional groups.

In some embodiments, the linker is selected from the group consisting of a cleavable linker, a non-cleavable linker, a peptide linker, a flexible linker, a rigid linker, a helical linker, and a non-helical linker.

In some embodiments, the linker comprises a peptide linker. The peptide linker can be made up of amino acid residues linked together by peptide bonds. In some embodiments, the peptide linker can further comprise one or more non-natural amino acids. In some embodiments, the peptide linker comprises an amino acid sequence having at least 1, 2, 3, 4, 5, 8, 10, 15, 20, 30, 50 or more amino acid residues, joined by peptide bonds and capable of linking two or more polypeptides. A peptide linker may or may not have a secondary structure.

Any suitable peptide linkers can be used. Many peptide linker sequences are known in the art, see, for example, Holliger et al., Proc. Natl. Acad. Sci. USA 90:6444-6448 (1993); Poljak et al., Structure 2:1121-1123 (1994). In some embodiments, the peptide linker may comprise or consist of amino acid residues selected from the amino acids glycine, serine, alanine, methionine, asparagine, and glutamine. In some embodiments, the peptide linker can be made up of a majority of amino acids that are sterically unhindered, such as glycine and alanine. In some embodiments, linkers are polyglycines, polyalanines, combinations of glycine and alanine (such as poly(Gly-Ala)), or combinations of glycine and serine (such as poly(Gly-Ser)).

In some embodiments, the linker comprises an amino acid sequence of ((G)nS)m, wherein m and n are independently an integer selected from 0 to 30, 1 to 29, 2 to 28, 3 to 27, 4 to 26, 5 to 25, 6 to 24, 7 to 23, 8 to 22, 9 to 21, 10 to 20, 11 to 19, 12 to 18, 13 to 17, 14 to 16 or 5. In some embodiments, m is 4 and n is 4.

In some embodiments, the linker comprises an amino acid sequence of SEQ ID NO: 68. In some embodiments, the linker comprises an amino acid sequence having at least 80%, at least 85%, at least 90%, at least 91%, at least 92%, at least 93%, at least 94%, at least 95%, at least 96%, at least 97%, at least 98%, at least 99% sequence identity to SEQ ID NO: 68.

### viii. Anti-PD-L1 Antibodies and Antigen-Binding Fragments Thereof

In some embodiments, the PD-L1-binding moiety of the bi-functional molecules provided herein comprises a moiety comprising an anti-PD-L1 antibody or antigen-binding fragments thereof. In some embodiments, the anti-PD-L1 antibody and antigen-binding fragments thereof are capable of specifically binding to PD-L1.

In some embodiments, the anti-PD-L1 antibodies and the antigen-binding fragments thereof provided herein specifically bind to human PD-L1 at an K_{D} value of no more than 0.8 nM, no more than 0.7 nM, no more than 0.6 nM, no more than 0.5 nM, or no more than 0.4 nM as measured by Biacore assay. Biacore assay is based on surface plasmon resonance technology, see, for example, Murphy, M. et al., Current protocols in protein science, Chapter 19, unit 19.14, 2006. In some embodiments, the K_{D} value is measured by the methods as described in Example 6 of the present disclosure.

Binding of the antibodies or the antigen-binding fragments thereof provided herein to human PD-L1 can also be represented by "half maximal effective concentration" (EC₅₀) value, which refers to the concentration of an antibody where 50% of its maximal binding is observed. The EC₅₀ value can be measured by binding assays known in the art, for example, direct or indirect binding assay such as enzyme-linked immunosorbent assay (ELISA), Fluorescence Activated Cell Sorting (FACS) assay, and other binding assay. In some embodiments, the antibodies and antigen-binding fragments thereof provided herein specifically bind to PD-L1 at an EC₅₀ (i.e. 50% binding concentration) of no more than 0.3 nM, no more than 0.2 nM, no more than 0.1 nM, or no more than 0.09 nM as measured by ELISA. In some embodiments, the antibodies and antigen-binding fragments thereof provided herein specifically bind to PD-L1 at an EC₅₀ (i.e. 50% binding concentration) of no more than 1.4 nM, no more than 1.3 nM, no more than 1.2 nM, no more than 1.1 nM, no more than 1.0 nM, no more than 0.3 nM, no more than 0.25 nM, or no more than 0.21 nM as measured by FACS assay.

In some embodiments, the anti-PD-L1 antibody or an antigen-binding fragment thereof provided herein specifically binds to PD-L1. In some embodiments, the anti-PD-L1 antibody or an antigen-binding fragment thereof provided herein does not bind to other members of B7 family.

In some embodiments, the anti-PD-L1 antibodies and antigen-binding fragments thereof provided herein are capable of blocking the interaction between the PD-L1 with its binding partner (e.g., PD-1 and B7-1) having an IC50 of no more than 2.2, 2.1, 2.0, 1.9, 1.8 or 1.2 ug/mL as measured by ELISA.

In some embodiments, the anti-PD-L1 antibodies and antigen-binding fragments thereof provided herein are capable of blocking the interaction between the PD-L1 with its binding partner (e.g., PD-1, ) having an EC50 of no more than 1.3, 1.2, 1.1, 1.0, 0.9, or 0.8 nM as measured by cell-based assay.

### ix. Illustrative Anti-PD-L1 Antibodies and Antigen-Binding Fragments Thereof

In some embodiments, the anti-PD-L1 antibodies (i.e., an antibody against PD-L1) and antigen-binding fragments thereof of the present disclosure comprise one or more (e.g. 1, 2, 3, 4, 5, or 6) CDRs comprising the sequences selected from the group consisting of **DYYMN** (SEQ ID NO: 1), **DINPNNX₁X₂TX₃YNHKFKG** (SEQ ID NO: 19), **WGDGPFAY** (SEQ ID NO: 3), **KASQNVX₄X₅X₆VA** (SEQ ID NO: 20), **SX₇SX₈RYT** (SEQ ID NO: 21), **QQYSNYPT** (SEQ ID NO: 6), wherein X₁ is G or A, X₂ is G or D or Q or E or L, X₃ is S or M or Q or L or V, X₄ is G or P or K, X₅ is A or G, X₆ is A or I, X₇ is A or N or R or V, X₈ is N or H or V or D.

In some embodiments, the heavy chain variable region comprises:
a) a HCDR1 comprising a sequence of SEQ ID NO: 1,
b) a HCDR2 comprising a sequence selected from group consisting of SEQ ID NO: 2, SEQ ID NO: 13, SEQ ID NO: 14, SEQ ID NO: 15, SEQ ID NO: 17, and SEQ ID NO: 18 and
c) a HCDR3 comprising a sequence of SEQ ID NO: 3,
and/or
a light chain variable region comprising:
d) a LCDR1 comprising a sequence selected from the group consisting of SEQ ID NO: 4, SEQ ID NO: 7, SEQ ID NO: 8 and SEQ ID NO: 9,
e) a LCDR2 comprising a sequence selected from the group consisting of SEQ ID NO: 5, SEQ ID NO: 10, SEQ ID NO: 11 and SEQ ID NO: 12, and
f) a LCDR3 comprising a sequence of SEQ ID NO: 6.

In some embodiments, the heavy chain variable region is selected from the group consisting of:
g) a heavy chain variable region comprising a HCDR1 comprising the sequence of SEQ ID NO: 1, a HCDR2 comprising the sequence of SEQ ID NO: 2, and a HCDR3 comprising the sequence of SEQ ID NO: 3;
h) a heavy chain variable region comprising a HCDR1 comprising the sequence of SEQ ID NO: 1, a HCDR2 comprising the sequence of SEQ ID NO: 13, and a HCDR3 comprising the sequence of SEQ ID NO: 3;
i) a heavy chain variable region comprising a HCDR1 comprising the sequence of SEQ ID NO: 1, a HCDR2 comprising the sequence of SEQ ID NO: 14, and a HCDR3 comprising the sequence of SEQ ID NO: 3;
j) a heavy chain variable region comprising a HCDR1 comprising the sequence of SEQ ID NO: 1, a HCDR2 comprising the sequence of SEQ ID NO: 15, and a HCDR3 comprising the sequence of SEQ ID NO: 3; and
k) a heavy chain variable region comprising a HCDR1 comprising the sequence of SEQ ID NO: 1, a HCDR2 comprising the sequence of SEQ ID NO: 17, and a HCDR3 comprising the sequence of SEQ ID NO: 3; and
l) a heavy chain variable region comprising a HCDR1 comprising the sequence of SEQ ID NO: 1, a HCDR2 comprising the sequence of SEQ ID NO: 18 and a HCDR3 comprising the sequence of SEQ ID NO: 3.

In some embodiments, the light chain variable region is selected from the group consisting of:
a) a light chain variable region comprising a LCDR1 comprising the sequence of SEQ ID NO: 4, a LCDR2 comprising the sequence of SEQ ID NO: 5, and a LCDR3 comprising the sequence of SEQ ID NO: 6;
b) a light chain variable region comprising a LCDR1 comprising the sequence of SEQ ID NO: 9, a LCDR2 comprising the sequence of SEQ ID NO: 5, and a LCDR3 comprising the sequence of SEQ ID NO: 6;
c) a light chain variable region comprising a LCDR1 comprising the sequence of SEQ ID NO: 8, a LCDR2 comprising the sequence of SEQ ID NO: 5, and a LCDR3 comprising the sequence of SEQ ID NO: 6;
d) a light chain variable region comprising a LCDR1 comprising the sequence of SEQ ID NO: 4, a LCDR2 comprising the sequence of SEQ ID NO: 12, and a LCDR3 comprising the sequence of SEQ ID NO: 6; and
e) a light chain variable region comprising a LCDR1 comprising the sequence of SEQ ID NO: 4, a LCDR2 comprising the sequence of SEQ ID NO: 11, and a LCDR3 comprising the sequence of SEQ ID NO: 6.

Antibody "4B6" as used herein refers to a monoclonal antibody comprising a heavy chain variable region having the sequence of SEQ ID NO: 46, and a light chain variable region having the sequence of SEQ ID NO: 47.

In some embodiments, the present disclosure provides anti-PD-L1 antibodies and antigen-binding fragments thereof comprising one or more (e.g. 1, 2, 3, 4, 5, or 6) CDR sequences of Antibody 4B6, or variants of Antibody 4B6. The CDR boundaries were defined or identified by the convention of Kabat.

In some embodiments, the present disclosure provides anti-PD-L1 antibodies and antigen-binding fragments thereof comprising HCDR1 comprising an amino acid sequence of SEQ ID NO: 1, HCDR2 comprising an amino acid sequence selected from the group consisting of SEQ ID NOs: 2, 13, 14, 15, 17, and 18, and HCDR3 comprising an amino acid sequence of SEQ ID NO: 3, and/or LCDR1 comprising an amino acid sequence selected from the group consisting of SEQ ID NOs: 4, 7, 8-9, LCDR2 comprising an amino acid sequence selected from the group consisting of SEQ ID NOs: 5, 10, 11-12, and LCDR3 comprising an amino acid sequence of SEQ ID NO: 6.

**Table 1. CDR amino acid sequences of the antibody 4B6.**

| **Antibody** | **Region** | **CDR1** | **CDR2** | **CDR3** |
|---|---|---|---|---|
| 4B6 | HCDR | **SEQ ID NO: 1** | **SEQ ID NO: 2** | **SEQ ID NO: 3** |
| | | DYYMN | DINPNNGGTSYNHKFKG | WGDGPFAY |
| | LCDR | **SEQ ID NO: 4** | **SEQ ID NO: 5** | **SEQ ID NO: 6** |
| | | KASQNVGAAVA | SASNRYT | QQYSNYPT |

**Table 2. Variable region amino acid sequences of the antibody 4B6.**

| **Antibody** | **VH** | **VL** |
|---|---|---|
| 4B6 | SEQ ID NO: 46 | SEQ ID NO: 47 |
| | EVQLQQSGPELVKPGASVKISCKAS GYVFTDYYMNWVKQSHGKSLEWI GDINPNNGGTSYNHKFKGKATVTV DKSSRTAYMELLSLTSEDSAVYYCV KWGDGPFAYWGQGTLVTVSA | DIVMTQSQKFMSTSVGDRVSI TCKASQNVGAAVAWYQQKPG QSPKLLIYSASNRYTGVPDRFT GSGSGTDFTLTISNMQSEDLA DYFCQQYSNYPTFGSGTKLGI K |

CDRs are known to be responsible for antigen binding. However, it has been found that not all of the 6 CDRs are indispensable or unchangeable. In other words, it is possible to replace or change or modify one or more CDRs in anti-PD-L1 antibody 4B6, yet substantially retain the specific binding affinity to PD-L1.

In some embodiments, the antibodies and antigen-binding fragments thereof provided herein comprise suitable framework region (FR) sequences, as long as the antibodies and antigen-binding fragments thereof can specifically bind to PD-L1. The CDR sequences provided in Table 1 above are obtained from mouse antibodies, but they can be grafted to any suitable FR sequences of any suitable species such as mouse, human, rat, rabbit, among others, using suitable methods known in the art such as recombinant techniques.

In some embodiments, the antibodies and antigen-binding fragments thereof provided herein are humanized. A humanized antibody or an antigen-binding fragment thereof is desirable in its reduced immunogenicity in human. A humanized antibody is chimeric in its variable regions, as non-human CDR sequences are grafted to human or substantially human FR sequences. Humanization of an antibody or antigen-binding fragment can be essentially performed by substituting the non-human (such as murine) CDR genes for the corresponding human CDR genes in a human immunoglobulin gene (see, for example, Jones et al. (1986) Nature 321:522-525; Riechmann et al. (1988) Nature 332:323-327; Verhoeyen et al. (1988) Science 239:1534-1536).

Suitable human heavy chain and light chain variable domains can be selected to achieve this purpose using methods known in the art. In an illustrative example, "best-fit" approach can be used, where a non-human (e.g. rodent) antibody variable domain sequence is screened or BLASTed against a database of known human variable domain sequences, and the human sequence closest to the non-human query sequence is identified and used as the human scaffold for grafting the non-human CDR sequences (see, for example, Sims et al., (1993) J. Immunol. 151:2296; Chothia et al. (1987) J. Mot. Biol. 196:901). Alternatively, a framework derived from the consensus sequence of all human antibodies may be used for the grafting of the non-human CDRs (see, for example, Carter et al. (1992) Proc. Natl. Acad. Sci. USA, 89:4285; Presta et al. (1993) J. Immunol.,151:2623).

In some embodiments, the present disclosure provides 12 humanized antibodies of 4B6, which are designated as Hu4B6_Hg.2La.1, Hu4B6_Hg.2La.2, Hu4B6_Hg.2La.4, Hu4B6_Hg.2La.6, Hu4B6_Hg.3La.1, Hu4B6_Hg.3La.2, Hu4B6_Hg.3La.4, Hu4B6_Hg.3La.6, Hu4B6_Hg.5La.1, Hu4B6_Hg.5La.2, Hu4B6_Hg.5La.4 and Hu4B6_Hg.5La.6, respectively. The SEQ ID NOs of the heavy and light chain variable regions of each humanized antibody of 4B6 are shown in Table 5. CDRs of each of the 12 humanized antibodies of 4B6 are shown in Table 5 (underlined sequences). The CDR boundaries were defined or identified by the convention of Kabat.

Table 3a below shows the amino acid sequences of the variant CDR for humanized 4B6, Table 3b below shows the FR for the humanized 4B6 heavy chain and light chain variable regions. Table 4 below shows the FR amino acid sequences for each heavy and light chains of 12 humanized antibodies for chimeric antibody 4B6, which are designated as Hu4B6_Hg.2La.1, Hu4B6_Hg.2La.2, Hu4B6_Hg.2La.4, Hu4B6_Hg.2La.6, Hu4B6_Hg.3La.1, Hu4B6_Hg.3La.2, Hu4B6_Hg.3La.4, Hu4B6_Hg.3La.6, Hu4B6_Hg.5La.1, Hu4B6_Hg.5La.2, Hu4B6_Hg.5La.4 and Hu4B6_Hg.5La.6, respectively. The heavy chain variable regions and light chain variable regions of these 12 humanized antibodies are shown in Table 5.

**Table 3a. Amino acid sequences of the CDR variants for humanized antibody of 4B6.**

| **SEQ ID NO.** | **Sequence** | **Annotation** |
|---|---|---|
| **7** | KASQNVGAIVA | 4B6-L-CDR1-1 |
| **8** | KASQNVPAAVA | 4B6-L-CDR1-2 |
| **9** | KASQNVKGAVA | 4B6-L-CDR1-3 |
| **10** | SNSHRYT | 4B6-L-CDR2-1 |
| **11** | SRSVRYT | 4B6-L-CDR2-2 |
| **12** | SVSDRYT | 4B6-L-CDR2-3 |
| **13** | DINPNNADTMYNHKFKG | 4B6-H-CDR2-1 |
| **14** | DINPNNAQTQYNHKFKG | 4B6-H-CDR2-2 |
| **15** | DINPNNAETLYNHKFKG | 4B6-H-CDR2-3 |
| **16** | DINPNNGLTSYNHKFKG | 4B6-H-CDR2-4 |
| **17** | DINPNNAQTVYNHKFKG | 4B6-H-CDR2-5 |
| **18** | DINPNNAGTSYNHKFKG | H-CDR2-WT (G55A) |

**Table 3b. Amino acid sequences of the FR sequences for 4B6 and humanized antibody of 4B6.**

| **SEQ ID NO.** | **Sequence** | **Annotation** |
|---|---|---|
| **22** | QVQLVQSGAEVKKPGASVKVSCKASGYTFT | HFR1 of human germline sequence |
| **23** | WVRQAPGQGLEWMG | HFR2 of human germline sequence |
| **24** | RVTMTRDTSISTAYMELSRLRSDDTAVYYCAR | HFR3 of human germline sequence |
| **25** | WGQGTLVTVSS | HFR4 of human germline sequence |
| **26** | DIQMTQSPSSLSASVGDRVTITC | LFR1 of human germline sequence |
| **27** | WYQQKPGKAPKLLIY | LFR2 of human germline sequence |
| **28** | GVPSRFSGSGSGTDFTFTISSLQPEDIATYYC | LFR3 of human germline sequence |
| **29** | FGQGTKLEIK | LFR4 of human germline sequence |
| **30** | QVQLVQSGAEVKKPGASVKVSCKASGYVFT | HFR1 variant |
| **31** | WVRQAPGQSLEWMG | HFR2 variant |
| **32** | RVTVTVDTSISTAYMELSRLRSDDTAVYYCAR | HFR3 variant 1 |
| **33** | RVTVTVDTSISTAYMELSRLRSDDTAVYYCVK | HFR3 variant 2 |
| **34** | RVTVTVDKSISTAYMELSRLRSDDTAVYYCAR | HFR3 variant 3 |
| **35** | RVTVTVDKSISTAYMELSRLRSDDTAVYYCVK | HFR3 variant 4 |
| **36** | WYQQKPGKSPKLLIY | LFR2 variant |
| **37** | GVPSRFSGSGSGTDFTLTISSLQPEDIATYYC | LFR3 variant 1 |
| **38** | GVPDRFSGSGSGTDFTLTISSLQPEDIATYYC | LFR3 variant 2 |
| **39** | GVPSRFSGSGSGTDFTLTISSLQPEDIATYYC | F73L mutation for variant 1-FR3 |
| **45** | GVPDRFSGSGSGTDFTLTISSLQPEDIATYYC | F73L, A43S, S60D for LC variant 2-FR3 |

**Table 4. The FR amino acid sequences for each humanized heavy and light chain variable regions for humanized antibody of 4B6.**

| **VH or VL Name** | **FR1 (SEQ ID NO.)** | **FR2 (SEQ ID NO.)** | **FR3 (SEQ ID NO.)** | **FR4 (SEQ ID NO.)** |
|---|---|---|---|---|
| **Hu4B6_Hg.2** | 30 | 31 | 35 | 25 |
| **AM4B6 Hg.3** | 30 | 31 | 35 | 25 |
| **AM4B6 Hg.5** | 30 | 31 | 35 | 25 |
| **AM4B6 La.1** | 26 | 27 | 37 | 29 |
| **AM4B6_La.2** | 26 | 27 | 37 | 29 |
| **AM4B6_La.4** | 26 | 27 | 37 | 29 |
| **AM4B6 La.6** | 30 | 31 | 35 | 25 |

Table 5 below shows the 3 variants of humanized 4B6 heavy chain variable regions (i.e. Hu4B6_Hg.2, Hu4B6_Hg.3 and Hu4B6_Hg.5) and 4 variants of humanized 4B6 light chain variable regions (i.e. AM4B6_La.1, AM4B6_La.2, AM4B6_La.4, AM4B6_La.6).

**Table 5. Amino acid sequences of the variable regions for humanized antibody of 4B6.**

| **Antibody** | **VH** | **VL** |
|---|---|---|
| **AM4B6_ Hg.2La.1** | **Hu4B6_Hg.2, SEQ ID NO: 58** | **AM4B6_La.1, SEQ ID NO: 62** |
| | QVQLVQSGAEVKKPGASVKVSC KASGYVFTDYYMNWVRQAPGQ SLEWMGDINPNNAQTQYNHKFK GRVTVTVDKSISTAYMELSRLRS DDTAVYYCVKWGDGPFAYWGQ GTLVTVSS | DIQMTQSPSSLSASVGDRVTITCKA SQNVKGAVAWYQQKPGKAPKLLI YSASNRYTGVPSRFSGSGSGTDFT LTISSLQPEDIATYYCQQYSNYPTF GQGTKLEIK |
| **AM4B6_ Hg.2La.2** | **Hu4B6_Hg.2, SEQ ID NO: 58** | **AM4B6_La.2, SEQ ID NO: 63** |
| | [?] QVQLVQSGAEVKKPGASVKVSC KASGYVFTDYYMNWVRQAPGQ SLEWMGDINPNNAQTQYNHKFK GRVTVTVDKSISTAYMELSRLRS DDTAVYYCVKWGDGPFAYWGQ GTLVTVSS | DIQMTQSPSSLSASVGDRVTITCKA SQNVPAAVAWYQQKPGKAPKLLI YSASNRYTGVPSRFSGSGSGTDFT LTISSLQPEDIATYYCQQYSNYPTF GQGTKLEIK |
| **AM4B6_ Hg.2La.4** | **Hu4B6Hg.2, SEQ ID NO: 58** | **AM4B6_La.4, SEQ ID NO: 64** |
| | QVQLVQSGAEVKKPGASVKVSC KASGYVFTDYYMNWVRQAPGQ SLEWMGDINPNNAQTQYNHKFK GRVTVTVDKSISTAYMELSRLRS DDTAVYYCVKWGDGPFAYWGQ GTLVTVSS | DIQMTQSPSSLSASVGDRVTITCKA SQNVGAAVAWYQQKPGKAPKLLI YSVSDRYTGVPSRFSGSGSGTDFT LTISSLQPEDIATYYCQQYSNYPTF GQGTKLEIK |
| **AM4B6_ Hg.2La.6** | **Hu4B6_Hg.2, SEQ ID NO: 58** | **AM4B6_La.6, SEQ ID NO: 65** |
| | QVQLVQSGAEVKKPGASVKVSC KASGYVFTDYYMNWVRQAPGQ SLEWMGDINPNNAQTQYNHKFK GRVTVTVDKSISTAYMELSRLRS DDTAVYYCVKWGDGPFAYWGQ GTLVTVSS | DIQMTQSPSSLSASVGDRVTITCKA SQNVGAAVAWYQQKPGKAPKLLI YSRSVRYTGVPSRFSGSGSGTDFT LTISSLQPEDIATYYCQQYSNYPTF GQGTKLEIK |
| **AM4B6_ Hg.3La.1** | **AM4B6_Hg.3, SEQ ID NO: 59** | **AM4B6_La.1, SEQ ID NO: 62** |
| | QVQLVQSGAEVKKPGASVKVSC KASGYVFTDYYMNWVRQAPGQ SLEWMGDINPNNAETLYNHKFK GRVTVTVDKSISTAYMELSRLRS DDTAVYYCVKWGDGPFAYWGQ GTLVTVSS | DIQMTQSPSSLSASVGDRVTITCKA SQNVKGAVAWYQQKPGKAPKLLI YSASNRYTGVPSRFSGSGSGTDFT LTISSLQPEDIATYYCQQYSNYPTF GQGTKLEIK |
| **AM4B6_ Hg.3La.2** | **AM4B6_Hg.3, SEQ ID NO: 59** | **AM4B6_La.2, SEQ ID NO: 63** |
| | QVQLVQSGAEVKKPGASVKVSC KASGYVFTDYYMNWVRQAPGQ SLEWMGDINPNNAETLYNHKFK GRVTVTVDKSISTAYMELSRLRS DDTAVYYCVKWGDGPFAYWGQ GTLVTVSS | DIQMTQSPSSLSASVGDRVTITCKA SQNVPAAVAWYQQKPGKAPKLLI YSASNRYTGVPSRFSGSGSGTDFT LTISSLQPEDIATYYCQQYSNYPTF GQGTKLEIK |
| **AM4B6_ Hg.3La.4** | **AM4B6_Hg.3, SEQ ID NO: 59** | **AM4B6_La.4, SEQ ID NO: 64** |
| | QVQLVQSGAEVKKPGASVKVSC KASGYVFTDYYMNWVRQAPGQ SLEWMGDINPNNAETLYNHKFK GRVTVTVDKSISTAYMELSRLRS DDTAVYYCVKWGDGPFAYWGQ GTLVTVSS | DIQMTQSPSSLSASVGDRVTITCKA SQNVGAAVAWYQQKPGKAPKLLI YSVSDRYTGVPSRFSGSGSGTDFT LTISSLQPEDIATYYCQQYSNYPTF GQGTKLEIK |
| **AM4B6_ Hg.3La.6** | **AM4B6_Hg.3, SEQ ID NO: 59** | **AM4B6_La.6, SEQ ID NO: 65** |
| | QVQLVQSGAEVKKPGASVKVSC KASGYVFTDYYMNWVRQAPGQ SLEWMGDINPNNAETLYNHKFK GRVTVTVDKSISTAYMELSRLRS DDTAVYYCVKWGDGPFAYWGQ GTLVTVSS | QVQLVQSGAEVKKPGASVKVSCK ASGYVFTDYYMNWVRQAPGQSLE WMGDINPNNAGTSYNHKFKGRVT VTVDKSISTAYMELSRLRSDDTAV YYCVKWGDGPFAYWGQGTLVTVS S |
| **AM4B6_ Hg.5La.1** | **AM4B6_Hg.5, SEQ ID NO: 60** | **AM4B6_La.1, SEQ ID NO: 62** |
| | QVQLVQSGAEVKKPGASVKVSC KASGYVFTDYYMNWVRQAPGQ SLEWMGDINPNNAQTVYNHKFK GRVTVTVDKSISTAYMELSRLRS DDTAVYYCVKWGDGPFAYWGQ GTLVTVSS | DIQMTQSPSSLSASVGDRVTITCKA SQNVKGAVAWYQQKPGKAPKLLI YSASNRYTGVPSRFSGSGSGTDFT LTISSLQPEDIATYYCQQYSNYPTF GQGTKLEIK |
| **AM4B6_ Hg.5La.2** | **AM4B6_Hg.5, SEQ ID NO: 60** | **AM4B6_La.2, SEQ ID NO: 63** |
| | QVQLVQSGAEVKKPGASVKVSC KASGYVFTDYYMNWVRQAPGQ SLEWMGDINPNNAQTVYNHKFK GRVTVTVDKSISTAYMELSRLRS DDTAVYYCVKWGDGPFAYWGQ GTLVTVSS | DIQMTQSPSSLSASVGDRVTITCKA SQNVPAAVAWYQQKPGKAPKLLI YSASNRYTGVPSRFSGSGSGTDFT LTISSLQPEDIATYYCQQYSNYPTF GQGTKLEIK |
| **AM4B6_ Hg.5La.4** | **AM4B6_Hg.5, SEQ ID NO: 60** | **AM4B6_La.4, SEQ ID NO: 64** |
| | QVQLVQSGAEVKKPGASVKVSC KASGYVFTDYYMNWVRQAPGQ SLEWMGDINPNNAQTVYNHKFK GRVTVTVDKSISTAYMELSRLRS DDTAVYYCVKWGDGPFAYWGQ GTLVTVSS | DIQMTQSPSSLSASVGDRVTITCKA SQNVGAAVAWYQQKPGKAPKLLI YSVSDRYTGVPSRFSGSGSGTDFT LTISSLQPEDIATYYCQQYSNYPTF GQGTKLEIK |
| **AM4B6_ Hg.5La.6** | **AM4B6_Hg.5, SEQ ID NO: 60** | **AM4B6_La.6, SEQ ID NO: 65** |
| | QVQLVQSGAEVKKPGASVKVSC KASGYVFTDYYMNWVRQAPGQ SLEWMGDINPNNAQTVYNHKFK GRVTVTVDKSISTAYMELSRLRS DDTAVYYCVKWGDGPFAYWGQ GTLVTVSS | QVQLVQSGAEVKKPGASVKVSCK ASGYVFTDYYMNWVRQAPGQSLE WMGDINPNNAGTSYNHKFKGRVT VTVDKSISTAYMELSRLRSDDTAV YYCVKWGDGPFAYWGQGTLVTVS S |

In some embodiments, the humanized anti-PD-L1 antibodies or antigen-binding fragments thereof provided herein are composed of substantially all human sequences except for the CDR sequences which are non-human. In some embodiments, the variable region FRs, and constant regions if present, are entirely or substantially from human immunoglobulin sequences. The human FR sequences and human constant region sequences may be derived from different human immunoglobulin genes, for example, FR sequences derived from one human antibody and constant region from another human antibody. In some embodiments, the humanized antibody or an antigen-binding fragment thereof comprises human heavy chain HFR1-4, and/or a light chain LFR1-4.

In some embodiments, the FR regions derived from human may comprise the same amino acid sequence as the human immunoglobulin from which it is derived. In some embodiments, one or more amino acid residues of the human FR are substituted with the corresponding residues from the parent non-human antibody. This may be desirable in some embodiments to make the humanized antibody or its fragment closely approximate the non-human parent antibody structure, so as to optimize binding characteristics (for example, increase binding affinity). In some embodiments, the humanized antibody or an antigen-binding fragment thereof provided herein comprises no more than 10, 9, 8, 7, 6, 5, 4, 3, 2, or 1 amino acid residue substitutions in each of the human FR sequences, or no more than 10, 9, 8, 7, 6, 5, 4, 3, 2, or 1 amino acid residue substitutions in all the FR sequences of a heavy or a light chain variable domain. In some embodiments, such change in amino acid residue could be present in heavy chain FR regions only, in light chain FR regions only, or in both chains. In some embodiments, one or more amino acids of the human FR sequences are randomly mutated to increase binding affinity. In some embodiments, one or more amino acids of the human FR sequences are back mutated to the corresponding amino acid(s) of the parent non-human antibody so as to increase binding affinity.

In some embodiments, the humanized anti-PD-L1 antibodies and antigen-binding fragments thereof of the present disclosure comprise a heavy chain HFR1 comprising the sequence of **QVQLVQSGAEVKKPGASVKVSCKASGYX₉FT** (SEQ ID NO: 40) or a homologous sequence of at least 80% sequence identity thereof, a heavy chain HFR2 comprising the sequence of **WVRQAPGQX₁₀LEWMG** (SEQ ID NO: 41) or a homologous sequence of at least 80% sequence identity thereof, a heavy chain HFR3 comprising the sequence of **RVTX₁₆TVDX₁₁SISTAYMELSRLRSDDTAVYYCX₁₂X₁₃** (SEQ ID NO: 42) or a homologous sequence of at least 80% sequence identity thereof, and a heavy chain HFR4 comprising the sequence of **WGQGTLVTVSS** (SEQ ID NO: 25) or a homologous sequence of at least 80% sequence identity thereof, wherein X₉ is T or V, X₁₀ is G or S, X₁₁ is T or K, X₁₂ is A or V, and X₁₃ is R or K.

In some embodiments, the humanized anti-PD-L1 antibodies and antigen-binding fragments thereof of the present disclosure comprise a light chain LFR1 comprising the sequence of **DIQMTQSPSSLSASVGDRVTITC** (SEQ ID NO: 26) or a homologous sequence of at least 80% sequence identity thereof, a light chain LFR2 comprising the sequence of **WYQQKPGKX₁₄PKLLIY** (SEQ ID NO: 43) or a homologous sequence of at least 80% sequence identity thereof, a light chain LFR3 comprising the sequence of **GVPX₁₅RFSGSGSGTDFTX₁₇TISSLQPEDIATYYC** (SEQ ID NO: 44) or a homologous sequence of at least 80% sequence identity thereof, and a light chain LFR4 comprising the sequence of **FGQGTKLEIK** (SEQ ID NO: 29) or a homologous sequence of at least 80% sequence identity thereof, wherein X₁₄ is A or S, X₁₅ is S or D, X₁₆ is M or V, and X₁₇ is F or L.

In some embodiments, the HFR1 comprises a sequence selected from the group consisting of SEQ ID NOs: 22 and 30, the HFR2 comprises a sequence selected from the group consisting of SEQ ID NOs: 23 and 31, the HFR3 comprises the sequence selected from the group consisting of SEQ ID NOs: 24 and 32-35, the HFR4 comprises a sequence of SEQ ID NOs: 25, the LFR1 comprises the sequence from the group consisting of SEQ ID NO: 26, the LFR2 comprises a sequence selected from the group consisting of SEQ ID NOs: 27 and 36, the LFR3 comprises a sequence selected from the group consisting of SEQ ID NOs: 28, and 37-38, 39, 45, and the LFR4 comprises a sequence of SEQ ID NO: 29.

In some embodiments, the humanized anti-PD-L1 antibodies and antigen-binding fragments thereof of the present disclosure comprise HFR1, HFR2, HFR3, and/or HFR4 sequences contained in a heavy chain variable region selected from a group consisting of: Hu4B6_Hg.2 (SEQ ID NO: 58), AM4B6_Hg.3 (SEQ ID NO: 59), AM4B6_Hg.5 (SEQ ID NO: 60).

In some embodiments, the humanized anti-PD-L1 antibodies and antigen-binding fragments thereof of the present disclosure comprise LFR1, LFR2, LFR3, and/or LFR4 sequences contained in a light chain variable region selected from a group consisting of: AM4B6_La.1 (SEQ ID NO: 62), AM4B6_La.2 (SEQ ID NO: 63), AM4B6_La.4 (SEQ ID NO: 64), and AM4B6_La.6 (SEQ ID NO: 65).

In some embodiments, the heavy chain variable region comprises a sequence selected from the group consisting of SEQ ID NO: 46, SEQ ID NO: 49, SEQ ID NO: 50, SEQ ID NO: 51, SEQ ID NO: 52, SEQ ID NO: 56, SEQ ID NO: 57, SEQ ID NO: 58, SEQ ID NO: 59, SEQ ID NO: 60, and a homologous sequence thereof having at least 80% sequence identity thereof. In some embodiments, the light chain variable region comprises a sequence selected from the group consisting of SEQ ID NO: 47, SEQ ID NO: 54, SEQ ID NO: 55, SEQ ID NO: 61, SEQ ID NO: 62, SEQ ID NO: 63, SEQ ID NO: 64, SEQ ID NO: 65, and a homologous sequence thereof having at least 80% sequence identity thereof. In some embodiments, the antibody against PD-L1 or an antigen-binding fragment thereof comprises a pair of heavy chain variable region and light chain variable region sequences selected from the group consisting of: SEQ ID NOs: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64, and 60/65.

These exemplary humanized anti-PD-L1 antibodies retained the specific binding capacity or affinity to PD-L1, and are better than, the parent mouse antibody 4B6 in that aspect.

In some embodiments, the anti-PD-L1 antibodies and antigen-binding fragments provided herein comprise all or a portion of the heavy chain variable domain and/or all or a portion of the light chain variable domain. In one embodiment, the anti-PD-L1 antibody or an antigen-binding fragment thereof provided herein is a single domain antibody which consists of all or a portion of the heavy chain variable domain provided herein. More information of such a single domain antibody is available in the art (see, e.g. U.S. Pat. No. 6,248,516).

In some embodiments, the anti-PD-L1 antibodies or the antigen-binding fragments thereof provided herein further comprise an immunoglobulin (Ig) constant region, which optionally further comprises a heavy chain and/or a light chain constant region. In some embodiments, the heavy chain constant region comprises CH1, hinge, and/or CH2-CH3 regions (or optionally CH2-CH3-CH4 regions). In some embodiments, the anti-PD-L1 antibodies or the antigen-binding fragments thereof provided herein comprises a heavy chain constant regions of human IgG1, IgG2, IgG3, IgG4, IgA1, IgA2 or IgM. In some embodiments, the light chain constant region comprises Cκ or Cλ. The constant region of the anti-PD-L1 antibodies or the antigen-binding fragments thereof provided herein may be identical to the wild-type constant region sequence or be different in one or more mutations.

In some embodiments, the anti-PD-L1 antibodies or the antigen-binding fragments thereof provided herein have a specific binding affinity to human PD-L1 which is sufficient to provide for diagnostic and/or therapeutic use.

The anti-PD-L1 antibodies or antigen-binding fragments thereof provided herein can be a monoclonal antibody, a polyclonal antibody, a humanized antibody, a chimeric antibody, a recombinant antibody, a bispecific antibody, a multi-specific antibody, a labeled antibody, a bivalent antibody, an anti-idiotypic antibody, or a fusion protein. A recombinant antibody is an antibody prepared in vitro using recombinant methods rather than in animals.

In some embodiments, the PD-L1 binding moiety comprises an anti-PD-L1 antibody or an antigen-binding fragment thereof, which competes for binding to PD-L1 with the antibody or an antigen-binding fragment thereof comprising a pair of heavy chain variable region and light chain variable region sequences selected from the group consisting of: SEQ ID NOs: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64, and 60/65.

### x. Antibody Variants

The anti-PD-L1 antibodies and antigen-binding fragments thereof provided herein also encompass various variants of the antibody sequences provided herein.

In some embodiments, the antibody variants comprise one or more modifications or substitutions in one or more of the CDR sequences provided in Table 1 above, one or more of the non-CDR sequences of the heavy chain variable region or light chain variable region provided in Tables 3a, 3b and 5 above, and/or the constant region (e.g. Fc region). Such variants retain binding specificity to PD-L1 of their parent antibodies, but have one or more desirable properties conferred by the modification(s) or substitution(s). For example, the antibody variants may have improved antigen-binding affinity, improved glycosylation pattern, reduced risk of glycosylation, reduced deamination, reduced or depleted effector function(s), improved FcRn receptor binding, increased pharmacokinetic half-life, pH sensitivity, and/or compatibility to conjugation (e.g. one or more introduced cysteine residues).

The parent antibody sequence may be screened to identify suitable or preferred residues to be modified or substituted, using methods known in the art, for example, "alanine scanning mutagenesis" (see, for example, Cunningham and Wells (1989) Science, 244:1081-1085). Briefly, target residues (e.g. charged residues such as Arg, Asp, His, Lys, and Glu) can be identified and replaced by a neutral or negatively charged amino acid (e.g. alanine or polyalanine), and the modified antibodies are produced and screened for the interested property. If substitution at a particular amino acid location demonstrates an interested functional change, then the position can be identified as a potential residue for modification or substitution. The potential residues may be further assessed by substituting with a different type of residue (e.g. cysteine residue, positively charged residue, etc.).

### xi. Affinity Variants

Affinity variants of antibodies may contain modifications or substitutions in one or more CDR sequences provided in Table 1 above, one or more FR sequences provided in Tables 3b and 4 above, or the heavy or light chain variable region sequences provided in Tables 5 above. FR sequences can be readily identified by a person skilled in the art based on the CDR sequences in Table 1 above and variable region sequences in Table 5 above, as it is well-known in the art that a CDR region is flanked by two FR regions in the variable region. The affinity variants retain specific binding affinity to PD-L1 of the parent antibody, or even have improved PD-L1 specific binding affinity over the parent antibody. In some embodiments, at least one (or all) of the substitution(s) in the CDR sequences, FR sequences, or variable region sequences comprises a conservative substitution.

A person skilled in the art will understand that in the CDR sequences provided in Table 1 and 3a above, and variable region sequences provided in Table 5 above, one or more amino acid residues may be substituted yet the resulting antibody or antigen-binding fragment still retain the binding affinity or binding capacity to PD-L1, or even have an improved binding affinity or capacity. Various methods known in the art can be used to achieve this purpose. For example, a library of antibody variants (such as Fab or scFv variants) can be generated and expressed with phage display technology, and then screened for the binding affinity to PD-L1. For another example, computer software can be used to virtually simulate the binding of the antibodies to PD-L1, and identify the amino acid residues on the antibodies which form the binding interface. Such residues may be either avoided in the substitution so as to prevent reduction in binding affinity, or targeted for substitution to provide for a stronger binding.

In some embodiments, the humanized anti-PD-L1 antibody or an antigen-binding fragment thereof provided herein comprises one or more amino acid residue substitutions in one or more of the CDR sequences, and/or one or more of the FR sequences. In some embodiments, an affinity variant comprises no more than 20, 15, 10, 9, 8, 7, 6, 5, 4, 3, 2, or 1 substitution in the CDR sequences and/or FR sequences in total.

In some embodiments, the anti-PD-L1 antibodies or antigen-binding fragments thereof comprise 1, 2, or 3 CDR sequences having at least 80% (e.g. at least 85%, 88%, 90%, 91%, 92%, 93%, 94%, 95%, 96%, 97%, 98%, 99%) sequence identity to that (or those) listed in Tables 1 and 3a above yet retaining the specific binding affinity to PD-L1 at a level similar to or even higher than its parent antibody.

In some embodiments, the anti-PD-L1 antibodies or antigen-binding fragments thereof comprise one or more variable region sequences having at least 80% (e.g. at least 85%, 88%, 90%, 91%, 92%, 93%, 94%, 95%, 96%, 97%, 98%, 99%) sequence identity to that (or those) listed in Table 5 above yet retaining the specific binding affinity to PD-L1 at a level similar to or even higher than its parent antibody. In some embodiments, a total of 1 to 10 amino acids have been substituted, inserted, or deleted in a variable region sequence listed in Table 5 above. In some embodiments, the substitutions, insertions, or deletions occur in regions outside the CDRs (e.g. in the FRs).

### xii. Glycosylation Variants

The anti-PD-L1 antibodies or antigen-binding fragments thereof provided herein also encompass glycosylation variants, which can be obtained to either increase or decrease the extent of glycosylation of the antibodies or antigen binding fragments thereof.

The anti-PD-L1 antibodies or antigen binding fragments thereof may comprise one or more modifications that introduce or remove a glycosylation site. A glycosylation site is an amino acid residue with a side chain to which a carbohydrate moiety (e.g. an oligosaccharide structure) can be attached. Glycosylation of antibodies is typically either N-linked or O-linked. N-linked refers to the attachment of the carbohydrate moiety to the side chain of an asparagine residue, for example, an asparagine residue in a tripeptide sequence such as asparagine-X-serine and asparagine-X-threonine, where X is any amino acid except proline. O-linked glycosylation refers to the attachment of one of the sugars N-aceylgalactosamine, galactose, or xylose to a hydroxyamino acid, most commonly to serine or threonine. Removal of a native glycosylation site can be conveniently accomplished, for example, by altering the amino acid sequence such that one of the above-described tripeptide sequences (for N-linked glycosylation sites) or serine or threonine residues (for O-linked glycosylation sites) present in the sequence in the is substituted. A new glycosylation site can be created in a similar way by introducing such a tripeptide sequence or serine or threonine residue.

In some embodiments, the anti-PD-L1 antibodies and antigen-binding fragments provided herein comprise one or more mutationsto remove one or more deamidation site. In some embodiments, the anti-PD-L1 antibodies and antigen-binding fragments provided herein comprise a mutation at G55 (for example, G55A) in the heavy chain. These mutations are tested and are believed not to negatively affect the binding affinity of the antibodies provided herein.

### xiii. Cysteine-engineered Variants

The anti-PD-L1 antibodies or antigen-binding fragments thereof provided herein also encompass cysteine-engineered variants, which comprise one or more introduced free cysteine amino acid residues.

A free cysteine residue is one which is not part of a disulfide bridge. A cysteine-engineered variant is useful for conjugation with for example, a cytotoxic and/or imaging compound, a label, or a radioisoptype among others, at the site of the engineered cysteine, through for example a maleimide or haloacetyl. Methods for engineering antibodies or antigen-binding fragments thereof to introduce free cysteine residues are known in the art, see, for example, WO2006/034488.

### xiv. Fc Variants

The anti-PD-L1 antibodies or antigen-binding fragments thereof provided herein also encompass Fc variants, which comprise one or more amino acid residue modifications or substitutions at the Fc region and/or hinge region, for example, to provide for altered effector functions such as ADCC and CDC. Methods of altering ADCC activity by antibody engineering have been described in the art, see for example, Shields RL. et al., J Biol Chem. 2001. 276(9): 6591-604; Idusogie EE. et al., J Immunol. 2000.164(8):4178-84; Steurer W. et al., J Immunol. 1995, 155(3): 1165- 74; Idusogie EE. et al., J Immunol. 2001, 166(4): 2571-5; Lazar GA. et al., PNAS, 2006, 103(11): 4005-4010; Ryan MC. et al., Mol. Cancer Ther., 2007, 6: 3009-3018; Richards JO,. et al., Mol Cancer Ther. 2008, 7(8): 2517-27; Shields R. L. et al., J. Biol. Chem, 2002, 277: 26733-26740; Shinkawa T. et al., J. Biol. Chem, 2003, 278: 3466-3473.

CDC activity of the antibodies or antigen-binding fragments provided herein can also be altered, for example, by improving or diminishing C1q binding and/or CDC (see, for example, WO99/51642; Duncan & Winter Nature 322:738-40 (1988); U.S. Pat. No. 5,648,260; U.S. Pat. No. 5,624,821); and WO94/29351 concerning other examples of Fc region variants. One or more amino acids selected from amino acid residues 329, 331 and 322 of the Fc region can be replaced with a different amino acid residue to alter C1q binding and/or reduced or abolished complement dependent cytotoxicity (CDC) (see, U.S. Pat. No. 6,194,551 by Idusogie et al.). One or more amino acid substitution(s) can also be introduced to alter the ability of the antibody to fix complement (see PCT Publication WO 94/29351 by Bodmer et al.).

In some embodiments, the Fc variants provided herein has reduced effector functions relative to a wildtype Fc (e.g. Fc of IgG1), and comprise one or more amino acid substitution(s) at a position selected from the group consisting of: 220, 226, 228, 229, 233, 234, 235, 236, 237, 238, 267, 268, 269, 270, 297, 309, 318, 320, 322, 325, 328, 329, 330, 331 and 332 of the Fc region (see, WO2016/196228; Richards et al. (2008) Mol. Cancer Therap. 7:2517; Moore et al. ( 2010) mAbs 2:181; and Strohl (2009) Current Opinion in Biotechnology 20:685-691), wherein the numbering of the residues in the Fc region is that of the EU index as in Kabat. Exemplary substitutions for reduced effector functions include, without limitation, 220S, 226S, 228P, 229S, 233P, 234V, 234G, 234A, 234F, 234A, 235A, 235G, 235E, 236E, 236R, 237A, 237K, 238S, 267R, 268A, 268Q, 269R, 297A, 297Q, 297G, 309L, 318A, 322A, 325L, 328R, 330S, 331S, or any combination thereof (see, WO2016/196228; and Strohl (2009) Current Opinion in Biotechnology 20:685-691).

In some embodiments, the anti-PD-L1 antibodies or antigen-binding fragments thereof provided herein has reduced effector functions, and comprise one or more amino acid substitution(s) in IgG1 at a position selected from the group consisting of: 234, 235, 237, 238, 268, 297, 309, 330, and 331. In some embodiments, the anti-PD-L1 antibodies or antigen-binding fragments thereof provided herein is of IgG1 isotype and comprise one or more amino acid substitution(s) selected from the group consisting of: N297A, N297Q, N297G, L235E, L234A, L235A, L234F, L235E, P331S, and any combination thereof.

In some embodiments, the anti-PD-L1 antibodies or antigen-binding fragments thereof provided herein is of IgG1 isotype and comprise a L234A and L235A mutation. In some embodiments, the anti-PD-L1 antibodies or antigen-binding fragments thereof provided herein is of IgG1 isotype and comprise L234F, L235E, and P331S. The L234F, L235E, and P331S set of substitutions (also referred as FES triple mutation) located in the CH2 region of the Fc domain can abrogate FCγR and C1q binding resulting in an antibody unable to elicit ADCC or CDC (Oganesyan et al., Acta Crystallogr. D 64:700-704 (2008)). PCT/US2013/36872 has shown that combining these mutations in a variant Fc domain, e.g., a variant Fc domain in an antibody result in an Fc domain having reduced thermal stability compared to a wild type parent molecule, e.g., a wild type IgG1 Fc. In some embodiments, the Fc variant comprises an amino acid sequence of SEQ ID NO: 81.

In some embodiments, the anti-PD-L1 antibodies or antigen-binding fragments thereof provided herein is of IgG2 isotype, and comprises one or more amino acid substitution(s) selected from the group consisting of: H268Q, V309L, A330S, P331S, V234A, G237A, P238S, H268A, and any combination thereof (e.g. H268Q/V309L/A330S/P331S, V234A/G237A/P238S/H268A/V309L/A330S/ P331S). In some embodiments, the anti-PD-L1 antibodies or antigen-binding fragments thereof provided herein is of IgG4 isotype, and comprises one or more amino acid substitution(s) selected from the group consisting of: S228P, N297A, N297Q, N297G, L235E, F234A, L235A, and any combination thereof. In some embodiments, the anti-PD-L1 antibodies or antigen-binding fragments thereof provided herein is of IgG2/ IgG4 cross isotype. Examples of IgG2/ IgG4 cross isotype is described in Rother RP et al., Nat Biotechnol 25:1256-1264 (2007).

In some embodiments, the anti-PD-L1 antibodies or antigen-binding fragments thereof comprise one or more amino acid substitution(s) that improves pH-dependent binding to neonatal Fc receptor (FcRn). Such a variant can have an extended pharmacokinetic half-life, as it binds to FcRn at acidic pH which allows it to escape from degradation in the lysosome and then be translocated and released out of the cell. Methods of engineering an antibody or an antigen-binding fragment thereof to improve binding affinity with FcRn are well-known in the art, see, for example, Vaughn, D. et al., Structure, 6(1): 63-73, 1998; Kontermann, R. et al., Antibody Engineering, Volume 1, Chapter 27: Engineering of the Fc region for improved PK, published by Springer, 2010; Yeung, Y. et al., Cancer Research, 70: 3269-3277 (2010); and Hinton, P. et al., J. Immunology, 176:346-356 (2006).

In some embodiments, anti-PD-L1 antibodies or antigen-binding fragments thereof comprise one or more amino acid substitution(s) in the interface of the Fc region to facilitate and/or promote heterodimerization. These modifications comprise introduction of a protuberance into a first Fc polypeptide and a cavity into a second Fc polypeptide, wherein the protuberance can be positioned in the cavity so as to promote interaction of the first and second Fc polypeptides to form a heterodimer or a complex. Methods of generating antibodies with these modifications are known in the art, e.g. as described in U.S. Pat. No. 5,731,168.

### xv. Antigen-binding Fragments

The PD-L1-binding moiety in the bi-functional molecules provided herein also encompass anti-PD-L1 antigen-binding fragments. Various types of antigen-binding fragments are known in the art and can be developed based on the anti-PD-L1 antibodies provided herein, including for example, the exemplary antibodies whose CDRs are shown in Tables 1 and 3a above, and variable sequences are shown in Tables 2, and 5, and their different variants (such as affinity variants, glycosylation variants, Fc variants, cysteine-engineered variants and so on).

In some embodiments, an anti-PD-L1 antigen-binding fragment provided herein is a diabody, a Fab, a Fab', a F(ab')2, a Fd, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a multispecific antibody, a camelized single domain antibody, a nanobody, a domain antibody, and a bivalent domain antibody.

Various techniques can be used for the production of such antigen-binding fragments. Illustrative methods include, enzymatic digestion of intact antibodies (see, e.g. Morimoto et al., Journal of Biochemical and Biophysical Methods 24:107-117 (1992); and Brennan et al., Science, 229:81 (1985)), recombinant expression by host cells such as *E. Coli* (e.g. for Fab, Fv and ScFv antibody fragments), screening from a phage display library as discussed above (e.g. for ScFv), and chemical coupling of two Fab'-SH fragments to form F(ab')₂ fragments (Carter et al., Bio/Technology 10:163-167 (1992)). Other techniques for the production of antibody fragments will be apparent to a person skilled in the art.

In some embodiments, the antigen-binding fragment is a scFv. Generation of scFv is described in, for example, WO 93/16185; U.S. Pat. Nos. 5,571,894; and 5,587,458. ScFv may be fused to an effector protein at either the amino or the carboxyl terminus to provide for a fusion protein (see, for example, Antibody Engineering, ed. Borrebaeck).

In some embodiments, the anti-PD-L1 antibodies or antigen-binding fragments thereof provided herein are bivalent, tetravalent, hexavalent, or multivalent. Any molecule being more than bivalent is considered multivalent, encompassing for example, trivalent, tetravalent, hexavalent, and so on.

A bivalent molecule can be monospecific if the two binding sites are both specific for binding to the same antigen or the same epitope. This, in some embodiments, provides for stronger binding to the antigen or the epitope than a monovalent counterpart. Similar, a multivalent molecule may also be monospecific. In some embodiments, in a bivalent or multivalent antigen-binding moiety, the first valent of binding site and the second valent of binding site are structurally identical (i.e. having the same sequences), or structurally different (i.e. having different sequences albeit with the same specificity).

A bivalent can also be bispecific, if the two binding sites are specific for different antigens or epitopes. This also applies to a multivalent molecule. For example, a trivalent molecule can be bispecific when two binding sites are monospecific for a first antigen (or epitope) and the third binding site is specific for a second antigen (or epitope).

### xvi. Bispecific Antibodies

In some embodiments, the anti-PD-L1 antibodies or antigen-binding fragments thereof is bispecific. In some embodiments, apart from the second moiety provided herein, the PD-L1 binding antibody or an antigen-binding fragment thereof is further linked to an additional functional domain having a different binding specificity from said anti-PD-L1 antibody, or antigen binding fragment thereof.

In some embodiments, the bispecific antibodies or antigen-binding fragments thereof provided herein are capable of specifically binding to a second antigen other than PD-L1 (and other than the target bound by the second moiety), or a second epitope on PD-L1 (or a second epitope on the target bound by the second moiety).

### xvii. Bi-functional Molecules

In some embodiments, the bi-functional molecule provided herein are capable of binding to both PD-L1 and the target bound by the second moiety. In some embodiments, the bi-functional molecule provided herein are capable of binding to both PD-L1 and TGFβ, or binding to both PD-L1 and IL-1, or binding to both PD-L1 and IL-1R, or binding to both PD-L1 and MHCII, or binding to both PD-L1 and CD47, or binding to both PD-L1 and SIRPα.

In some embodiments, the bi-functional molecule targeting PD-L1 and TGFβ of the present disclosure specifically binding to human TGFβ1 at an EC₅₀ of no more than 2.0 nM (e.g. no more than 2.0 nM, no more than 1.2 nM, no more than 1.1 nM, no more than 1.0 nM, no more than 0.9 nM, no more than 0.8 nM) as measured by ELISA assay. In some embodiments, the protein targeting PD-L1 and TGFβ of the present disclosure is capable of simultaneously binding to PD-L1 and TGFβ as measured by ELISA assay. In some embodiments, the bi-functional molecule targeting PD-L1 and TGFβ of the present disclosure is capable of specifically binding to human PD-L1 at a K_{D} value of no more than 0.8 nM, no more than 0.7 nM, no more than 0.6 nM, no more than 0.5 nM, or no more than 0.4 nM as measured by Biacore assay. In some embodiments, the bi-functional molecule targeting PD-L1 and TGFβ of the present disclosure is capable of specifically binding to human TGFβ1 at a K_{D} value of no more than 2.0 nM (e.g. no more than 2.0 nM, no more than 1.2 nM, no more than 1.1 nM, no more than 1.0 nM, no more than 0.9 nM, no more than 0.8 nM) as measured by ELISA assay.

In some embodiments, the bi-functional molecule targeting PD-L1 and TGFβ of the present disclosure is capable of exhibiting synergistic effect on tumor growth inhibition at a dose dependent manner.

In some embodiments, the bi-functional molecule targeting PD-L1 and TGFβ of the present disclosure is capable of exhibiting enhanced infiltration of anti-tumor immune cells into a tumor microenvironment as compared to a molecule comprising the immune checkpoint molecule only.

In some embodiments, the bi-functional molecule targeting PD-L1 and TGFβ of the present disclosure is capable of selectively reducing at least 90% (e.g. at least 80%, 70%, 60%, 50%, 40%, 30%, or 20%) of TGFβ1 in plasma and such reduction can be maintained for at least 10, 14, or 21 days.

In some embodiments, the bi-functional molecule comprises heterodimeric heavy chains. The heavy chains are heterodimeric with respect to presence or position of the second moiety. In some embodiments, the heterodimeric heavy chains comprise one heavy chain having the second moiety but the other heavy chain having not, wherein the second moiety comprises a CD47 binding domain (e.g. soluble SIRP α) or a SIRPα binding domain.

In the bi-functional molecule, the heterodimeric heavy chains comprise one heavy chain having the second moiety but the other heavy chain having not. The heterodimeric heavy chains can further comprise heterodimeric Fc regions that associate in a way that discourages homodimerization and/or favors heterodimerization. For example, the heterodimeric Fc regions can be selected so that they are not identical and that they preferentially form heterodimers between each other rather than to form homodimers within themselves. In some embodiments, the heterodimeric Fc regions are capable of associating into heterodimers via formation of knob-into-hole, hydrophobic interaction, electrostatic interaction, hydrophilic interaction, or increased flexibility. In some embodiments, heterodimeric Fc regions comprise CH2 and/or CH3 domains which are respectively mutated to be capable of forming a knobs-into-holes. A knob can be obtained by replacement of a small amino acid residue with a larger one in the first CH2/CH3 polypeptide, and a hole can be obtained by replacement of a large residue with a smaller one. In some embodiments, heterodimeric Fc regions comprise a first CH3 domain of the IgG1 isotype containing S354C and T366W substitution (SEQ ID NO: 96, knob) and a second CH3 domain of the IgG1 isotype containing Y349C, T366S, L368A and Y407V substitution (SEQ ID NO: 97, hole).

In some embodiments, the bi-functional molecule comprises a heavy chain comprising an amino acid sequence of SEQ ID NO: 118 or SEQ ID NO: 120, and/or a light chain comprising an amino acid sequence of SEQ ID NO: 119 or SEQ ID NO: 121.

### xviii. Conjugates

In some embodiments, the bi-functional molecule further comprises one or more conjugate moieties. The conjugate moiety can be linked to the bi-functional molecule. A conjugate moiety is a moiety that can be attached to the bi-functional molecule. It is contemplated that a variety of conjugate moieties may be linked to the bi-functional molecules provided herein (see, for example, "Conjugate Vaccines", Contributions to Microbiology and Immunology, J. M. Cruse and R. E. Lewis, Jr. (eds.), Carger Press, New York, (1989)). These conjugate moieties may be linked to the bi-functional molecule by covalent binding, affinity binding, intercalation, coordinate binding, complexation, association, blending, or addition, among other methods. In some embodiments, the bi-functional molecule can be linked to one or more conjugates via a linker.

In some embodiments, the bi-functional molecule provided herein may be engineered to contain specific sites outside the epitope binding portion that may be utilized for binding to one or more conjugate moieties. For example, such a site may include one or more reactive amino acid residues, such as for example cysteine or histidine residues, to facilitate covalent linkage to a conjugate moiety.

In some embodiments, the bi-functional molecules may be linked to a conjugate moiety indirectly, or through another conjugate moiety. For example, the bi-functional molecules provided herein may be conjugated to biotin, then indirectly conjugated to a second conjugate that is conjugated to avidin. In some embodiments, the conjugate moiety comprises a clearance-modifying agent (e.g. a polymer such as PEG which extends half-life), a chemotherapeutic agent, a toxin, a radioactive isotope, a lanthanide, a detectable label (e.g. a luminescent label, a fluorescent label, an enzyme-substrate label), a DNA-alkylator, a topoisomerase inhibitor, a tubulin-binder, a purification moiety or other anticancer drugs.

A "toxin" can be any agent that is detrimental to cells or that can damage or kill cells. Examples of toxin include, without limitation, taxol, cytochalasin B, gramicidin D, ethidium bromide, emetine, mitomycin, etoposide, tenoposide, vincristine, MMAE, MMAF, DM1, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin and analogs thereof, antimetabolites (e.g. methotrexate, 6-mercaptopurine, 6-thioguanine, cytarabine, 5-fluorouracil decarbazine), alkylating agents (e.g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g. daunorubicin (formerly daunomycin) and doxorubicin), antibiotics (e.g. dactinomycin (formerly actinomycin), bleomycin, mithramycin, and anthramycin (AMC)), anti-mitotic agents (e.g. vincristine and vinblastine), a topoisomerase inhibitor, and a tubulin-binders.

Examples of detectable label may include a fluorescent labels (e.g. fluorescein, rhodamine, dansyl, phycoerythrin, or Texas Red), enzyme-substrate labels (e.g. horseradish peroxidase, alkaline phosphatase, luceriferases, glucoamylase, lysozyme, saccharide oxidases or β-D-galactosidase), radioisotopes (e.g. 123I, 124I, 125I, 131I, 35S, 3H, 111In, 112In, 14C, 64Cu, 67Cu, 86Y, 88Y, 90Y, 177Lu, 211At, 186Re, 188Re, 153Sm, 212Bi, and 32P, other lanthanides), luminescent labels, chromophoric moieties, digoxigenin, biotin/avidin, DNA molecules or gold for detection.

In some embodiments, the conjugate moiety can be a clearance-modifying agent which helps increase half-life of the bi-functional molecule. Illustrative examples include water-soluble polymers, such as PEG, carboxymethylcellulose, dextran, polyvinyl alcohol, polyvinyl pyrrolidone, copolymers of ethylene glycol/propylene glycol, and the like. The polymer may be of any molecular weight, and may be branched or unbranched. The number of polymers attached to the antibody may vary, and if more than one polymer are attached, they can be the same or different molecules.

In some embodiments, the conjugate moiety can be a purification moiety such as a magnetic bead.

In some embodiments, the bi-functional molecules provided herein is used as a base for a conjugate.

### III. Pharmaceutical Formulations

The formulations of the present disclosure may also contain other pharmaceutically acceptable excipients such as vehicles (aqueous or non-aqueous), osmotic pressure regulators, viscosity reducing excipients, preservatives, wetting agents, emulsifying agents and dispersing agents. The absence of microorganisms can be ensured by sterilization procedures and by the addition of various antibacterial and antifungal agents, for example, parabens, chlorobutanol, phenol, sorbic acid, and the like. Preservatives are typically used at concentrations of about 0.001% (w/v) to about 2% (w/v). Preservatives include, but are not limited to, ethanol, benzyl alcohol, phenol, m-cresol, p-chloro-m-cresol, methyl or propyl paraben, benzalkonium chloride.

The formulations of the present disclosure exhibit good stability and ease of application. In some embodiments of the present disclosure, bi-functional molecules, particularly PD-L1/TGF-β proteins, can remain stable for long periods of time, meeting clinical use needs. In addition, the embodiments provided by the disclosure also solve the problem of degradation of polysorbate 80 and avoid the influence of degradation products of polysorbate 80 on protein quality.

In some embodiments, the formulations of the present disclosure are liquid formulations. For example, the liquid formulation is an injection. For example, the liquid formulation comprises water for injection.

In some embodiments, the purity of the bi-functional protein molecule in the formulation decreases by no more than 10%, e.g., no more than 5%, 4%, 3%, 2%, 1%, 0.5% or 0.1%, after storage of the formulation of the disclosure, such as liquid formulation at about -80°C to about 45°C, e.g., -80°C, about -30°C, about -20°C, about 0°C, about 5°C, about 25°C, about 35°C, about 37°C, about 42°C or about 45°C, for at least 14 days, at least 28 days, at least 1 months, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months or longer, as measured by SEC.

In one embodiment, the purity of the bi-functional protein molecule decreases by no more than 10%, e.g., no more than 5%, 4%, 3%, 2%, 1%, 0.5% or 0.1%, after storage of the formulation of the disclosure, such as liquid formulation at about -80°C to about 45°C, e.g., - 80°C, about -30°C, about -20°C, about 0°C, about 5°C, about 25°C, about 35°C, about 37°C, about 42°C or about 45°C for at least 14 days, at least 28 days, at least 1 months, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months or longer, as measured by the non-reducing CE-SDS method.

### IV. Methods of treatment and uses

The present disclosure provides methods of treating, preventing, or ameliorating a PD-L1-associated disease in a subject, comprising administering to the subject a therapeutically effective amount of a formulation provided herein.

In some embodiments, the subject is human.

PD-1-related conditions and disorders can be immune related disease or disorder, cancers, autoimmune diseases, or infectious disease.

In some embodiments, the PD-1-related conditions and disorders include cancers, for example, non-small cell lung cancer, small cell lung cancer, renal cell cancer, colorectal cancer, ovarian cancer, breast cancer, pancreatic cancer, gastric carcinoma, bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies, such as classical Hodgkin lymphoma (CHL), primary mediastinal large B-cell lymphoma, T-cell/histiocyte-rich B-cell lymphoma, EBV-positive and -negative PTLD, and EBV-associated diffuse large B-cell lymphoma (DLBCL), plasmablastic lymphoma, extranodal NK/T-cell lymphoma, nasopharyngeal carcinoma, and HHV8-associated primary effusion lymphoma, Hodgkin's lymphoma, neoplasm of the central nervous system (CNS), such as primary CNS lymphoma, spinal axis tumor, brain stem glioma. In some embodiments, the tumors and cancers are metastatic, especially metastatic tumors expressing PD-L1.

In some embodiments, the PD-1-related conditions and disorders include autoimmune diseases. Autoimmune diseases include, but are not limited to, Acquired Immunodeficiency Syndrome (AIDS, which is a viral disease with an autoimmune component), alopecia areata, ankylosing spondylitis, antiphospholipid syndrome, autoimmune Addison's disease, autoimmune diabetes, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pemacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo and Wegener's granulomatosis.

In some embodiments, the PD-1-related conditions and disorders include infectious disease. Infectious disease include, for example, chronic viral infection, for example, fungus infection, parasite/protozoan infection or chronic viral infection, for example, malaria, coccidioiodmycosis immitis, histoplasmosis, onychomycosis, aspergilosis, blastomycosis, candidiasis albicans, paracoccidioiomycosis, microsporidiosis, Acanthamoeba keratitis, Amoebiasis, Ascariasis, Babesiosis, Balantidiasis, Baylisascariasis, Chagas disease, Clonorchiasis, Cochliomyia, Cryptosporidiosis, Diphyllobothriasis, Dracunculiasis, Echinococcosis, Elephantiasis, Enterobiasis, Fascioliasis, Fasciolopsiasis, Filariasis, Giardiasis, Gnathostomiasis, Hymenolepiasis, Isosporiasis, Katayama fever, Leishmaniasis, Lyme disease, Metagonimiasis, Myiasis, Onchocerciasis, Pediculosis, Scabies, Schistosomiasis, Sleeping sickness, Strongyloidiasis, Taeniasis, Toxocariasis, Toxoplasmosis, Trichinosis, Trichuriasis, Trypanosomiasis, helminth infection, infection of hepatitis B (HBV), hepatitis C (HCV), herpes virus, Epstein-Barr virus, HIV-1, HIV-2, cytomegalovirus, herpes simplex virus type I, herpes simplex virus type II, human papilloma virus, adenovirus, Kaposi West sarcoma associated herpes virus epidemics, thin ring virus (Torquetenovirus), human T lymphotrophic viruse I, human T lymphotrophic viruse II, varicella zoster, JC virus or BK virus.

In some embodiments, the PD-L1-related disease is a PD-L1-expressing cancer, or a PD-L1-overexpressing cancer. A "PD-L1-expressing cancer" is one that involves cancer cells or tumor cells having PD-L1 protein present at their cell surface. A "PD-L1-overexpressing cancer" is one which has significantly higher levels of a PD-L1, at the cell surface of a cancer or tumor cell, compared to a noncancerous cell of the same tissue type.

PD-L1 expression or overexpression may be determined in a diagnostic or prognostic assay by evaluating increased levels of the PD-L1 present on the surface of a cell (e.g. via an immunohistochemistry assay; IHC). Alternatively, or additionally, one may measure levels of PD-L1-encoding nucleic acid in the cell, e.g. via fluorescent in situ hybridization (FISH; see WO98/45479 published October, 1998), southern blotting, or polymerase chain reaction (PCR) techniques, such as real time quantitative PCR (RT-PCR). One may also study PD-L1 overexpression by measuring shed antigen (e.g., PD-L1 ectodomain or soluble PD-L1) in a biological fluid such as serum. Aside from the above assays, various in vivo assays are available to the skilled practitioner. For example, one may expose cells within the body of the patient to an anti-PD-L1 antibody which is optionally labeled with a detectable label, e.g. a radioactive isotope, and binding of the antibody to cells in the patient can be evaluated, e.g. by external scanning for radioactivity or by analyzing a biopsy taken from a patient previously exposed to the antibody.

In some embodiments, the subject has been identified as being likely to respond to a PD-1 antagonist. The presence or level of PD-L1 on an interested biological sample can be indicative of whether the subject from whom the biological sample is derived could likely respond to a PD-1 antagonist. In some embodiments, the test sample is derived from a cancer cell or tissue, or tumor infiltrating immune cells. In some embodiments, presence or up-regulated level of the PD-L1 in the test biological sample indicates likelihood of responsiveness. The term "up-regulated" as used herein, refers to an overall increase of no less than 10%, 15%, 20%, 25%, 30%, 35%, 40%, 45%, 50%, 55%, 60%, 65%, 70%, 75%, 80% or greater, in the protein level of PD-L1 in the test sample, as compared to the PD-L1 protein level in a reference sample as detected using the same antibody. The reference sample can be a control sample obtained from a healthy or non-diseased individual, or a healthy or non-diseased sample obtained from the same individual from whom the test sample is obtained. For example, the reference sample can be a non-diseased sample adjacent to or in the neighborhood of the test sample (e.g. tumor).

In some embodiments, the PD-L1-related disease is resistant to PD-L1/PD-1 monotherapy. "PD-L1/PD-1 monotherapy" as used herein refers to a monotherapy that acts by inhibiting or reducing PD-L1 and PD-1 interaction or signaling. Exemplary PD-L1/PD-1 monotherapy may include anti-PD-L1 antibody therapy, anti-PD-1 antibody therapy, or monotherapy involving small molecule inhibitors directed to PD-1 or PD-L1. By "resistant" it is meant that the disease has no or reduced responsiveness or sensitivity to a PD-L1/PD-1 monotherapy. Reduced responsiveness can be indicated by, for example, requirement of an increased dose to achieve a given efficacy. In some embodiments, the disease can be non-responsive to PD-L1/PD-1 monotherapy. For example, the cancer cells or tumor size increases despite of the treatment with the PD-L1/PD-1 monotherapy, or the disease showed regression back to its former state, for example, return of previous symptoms following partial recovery. The resistance to PD-L1/PD-1 monotherapy can be de novo or acquired.

In another aspect, the present disclosure provides a method of treating, preventing, or alleviating a disease or condition in a subject, the disease or condition would benefit from suppression of an immunosuppressive cytokine, from induction of sustained immune responses, or from stimulation of anti-tumor immunity, said method comprising administering an effective amount of the formulation provided herein.

In some embodiments, the immunosuppressing cytokine is a TGFβ or IL-1. In some embodiments, the immunosuppressing cytokine is a TGFβ1 or IL-1β.

In some embodiments, the disease or condition is a TGFβ-related disease or condition. In some embodiments, the TGFβ-related disease is cancer, fibrotic disease, or kidney disease.

In some embodiments, the TGFβ-related disease is cancer. In some embodiments, the cancer is selected from the group consisting of: colorectal, breast, ovarian, pancreatic, gastric, prostate, renal, cervical, myeloma, lymphoma, leukemia, thyroid, endometrial, uterine, bladder, neuroendocrine, head and neck, liver, nasopharyngeal, testicular, small cell lung, cancer, non-small cell lung cancer, melanoma, basal cell, skin cancer, squamous cell skin cancer, dermatofibrosarcoma protuberans, Merkel cell carcinoma, glioblastoma, glioma, sarcoma, mesothelioma, and myelodisplastic syndromes.

In some embodiments, the TGFβ□related disease is fibrotic disease. Fibrotic disease is a disease or condition that involves fibrosis. Fibrosis is a scarring process that is a common feature of chronic organ injury, for example in lungs, liver, kidney, skin, heart, gut or muscle. Fibrosis is characterized by elevated activity of transforming growth factor-beta (TGF-β) resulting in increased and altered deposition of extracellular matrix and other fibrosis-associated proteins.

Fibrotic disease can include fibrotic disease in lungs, liver, kidney, eyes, skin, heart, gut or muscle. Examples of fibrotic disease in lungs include pulmonary fibrosis, cystic fibrosis, pulmonary hypertension, progressive massive fibrosis, bronchiolitis obliterans, airway remodeling associated with chronic asthma or idiopathic pulmonary. Examples of fibrotic disease in liver include cirrhosis or non-alcoholic steatohepatitis. Examples of fibrotic disease in kidney include such as renal fibrosis, ischemic renal injury, tubulointerstitial fibrosis, diabetic nephropathy, nephrosclerosis, or nephrotoxicity. Examples of fibrotic disease in eyes include such as corneal fibrosis, subretinal fibrosis. Examples of fibrotic disease in skin include such as nephrogenic systemic fibrosis, keloid or scleroderma. Examples of fibrotic disease in heart include endomyocardial fibrosis or old myocardial infarction.

In some embodiments, the disease or condition is an IL-1-related disease or condition. In some embodiments, the IL-1-related disease is autoinflammatory disease, metabolic syndrome, acute inflammation, chronic inflammation or malignancy.

In some embodiments, the disease or condition would benefit from induction of sustained immune responses by stimulating MHCII signaling with an immunostimulatory polypeptide, e.g., soluble LAG-3. In some embodiments, the disease or condition is cancer, viral infection, parasite infection, or a combination thereof.

In some embodiments, the disease or condition would benefit from stimulation of anti-tumor immunity by inhibiting an immunoinhibitory receptor signaling. In some embodiments, the immunoinhibitory receptor is SIRPα. In some embodiments, the disease, disorder or condition is SIRPα related, such as cancer, solid tumor, a chronic infection, an inflammatory disease, multiple sclerosis, an autoimmune disease, a neurologic disease, a brain injury, a nerve injury, a polycythemia, a hemochromatosis, a trauma, a septic shock, fibrosis, atherosclerosis, obesity, type II diabetes, a transplant dysfunction, or arthritis. In some embodiments, the cancer is anal cancer, appendix cancer, astrocytoma, basal cell carcinoma, gallbladder cancer, gastric cancer, lung cancer, bronchial cancer, bone cancer, liver and bile duct cancer, pancreatic cancer, breast cancer, liver cancer, ovarian cancer, testicle cancer, kidney cancer, renal pelvis and ureter cancer, salivary gland cancer, small intestine cancer, urethral cancer, bladder cancer, head and neck cancer, spine cancer, brain cancer, cervix cancer, uterine cancer, endometrial cancer, colon cancer, colorectal cancer, rectal cancer, anal cancer, esophageal cancer, gastrointestinal cancer, skin cancer, prostate cancer, pituitary cancer, vagina cancer, thyroid cancer, throat cancer, glioblastoma, melanoma, myelodysplastic syndrome, sarcoma, teratoma, chronic lymphocytic leukemia (CLL), chronic myeloid leukemia (CML), acute lymphocytic leukemia (ALL), acute myeloid leukemia (AML), Hodgkin lymphoma, non-Hodgkin lymphoma, multiple myeloma, T or B cell lymphoma, **GI** organ interstitialoma, soft tissue tumor, hepatocellular carcinoma, and adenocarcinoma. **In** some embodiments, the cancer is a CD47-expressing cancer, or a CD47-overexpressing cancer.

The therapeutically effective amount of a bi-functional molecule provided herein will depend on various factors known in the art, such as for example body weight, age, past medical history, present medications, state of health of the subject and potential for cross-reaction, allergies, sensitivities and adverse side-effects, as well as the administration route and extent of disease development.

**In** some embodiments, the bi-functional molecule provided herein may be administered at a therapeutically effective dosage of about 0.01 mg/kg to about 100 mg/kg.

The formulation provided herein may be administered by routes known in the art, for example parenteral (e.g. subcutaneous, intraperitoneal, intravenous, including intravenous infusion, intramuscular, or intradermal injection) or non-parenteral (e.g. oral, intranasal, intraocular, sublingual, rectal, or topical) routes.

**In** some embodiments, the bi-functional molecule provided herein may be administered alone or in combination with a therapeutically effective amount of a second therapeutic agent. For example, the bi-functional molecule disclosed herein may be administered in combination with a second therapeutic agent, for example, a chemotherapeutic agent, an anti-cancer drug, radiation therapy, an immunotherapy agent, an anti-angiogenesis agent, a targeted therapy, a cellular therapy, a gene therapy, a hormonal therapy, an antiviral agent, an antibiotic, an analgesics, an antioxidant, a metal chelator, or cytokines.

The term "immunotherapy" as used herein, refers to a type of therapy that stimulates immune system to fight against disease such as cancer or that boosts immune system in a general way. Examples of immunotherapy include, without limitation, checkpoint modulators, adoptive cell transfer, cytokines, oncolytic virus and therapeutic vaccines.

"Targeted therapy" is a type of therapy that acts on specific molecules associated with cancer, such as specific proteins that are present in cancer cells but not normal cells or that are more abundant in cancer cells, or the target molecules in the cancer microenvironment that contributes to cancer growth and survival. Targeted therapy targets a therapeutic agent to a tumor, thereby sparing of normal tissue from the effects of the therapeutic agent.

In some embodiments, a bi-functional molecule provided herein that is administered in combination with one or more additional therapeutic agents may be administered simultaneously with the one or more additional therapeutic agents, and in some of these embodiments the bi-functional molecule and the additional therapeutic agent(s) may be administered as part of the same pharmaceutical composition. However, a bi-functional molecule administered "in combination" with another therapeutic agent does not have to be administered simultaneously with or in the same composition as the agent. A bi-functional molecule administered prior to or after another agent is considered to be administered "in combination" with that agent as the phrase is used herein, even if the antibody or antigen-binding fragment and the second agent are administered via different routes.

In another aspect, the present disclosure also provides use of the bi-functional molecule provided herein and/or the pharmaceutical composition, pharmaceutical formulation provided herein in the manufacture of a medicament for treating a PD-L1-related disease, and/or a TGF-β-related disease and/or an IL-1-related disease and/or a CD47-related disease in a subject.

The following examples are provided to better illustrate the claimed invention and are not to be interpreted as limiting the scope of the present disclosure. All specific preparations or formulations, materials, and methods described below, in whole or in part, fall within the scope of the present disclosure. These specific preparations or formulations, materials, and methods are not intended to limit the disclosure, but merely to illustrate specific embodiments falling within the scope of the disclosure. A person skilled in the art may develop equivalent compositions, materials, and methods without the exercise of inventive work and without departing from the scope of the invention. It will be understood that many variations can be made in the embodiments herein described. Such variations are included within the scope of the invention.

### EXAMPLES

### Example A: Preparation, characterization and activity of antibody and bi-functional protein

### Example 1: Generation, expression and purification of humanzied 4B6 antibodies

The anti-PD-L1 mAb 4B6, which originated in Patent WO2017161976A1 comprising a VH sequence of SEQ ID NO: 46 and a VL sequence of SEQ ID NO: 47 shown below, was a potent PD-1/PD-L1 blocker. This antibody was generated from mouse hybridoma antibody therefore it needed an appropriate humanization. The sequence of the variable domain of mouse antibody 4B6 was used to identify the germline sequence with the highest homology to their respective murine framework. Computer-modelling was used for designing the humanzied variants with complementarity-determining region (CDR) grafting and back mutations.

Mouse/chimeric heavy chain variable region (SEQ ID NO: 46):

EVQLQQSGPELVKPGASVKISCKASGYVFTDYYMNWVKQSHGKSLEWIGDINPN
NGGTSYNHKFKGKATVTVDKSSRTAYMELLSLTSEDSAVYYCVKWGDGPFAYWGQGT
LVTVSA

Mouse/chimeric light chain variable region (SEQ ID NO: 47):

DIVMTQSQKFMSTSVGDRVSITCKASQNVGAAVAWYQQKPGQSPKLLIYSASNR
YTGVPDRFTGSGSGTDFTLTISNMQSEDLADYFCQQYSNYPTFGSGTKLGIK

NOTE: The italic portion represents framework (FR), and the underlined portion represents CDR sequences. The order is FR1-CDR1-FR2-CDR2-FR3-CDR3-FR4.

Human germline framework sequences VK/1-33 for light chain and VH/1-2 for heavy chain were used for CDR grafting, respectively.

Heavy chain variants 1, 2, 3 and 4 (i.e., VH variant 1, 2, 3, and 4) were obtained by direct grafting the three CDRs to the VH germline sequence (SEQ ID NO: 48), and in addition the back mutations of M69V, R71V for VH variant 1 (SEQ ID NO: 49), M69V, R71V, A93V, R94K for VH variant 2 (SEQ ID NO: 50), M69V, R71V, T73K, T28V for VH variant 3 (SEQ ID NO: 51) and M69V, R71V, A93V, R94K, T73K, T28V, G44S for VH variant 4 (SEQ ID NO: 52), respectively.

Germline sequence for 4B6 VH:
VH/1-2 (4B6-VH germline, SEQ ID NO: 48): 
QVQLVQSGAEVKKPGASVKVSCKASGYTFTGYYMHWVRQAPGQGLEWMG
WINPNSGGTNYAQKFQGRVTMTRDTSISTAYMELSRLRSDDTAVYYCAR
VH/1-2 variant 1 (4B6_Ha, SEQ ID NO: 49): 
QVQLVQSGAEVKKPGASVKVSCKASGYTFTDYYMNWVRQAPGQGLEWMG
DINPNNGGTSYNHKFKGRVTVTVDTSISTAYMELSRLRSDDTAVYYCARWGDGPFA
YWGQGTLVTVSS
VH/1-2 variant 2 (4B6_Hb, SEQ ID NO: 50): 
QVQLVQSGAEVKKPGASVKVSCKASGYTFTDYYMNWVRQAPGQGLEWMG
DINPNNGGTSYNHKFKGRVTVTVDTSISTAYMELSRLRSDDTAVYYCVKWGDGPFA
YWGQGTLVTVSS
VH/1-2 variant 3 (Hu4B6 Hc, SEQ ID NO: 51): 
QVQLVQSGAEVKKPGASVKVSCKASGYVFTDYYMNWVRQAPGQGLEWMG
DINPNNGGTSYNHKFKGRVTVTVDKSISTAYMELSRLRSDDTAVYYCARWGDGPFA
YWGQGTLVTVSS
VH/1-2 variant 4 (Hu4B6 Hd, SEQ ID NO: 52):

QVQLVQSGAEVKKPGASVKVSCKASGYVFTDYYMNWVRQAPGQSLEWMG
DINPNNGGTSYNHKFKGRVTVTVDKSISTAYMELSRLRSDDTAVYYCVKWGDGPFA
YWGQGTLVTVSS

Light chain vairants 1 and 2 (VL variant 1 and 2) were obtained by direct grafting the three CDRs to the germline seqeunce (SEQ ID NO : 53), and in addition the back mutation of F73L mutation for VL variant 1 (SEQ ID NO: 54) and F73L, A43S, S60D for VL variant 2 (SEQ ID NO: 55), respectively.

Germline sequence for 4B6 VL:
VK/1-33 (4B6-VL-germline, SEQ ID NO: 53) : 
DIQMTQSPSSLSASVGDRVTITCQASQDISNYLNWYQQKPGKAPKLLIYDASN
LETGVPSRFSGSGSGTDFTFTISSLQPEDIATYYCQQYDNLP
VK/1-33 variant 1 (Hu4B6 La, SEQ ID NO: 54) : 
DIQMTQSPSSLSASVGDRVTITCKASQNVGAAVAWYQQKPGKAPKLLIYSAS
NRYTGVPSRFSGSGSGTDFTLTISSLQPEDIATYYCQQYSNYPTFGQGTKLEIK
VK/1-33 variant 2 (Hu4B6 Lb, SEQ ID NO: 55) :

DIQMTQSPSSLSASVGDRVTITCKASQNVGAAVAWYQQKPGKSPKLLIYSASN
RYTGVPDRFSGSGSGTDFTLTISSLQPEDIATYYCQQYSNYPTFGQGTKLEIK

cDNAs of the variable regions of the above heavy chains and light chains were synthesized and then fused with the sequences of the constatnt region of human IgG1 and human kappa. The resulting antibody gene sequences were cloned into an expression vector. Large-scale DNA was prepared by using Plasmid Maxiprep System from Qiagen for humanized 4B6 variants expression, as shown in Table 6, and cell transfection was carried out using the ExpiFectamine^{™} CHO Reagent from Invitrogen according to the manufacturer's protocol. Supernatant was harvested when cell viability was more than 60% and filtered through 0.22um filtration capsule to remove cell debris. The filtered supernatant was subsequently loaded onto a pre-equilibrated Protein-A affinity column. Protein A resin was washed with equilibration buffer (PBS), and 25 mM citrate (pH3.5) was then used to elute antibody. The purified antibody solution was adjusted to pH 6.0-7.0 by using 1M Tris-base (pH 9.0). The endotoxin was controlled below 1EU/mg. Finally, the purified antibody was characterized by SDS-PAGE.

**Table 6. Expression of humanized 4B6 variants**

| Humanized sequence combinations for murine antobody 4B6 | | | |
|---|---|---|---|
| | Hu4B6_L0 | Hu4B6_La (SEQ ID NO : 54) | Hu4B6_Lb (SEQ ID NO : 55) |
| Hu4B6_H0 | | | |
| Hu4B6_Ha (SEQ ID NO : 49) | | Hu4B6_HaLa | Hu4B6_HaLb |
| Hu4B6_Hb (SEQ ID NO : 50) | | Hu4B6_HbLa | Hu4B6_HbLb |
| Hu4B6_Hc (SEQ ID NO : 51) | | Hu4B6_HcLa | Hu4B6_HcLb |
| Hu4B6_Hd (SEQ ID NO : 52) | | Hu4B6_HdLa | Hu4B6_HdLb |

| | | | |
|---|---|---|---|
| NOTE: This table shows various sequence combinations of different mutations. For example, Hu4B6_HaLa indicates that two kinds of mutation (heavy chain Hu4B6_Ha and light chain Hu4B6_La) are present on the humanized murine antibody Hu4B6_HaLa, and so on. Hu4B6_L0 and Hu4B6_H0 are obtained by CDR-grafting, which are lack of the key back mutations, so they are not used for expression. | | | |

### Example 2: Binding to human PD-L1 by an ELISA assay

Binding of the humanized antibodies were evaluated by an ELISA method. Briefly, human PD-L1-His was immobilized on the plate. Humanized 4B6 antibodies set forth in Table 6 were serial diluted in PBS and added for 1h incubation. Next, Goat pAb to human IgG-HRP and TMB were added for detection of binding at OD450nm.

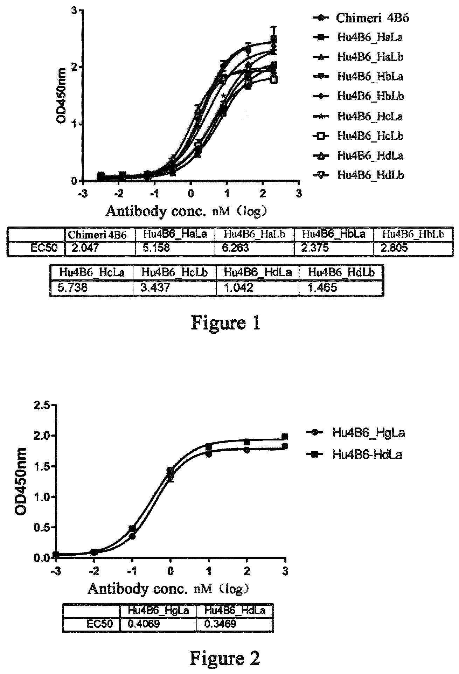

As shown in Figure 1, all humanized variants were tested in order to screen the best one. All the variants retained their binding activity, and Hu4B6_HdLa showed a better binding activity than the others.

We analyzed the CDR sequence of this antibody and found that there is a NG motif in CDR2 of the heavy chain. There may be a risk of deamination in expression and purification. To remove the deamidation hot spot, we introduced the mutation of G55A (bolded and enlarged below) into the Hu4B6_Hd. Then Hu4B6_Hg (SEQ ID NO: 56) was obtained, and the affinty to human PD-L1 was not affected, as shown in Figure 2.

Hu4B6_Hg (SEQ ID NO: 56) :

QVQLVQSGAEVKKPGASVKVSCKASGYVFTDYYMNWVRQAPGQSLEWMG
DINPNNAGTSYNHKFKGRVTVTVDKSISTAYMELSRLRSDDTAVYYCVKWGDGPFA
YWGQGTLVTVSS

### Example 3: Monoclonal phage ELISA and sequence analysis

Although Hu4B6-HgLa has retained activity from chimeric 4B6, as an antagonist drug, a higher affinity was preferable. Based on the Hu4B6-HgLa sequence, site-directed mutagenesis in the CDRs and several cycles of panning for off-rate-dependent selection in vitro was further used for affinity maturation. First of all, the VL and VH domains of 4B6-HgLa, were amplified and attached by a peptide linker(G₄S)3 to form the scFv by overlapping PCR, then subcloned into the phagemid vector pComb3x (Wuhan MiaoLingBio, P0862), as a wild-type sequence for affinity maturation via SfiI cleavage sites.

To investigate the individual contributions of CDR1 and CDR2 of both heavy and light chain to 4B6 affinity maturation, one SPM (small perturbation mutagenesis) phage library for each CDR above would be constructed, as antibody CDR3 of both chains usually plays an important role in antigen binding. The CDR1 and CDR2 sequences of both chains were aligned with germline sequences and the germline of variable region of heavy chain and light chain was IGHV1-2 and IGKV1-33, respectively. The bioinformatics analysis results of the germline CDR sequences are used to guide design of the library.

After determining amino acid mutation sites and substitution sequences, the degenerate primers were designed for increasing diversity of mutation library. The diversified CDR fragment was amplified to construct 4B6 scFv gene mutant library. The scFv genes were ligated with pComb3XSS phage display vector to generate the scFv libraries. The codon-based primers of each CDR (including HCDR1, HCDR2, LCDR1 and LCDR2, listed in Table 2) was established as an independent library, and 4B6 affinity maturation library was divided into 4 libraries. The capacities were 1.76×10⁸ CFM for HCDR1, 1.81×10⁸ CFM for HCDR2, 2.34×10⁸ CFM for LCDR1 and 2.00×10⁸ CFM for LCDR2. 5 or 6 clones of each library were picked randomly for sequencing of colony. The results showed that the insertion rate of the constructed library was 100%.

10 µg/mL hPD-L1 (Acro Biosystems, PD1-H5229) antigens was coated to the ELISA plate and were reacted with 200 µL of phages (1×10¹⁰ pfu/mL of phage display library) at 37°C for 1 h. After washing, TG1 (Lucigen, 60502-2) with OD600 around 0.5 was added into the well directly for infection and incubated with phage 15 min. Sufficient volume of M13KO7 helper phage (NEB, N0315S) to mid-log phase culture for library phagemid rescue, and the phages were generated and purified for the next round of screening. The screening process was repeated for 3 rounds, and concentration of antigen was reduced to 2.5 µg/mL for the 2^{nd} round and 1 µg/mL for the 3^{rd} round.

ELISA binding assay was carried out for detecting titer of these polyclonal phage variants. After 3 rounds of panning, 3 libraries, including 4B6-H-CDR2, 4B6-L-CDR1 and 4B6-L-CDR2, are obviously enriched.

For these 3 libraries, 96 clones of each library were picked and subjected to phage ELISA to detect their binding activity. Briefly, 1 µg/mL hPD-L1 (Acro Biosystems, PD1-H5229) antigens was coated to the ELISA plate and left overnight at 4°C. Then 300 µL of 3% (w/v) skim milk was added for blocking at room temperature for 1 h. After 1 h, 100 µL of supernatant containing monoclonal antibody fragment phage was added with PBS as a negative control, and incubated at 37°C for 1h. 0.5% PBS+Tween-20 were used for washing for 3 times, and 100 µL HRP coupled anti-M13 mAb (1:20000, Sino Biological, 11973-MM05T-H) was added. After incubation at room temperature for 1h, mixed TMB (InnoReagents, TMB-S-003) substrate reagent was added and the plate was incubated at room temperature for 5 min. 0.1M H₂SO₄ was added for stopping reaction, then OD450 nm was recorded. The positive clones were picked for DNA sequencing by Genewiz (Suzhou, China). The sequences were shown in Table 7.

**Table 7. The sequences of positive clones of 4B6 scFv phage library**

| | | |
|---|---|---|
| AM4B6_Hg | | QVQLVQSGAEVKKPGASVKVSCKASGYVFTDYYMNWVRQAP GQSLEWMGDINPNNAGTSYNHKFKGRVTVTVDKSISTAYMEL SRLRSDDTAVYYCVKWGDGPFAYWGQGTLVTVSS(SEQ ID NO: 56) |
| Hu4B6_Hg.2 | G57Q, S59Q | QVQLVQSGAEVKKPGASVKVSCKASGYVFTDYYMNWVRQAP GQSLEWMGDINPNNAQTQYNHKFKGRVTVTVDKSISTAYMEL SRLRSDDTAVYYCVKWGDGPFAYWGQGTLVTVSS(SEQ ID NO: 58) |
| AM4B6_Hg.3 | G57E, S59L | QVQLVQSGAEVKKPGASVKVSCKASGYVFTDYYMNWVRQAP GQSLEWMGDINPNNAETLYNHKFKGRVTVTVDKSISTAYMEL SRLRSDDTAVYYCVKWGDGPFAYWGQGTLVTVSS(SEQ ID NO: 59) |
| AM4B6_Hg.5 | G57Q, S59V | QVQLVQSGAEVKKPGASVKVSCKASGYVFTDYYMNWVRQAP GQSLEWMGDINPNNAQTVYNHKFKGRVTVTVDKSISTAYMEL SRLRSDDTAVYYCVKWGDGPFAYWGQGTLVTVSS(SEQ ID NO: 60) |
| Hu4B6_La | | DIQMTQSPSSLSASVGDRVTITCKASQNVGAAVAWYQQKPGK APKLLIYSASNRYTGVPSRFSGSGSGTDFTLTISSLQPEDIATYY CQQYSNYPTFGQGTKLEIK(SEQ ID NO: 61) |
| AM4B6_La.1 | G30K, A31G | DIQMTQSPSSLSASVGDRVTITCKASQNVKGAVAWYQQKPGK APKLLIYSASNRYTGVPSRFSGSGSGTDFTLTISSLQPEDIATYY CQQYSNYPTFGQGTKLEIK(SEQ ID NO: 62) |
| AM4B6_La.2 | G30P | DIQMTQSPSSLSASVGDRVTITCKASQNVPAAVAWYQQKPGK APKLLIYSASNRYTGVPSRFSGSGSGTDFTLTISSLQPEDIATYY CQQYSNYPTFGQGTKLEIK(SEQ ID NO: 63) |
| AM4B6_La.4 | A51V, N53D | DIQMTQSPSSLSASVGDRVTITCKASQNVGAAVAWYQQKPGK APKLLIYSVSDRYTGVPSRFSGSGSGTDFTLTISSLQPEDIATYY CQQYSNYPTFGQGTKLEIK(SEQ ID NO: 64) |

| | | |
|---|---|---|
| Note: the sequences underlined refer to CDR sequences, and the amino acids bolded refer to mutated amino acids. | | |

### Example 4: Bio-lay interferometry (BLI) for detection of binding affinity

Bio-Lay interferometry (BLI) was used for testing the binding affinity of 4B6 scFv variants to human PD-L1-Fc (Sino Biological, 70110-D02H) antigen. The materials and procedure were shown in Table 8 and Table 9, respectively. The results were shown in Tables 10-12. According to the binding affinity results, 4 light chain variants (L-CDR1-2, L-CDR1-3, L-CDR2-2, L-CDR2-3) and 3 heavy chain variants (H-CDR2-2, H-CDR2-3, H-CDR2-5) were selected for future construction.

**Table 8. Samples and materials used in BLI assay**

| Well Type | Sample ID |
|---|---|
| Baseline | KD buffer |
| Load | PD-L1-FC (3.25 µg/mL) |
| Baseline2 | KD buffer |
| Sample | 4B6 scFvs (5.2 µg/mL) |
| Reference Well | KD buffer |
| Regeneration | Regeneration buffer |
| Neutralization | KD buffer |

**Table 9. BLI assay procedure**

| Step Type | Assay Time |
|---|---|
| Baseline | 60s |
| Loading | 180s |
| Baseline2 | 120s |
| Association | 180s |
| Dissociation | 240s |
| Regeneration | 5s |

**Table 10. Binding affinity ranking of 4B6-L-CDR1 mutation variants**

| **Sample No.** | **Sequence** | **Kₒₙ (×10⁵M⁻¹s⁻¹)** | **K_{off} (×10⁻³M⁻¹s⁻¹)** | **K_{d} (×10⁻⁹M)** | **Fold increase** |
|---|---|---|---|---|---|
| L-CDR1-WT | KASQNVGAAVA (SEQ ID NO: 4) | 0.358 | 1.01 | 28.2 | ---- |
| L-CDR1-1 | KASQNVGAIVA (SEQ ID NO: 7) | 0.412 | 0.285 | 6.91 | 4.08 |
| L-CDR1-2 | KASQNVPAAVA (SEQ ID NO: 8) | 2.71 | 1.54 | 5.68 | 4.96 |
| L-CDR1-3 | KASQNVKGAVA (SEQ ID NO: 9) | 0.911 | 0.253 | 2.78 | 10.14 |

**Table 11. Binding affinity ranking of 4B6-L-CDR2 mutation variants**

| **Sample No.** | **Sequence** | **Kₒₙ (×10⁵M⁻¹s⁻¹)** | **K_{off} (×10⁻³M⁻¹s⁻¹)** | **K_{d} (×10⁻⁹M)** | **Fold increase** |
|---|---|---|---|---|---|
| L-CDR2-WT | SASNRYT (SEQ ID NO: 5) | 0.381 | 1.05 | 27.6 | ---- |
| L-CDR2-1 | SNSHRYT (SEQ ID NO: 10) | 0.468 | 0.377 | 8.06 | 3.42 |
| L-CDR2-2 | SRSVRYT (SEQ ID NO: 11) | 1.62 | 0.540 | 3.34 | 8.26 |
| L-CDR2-3 | SVSDRYT (SEQ ID NO: 12) | 1.24 | 0.543 | 4.37 | 6.32 |

**Table 12. Binding affinity ranking of 4B6-H-CDR2 mutation variants**

| **Sample No.** | **Sequence** | **Kₒₙ (×10⁵M⁻¹s⁻¹)** | **K_{off} (×10⁻³M⁻¹s⁻¹)** | **K_{d} (×10⁻⁹M)** | **Fold increase** |
|---|---|---|---|---|---|
| H-CDR2-WT (G55A) | DINPNNAGTSYNHKFKG (SEQ ID NO: 18) | 0.463 | 1.24 | 26.7 | ---- |
| H-CDR2-1 | DINPNNADTMYNHKFKG (SEQ ID NO: 13) | 1.41 | 0.495 | 3.52 | 7.6 |
| H-CDR2-2 | DINPNNAQTQYNHKFKG (SEQ ID NO: 14) | 1.01 | 0.244 | 2.42 | 11.03 |
| H-CDR2-3 | DINPNNAETLYNHKFKG (SEQ ID NO: 15) | 1.16 | 0.283 | 2.43 | 10.99 |
| H-CDR2-4 | DINPNNGLTSYNHKFKG (SEQ ID NO: 16) | 1.17 | 0.517 | 4.43 | 6.03 |
| H-CDR2-5 | DINPNNAQTVYNHKFKG (SEQ ID NO: 17) | 1.02 | 0.245 | 2.40 | 11.13 |

### Example 5: Construction and expression of AM-4B6-hIgG1-TGFβRII (1-136) fusion protein

The selected heavy chain and light chain variants were cross combined and expressed with hIgG1-TGFβRII (1-136) fusion protein. The TGFβRII (1-136) has an amino acid sequence set forth in SEQ ID NO: 79:

TIPPHVQKSVNNDMIVTDNNGAVKFPQLCKFCDVRFSTCDNQKSCMSNCSITS
ICEKPQEVCVAVWRKNDENITLETVCHDPKLPYHDFILEDAASPKCIMKEKKKPGETF
FMCSCSSDECNDNIIFSEEYNTSNPD

The amino acid sequence of hIgG1 is as follows (SEQ ID NO: 80):

The TGFβRII (1-136) was linked to the carboxyl terminus of the hIgG1 via a peptide linker (G4S)₄G (SEQ ID NO: 68).

The amino acid sequence of hKappa is as follows (SEQ ID NO: 82):

The intact antibody variants were prefixed by AM (affinity matured). The sequence construct of heavy chain and light chain was shown in Table 13 and the design of intact antibody was shown in Table 14. For example, as shown in Table 13 and Table 14, the bi-functional molecule "AM-4B6-hIgG1-TGFβRII variant 1" has a heavy chain and a light chain, where the heavy chain from N terminus to C terminus includes: Hu4B6_Hg.2 -_hIgG1 - (G4S)4G- TGFβRII (1-136); and the light chain from N terminus to C terminus includes: Hu4B6 _La.1 - hKappa. The same nomenclature applies to the other variants in Table 14.

The co-transfection of heavy chain and light chain was carried out using the ExpiFectamine^{™} CHO Reagent (Thermo, A29129) from Invitrogen according to the manufacturer's protocol. The supernatant was harvested on day 10 and purified by affinity chromatography.

**Table 13. The list of AM-4B6-hIgG1-TGFβRII heavy chain and light chain variants**

| Name | Mutation site | Region | Sequence |
|---|---|---|---|
| Hu4B6_Hg.2_hIgG1 (G4S)4G- TGFβRII | - G57Q, S59Q | H-CDR2-2 | DINPNNAQTQYNHKFKG |
| Hu4B6_Hg.3_hIgG1 (G4S)4G- TGFβRII | - G57E, S59L | H-CDR2-3 | DINPNNAETLYNHKFKG |
| Hu4B6_Hg.5_hIgG1 (G4S)4G- TGFβRII | - G57Q, S59V | H-CDR2-5 | DINPNNAQTVYNHKFKG |
| Hu4B6_La.1_hKappa | G30K, A31G | L-CDR1-3 | KASQNVKGAVA |
| Hu4B6_La.2_hKappa | G30P | L-CDR1-2 | KASQNVPAAVA |
| Hu4B6_La.4_hKappa | A51V, N53D | L-CDR2-2 | SRSVRYT |
| Hu4B6_La.6_hKappa | A51R, N53V | L-CDR2-3 | SVSDRYT |

**Table 14. The list of AM-4B6-hIgG1-TGFβRII antibody variants**

| **Sample No.** | **Heavy chain** | **Light chain** |
|---|---|---|
| AM-4B6-hIgG1-TGFβRII variant 1 | Hu4B6_Hg.2 (SEQ ID NO: 58) | Hu4B6_La.1 (SEQ ID NO: 62) |
| AM-4B6-hIgG1-TGFβRII variant 2 | | Hu4B6_La.2 (SEQ ID NO: 63) |
| AM-4B6-hIgG1-TGFβRII variant 3 | | Hu4B6_La.4 (SEQ ID NO: 64) |
| AM-4B6-hIgG1-TGFβRII variant 4 | | Hu4B6_La.6 (SEQ ID NO: 66) |
| AM-4B6-hIgG1-TGFβRII variant 5 | Hu4B6_Hg.3 (SEQ ID NO: 59) | Hu4B6_La.1 (SEQ ID NO: 62) |
| AM-4B6-hIgG1-TGFβRII variant 6 | | Hu4B6_La.2 (SEQ ID NO: 63) |
| AM-4B6-hIgG1-TGFβRII variant 7 | | Hu4B6_La.4 (SEQ ID NO: 64) |
| AM-4B6-hIgG1-TGFβRII variant 8 | | Hu4B6_La.6 (SEQ ID NO: 65) |
| AM-4B6-hIgG1-TGFβRII variant 9 | Hu4B6_Hg.5 (SEQ ID NO: 60) | Hu4B6_La.1 (SEQ ID NO: 62) |
| AM-4B6-hIgG1-TGFβRII variant 10 | | Hu4B6_La.2 (SEQ ID NO: 63) |
| AM-4B6-hIgG1-TGFβRII variant 11 | | Hu4B6_La.4 (SEQ ID NO: 64) |
| AM-4B6-hIgG1-TGFβRII variant 12 | | Hu4B6_La.6 (SEQ ID NO: 65) |

### Example 6: Binding affinity of AM-4B6-hIgG1-TGFβRII variants to hPD-L1

### Binding to hPD-L1 by an ELISA assay

1 µg/mL hPD-L1 (Acro Biosystems, PD1-H5229) antigens was coated to the ELISA plate and left overnight at 4°C. Then 300 µL of 3% (w/v) skim milk was added for blocking at room temperature for 1 h. After 1 h, 100 µL of AM-4B6-hIgG1-TGFβRII variants or original 4B6-hIgG1-TGFβRII at concentrations ranging from 100 nM to 0.006 nM (four-fold serial dilutions) were added with PBS as a negative control, and incubated at room temperature for 1 h. 0.5% PBS+Tween-20 were used for washing for 3 times, and 100 µL HRP-conjugated anti-human Fc antibody (1:20000,Abcam, ab98624) was added After incubation at room temperature for 1 h, mixed TMB substrate reagent (InnoReagents, TMB-S-003) was added and incubated at room temperature for 5 min, and then stopped by adding 0.1M H₂SO₄. OD450 nm was recorded by Microplate Reader.

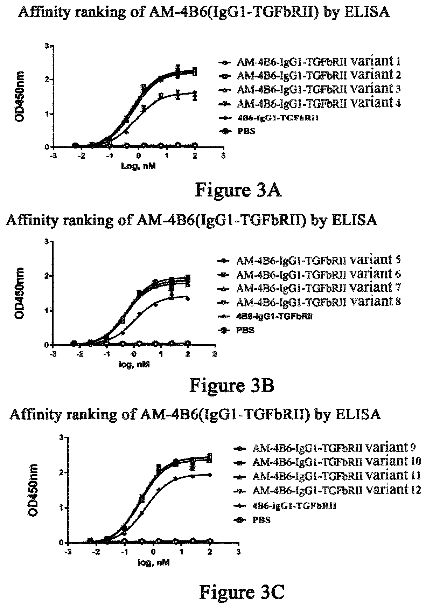

As shown in Figure 3A - Figure 3C, when compared to the original 4B6-hIgG1-TGFβRII, all of the variants had enhanced binding signals and affinities. Given the limited differences between these variants, Surface Plasmon Resonance technology was used for further evaluation of their binding affinity.

### Binding to hPD-L1 by Biacore

4 µg/mL AM-4B6-hIgG1-TGFβRII variants or original 4B6-hIgG1-TGFβRII were immobilized on the surface of S series Protein A chip. The human PD-L1 was diluted to an appropriated concentration gradient (0 nM, 1.875 nM, 3.75 nM, 7.5 nM, 15 nM, 30 nM, 60 nM) and injected into the sample channel of Biacore 2000. The results are shown in Table 15. The binding affinity of AM-4B6-hIgG1-TGFβRII variant 7 to human PD-L1 was improved about 15 folds than that of original 4B6-hIgG1-TGFβRII.

**Table 15. Binding affinity of AM-4B6-hIgG1-TGFβRII to hPD-L1 by Biacore**

| **Analyte** | **Sample** NO. | **Sample name** | **ka(1/Ms)** | **kd(1/s)** | **KD(M)** |
|---|---|---|---|---|---|
| **PD-L1** his | 1 | AM-4B6-hIgG1-TGFβRII variant 5 | 2.060E+5 | 1.487E-4 | 7.218E-10 |
| | 2 | AM-4B6-hIgG1-TGFβRII variant 7 | 3.815E+5 | 1.479E-4 | 3.876E-10 |
| | 3 | AM-4B6-hIgG1-TGFβRII variant 2 | 3.231E+5 | 1.676E-4 | 5.189E-10 |
| | 4 | AM-4B6-hIgG1-TGFβRII variant 10 | 2.444E+5 | 1.476E-4 | 6.039E-10 |
| | 5 | AM-4B6-hIgG1-TGFβRII variant 11 | 2.688E+5 | 1.366E-4 | 5.083E-10 |
| | 6 | 4B6-hIgG1-TGFβRII | 2.107E+5 | 0.001156 | 5.489E-9 |

### Binding to PD-L1 expressing on cell surface by a FACS assay

293T-PD-L1-CD3L cell was generated by MabSpace Bioscience for characterization of PD-L1 antibodies. The cell was transfected with both human PD-L1 and anti-CD3 scFv. AM-4B6-hIgG1-TGFβRII variants or original 4B6-hIgG1-TGFβRII were serially diluted (5-fold dilutions) to obtain 8 concentrations in dilution buffer (PBS with 2%BSA). 293T-PD-L1-CD3L cells were harvested and centrifuged. They were resuspended in PBS with a density of 2×10⁶ cells/mL and then added to the plate with 100 ul per well. After centrifugation and removal of supernatant, the diluted antibodies were added to the plate and incubated in 4°C for 30min. After washing twice with dilution buffer, PE conjugated donkey anti-human IgG(H+L)(Jacksonimmuno, 709-116-149) was added to the plate and incubated in 4°C for 30 min. After washing, cells were resuspended in 200 µL PBS and analyzed by flow cytometry.

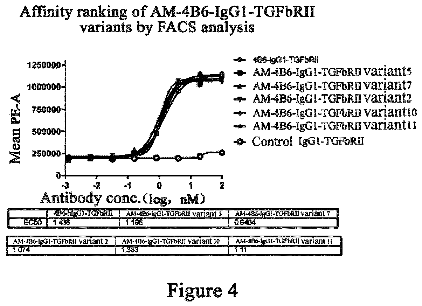

As shown in Figure 4, these 5 variants bound to PD-L1 expressed on surface of 293T-PD-L1-CD3L cells with a similar EC50. Variant 7 had a slight lower EC50 than others, which was consistent with the binding affinity results measured by Biacore.

### Example 7: PD-1/PD-L1 blockade activity of AM-4B6-hIgG1-TGFbRII variants

### PD-1/PD-L1 and B7-1/PD-L1 blockade by an ELISA assay

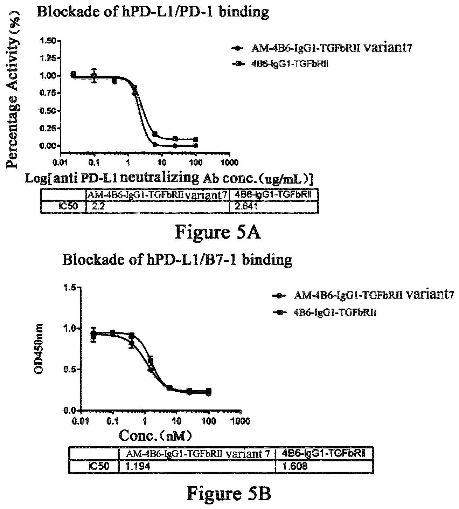

To test ligand/receptor blocking activity, 0.5 µg/mL hPD-L1-Fc antigen was coated to the ELISA plate and left overnight at 4°C. 300 µL blocking buffer was added for blocking at room temperature for 1 h. After 1 h, 50 µL of AM-4B6-hIgG1-TGFβRII variant 7 or original 4B6-hIgG1-TGFβRII at concentrations ranging from 100 nM to 0.024 nM (four-fold serial dilutions) with 50 µL PD-L1-his, concentration of which is 1 µg/mL, were added and incubated at room temperature for 1 h. 0.5% PBS+Tween-20 were used for washing for 3 times, and 100 µL HRP-conjugated streptavidin (1:5000, Abcam, cat#ab7403) was added. After incubation at room temperature for 1.5 h, mixed TMB substrate reagent was added and incubated at room temperature for 5 min, then stopped by adding 0.1 M H₂SO₄. OD450nm was recorded by Microplate Reader. As shown in Figure 5A - Figure 5B, both samples could block PD-L1/PD-1 or PD-L1/B7-1 while AM-4B6-hIgG1-TGFβRII variant 7 with a lower IC50, indicating variant 7 has the improved activity.

### PD-1/PD-L1 blockade by a cell-based assay

In this assay, 293T-PD-L1-CD3L cell expresses PD-L1 and anti-CD3 scFv and Jurkat-NFAT-Luc-PD1 cell expresses PD-1 and carrying NFAT signal which can be activated with CD3 stimulation. NFAT activation leads to downstream luciferase gene transcription and expression, which can be detected by its substrate. The two cells were generated by MabSpace Bioscience.

Briefly, 293T-PD-L1-CD3L cells were harvested and resuspended at a density of 2×10⁶ cells/mL. 20 µL cells per well were added into half well plate. AM-4B6-hIgG1-TGFβRII variants or original 4B6-hIgG1-TGFβRII were serially diluted (3-fold dilutions) to obtain 8 concentrations in RPMI medium with 2%FBS. 20 µL antibodies per well were added into half well plate, and the plate was incubated at 37°C, 5% CO₂ for 30min. Jurkat-NFAT-Luc-PD1 cells were harvested and resuspended at a density of 4×10⁶ cells/mL in RPMI medium with 2%FBS. Finally, 20 µL cells per well with 5 ng/mL TGF-beta (R&D, 240-B-010) were added into half well plate and incubated in 37°C, 5% CO₂ for 5 h. 60 µL OneGlo detection reagent (Promega, E6120) was added to each well and incubated at room temperature for 5 minutes. The luminescent signal was read by Microplate Reader. The data was analyzed by GraphPad Prism.

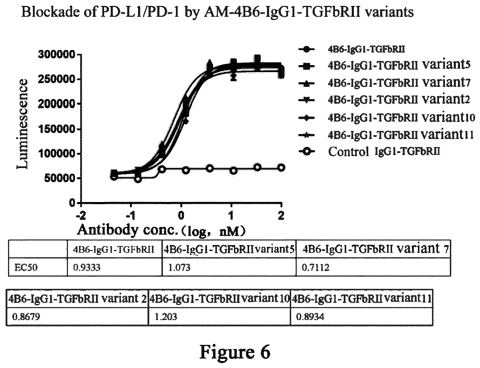

As shown in Figure 6, consistent with the previous ELISA results, variant 7 had the most potent blockade activity in this cell-based assay as compared with the other variants. Therefore, the 4B6 Fab part of AM-4B6-hIgG1-TGFβRII variant 7 was abbreviated as AM4B6, and AM4B6-hIgG1-TGFβRII fusion protein was further evaluated in the following experiments.

### Example 8: Generation and characterization of AM4B6-hIgG1-TGFβRII' in vitro

### AM4B6-hIgG1-TGFβRII cloning and expression

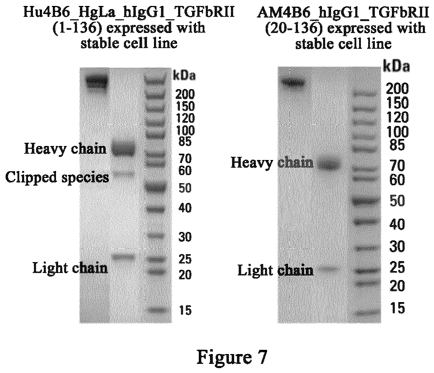

It was reported that the truncated TGFβRII ECD_20-136 was soluble and retained the ability to bind TGFβ1 (Kim-Ming Lo, et al, US9676863 B2, 2017; Christian C., et al), Protein Expression and Purification, 2000, 20: 98-104). Next, we replaced the full length of extracellular domain of TGFβRII_1-136 with the truncated one and evaluated the developability and stability. The SDS-PAGE results from the stable cell line showed that the protein expression is good for both truncated and full-length TGFβRII ECD, but the protein stability is much better for truncated TGFβRII ECD_20-136 than full length TGFβRII ECD (Figure 7). The sequences of the truncated TGFβRII ECD_20-136 are as below:
Sequence of stable TGF-β trap, TGF-βRII extracellular domain (20-136) 
GAVKFPQLCKFCDVRFSTCDNQKSCMSNCSITSICEKPQEVCVAVWRKNDENI
TLETVCHDPKLPYHDFILEDAASPKCIMKEKKKPGETFFMCSCSSDECNDNIIFSEEYN
TSNPD
 (SEQ ID NO : 66)

The bi-functional molecules comprising the truncated TGF-βRII (i.e. TGF-βRII (20-136), SEQ ID NO: 66) was used in this example and also in Examples 9-11. Such bi-functional molecules' names were indicated with TGFβRII', to distinguish from the TGF-βRII (1-136). For example, AM4B6-hIgG1-TGFβRII' indicates a molecule having TGF-βRII (20-136).

### Binding to PD-L1 from various species by an ELISA assay

1 µg/mL human PD-L1 (Acro Biosystems, PD1-H5229) or cyno PD-L1 antigens were coated to the ELISA plate and left overnight at 4°C. Then 300 µL of 3%(w/v) skim milk was added for blocking at room temperature for 1 h. After 1 h, 100 µL of AM4B6-hIgG1-TGFβRII' or control hIgG1-TGFβRII' at concentrations ranging from 100 nM to 0.02 nM (four-fold serial dilutions) were added, and incubated at room temperature for 1 h. 0.5% PBS+Tween-20 were used for washing for 3 times, and 100 µL HRP-conjugated anti-human Fc antibody (1:20000,Abcam, ab98624) was added, After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader.

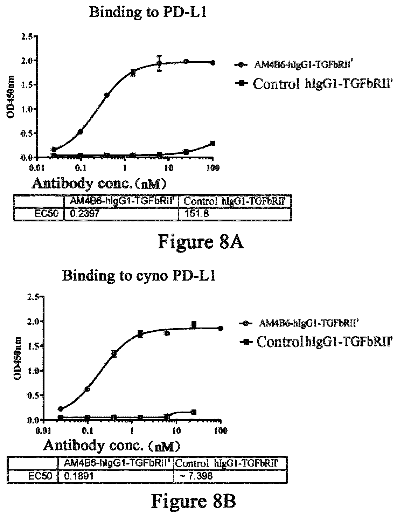

As shown in Figure 8A and Figure 8B, AM4B6-hIgG1-TGFβRII' cross-reacted with cyno PD-L1 with a similar EC50 to that of human PD-L1.

### Binding to TGFβ1, TGFβ2 and TGFβ3 from various species by an ELISA

According to the sequences of TGFβ1, TGFβ2 and TGFβ3 from 4 common species: human, cynomolgus, mouse and rat, which were published on the Uniport website (https://www.uniprot.org/), TGFβ□members are quite conservative. The sequences of human TGFβ1 and cynomolgus TGFβ1 are identical; mouse TGFβ1 and rat TGFβ1 are identical; human TGFβ2 and cynomolgus TGFβ2 are identical; mouse TGFβ2 and rat TGFβ2 are identical; human TGFβ3, cynomolgus TGFβ3 and mouse TGFβ3 are identical.

For TGFβ1 and TGFβ3, the procedure is as below: 0.5 µg/mL human TGFβ1 (Sino Biological, 10804-HNAC) or Mouse TGFβ1 (Novoprotein, CK33) or Human TGFβ3 (Genscript, Z03430) or Rat TGFβ3 (Novoprotein, CJ44) antigens were coated to the ELISA plate and left overnight at 4°C. Then 300 µL of 3%(w/v) skim milk was added for blocking at room temperature for 1 h. After 1 h, 100 µL of AM4B6-hIgG1-TGFβRII' or control hIgG1-TGFβRII' at concentrations ranging from 100 nM to 0.006 nM (four-fold serial dilutions) were added, and incubated at room temperature for 1 h. 0.5% PBS+Tween-20 were used for washing for 3 times, and 100 µL HRP-conjugated anti-human Fc antibody (1:20000, Abcam, ab98624) was added, After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader.

For TGFβ2, the test procedure is different: 2 µg/mL AM4B6-hIgG1-TGFβRII' or control hIgG1-TGFβRII' were coated to the ELISA plate and left overnight at 4°C. Then 300 µL of 3%(w/v) skim milk was added for blocking at room temperature for 1 h. After 1 h, 100 µL of human TGFβ2 or mouse TGFβ2 at concentrations ranging from 39.4 nM to 0.3 nM (two-fold serial dilutions) were added, and incubated at room temperature for 1 h. 0.5% PBS+Tween-20 were used for washing for 3 times, and 100 µL TGFβ2 Biotinylated antibody (1:10000, R&D, BAF302) was added. After incubation at room temperature for 1 h and washing, 100 µL HRP-streptavidin (1:5000, Abcam, ab7403) was added and the plate was incubated at room temperature for 1 h. After washing, mixed TMB substrate reagent (InnoReagents, TMB-S-003) was added and incubated at room temperature for 5 min and stopped by adding 0.1M H₂SO₄. OD450 nm was recorded by Microplate Reader.

Results were summarized in Table 16. For binding affinity to TGFβ1, the EC50 values were quite similar among different species. Furthermore, the binding affinity to TGFβ1 and TGFβ3 was significantly higher than TGFβ2, indicating the blocking activity to TGFβ1 and TGFβ3 may be more potent than TGFβ2.

**Table 16. Binding of AM4B6-hIgG1-TGFβRII' to TGFβ1, TGFβ2 and TGFβ3**

| Binding to TGFβ1, TGFβ2 and TGFβ3 from various species by ELISA (EC₅₀, nM) | | | | | |
|---|---|---|---|---|---|
| TGFβ1 | | TGFβ2 | | TGFβ3 | |
| Human | Mouse | **Human** | Mouse | Human | Rat |
| 0.748 | 0.800 | 8.459 | 8.884 | 5.257 | 3.733 |

### Binding to other members within the B7 family or TGFβ superfamily by an ELISA assay

For B7 family, 0.5 µg/mL hPD-L1 (Acro Biosystems, PD1-H5229) or hPD-L2 or B7-2 or B7-1 or B7-H2 or B7-H3 or B7-H4 or VISTA were coated to the ELISA plate and left overnight at 4°C. For TGFβ superfamily, 0.5 µg/mL human Activin A, BMP-2, LAP or TGFβ1 were coated at 4°C overnight. Then 300 µL of 3%(w/v) skim milk was added for blocking at room temperature for 1 h. After 1 h, 100 µL of AM4B6-hIgG1-TGFβRII' at concentrations ranging from 100 nM to 0.006 nM (serial diluted) were added, and incubated at room temperature for 1 h. 0.5% PBS+Tween-20 were used for washing for 3 times, and 100 µL HRP-conjugated anti-human Fc antibody (1:20000,Abcam, ab98624) was added, After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader.

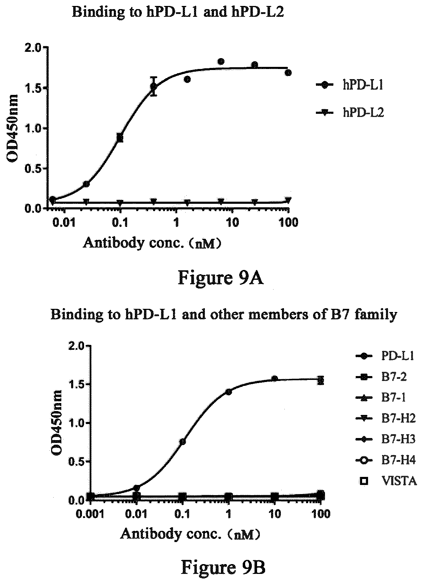

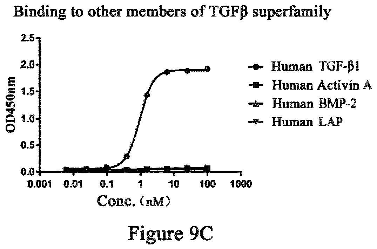

As shown in Figure 9A - Figure 9B, AM4B6-hIgG1-TGFβRII' specifically bound to PD-L1 rather than the other antigens that also belong to the B7 family. As shown in Figure 9C, AM4B6-hIgG1-TGFβRII' specifically bound to TGF-β1 rather than the other antigens that also belong to the TGFβ superfamily.

### Binding to PD-L1 expressing cells of AM4B6-hIgG1-TGFβRII'

MC38/hPD-L1 was generated by deleting mPD-L1 via CRISPR-Cas9 system, followed by transduction of hPD-L1 using lenti-virus. This cell line was a courtesy of Professor Qin Xiaofeng's laboratory at the Center of Systems Medicine, Chinese Academy of Medical Sciences Suzhou Institute of Systems Medicine (Huang, Anfei, et al. Scientific Reports 7 (2017): 42687.). MC-38/hPD-L1 cells were cultured in RPMI1640+10%FBS. EMT-6/hPD-L1, is a mouse breast cancer cell line that stably expresses transfected human PD-L1 gene. EMT-6/hPD-L1 cells were cultured in Waymouth's (1×) MB752/1 +15% FBS. NCI-H460 cells were purchased from COBIOER Ltd. It's a human lung epithelial tumor cell line with PD-L1 expression. NCI-H460 cells were cultured in RPMI1640+10%FBS. NCI-H292 cells were purchased from COBIOER Ltd. It's a human lung epithelial tumor cell line with PD-L1 expression. NCI-H292 cells were cultured in RPMI1640+10%FBS+ 1nM sodium pyruvate solution. The protocol of FACS analysis was the same with Example 6 section 3.

Human or cynomolgus PBMC (TPCS, Cat#PB025C) was recovered from liquid nitrogen and resuspended in RPMI1640 with 10%FBS. 5µg/mL PHA (Sigma, Cat# L8902) was added to stimulate PBMC activation and cells were cultured for 3 days. Activated PBMC were harvested and centrifuged and resuspended in PBS with density of 2×10⁶ cells/mL and added to the plate with 100 ul per well. AM4B6-hIgG1-TGFβRII' or AM4B6 or control hIgG1-TGFβRII' were serially diluted (5-fold dilutions) to obtain 10 concentrations in dilution buffer (PBS with 2%BSA). After centrifugation and removal of the supernatant in the plate, the diluted antibodies were added to the plate with the activated PBMC and incubated in 4°C for 1 hour. After washing twice with dilution buffer, Alexa488-labeled mouse anti-human CD3 (Biolegend, Cat#300320) and APC-labeled anti-human IgG secondary antibody (BD, Cat#550931) were added and incubate at 4°C for 30 mins. After washing, cells were resuspended in 150 µL PBS and analyzed by flow cytometry.

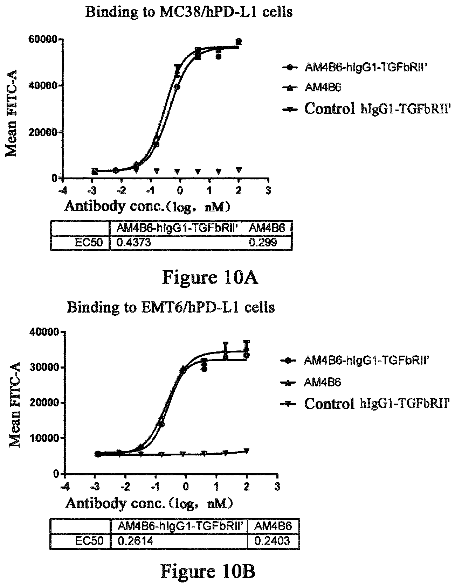

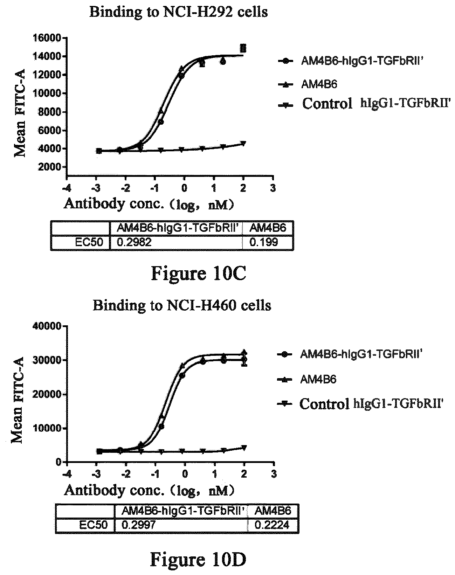

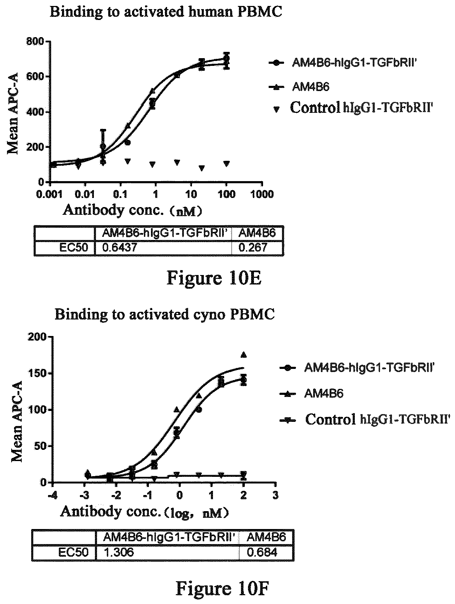

As shown in Figure 10A - Figure 10F, AM4B6-hIgG1-TGFβRII' could bind to PD-L1 expressed on these cancer cell lines and the activated human or cynomolgus T cells with the similar affinity to AM4B6 mAb alone.

### Binding to activated human T cells

Human PBMC (TPCS, Cat#PB025C) was recovered from liquid nitrogen and resuspended in RPMI1640 with 10%FBS. 5µg/mL PHA (Sigma, Cat# L8902) was added to stimulate PBMC activation and cells were cultured for 3 days. Activated PBMC were harvested and centrifuged and resuspended in PBS with density of 2×10⁶ cells/mL and added to the plate with 100 ul per well. AM4B6-hIgG1-TGFβRII' or AM4B6 or control hIgG1-TGFβRII' were serially diluted (5-fold dilutions) to obtain 10 concentrations in dilution buffer (PBS with 2%BSA). After centrifugation and removal of the supernatant in the plate, the diluted antibodies were added to the plate with the activated PBMC and incubated in 4°C for 1 hour. After washing twice with dilution buffer, Alexa488-labeled mouse anti-human CD3 (Biolegend, Cat#300320) and APC-labeled anti-human IgG secondary antibody (BD, Cat#550931) were added and incubate at 4°C for 30 mins. After washing, cells were resuspended in 150 µL PBS and analyzed by flow cytometry.

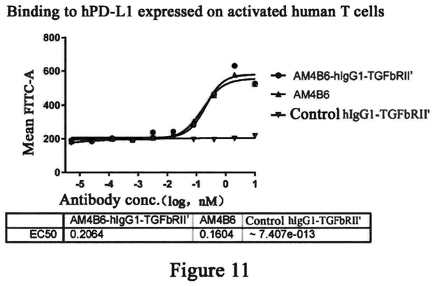

As shown in Figure 11, AM4B6-hIgG1-TGFβRII' could bind to PD-L1 expressed on the activated human T cells.

### Blockade of hPD-L1/hPD-1 and cynoPD-L1/cynoPD-1 by an ELISA assay

0.5 µg/mL hPD-L1-Fc or 0.5 µg/mL cynoPD-L1-Fc was coated to the ELISA plate and left overnight at 4°C. 300 µL of 3%(w/v) skim milk was added for blocking at room temperature for 1 h. After 1 h, 100 µL of AM4B6-hIgG1-TGFβRII' or AM4B6 at concentrations ranging from 100 nM to 0.02 nM (four-fold serial dilutions) with 0.5 µg/mL hPD-1-Fc-biotin or cyno PD-1-Fc-biotin were added and incubated at room temperature for 1 h. 0.5% PBS+Tween-20 were used for washing for 3 times, and 100 µL HRP-conjugated streptavidin (1:5000) was added. After incubation at room temperature for 1 h, mixed TMB substrate reagent was added and incubated at room temperature for 5 min, then stopped by adding 0.1 M H₂SO₄. OD450nm was recorded by Microplate Reader.

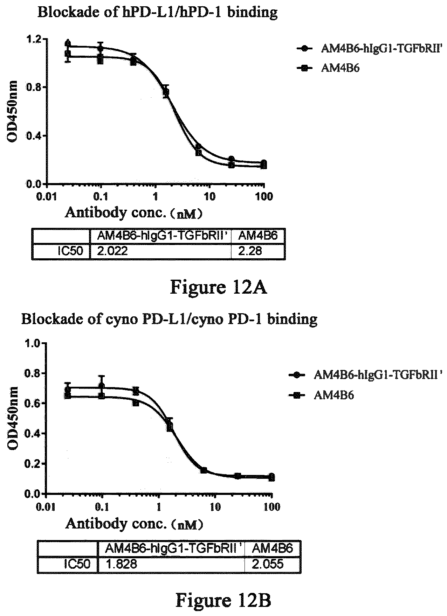

As shown in Figure 12A - Figure 12B, AM4B6-hIgG1-TGFβRII' could also completely block cyno PD-L1/cyno PD-1 with a similar IC50 to that of blocking human PD-L1/human PD-1.

### Simultaneously binding to hPD-L1 and hTGFβ1

0.5 µg/mL hTGFβ-1 was coated to the ELISA plate and left overnight at 4°C. Then 300 µL of blocking buffer was added for blocking at room temperature for 1 h. After 1 h, 100 µL of AM4B6-hIgG1-TGFβRII' or AM4B6 or control hIgG1-TGFβRII' at concentrations ranging from 100 nM to 0.02 nM (four-fold serial dilutions) were added, and incubated at room temperature for 1 h. 0.5% PBS+Tween-20 were used for washing for 3 times, and then 0.5 µg/mL hPD-L1-biotin was added into each well. 1h later, 100 µL HRP-conjugated streptavidin (1:5000) was added. After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader.

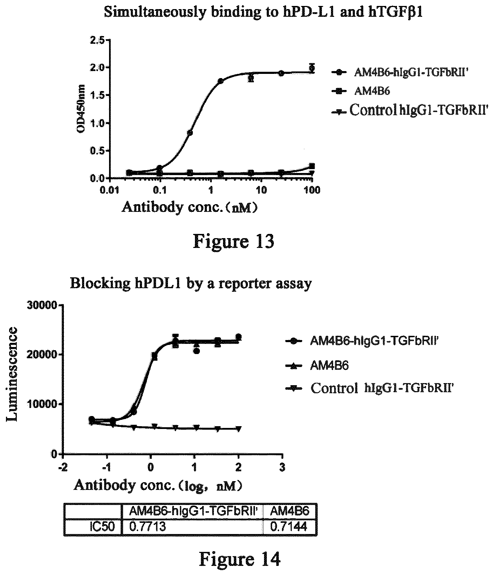

As shown in Figure 13, AM4B6-hIgG1-TGFβRII', which was composed of anti-PD-L1 antibody AM4B6 and TGFβRII', could bind the two targets at the same time, indicating its bispecific or bifunctional character.

### Blockade of hPD-L1/hPD-1 by a reporter cell assay

Protocol was the same as Example 7 section 2. As shown in Figure 14, AM4B6-hIgG1-TGFβRII could block the inhibition effect of PD-L1/PD-1 and subsequently reverse the signaling activation, same as AM4B6 mAb alone.

### Blockade of TGFβ1 signaling by a reporter cell assay

TGFβ reporter HEK-293 cell line was purchased from Genomeditech (Cat: GM-C05346) and cultured in DMEM media containing 10% FBS, 4 µg/mL blasticidin, 400 µg/mL neomycin, 125µg/mL hygromycin, 0.75 µg/mL puromycin, and 1% Pen/Strep in 37 °C incubator with 5% carbon dioxide.

Cells were collected in the log-growth phase and resuspended in DMEM media and planted in 96-well plate in density of 2×10^4 cells/100 µL per well. After cells were cultured overnight, the medium was replaced with 75 µL of culture media containing 10 ng/mL of human TGFβ1. 75 µL of AM4B6-hIgG1-TGFβRII' or AM4B6 were added at the final concentration of ranging from 100 nM to 0.02 nM (three-fold serial dilutions). The plate was incubated at 37 °C incubator for 16 hours. The ONE-GloTM luciferase assay system was added at 150 µL/well and after incubation at room temperature for 10 minutes, the plate was read with the microplate reader.

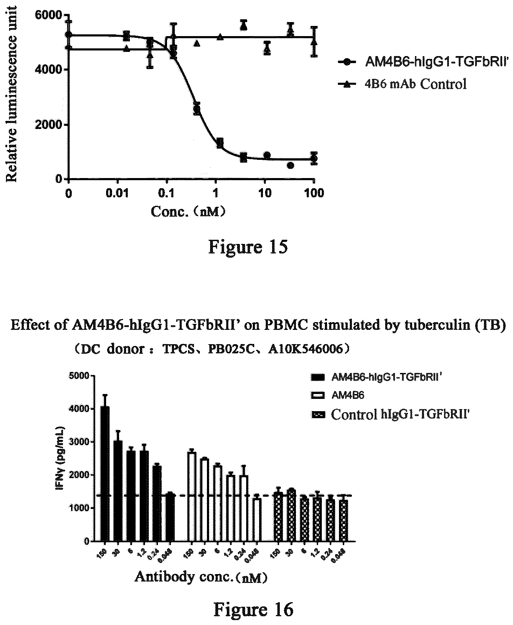

As shown in Figure 15, AM4B6-hIgG1-TGFβRII' displayed a potent blocking activity on TGFβ1 signaling with an IC50 of 0.35nM, while AM4B6 mAb alone had no blocking activity, indicating the blocking activity is TGFβ1 specific.

### Effect of AM4B6-hIgG1-TGFβRII' on IFN-y release of PBMC stimulated by tuberculin (TB)

Human PBMC was recovered from liquid nitrogen and resuspend the cells at density of 2×10^6/mL. Add TB to a final concentration of 1.33µg/mL; cultured at 37°C for 5 days. On the sixth day, the induced PBMC were collected and centrifuged, washed once with PBS, resuspended in fresh medium, adjusted to a density of 1×10^6/mL, and seeded into a 96-well cell plate, 180µL/well. Add diluted antibodies to the 96-well cell culture plate, 20µL/well. Control group and blank group were added with 20µL PBS. Cell culture plates were incubated at 37°C for 3 days in a 5% CO₂ incubator. At the end of incubation, the cell supernatant was diluted 10-fold, and the secretion level of IFNγ was detected with an IFN-γ ELISA detection kit (R&D, DY285B).

As shown in Figure 16, AM4B6-hIgG1-TGFβRII' induced a significantly higher level of IFN-γ release than AM4B6 mAb alone, indicating its activation activity is more potent due to its bispecific binding and blocking activity.

### ADCC/CDC activity of AM4B6-hIgG1-TGFβRII'

For ADCC assay, the effector cell: Jurkat-NFAT Luc-FcγRIIIa-158V cell line was constructed by Mabspace Biosciences (Suzhou) Co., Limited. The target cell: HEK-293T-hPD-L1 cells (purchased from Crown Biosciences Inc., Cat: 2005).

HEK-293T-hPD-L1 cells were added to the cell culture plates at 10,000 cells/12.5 µL per well. AM4B6-hIgG1-TGFβRII' dilutions at final concentrations ranging from 200nM to 0.003nM were then added at 12.5 µL/well. The plates were then placed in the incubator at 37 °C to allow the antibody and cells incubation for 30 minutes. Then Jurkat-NFAT Luc-FcγRIIIa-158V cells were added to the wells at 60,000 cells/25 µL per well. The plates were then placed in the incubator at 37 °C for 6 hours. The ONE-GloTM luciferase assay system was added at 50 µL/well and after incubation at room temperature for 10 minutes, the plate was read with the microplate reader.

For CDC assay, the target cell is also HEK-293T-hPD-L1 cells. HEK-293T-hPD-L1 cells were added to the cell culture plates at 10,000 cells/25 per well. AM4B6-hIgG1-TGFβRII' dilutions at final concentrations ranging from 200nM to 0.3nM were then added at 12.5 µL/well. The plates were then placed in the incubator at 37 °C to allow the antibody and cells incubation for 30 minutes. The HEK-293T-hPD-L1 cells were treated with 40% complements at 50 µL/well (final concentration is 20%), then incubated at 37 °C for 80 min. The ONE-GloTM luciferase assay system was added at 100 µL/well and after incubation at room temperature for 10 minutes, the plate was read with the microplate reader.

The results suggested that AM4B6-hIgG1-TGFβRII' had neither ADCC nor CDC activity on HEK-293T-hPD-L1 cells (data not shown).

### Example 9: Efficacy of AM4B6-hIgG1-TGFβRII' in vivo

### MC38-hPD-L1 tumor model on C57BL/6 mice

Endogenous mouse PD-L1 in mouse tumor cell line MC38 (ATCC) was knocked out using a highly efficient CRISPR/Cas9 system we recently developed. Briefly, sgRNA targeting the first coding exon of mouse PD-L1 gene was designed, and the cells were transfected by hit-and-run CRISPR/Cas9+sgRNA constructs and selected for knock out cells. The cells with complete knock out of endogenous mouse PD-L1 were identified by FACS analysis for cell surface expression of PD-L1 in steady state or stimulated by interferon gamma, and subsequently verified by TA cloning and sequencing of the targeted genomic region. To generate human PD-L1 replacement cell line, the coding sequence of human PD-L1 cDNA was cloned into a FG12 derived lentiviral vector. The mouse PD-L1 knock out cells were then infected with the human PD-L1 expressing lentivirus, and a high level and stable expression of human PD-L1 in the established cell line was confirmed by FACS analysis. This engineered cells of MC38 was named as MC38-hPD-L1.

MC38-hPD-L1 cells were maintained in vitro as a monolayer culture in RPMI1640 medium supplemented with 10% heat inactivated fetal bovine serum at 37°C in an atmosphere with 5% CO₂ in air. The tumor cells growing in an exponential growth phase were harvested and counted for tumor inoculation. Each female SPF grade C57BL/6 mouse was inoculated with mixed 2×10⁶ MC38-hPD-L1 cells with 50% matri-gel. When the tumor size around 90 mM^3, tumor bearing mice were selected and randomized to 5 groups (n=8). Animals were treated with 2.5mg/kg isotype control, 3mg/kg isotype control-TGFβRII', 2.5mg/kg AM4B6, 0.3mg/kg AM4B6-hIgG1-TGFβRII', 1mg/kg AM4B6-hIgG1-TGFβRII' and 3mg/kg AM4B6-hIgG1-TGFβRII'. All the antibodies were administrated twice a week for 4 weeks by i.p. injection. Tumor size was measured twice or triple times a week in two dimensions using a caliper (INSIZE) and the volume was expressed in mM^3 using the formula: V=0.5 a×b^2 where a and b ate the long and shirt diameters of the tumor, respectively. Results were analyzed using Prism GraphPad and expressed as mean±S.E.M. Comparisons between two groups were made by T-test, and the difference is considered significant if p is *<0.05 and **<0.01.

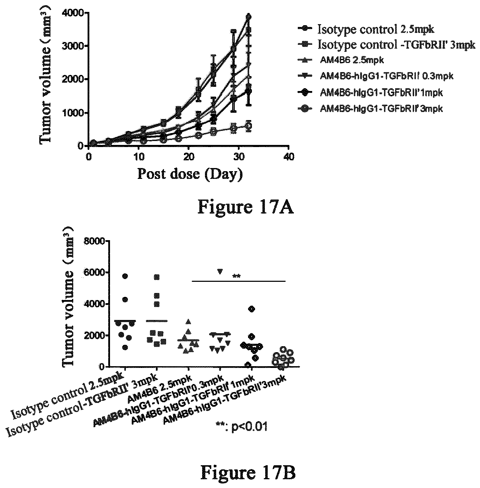

As shown in Figure 17A- Figure 17B, 3mg/kg isotype control-TGFβRII' didn't inhibit tumor growth at all, indicating TGFβRII' alone had little efficacy. 2.5mg/kg AM4B6 had only partial inhibition effect, similar to that of 0.3mg/kg AM4B6-hIgG1-TGFβRII', which seemed not sufficient to control tumor growth. With the increase of dosage, the tumor volumes were getting smaller and smaller. 3mg/kg AM4B6-hIgG1-TGFβRII' almost completely stopped the tumor growth, with 84% TGI (Table 17).

**Table 17. Tumor Growth Inhibition (TGI) of antibodies in MC38-hPD-L1 tumor model on Day 32 (mean±S.E.M., n=8)**

| Treatment | Tumor size (±SEM, mM^3) | TGI (%) | p value vs. Isotype Control |
|---|---|---|---|
| Isotype Control 2.5 mg/kg | 3877.77±712.67 | | |
| Isotype control-TGFβRII' 3 mg/kg | 3489.60±880.53 | 10.01 | 0.6713 |
| AM4B6 2.5 mg/kg | 2105.51±443.56 | 45.70 | 0.0183 |
| AM4B6-hIgG1-TGFβRII' 0.3 mg/kg | 2411.61±742.60 | 37.81 | 0.0931 |
| AM4B6-hIgG1-TGFβRII' 1 mg/kg | 1646.12±517.06 | 57.55 | 0.0063 |
| AM4B6-hIgG1-TGFβRII' 3 mg/kg | 601.31±197.10 | 84.49 | 0.00006 |

### H460 tumor and human PBMC co-inoculated model on NOD-SCID mice

H460 cell was purchased from COBIOER Ltd. H460 cells were maintained in vitro as a monolayer culture in RPMI1640 medium supplemented with 10% heat inactivated fetal bovine serum at 37°C in an atmosphere with 5% CO₂ in air. The tumor cells growing in an exponential growth phase were harvested and counted for tumor inoculation. Each female SPF grade NOD-SCID mouse was inoculated with mixed 3×10^6 H460 cells (Model group) or 3×10^6 H460 cells mixed with 1.5×10^6 human PBMC. All the cell suspension was mixed well with Matrigel as 1:1 ratio before inoculation. Approximately 4 hours after inoculations, animals were grouped to 7 groups (n=10) and dosed differently. Animals were treated with either 16.7mg/kg control hIgG1, or 20mg/kg control hIgG1-TGFβRII', or 16.7mg/kg AM4B6, or 5mg/kg AM4B6-hIgG1-TGFβRII', or 10mg/kg AM4B6-hIgG1-TGFβRII', or 20mg/kg AM4B6-hIgG1-TGFβRII'. Model group was treated with nothing. All the antibodies were administrated twice a week for 5 weeks by i.p. injection. Tumor size was measured twice or triple times a week in two dimensions using a caliper (INSIZE) and the volume was expressed in mM^3 using the formula: V=0.5 a×b^2 where a and b are the long and short diameters of the tumor, respectively. Results were analyzed using GraphPad Prism and expressed as mean±S.E.M. Comparisons between two groups were made by T-test, and the difference is considered significant if p is *<0.05 and **<0.01.

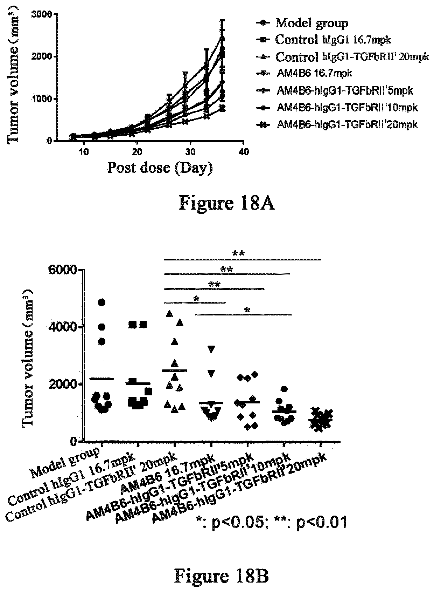

As shown in Figure 18A - Figure 18B, 20mg/kg isotype control-TGFβRII' didn't inhibit tumor growth at all. 16.7mg/kg AM4B6 had only partial inhibition effect, while 5mg/kg AM4B6-hIgG1-TGFβRII' already had significantly better tumor inhibition, indicating TGFβRII fusion increased the anti-tumor efficacy of AM4B6 mAb alone. In addition, an obvious dose-response of AM4B6-hIgG1-TGFβRII' was observed in this model, again suggesting the efficacy is depending on AM4B6-hIgG1-TGFβRII'.

### EMT6-hPD-L1 tumor model on C57BL/6 mice

Endogenous mouse PD-L1 in mouse tumor cell line EMT6 (ATCC) was knocked out and human PD-L1 was knocked in the cells, the engineered cells of EMT6 were named as EMT6-hPD-L1.

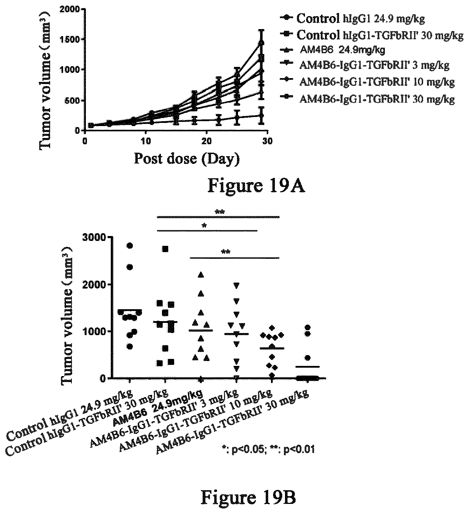

Mice were subcutaneously inoculated with EMT6/hPD-L1 tumor cells and randomly divided into 7 groups thereafter according to the tumor volume with 10 mice per group. After grouping, animals from group 1 to 7 were administered with 24.9 mg/kg Control hIgG1, 30 mg/kg control hIgG1-TGFβRII', 24.9 mg/kg AM4B6, 3 mg/kg AM4B6-hIgG1-TGFβRII', 10 mg/kg AM4B6-hIgG1-TGFβRII' or 30 mg/kg AM4B6-hIgG1-TGFβRII' respectively, by intraperitoneal injection twice a week for 4 weeks. The tumor volume and body weight of tumor bearing mice were observed twice weekly. As shown in Figure 19 A - Figure 19B, AM4B6-hIgG1-TGFβRII' dose-dependently inhibited tumor growth with TGI of 21.43%, 46.83% and 79.39% at 3, 10 and 30 mg/kg respectively on Day 29 post dose. At equal molar quantity, the anti-tumor activity of AM4B6-hIgG1-TGFβRII' at 30 mg/kg was more pronounced than AM4B6 at 24.9 mg/kg, in that group, TGI was 29.67% on Day 29.

### Example 10: Impact of AM4B6-hIgG1-TGFβRII' treatment on tumor infiltrating lymphocytes (TIL) in MC38-hPD-L1 tumor model

MC38-hPD-L1 tumor cells were cultured and inoculated following the same process of Example 9. When the tumor size was 250-300 mM^3, tumor bearing mice were selected and randomized to 4 groups (n=6). Animals were treated with PBS, or 3mg/kg isotype control-TGFβRII', or 2.5mg/kg AM4B6, or 3mg/kg AM4B6-hIgG1-TGFβRII'. All the antibodies were administrated twice a week for 1 or 2 weeks by i.v. injection. Tumors were harvested 24 hours after the 2^{nd} dosing and 24 hours after the 4^{th} dosing, respectively, followed by dissociation with gentle MACS Dissociator (Miltenyi Biotec, 130-093-235) and digested with mouse Tumor Dissociation Kit (Miltenyi Biotec, 130-096-730) for 40 min at 37°C. Isolated single tumor cell suspension of each group was analyzed for TIL sub-population percentage using FACS after being stained by PE anti-mouse CD45 (BD bioscience, Cat#553081), APC anti-mouse CD8a (Biolegend, Cat#100712), APC anti-mouse NK1.1 (Biolegend, Cat#108710), FITC anti-mouse Granzyme B (Biolegend, Cat#515403) and FITC anti-mouse IFN gamma (Invitrogen, Cat#11-7311-82), shown in Table 18 and Table 19.

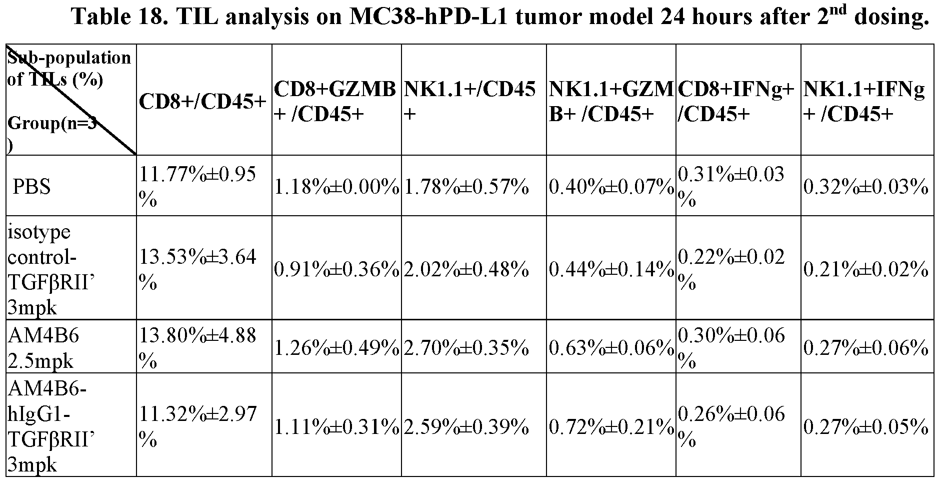

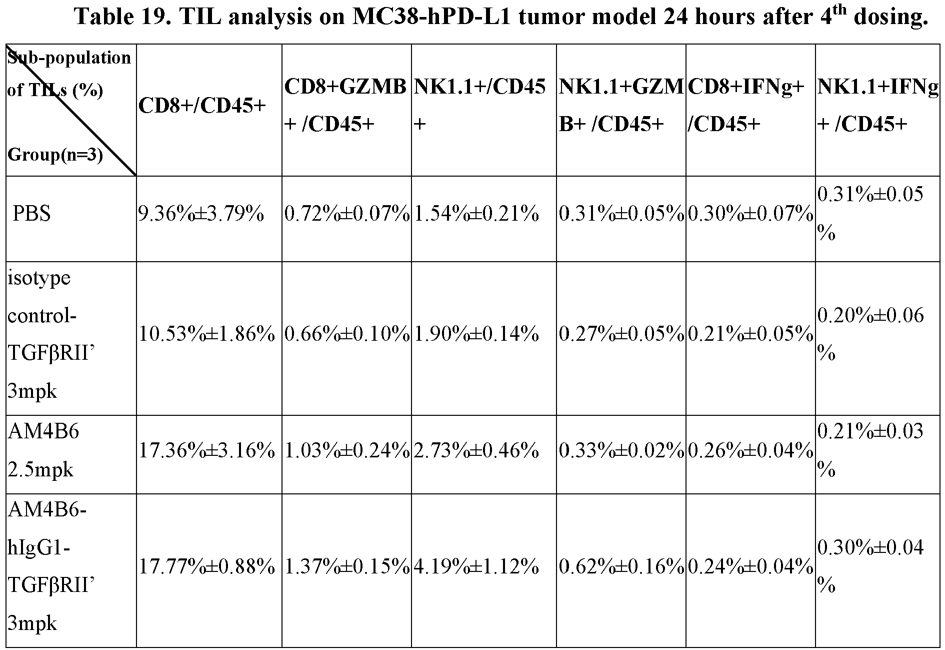

After the 2^{nd} dosing, there was no significant changes in percentage of sub-population of TILs among different treatment groups (Table 18). But after the 4^{th} dosing, CD8+/CD45+% of AM4B6 group and AM4B6-hIgG1-TGFβRII' group significantly increased, comparing to that of the isotype control-TGFβRII' group (Table 19). CD8+GZMB+% and NK1.1+% also increased a lot, as compared to that of the isotype control-TGFβRII' group. These findings indicates the CD8+T cells and NK1.1 T cells might be stimulated by AM4B6 or AM4B6-hIgG1-TGFβRII' to activate and proliferate, and also enriched in tumor microenvironment to facilitate tumor cell killing. When compared to AM4B6, AM4B6-hIgG1-TGFβRII' had an even higher CD8+GZMB+% and NK1.1+%, which was correlated with its more potent anti-tumor activity as measured by TGI above.

### Example 11: Pharmacokinetics and pharmacodynamics study of AM4B6-hIgG1-TGFβRII' in vivo

C57BL/6 female mice were randomized to 6 groups (n=3). Animals were treated with 3mg/kg isotype control-TGFβRII', or 2.5mg/kg AM4B6, or 0.3mg/kg AM4B6-hIgG1-TGFβRII', or 1mg/kg AM4B6-hIgG1-TGFβRII', or 3mg/kg AM4B6-hIgG1-TGFβRII', or 3mg/kg M7824-analog. M7824-analog was generated by MabSpace Biosciences according to the sequence disclosed in US9676863. All the antibodies were administrated by i.v. single injection. After injection, 200µL blood of each mouse was collected at different time points: Predose, 30 min, 2 h, 8 h, 24 h, 48 h, D4, D7, D10, D14, D21 post injection. 80µL plasma of each mouse was collected and tested antibody concentration.

To measure antibody concentration in plasma, two methods were used. The first one is to detect whole bi-functional molecule, including both AM4B6 and TGFβRII' arms. Generally, 1 µg/mL of human PD-L1-his was coated on the 96-well ELISA plate at room temperature for 2 hours. After blocking, serially diluted standard and plasma samples were added and incubated for 1.5 hours. After washing, 0.1 µg/mL biotinylated anti-human TGFβRII' was added, and then after washing, streptavidin-HRP was added. Finally, TMB was added to develop color, which was stopped by diluted sulfuric acid. The plates were read of OD450nm and OD620nm by a microplate reader. Data were analyzed by OD450nm-OD620nm.

The second one is to only detect AM4B6 antibody arm. Similar to the procedure above, 1 µg/mL of human PD-L1-his was coated, and serially diluted standard and plasma samples were added, and incubated for 1.5 hours. After washing, diluted goat HRP conjugated anti-human IgG Fc antibody was added. Finally, TMB was added to develop color, which was stopped by diluted sulfuric acid. The plates were read of OD450nm and OD620nm by a microplate reader. Data were analyzed by OD450nm-OD620nm.

To evaluate the correlation between antibody concentration and change of TGFβ in plasma, the concentration changes of TGFβ1 and TGFβ2 in plasma were tested. Briefly, 4 µg/mL of mouse TGF-β1 capture antibody or 2 µg/mL of mouse TGF-β2 capture antibody was coated on the 96-well ELISA plate at room temperature for 2 hours. 10 µL of 1 N HCl were added to 50 µL of each plasma sample and incubated for 10 minutes at room temperature. The acidified samples were neutralized by adding 10 µL of 1.2N NaOH/0.5M HEPES to ensure the final pH within 7.2-7.6. After blocking, serially diluted standard and plasma samples were added and incubated for 1.5 hours. After washing, TGF-β1 or TGF-β2 detection antibody was added, and then after washing, streptavidin-HRP was added. Finally, TMB was added to develop color, which was stopped by diluted sulfuric acid. The plates were read of OD450nm and OD620nm by a microplate reader. Data were analyzed by OD450nm-OD620nm.

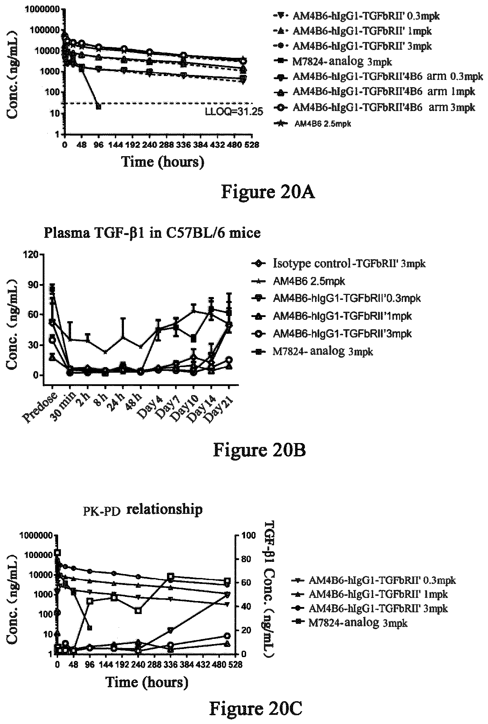

Figure 20A showed the antibody concentration change in plasma. There was no significant difference in PK profiles using the two methods, indicating the whole bifunctional molecule AM4B6-hIgG1-TGFβRII' was quite stable without abnormal cleavage and clearance in vivo, like that of AM4B6 mAb. And at the same time, AM4B6-hIgG1-TGFβRII' depleted TGF-β1 within 30min after i.v. injection even at the lowest dose of 0.3mg/kg, as shown in Figure 20B. M7824-analog and isotype control-TGFβRII' also depleted TGF-β1, but M7824-analog could not maintain that effect from Day 2 while AM4B6-hIgG1-TGFβRII' could maintain a low level of TGF-β1 to Day 21. This results also corresponded to their PK exposure (Figure 20C), indicating TGF-β1 may serve as a good pharmacodynamic marker for AM4B6-hIgG1-TGFβRII' target engagement in plasma. No obvious depletion of TGF-β2 in mice plasma was detected (data now shown).

### Example 12: Construction and Expression of AM4B6-hIgG1-IL-1RA fusion protein

The selected heavy chain and light chain variants were cross combinated and expressed with hIgG1-IL-1RA (34-177) (UniProtKB, P18510) fusion protein. The sequence of heavy chain and light chain was shown in Table 20.

**Table 20. The list of AM4B6-hIgG1-IL-1RA heavy chain and light chain variants**

| Name | | Mutation site | Region | Sequence |
|---|---|---|---|---|
| AM4B6_Hg.3_hIgG1 IL-1RA | -(G4S)4G- | G57E, S59L | H-CDR2-3 | DINPNNAETLYNHKFKG |
| AM4B6_La.4_hKappa | | A51V, N53D | L-CDR2-2 | SRSVRYT |

Similar to AM4B6-hIgG1-TGFβRII' bi-functional molecule, the truncated human IL-1RA _34-177 was fused with AM4B6 to obtain better activity and stability. AM4B6-hIgG1-IL-1RA was short for AM4B6-hIgG1-IL-1RA (34-177). The sequences of the truncated human IL-1RA _34-177 are as below: (SEQ ID NO: 67)

KMQAFRIWDVNQKTFYLRNNQLVAGYLQGPNVNLEEKIDVVPIEPHALFLGI
HGGKMCLSCVKSGDETRLQLEAVNITDLSENRKQDKRFAFIRSDSGPTTSFESAACPG
WFLCTAMEADQPVSLTNMPDEGVMVTKFYFQEDE

The co-transfection of heavy chain and light chain was carried out using the ExpiFectamine^{™} CHO Reagent (Thermo, A29129) from Invitrogen according to the manufacturer's protocol. The supernatant was harvested on day 10 and purified by affinity chromatography.

### Example 13: Affinity of AM4B6- hIgG1-IL-1RA bi-functional molecule to hPD-L1

### Binding to human PD-L1 based on ELISA assay

1 µg/mL hPD-L1 (Acro Biosystems, PD1-H5229) antigens was coated to the ELISA plate and coated overnight at 4°C. Then 300 µL of 2% (w/v) BSA was added for blocking at room temperature for 1 h. After 1h incubation, 100 µL of AM4B6-hIgG1-IL-1RA bi-functional molecule or AM4B6-hIgG1 monoclonal antibody at concentrations ranging from 10 nM to 0.00017 nM (three-fold serial dilutions) were added with PBST as negative control, and incubated at room temperature for 1 h. PBS with 0.5% Tween-20 were used for washing for 3 times, and 100 µL HRP-conjugated anti-human Fc antibody (1:20000, Abcam, ab98624) was added, After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader. The data was analyzed by Graphpad prism.

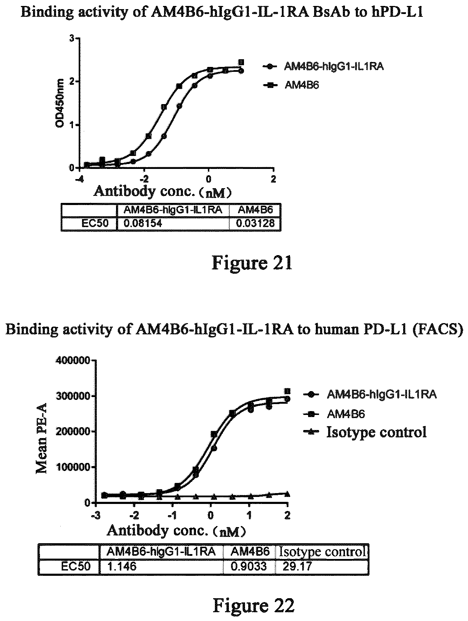

As shown in Figure 21, comparing to the AM4B6 monoclonal antibody, AM4B6-hIgG1-IL-1RA bi-functional molecule have similar binding signals and affinities.

### Binding to PD-L1 expressing on cell surface of AM4B6-hIgG1-IL-1RA by a FACS assay

293T-PD-L1-CD3L cell was generated by MabSpace Biosciences for characterization of PD-L1 antibodies. The cell was transfected with both human PD-L1 and anti-CD3 scFv. AM4B6-hIgG1-IL-1RA bi-functional molecule or AM4B6 monoclonal antibody were serially diluted with 3-fold dilutions to obtain 11 concentrations in dilution buffer (PBS with 2%BSA). 293T-PD-L1-CD3L cells were harvested and centrifuged. Then they were resuspended in PBS with density of 2×10⁶ cells/mL and added to the plate with 100 µL per well. After centrifugation and removing the supernatant, the diluted antibodies were added to the plate and incubated in 4°C for 30min. After washing twice with dilution buffer, PE conjugated donkey anti-human IgG (H+L) (Jacksonimmuno, 709-116-149) was added to the plate and incubated in 4°C for 30 min. After washing, cells were resuspended in 200 µL PBS and analyzed by flow cytometry. The data was analyzed by Graphpad prism.

As shown in Figure 22, AM4B6-hIgG1-IL-1RA bi-functional molecule and AM4B6-hIgG1 could bind to PD-L1 expressed on surface of cells with similar EC50 which was consistent with affinity results measured by ELISA.

### Example 14: PD1/PD-L1 blockade activity of AM4B6-hIgG1-IL-1RA

In this assay, 293T-PD-L1-CD3L cell was expressing PD-L1 and anti-CD3 scFv, and Jurkat-NFAT-Luc-PD1 cell was expressing PD-1 and carrying NFAT signal which can be activated by CD3 stimulation. NFAT activation will lead to luciferase gene transcription and expression, which can be detected by its substrate. Both two cells were generated by MabSpace Biosciences.

Briefly, 293T-PD-L1-CD3L cells was harvested and resuspended at density of 2×10⁶ cells/mL. 20 µL cells per well was added into half well plate. AM4B6-hIgG1-IL-1RA bi-functional molecule and AM4B6-hIgG1 were serially diluted (3-fold dilutions) to obtain 8 concentrations in RPMI medium with 2%FBS. 20 µL antibodies per well was added into half well plate, and the plate was incubated at 37°C, 5% CO₂ for 30min. Jurkat-NFAT-Luc-PD1 cells were harvested and resuspended at density of 4×10⁶ cells/mL in RPMI medium with 2%FBS. Finally, 20 µL cells per well was added into half well plate and incubated in 37°C, 5% CO₂ for 5 h. 60 µL OneGlo detection reagent (Promega, E6120) was added to each well and incubated at room temperature for 5 minutes. The luminescent signal was read by Microplate Reader. The data was analyzed by GraphPad Prism.

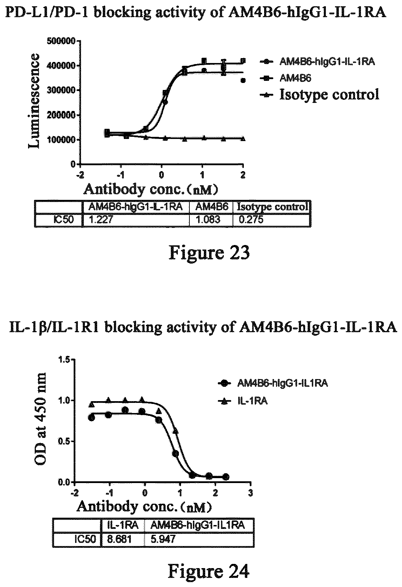

As shown in Figure 23, AM4B6-hIgG1-IL-1RA bi-functional molecule and AM4B6-hIgG1 had similar blockade activity to PD-L1 in this cell-based assay.

### Example 15: Blocking activity of AM4B6-hIgG1-IL-1RA to human IL-1β

### Blocking activity of AM4B6-hIgG1-IL-1RA BsAb to hIL-1β based on ELISA

To test ligand/receptor blocking activity, 5 µg/mL Human IL-1β protein (Sino Biological, Cat#10139) was coated to the ELISA plate and incubated overnight at 4°C. 300 µL blocking buffer was added for blocking at room temperature for 1 h. After 1 h, 50 µL of AM4B6-hIgG1-IL-1RA BsAb or IL-1RA protein(Sino Biological, Cat#10123-HNAE) at serial concentrations ranging from 200 nM to 0.03 nM (three-fold serial dilutions) with 50µl 10nM Human IL-1RI-his (Sino Biological, Cat#10126-H08H) were added to the well and incubate 1hr at room temperature. PBS with 0.5% Tween-20 were used for washing for 3 times, and 100 µL HRP-conjugated his-tag Antibody (1:2000 dilution, Genscript, Cat#A00612) was added, incubate for 1hr in room temperature. Then, mixed TMB substrate reagent (InnoReagents, Cat#: TMB-S-003) was added and incubated at room temperature for 5 min, then stopped by adding 0.1 M H₂SO₄. OD450nm was recorded by Microplate Reader. The data was analyzed by Graphpad prism.

As shown in Figure 24, AM4B6-hIgG1-IL-1RA can block IL-1β dose dependently, and the blocking activity of AM4B6-hIgG1-IL-1RA to IL-1RI was better than that of IL-1RA protein.

### Blocking activity of AM4B6-hIgG1-IL-1RA Bi-Functional Molecule to hIL-1β on reporter cell

In this assay, HEK-Blue^{™} CD40L cells were purchase from Invivogen (Cat# hkb-cd40), These cells were generated by stable transfection of HEK293 cells with the human CD40 gene and an NF-kB inducible SEAP construct. Binding of CD40L to its receptor CD40 triggers cascade leading to the activation of NF-kB and subsequent production of SEAP which can monitored by QUANTI-Blue. HEK293 cells express endogenously the receptor for the cytokines IL-1β which share a common signaling pathway with CD40L. So, IL-1b - mediated SEAP production can be blocked using neutralizing antibody.

Briefly, collect HEK293-CD40L cells at log phase cells and seed cells at density of 5×10^4/well (100µl/well) into 96-well plate to adhere overnight. AM4B6-hIgG1 -IL-1RA bispecific antibody and IL-1RA protein were serially diluted (5-fold dilutions) to obtain 10 concentrations in complete culture medium. Add 50µl/well diluted antibody (or IL-1RA protein) and 50µl/well human IL-1β to the cells, incubate at 37°C for 24h. Next day add 160µl of QUANTI-Blue ^{™} Solution (Invivogen, Cat#rep-qbs) to a new plate, and 40µl cell culture supernatant were added the plates. Incubate the plates at 37°C for 2h. Determine SEAP level using a spectrophotometer at 620nm. The The data was analyzed by Graphpad prism.

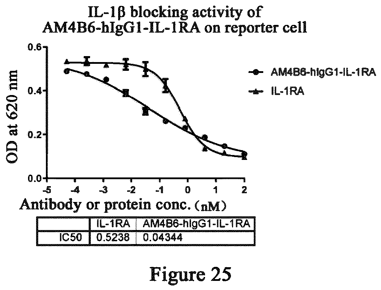

As shown in Figure 25, AM4B6-hIgG1-IL-1RA can block IL-1β in a dose dependent manner, and the blocking activity of AM4B6-hIgG1-IL-1RA to IL-1β was stronger than IL-1RA protein, which was consistent with blocking results measured by ELISA.

### Example 16: Construction, expression, purification of AM4B6-SIRPα bifunctional antibodies

The SIRPα_CV1 is an engineered high-affinity SIRPα variant, which potently antagonized CD47 on cancer cells but did not induce macrophage phagocytosis on their own (Kipp Weiskopf et al. Science 341, 88 (2013)). We invented bifunctional antibodies targeting both PD-L1 and CD47, including symmetrical antibodies (AM4B6-hIgG1-SIRPα and 3280A-hIgG1-SIRPα) and asymmetric antibodies (AM4B6-hIgG1-SIRPα (KIH) and 3280A-hIgG1-SIRPα(KIH)), wherein KIH is short for knob into hole. The constructions of these molecules are described in Table 21, and the SIRPα_CV1 sequenced is also listed below.

SIRPα_CV1 sequence (SEQ ID NO: 84):

EEELQIIQPDKSVLVAAGETATLRCTITSLFPVGPIQWFRGAGPGRVLIYNQRQ
GPFPRVTTVSDTTKRNNMDFSIRIGNITPADAGTYYCIKFRKGSPDDVEFKSGAGTELS
VRAKPS

**Table 21. construction of anti-PD-L1-SIRPα bifunctional antibodies**

| ID | Light chain sequence | Heavy chain sequence 1 | Heavy chain sequence 2 | Note |
|---|---|---|---|---|
| AM4 B6-hIgG1 - SIRPα | AM4B6-La.4-hKappa SEQ ID NO: 90 | AM4B6_Hg.3_hIgG1_L2 34F_L235E_P331S - (G4S)4G - SIRPa_CV1 SEQ ID NO: 91 | NA | SIRPα_CV1 sequence is from Kipp Weiskopf et al. Science 341, 88 (2013) |
| 3280 A-hIgG1 SIRPα | 3280A-L-hKappa SEQ ID NO: 92 | 3280A-H-hIgG1(N297A)-(G4S)4G- SIRPα_CV1 SEQ ID NO: 93 | NA | 3280A is short for Atezolizumab, anti-PD-L1 antibody of Roche |
| AM4 B6-hIgG1 - SIRPα (KIH) | AM4B6-La.4-hKappa SEQ ID NO: 90 | AM4B6_Hg.3_hIgG1_L2 34F_L235E_P331S (knob) - SIRPa_CV1 SEQ ID NO: 94 | AM4B6_Hg.3 _hIgG1_L234 F_L235E_P33 1S (hole) SEQ ID NO: 95 | Knob: S354C, T366W Hole: Y349C, T366S, L368A, Y407V |
| 3280 A-hIgG1 - SIRPα (KIH) | 3280A-L-hKappa 3280A-L-hKappa SEQ ID NO: 92 | 3280A_H_hIgG1_L234F_ L235E_P331S (knob) - SIRPa_CV1 SEQ ID NO: 96 | 3280A_H_hIg G1_L234F_L2 35E_P331S (hole) SEQ ID NO: 97 | 3280A is short for Atezolizumab, anti-PD-L1 antibody of Roche |

All of the 4 bifunctional antibodies were expressed with Expi-CHO cell according to the manufacture's protocol. For the two symmetrical bifunctional antibodies, high purity antibodies were obtained with one-step Mabselect SuRe purification, but for the asymmetric bifunctional antibodies, high purity antibody cannot be obtained by conventional one-step Mabselect SuRe purification. To obtain the high purity asymmetric antibodies, we used a HiTrap PrismA resin to polish purify the antibodies, and the purity of the asymmetric antibodies was better than 95%. The polish purification procedure is described as follows.

Buffer used:
Equilibration buffer: 50 mM Tris-HAc, 150 mM NaCl, pH7.4.
Wash buffer: 50 mM NaAc/HAc, 500 mM NaCl, 5% PEG, pH5.5.
Elution buffer: 50 mM HAc, 500 mM NaCl, 5% PEG, pH 3.0.
Equilibrate the HiTrap PrismA column with equilibration buffer for at least 5 column volumes (CV), until the UV baseline, eluent pH, and conductivity are unchanged.
Load the sample onto a pre-equilibrated HiTrap PrismA column.
Wash with 5 to 10 CV wash buffer, until the UV trace returns to baseline.
Elute with 0-100% elution buffer in 10-20 CV and collect 5-10 fractions with several tubes separately. The pH was adjusted to about 6.0-7.0 with 1M Tris-base (pH 9.0)

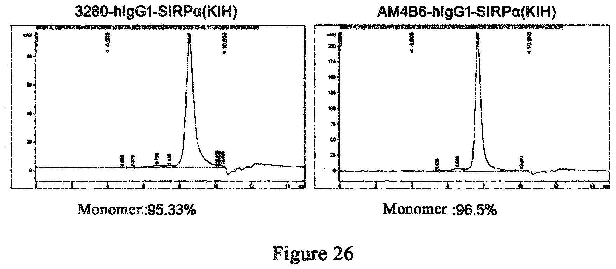

The fractions are then characterized by SEC-HPLC. Figure 26 shows that the purity of 3280A-hIgG1-SIRPα (KIH) and AM4B6-hIgG1-SIRPα (KIH) is 95.33% and 96.5%, respectively.

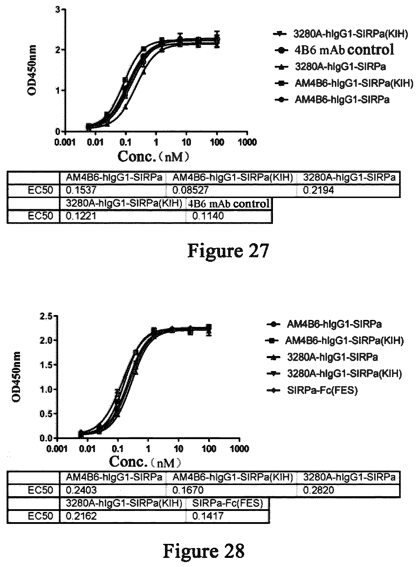

The affinity to human PD-L1 or human CD47 were tested with ELISA. Figures 27 and 28 show that the affinities to antigen of bifunctional antibodies are comparable with that of parent monoclonal antibody (4B6 mAb control) or fusion proteins (SIRPα-Fc (FES)).

### Example 17: Construction and Expression of IgG-scFv-ACZ885-AM4B6 and IgG-scFv-XOMA052-AM4B6 bispecific antibodies

The sequence of single chain fragments (scFvs) AM4B6 are shown in the following table. The anti-IL-1β antibodies Gevokizumab (XOMA052) and Canakinumab (ACZ885) were from Novartis. The scFvs of AM4B6 were connected to anti-IL-1β antibody heavy chain C-terminal to obtain better activity and stability. The scFvs have the GS linker (GGGGSGGGGSGGGGSGGGGS) that connected VH to VL, and contain an interdomain disulfide bond between the residues H44C and L100C (Kabat numbering). The sequence of the anti-IL-1β antibodies (XOMA052 and ACZ885) are shown in Table 22. The constructed bispecific antibodies were named IgG-scFv-ACZ885-AM4B6 and IgG-scFv-XOMA052-AM4B6, respectively.

The co-transfection of heavy chain and light chain of the bispecific antibody were carried out using the ExpiFectamine^{™} CHO Reagent (Thermo, A29129) from Invitrogen according to the manufacturer's protocol. The supernatant was harvested on day 10 and purified by affinity chromatography.

### Example 18: Binding activities of IgG-scFv-ACZ885-AM4B6 and IgG-scFv-XOMA052-AM4B6 bispecific antibody to hIL-1β

### Binding to human IL-1β protein based on ELISA assay

100µL 1 µg/mL hIL-1β protein (SinoBiological, Cat# 10139 -HNAE) antigen was coated to the ELISA plate and coated overnight at 4°C. Then 200 µL of 2% (w/v) BSA was added for blocking at room temperature for 2 h. After the incubation, 100 µL of IgG-scFv-ACZ885-AM4B6, IgG-scFv-XOMA052-AM4B6 bispecific antibody, ACZ885 or XOMA052 at the concentrations ranging from 20 nM to 0.000339 nM (three-fold serial dilutions) were added with PBST as negative control, and incubated at room temperature for 1 h. PBS with 0.5% Tween-20 were used for washing for 3 times, and 100 µL HRP-coupled anti-human Fc antibody (1:20000, Abcam, ab98624) was added. After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader. The data was analyzed by Graphpad prism.

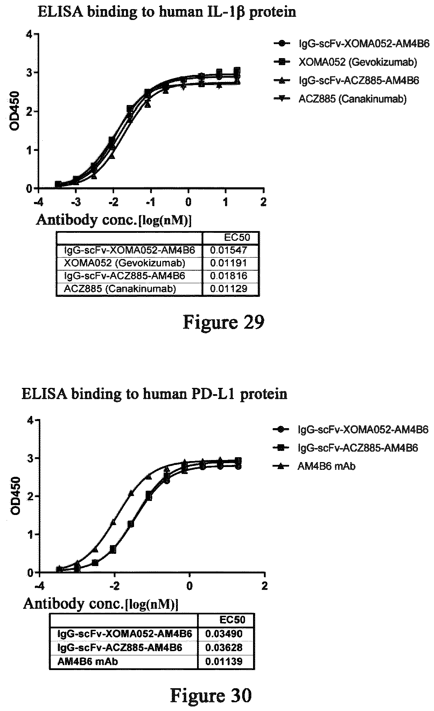

As shown in Figure 29, comparing to the ACZ885 and XOMA052 monoclonal antibody, IgG-scFv-ACZ885-AM4B6 and IgG-scFv-XOMA052-AM4B6 bsAbs bi-functional molecule have similar activity of binding to hIL-1β protein, respectively.

### Example 19: Binding activities of IgG-scFv-ACZ885-AM4B6 and IgG-scFv-XOMA052-AM4B6 bispecific antibody to hPD-L1

### Binding to human PD-L1 based on ELISA assay

100µL 1 µg/mL hPD-L1 (Acro Biosystems, PD1-H5229) antigen was coated to the ELISA plate and coated overnight at 4°C. Then 300 µL of 2% (w/v) BSA was added for blocking at room temperature for 1 h. After 1h incubation, 100 µL of IgG-scFv-ACZ885-AM4B6, IgG-scFv-XOMA052-AM4B6 bispecific antibody or AM4B6-hIgG1 monoclonal antibody (AM4B6 mAb) at the concentrations ranging from 20 nM to 0.000339 nM (three-fold serial dilutions) were added with PBST as negative control, and incubated at room temperature for 1 h. PBS with 0.5% Tween-20 were used for washing for 3 times, and 100 µL HRP-coupled anti-human Fc antibody (1:20000, Abcam, ab98624) was added. After incubation at room temperature for 1 h, mixed TMB substrate reagent (InnoReagents, TMB-S-003) was added and incubated at room temperature for 5 min, and stopped by adding 0.1M H₂SO₄. OD450 nm was recorded by Microplate Reader. The data was analyzed by Graphpad prism.

As shown in Figure 30, comparing to the AM4B6 monoclonal antibody, IgG-scFv-ACZ885-AM4B6, IgG-scFv-XOMA052-AM4B6 bispecific antibody have similar activity of binding to hPD-L1 protein.

### Binding of IgG-scFv-ACZ885-AM4B6 and IgG-scFv-XOMA052-AM4B6 to PD-L1 expressing 293T cells by FACS assay

293T-PD-L1-CD3L cell was generated by MabSpace Biosciences for characterization of PD-L1 antibodies. The cell was transfected with both human PD-L1 and anti-CD3 scFv. IgG-scFv-ACZ885-AM4B6, IgG-scFv-XOMA052-AM4B6 bispecific antibody AM4B6 mAb were serially diluted (4-fold) to obtain 9 concentrations in dilution buffer (PBS with 2%BSA). 293T-PD-L1-CD3L cells were harvested and centrifuged. Then they were resuspended in PBS with density of 2×10⁶ cells/mL and added to the plate with 100 µL per well. After centrifugation and removing the supernatant, the diluted antibodies were added to the plate and incubated in 4°C for 30min. After washing twice with dilution buffer, PE conjugated donkey anti-human IgG (H+L) (Jacksonimmuno, 709-116-149) was added to the plate and incubated in 4°C for 30 min. After washing, cells were resuspended in 200 µL PBS and analyzed by flow cytometry. The data was analyzed by Graphpad prism.

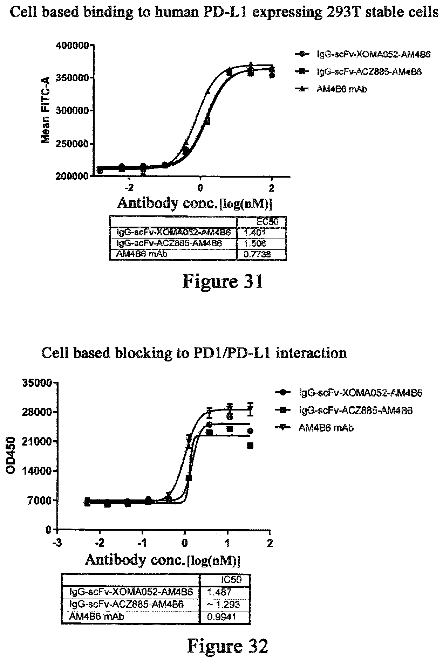

As shown in Figure 31, IgG-scFv-ACZ885-AM4B6 and IgG-scFv-XOMA052-AM4B6 could bind to PD-L1 expressed on surface of cells with similar EC₅₀ which was consistent with affinity results measured by ELISA.

### Example 20: PD1/PD-L1 blockade activity of IgG-scFv-ACZ885-AM4B6 and IgG-scFv-XOMA052-AM4B6

In this assay, 293T-PD-L1-CD3L cell was expressing PD-L1 and anti-CD3 scFv, and Jurkat-NFAT-Luc-PD1 cell was expressing PD-1 and carrying NFAT signal which can be activated by CD3 stimulation. NFAT activation leads to luciferase gene transcription and expression, which can be detected by its substrate. The two cells were generated by MabSpace Biosciences.

Briefly, 293T-PD-L1-CD3L cells was harvested and resuspended at density of 2×10⁶ cells/mL. 20 µL cells per well was added into half well plate. IgG-scFv-ACZ885-AM4B6, IgG-scFv-XOMA052-AM4B6 bispecific antibody and AM4B6-hIgG1 were serially diluted (3-fold dilutions) to obtain 9 concentrations in RPMI medium with 2%FBS. 20 µL antibodies per well was added into half well plate, and the plate was incubated at 37°C, 5% CO₂ for 30min. Jurkat-NFAT-Luc-PD1 cells were harvested and resuspended at density of 4×10⁶ cells/mL in RPMI medium with 2%FBS. Finally, 20 µL cells per well was added into half well plate and incubated in 37°C, 5% CO₂ for 5 h. 60 µL OneGlo detection reagent (Promega, E6120) was added to each well and incubated at room temperature for 5 minutes. The luminescent signal was read by Microplate Reader. The data was analyzed by GraphPad Prism.

As shown in Figure 32, IgG-scFv-ACZ885-AM4B6, IgG-scFv-XOMA052-AM4B6 and AM4B6-hIgG1 had similar blockade activity to PD-L1 in this cell-based assay.

### Example 21: Blocking activity of IgG-scFv-XOMA052-AM4B6 to human IL-1β on human dermal fibroblast (HDF) cells

### Blocking activity of IgG-scFv-XOMA052-AM4B6 to human IL-1β on HDF cells

To test ligand/receptor blocking activity, 4×10⁴/mL HDF cells with 100 µL/well were stimulated with 50 pg/mL of recombinant human IL-1β protein (Sino Biological, Cat#10139) while cells without IL-1β stimulation as the negative control. Then, 100 uL/well IgG-scFv-XOMA052-AM4B6 and XOMA052 at serial concentrations ranging from 100 nM to 0.00038 nM (four-fold serial dilutions) were added to the cultures and incubated overnight (16 -17 hr) at room temperature. After stimulation, IL-6 release in the cell cultured suspension was detected using IL-6 ELISA Kit (R&D, DY206, P209026) guided by the kit instruction.

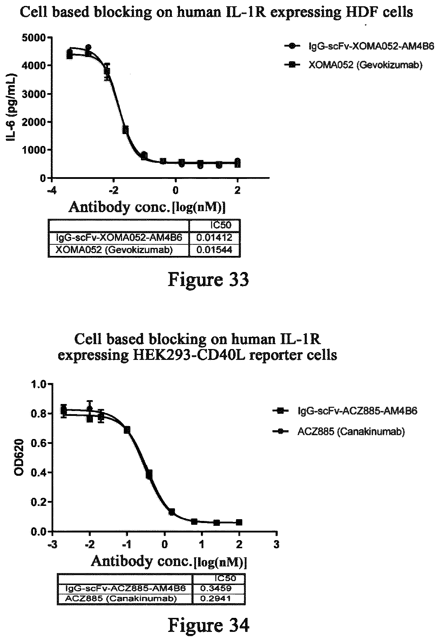

As shown in Figure 33, IgG-scFv-XOMA052-AM4B6 and XOMA052 can block IL-1β dose dependently, and the blocking activity of IgG-scFv-XOMA052-AM4B6 to HDF cells was similar to that of XOMA052.

### Blocking activity of IgG-scFv-ACZ885-AM4B6 to hIL-1β on reporter cell

In this assay, HEK-Blue^{™} CD40L cells were purchase from Invivogen (Cat# hkb-cd40), These cells were generated by stable transfection of HEK293 cells with the human CD40 gene and an NF-kB inducible SEAP construct. Binding of CD40L to its receptor CD40 triggers cascade leading to the activation of NF-kB and subsequent production of SEAP which can be monitored by QUANTI-Blue. HEK293 cells express endogenously the receptor for the cytokines IL-1β which share a common signaling pathway with CD40L. So, IL-1β-mediated SEAP production can be blocked using neutralizing antibody.

Briefly, collect HEK293-CD40L cells at log phase cells and seed cells at density of 5×10^4/well (100µl/well) into 96-well plate to adhere overnight. IgG-scFv-ACZ885-AM4B6and ACZ885 were serially diluted from 100 nM (4-fold dilutions) to obtain 9 concentrations in complete culture medium. Add 50µl/well diluted antibodies and 50µl/well human IL-1β (1ng/mL) to the cells, incubate at 37°C for 24h. Next day, add 160µl of QUANTI-Blue ^{™} Solution (Invivogen, Cat#rep-qbs) to a new plate, and 40µl cell culture supernatant were added the plates. Incubate the plates at 37°C for 2h. Determine SEAP level using a spectrophotometer at 620nm. The data was analyzed by Graphpad prism.

As shown in Figure 34, IgG-scFv-ACZ885-AM4B6 and ACZ885 (Canakinumab) can block IL-1β in a dose dependent manner, and the blocking activity of IgG-scFv-ACZ885-AM4B6 was similar to ACZ885 (Canakinumab) on HEK293-CD40L reporter cells.

**Table 22. Amino acid sequences mentioned in the present disclosure**

| SED ID NO. | Sequences | Region |
|---|---|---|
| 1 | DYYMN | 4B6_HCDR1 |
| 2 | DINPNNGGTSYNHKFKG | 4B6_HCDR2 |
| 3 | WGDGPFAY | 4B6_HCDR3 |
| 4 | KASQNVGAAVA | 4B6_LCDR1 |
| 5 | SASNRYT | 4B6_LCDR2 |
| 6 | QQYSNYPT | 4B6_LCDR3 |
| 7 | KASQNVGAIVA | 4B6-L-CDR1-1 |
| 8 | KASQNVPAAVA | 4B6-L-CDR1-2 |
| 9 | KASQNVKGAVA | 4B6-L-CDR1-3 |
| 10 | SNSHRYT | 4B6-L-CDR2-1 |
| 11 | SRSVRYT | 4B6-L-CDR2-2 |
| 12 | SVSDRYT | 4B6-L-CDR2-3 |
| 13 | DINPNNADTMYNHKFKG | 4B6-H-CDR2-1 |
| 14 | DINPNNAQTQYNHKFKG | 4B6-H-CDR2-2 |
| 15 | DINPNNAETLYNHKFKG | 4B6-H-CDR2-3 |
| 16 | DINPNNGLTSYNHKFKG | 4B6-H-CDR2-4 |
| 17 | DINPNNAQTVYNHKFKG | 4B6-H-CDR2-5 |
| 18 | DINPNNAGTSYNHKFKG | H-CDR2-WT (G55A) |
| 19 | DINPNNX₁X₂TX₃YNHKFKG | HCDR2 |
| 20 | KASQNVX₄X₅X₆VA | LCDR1 |
| 21 | SX₇SX₈RYT | LCDR2 |
| 22 | QVQLVQSGAEVKKPGASVKVSCKASGYTFT | HFR1 |
| 23 | WVRQAPGQGLEWMG | HFR2 |
| 24 | RVTMTRDTSISTAYMELSRLRSDDTAVYYCAR | HFR3 |
| 25 | WGQGTLVTVSS | HFR4 |
| 26 | DIQMTQSPSSLSASVGDRVTITC | LFR1 |
| 27 | WYQQKPGKAPKLLIY | LFR2 |
| 28 | GVPSRFSGSGSGTDFTFTISSLQPEDIATYYC | LFR3 |
| 29 | FGQGTKLEIK | LFR4 |
| 30 | QVQLVQSGAEVKKPGASVKVSCKASGYVFT | HFR1 variant |
| 31 | WVRQAPGQSLEWMG | HFR2 variant |
| 32 | RVTVTVDTSISTAYMELSRLRSDDTAVYYCAR | HFR3 variant 1 |
| 33 | RVTVTVDTSISTAYMELSRLRSDDTAVYYCVK | HFR3 variant 2 |
| 34 | RVTVTVDKSISTAYMELSRLRSDDTAVYYCAR | HFR3 variant 3 |
| 35 | RVTVTVDKSISTAYMELSRLRSDDTAVYYCVK | HFR3 variant 4 |
| 36 | WYQQKPGKSPKLLIY | LFR2 variant |
| 37 | GVPSRFSGSGSGTDFTLTISSLQPEDIATYYC | LFR3 variant 1 |
| 38 | GVPDRFSGSGSGTDFTLTISSLQPEDIATYYC | LFR3 variant 2 |
| 39 | GVPSRFSGSGSGTDFTLTISSLQPEDIATYYC | F73L mutation for variant 1-FR3 |
| 40 | QVQLVQSGAEVKKPGASVKVSCKASGYX₉FT | HFR1 |
| 41 | WVRQAPGQX₁₀LEWMG | HFR2 |
| 42 | RVTX₁₆TVDX₁₁SISTAYMELSRLRSDDTAVYYCX₁₂X₁₃ | HFR3 |
| 43 | WYQQKPGKX₁₄PKLLIY | LFR2 |
| 44 | GVPX₁₅RFSGSGSGTDFTX₁₇TISSLQPEDIATYYC | LFR3 |
| 45 | GVPDRFSGSGSGTDFTLTISSLQPEDIATYYC | F73L, A43S, S60D for LC variant 2-FR3 |
| 46 | EVQLQQSGPELVKPGASVKISCKASGYVFTDYYMN WVKQSHGKSLEWIGDINPNNGGTSYNHKFKGKATV TVDKSSRTAYMELLSLTSEDSAVYYCVKWGDGPFA YWGQGTLVTVSA | 4B6_VH |
| 47 | DIVMTQSQKFMSTSVGDRVSITCKASQNVGAAVAW YQQKPGQSPKLLIYSASNRYTGVPDRFTGSGSGTDFT LTISNMQSEDLADYFCQQYSNYPTFGSGTKLGIK | 4B6_VL |
| 48 | QVQLVQSGAEVKKPGASVKVSCKASGYTFTGYYM HWVRQAPGQGLEWMGWINPNSGGTNYAQKFQGRV TMTRDTSISTAYMELSRLRSDDTAVYYCAR | Humanized 4B6, VH germline |
| 49 | QVQLVQSGAEVKKPGASVKVSCKASGYTFTDYYM NWVRQAPGQGLEWMGDINPNNGGTSYNHKFKGRV TVTVDTSISTAYMELSRLRSDDTAVYYCARWGDGPF AYWGQGTLVTVSS | Humanized 4B6, VH variant 1 |
| 50 | QVQLVQSGAEVKKPGASVKVSCKASGYTFTDYYM NWVRQAPGQGLEWMGDINPNNGGTSYNHKFKGRV TVTVDTSISTAYMELSRLRSDDTAVYYCVKWGDGP FAYWGQGTLVTVSS | Humanized 4B6, VH variant 2 |
| 51 | QVQLVQSGAEVKKPGASVKVSCKASGYVFTDYYM NWVRQAPGQGLEWMGDINPNNGGTSYNHKFKGRV TVTVDKSISTAYMELSRLRSDDTAVYYCARWGDGP FAYWGQGTLVTVSS | Humanized 4B6, VH variant 3 |
| 52 | QVQLVQSGAEVKKPGASVKVSCKASGYVFTDYYM NWVRQAPGQSLEWMGDINPNNGGTSYNHKFKGRV TVTVDKSISTAYMELSRLRSDDTAVYYCVKWGDGP FAYWGQGTLVTVSS | Humanized 4B6, VH variant 4 |
| 53 | DIQMTQSPSSLSASVGDRVTITCQASQDISNYLNWY QQKPGKAPKLLIYDASNLETGVPSRFSGSGSGTDFTF TISSLQPEDIATYYCQQYDNLP | Humanized 4B6, VL germline |
| 54 | DIQMTQSPSSLSASVGDRVTITCKASQNVGAAVAWY QQKPGKAPKLLIYSASNRYTGVPSRFSGSGSGTDFTL TISSLQPEDIATYYCQQYSNYPTFGQGTKLEIK | Humanized 4B6, VL variant 1 |
| 55 | DIQMTQSPSSLSASVGDRVTITCKASQNVGAAVAWY QQKPGKSPKLLIYSASNRYTGVPDRFSGSGSGTDFTL TISSLQPEDIATYYCQQYSNYPTFGQGTKLEIK | Humanized 4B6, VL variant 2 |
| 56 | QVQLVQSGAEVKKPGASVKVSCKASGYVFTDYYM NWVRQAPGQSLEWMGDINPNNAGTSYNHKFKGRV TVTVDKSISTAYMELSRLRSDDTAVYYCVKWGDGP FAYWGQGTLVTVSS | Hu4B6_Hg |
| 57 | QVQLVQSGAEVKKPGASVKVSCKASGYVFTDYYM NWVRQAPGQSLEWMGDINPNNAGTSYNHKFKGRV TVTVDKSISTAYMELSRLRSDDTAVYYCVKWGDGP FAYWGQGTLVTVSS | AM4B6_Hg |
| 58 | QVQLVQSGAEVKKPGASVKVSCKASGYVFTDYYM NWVRQAPGQSLEWMGDINPNNAQTQYNHKFKGRV TVTVDKSISTAYMELSRLRSDDTAVYYCVKWGDGP FAYWGQGTLVTVSS | Hu4B6_Hg.2 |
| 59 | QVQLVQSGAEVKKPGASVKVSCKASGYVFTDYYM NWVRQAPGQSLEWMGDINPNNAETLYNHKFKGRV TVTVDKSISTAYMELSRLRSDDTAVYYCVKWGDGP FAYWGQGTLVTVSS | AM4B6_Hg.3 |
| 60 | QVQLVQSGAEVKKPGASVKVSCKASGYVFTDYYM NWVRQAPGQSLEWMGDINPNNAQTVYNHKFKGRV TVTVDKSISTAYMELSRLRSDDTAVYYCVKWGDGP FAYWGQGTLVTVSS | AM4B6_Hg.5 |
| 61 | DIQMTQSPSSLSASVGDRVTITCKASQNVGAAVAWY QQKPGKAPKLLIYSASNRYTGVPSRFSGSGSGTDFTL TISSLQPEDIATYYCQQYSNYPTFGQGTKLEIK | Hu4B6_La |
| 62 | DIQMTQSPSSLSASVGDRVTITCKASQNVKGAVAWY QQKPGKAPKLLIYSASNRYTGVPSRFSGSGSGTDFTL TISSLQPEDIATYYCQQYSNYPTFGQGTKLEIK | AM4B6_La.1 |
| 63 | DIQMTQSPSSLSASVGDRVTITCKASQNVPAAVAWY QQKPGKAPKLLIYSASNRYTGVPSRFSGSGSGTDFTL TISSLQPEDIATYYCQQYSNYPTFGQGTKLEIK | AM4B6_La.2 |
| 64 | DIQMTQSPSSLSASVGDRVTITCKASQNVGAAVAWY QQKPGKAPKLLIYSVSDRYTGVPSRFSGSGSGTDFTL TISSLQPEDIATYYCQQYSNYPTFGQGTKLEIK | AM4B6_La.4 |
| 65 | DIQMTQSPSSLSASVGDRVTITCKASQNVGAAVAWY QQKPGKAPKLLIYSRSVRYTGVPSRFSGSGSGTDFTL TISSLQPEDIATYYCQQYSNYPTFGQGTKLEIK | AM4B6_La.6 |
| 66 | GAVKFPQLCKFCDVRFSTCDNQKSCMSNCSITSICEK PQEVCVAVWRKNDENITLETVCHDPKLPYHDFILED AASPKCIMKEKKKPGETFFMCSCSSDECNDNIIFSEE YNTSNPD | truncated TGF-beta receptor II sequence 20-136 |
| 67 | KMQAFRIWDVNQKTFYLRNNQLVAGYLQGPNVNLE EKIDVVPIEPHALFLGIHGGKMCLSCVKSGDETRLQL EAVNITDLSENRKQDKRFAFIRSDSGPTTSFESAACPG WFLCTAMEADQPVSLTNMPDEGVMVTKFYFQEDE | truncated IL-1 R-34-177 |
| 68 | (G4S)4G | linker |
| 69 | LQCFCHLCTKDNFTCVTDGLCFVSVTETTDKVIHNSM CIAEIDLIPRDRPFVCAPSSKTGSVTTTYCCNQDHCNKI ELPTTVKSSPGLGPVELAAVIAGPVCFVCISLMLMVYI CHNRTVIHHRVPNEEDPSLDRPFISEGTTLKDLIYDMT TSGSGSGLPLLVQRTIARTIVLQESIGKGRFGEVWRGK WRGEEVAVKIFSSREERSWFREAEIYQTVMLRHENIL GFIAADNKDNGTWTQLWLVSDYHEHGSLFDYLNRYT VTVEGMIKLALSTASGLAHLHMEIVGTQGKPAIAHRD LKSKNILVKKNGTCCIADLGLAVRHDSATDTIDIAPNH RVGTKRYMAPEVLDDSINMKHFESFKRADIYAMGLV FWEIARRCSIGGIHEDYQLPYYDLVPSDPSVEEMRKV VCEQKLRPNIPNRWQSCEALRVMAKIMRECWYANGA ARLTALRIKKTLSQLSQQEGIKM | TGFβRI |
| 70 | TIPPHVQKSVNNDMIVTDNNGAVKFPQLCKFCDVRF STCDNQKSCMSNCSITSICEKPQEVCVAVWRKNDEN ITLETVCHDPKLPYHDFILEDAASPKCIMKEKKKPGE TFFMCSCSSDECNDNIIFSEEYNTSNPDLLLVIFQVTG ISLLPPLGVAISVIIIFYCYRVNRQQKLSSTWETGKTR KLMEFSEHCAIILEDDRSDISSTCANNINHNTELLPIEL DTLVGKGRFAEVYKAKLKQNTSEQFETVAVKIFPYE EYASWKTEKDIFSDINLKHENILQFLTAEERKTELGK QYWLITAFHAKGNLQEYLTRHVISWEDLRKLGSSLA RGIAHLHSDHTPCGRPKMPIVHRDLKSSNILVKNDLT CCLCDFGLSLRLDPTLSVDDLANSGQVGTARYMAPE VLESRMNLENVESFKQTDVYSMALVLWEMTSRCNA VGEVKDYEPPFGSKVREHPCVESMKDNVLRDRGRP EIPSFWLNHQGIQMVCETLTECWDHDPEARLTAQCV AERFSELEHLDRLSGRSCSEEKIPEDGSLNTTK | TGFβRII isoform A |
| 71 | TIPPHVQKSDVEMEAQKDEIICPSCNRTAHPLRHINND MIVTDNNGAVKFPQLCKFCDVRFSTCDNQKSCMSNCS ITSICEKPQEVCVAVWRKNDENITLETVCHDPKLPYHD FILEDAASPKCIMKEKKKPGETFFMCSCSSDECNDNIIFS EEYNTSNPDLLLVIFQVTGISLLPPLGVAISVIIIFYCYRV NRQQKLSSTWETGKTRKLMEFSEHCAIILEDDRSDISST CANNINHNTELLPIELDTLVGKGRFAEVYKAKLKQNTS EQFETVAVKIFPYEEYASWKTEKDIFSDINLKHENILQF LTAEERKTELGKQYWLITAFHAKGNLQEYLTRHVISW EDLRKLGSSLARGIAHLHSDHTPCGRPKMPIVHRDLKS SNILVKNDLTCCLCDFGLSLRLDPTLSVDDLANSGQVG TARYMAPEVLESRMNLENVESFKQTDVYSMALVLWE MTSRCNAVGEVKDYEPPFGSKVREHPCVESMKDNVL RDRGRPEIPSFWLNHQGIQMVCETLTECWDHDPEARL TAQCVAERFSELEHLDRLSGRSCSEEKIPEDGSLNTTK | TGFβRII isoform B |
| 72 | GPEPGALCELSPVSASHPVQALMESFTVLSGCASRGTT GLPQEVHVLNLRTAGQGPGQLQREVTLHLNPISSVHIH HKSVVFLLNSPHPLVWHLKTERLATGVSRLFLVSEGSV VQFSSANFSLTAETEERNFPHGNEHLLNWARKEYGAV TSFTELKIARNIYIKVGEDQVFPPKCNIGKNFLSLNYLA EYLQPKAAEGCVMSSQPQNEEVHIIELITPNSNPYSAFQ VDITIDIRPSQEDLEVVKNLILILKCKKSVNWVIKSFDV KGSLKIIAPNSIGFGKESERSMTMTKSIRDDIPSTQGNLV KWALDNGYSPITSYTMAPVANRFHLRLENNAEEMGD EEVHTIPPELRILLDPGALPALQNPPIRGGEGQNGGLPFP FPDISRRVWNEEGEDGLPRPKDPVIPSIQLFPGLREPEEV QGSVDIALSVKCDNEKMIVAVEKDSFQASGYSGMDVT LLDPTCKAKMNGTHFVLESPLNGCGTRPRWSALDGVV YYNSIVIQVPALGDSSGWPDGYEDLESGDNGFPGDMD EGDASLFTRPEIVVFNCSLQQVRNPSSFQEQPHGNITFN MELYNTDLFLVPSQGVFSVPENGHVYVEVSVTKAEQE LGFAIQTCFISPYSNPDRMSHYTIIENICPKDESVKFYSP KRVHFPIPQADMDKKRFSFVFKPVFNTSLLFLQCELTL CTKMEKHPQKLPKCVPPDEACTSLDASIIWA MMQNKKTFTKPLAVIHHEAESKEKGPSMKEPNPISPPIF HGLDTLTVMGIAFAAFVIGALLTGALWYIYSHTGETAG RQQVPTSPPASENSSAAHSIGSTQSTPCSSSSTA | TGFβRIII |
| 73 | LEADKCKEREEKIILVSSANEIDVRPCPLNPNEHKGTI TWYKDDSKTPVSTEQASRIHQHKEKLWFVPAKVED SGHYYCVVRNSSYCLRIKISAKFVENEPNLCYNAQAI FKQKLPVAGDGGLVCPYMEFFKNENNELPKLQWYK DCKPLLLDNIHFSGVKDRLIVMNVAEKHRGNYTCH ASYTYLGKQYPITRVIEFITLEENKPTRPVIVSPANET MEVDLGSQIQLICNVTGQLSDIAYWKWNGSVIDEDD PVLGEDYYSVENPANKRRSTLITVLNISEIESRFYKHP FTCFAKNTHGIDAAYIQLIYPVTNFQKHMIGICVTLT VIIVCSVFIYKIFKIDIVLWYRDSCYDFLPIKASDGKT YDAYILYPKTVGEGSTSDCDIFVFKVLPEVLEKQCG YKLFIYGRDDYVGEDIVEVINENVKKSRRLIIILVRET SGFSWLGGSSEEQIAMYNALVQDGIKVVLLELEKIQ DYEKMPESIKFIKQKHGAIRWSGDFTQGPQSAKTRF WKNVRYHMPVQRRSPSSKHQLLSPATKEKLQREAH VPLG | IL-1RI |
| 74 | SERCDDWGLDTMRQIQVFEDEPARIKCPLFEHFLKF NYSTAHSAGLTLIWYWTRQDRDLEEPINFRLPENRIS KEKDVLWFRPTLLNDTGNYTCMLRNTTYCSKVAFP LEVVQKDSCFNSPMKLPVHKLYIEYGIQRITCPNVDG YFPSSVKPTITWYMGCYKIQNFNNVIPEGMNLSFLIA LISNNGNYTCVVTYPENGRTFHLTRTLTVKVVGSPK NAVPPVIHSPNDHVVYEKEPGEELLIPCTVYFSFLMD SRNEVWWTIDGKKPDDITIDVTINESISHSRTEDETRT QILSIKKVTSEDLKRSYVCHARSAKGEVAKAAKVKQ KVPAPRYTVELACGFGATVLLVVILIVVYHVYWLE MVLFYRAHFGTDETILDGKEYDIYVSYARNAEEEEF VLLTLRGVLENEFGYKLCIFDRDSLPGGIVTDETLSFI QKSRRLLVVLSPNYVLQGTQALLELKAGLENMASR GNINVILVQYKAVKETKVKELKRAKTVLTVIKWKG EKSKYPQGRFWKQLQVAMPVKKSPRRSSDEQGLS YSSLKNV | IL-1RAP |
| 75 | FTLQPAAHTGAARSCRFRGRHYKREFRLEGEPVALR CPQVPYWLWASVSPRINLTWHKNDSARTVPGEEET RMWAQDGALWLLPALQEDSGTYVCTTRNASYCDK MSIELRVFENTDAFLPFISYPQILTLSTSGVLVCPDLSE FTRDKTDVKIQWYKDSLLLDKDNEKFLSVRGTTHLL VHDVALEDAGYYRCVLTFAHEGQQYNITRSIELRIK KKKEETIPVIISPLKTISASLGSRLTIPCKVFLGTGTPLT TMLWWTANDTHIESAYPGGRVTEGPRQEYSENNEN YIEVPLIFDPVTREDLHMDFKCVVHNTLSFQTLRTTV KEASSTFSWGIVLAPLSLAFLVLGGIWMHRRCKHRT GKADGLTVLWPHHQDFQSYPK | IL-1RII |
| 76 | RPSGRKSSKMQAFRIWDVNQKTFYLRNNQLVAGYL QGPNVNLEEKIDVVPIEPHALFLGIHGGKMCLSCVKS GDETRLQLEAVNITDLSENRKQDKRFAFIRSDSGPTT SFESAACPGWFLCTAMEADQPVSLTNMPDEGVMVT KFYFQEDE | IL-1RA |
| 77 | LQCFCHLCTKDNFTCVTDGLCFVSVTETTDKVIHNS MCIAEIDLIPRDRPFVCAPSSKTGSVTTTYCCNQDHC NKIELPTTVKSSPGLGPVEL | ECD of human TGFβRI 34-126 |
| 78 | IPPHVQKSVNNDMIVTDNNGAVKFPQLCKFCDVRFS TCDNQKSCMSNCSITSICEKPQEVCVAVWRKNDENI TLETVCHDPKLPYHDFILEDAASPKCIMKEKKKPGET FFMCSCSSDECNDNIIFSEEYNTSNPD | ECD of human TGFβRII 24-159 |
| 79 | GPEPGALCELSPVSASHPVQALMESFTVLSGCASRGT TGLPQEVHVLNLRTAGQGPGQLQREVTLHLNPISSV HIHHKSVVFLLNSPHPLVWHLKTERLATGVSRLFLV SEGSVVQFSSANFSLTAETEERNFPHGNEHLLNWAR KEYGAVTSFTELKIARNIYIKVGEDQVFPPKCNIGKN FLSLNYLAEYLQPKAAEGCVMSSQPQNEEVHIIELIT PNSNPYSAFQVDITIDIRPSQEDLEVVKNLILILKCKK SVNWVIKSFDVKGSLKIIAPNSIGFGKESERSMTMTK SIRDDIPSTQGNLVKWALDNGYSPITSYTMAPVANR FHLRLENNAEEMGDEEVHTIPPELRILLDPGALPALQ NPPIRGGEGQNGGLPFPFPDISRRVWNEEGEDGLPRP KDPVIPSIQLFPGLREPEEVQGSVDIALSVKCDNEKMI VAVEKDSFQASGYSGMDVTLLDPTCKAKMNGTHFV LESPLNGCGTRPRWSALDGVVYYNSIVIQVPALGDS SGWPDGYEDLESGDNGFPGDMDEGDASLFTRPEIVV FNCSLQQVRNPSSFQEQPHGNITFNMELYNTDLFLVP SQGVFSVPENGHVYVEVSVTKAEQELGFAIQTCFISP YSNPDRMSHYTIIENICPKDESVKFYSPKRVHFPIPQA DMDKKRFSFVFKPVFNTSLLFLQCELTLCTKMEKHP QKLPKCVPPDEACTSLDASIIWA MMQNKKTFTKPLAVIHHEAESKEKGPSMKEPNPISP PIFHGLDTLTV | ECD of human TGFβRIII 21-787 |
| 80 | | hIgG1 aa sequence: |
| 81 | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCP PCPAPEFEGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPASIEK TISKAKGQPREPQVYTLPPSRDELTKNQVSLTCLVKG FYPSDIAVEWESNGQPENNYKTTPPVLDSDGSFFLYS KLTVDKSRWQQGNVFSCSVMHEALHNHYTQKSLSL SPGA | hIgG1_FES (L234F/L235E/P331S) |
| 82 | | hKappa aa sequence |
| 83 | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCP PCPAPEFEGGPSVFLFPPKPKDTLMISRTPEVTCVVVD VSHEDPEVKFNWYVDGVEVHNAKTKPREEQYNSTY RVVSVLTVLHQDWLNGKEYKCKVSNKALPASIEKTI SKAKGQPREPQVYTLPPSRDELTKNQVSLTCLVKGF YPSDIAVEWESNGQPENNYKTTPPVLDSDGSFFLYSK LTVDKSRWQQGNVFSCSVMHEALHNHYTQKSLSLS PGA | hIgG1_FES (L234F/L235E/P331S) |
| 84 | EEELQIIQPDKSVLVAAGETATLRCTITSLFPVGPIQWF RGAGPGRVLIYNQRQGPFPRVTTVSDTTKRNNMDFS IRIGNITPADAGTYYCIKFRKGSPDDVEFKSGAGTELS VRAKPS | SIRPa_CV1 |
| 85 | | 3280A Light chain variable region |
| 86 | | 3280A Heavy chain variable region |
| 87 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APELLGGPSVFLFPPKPKDTLMISRTPEVTCVVVDVSH EDPEVKFNWYVDGVEVHNAKTKPREEQYASTYRVVS VLTVLHQDWLNGKEYKCKVSNKALPAPIEKTISKAKG QPREPQVYTLPPSREEMTKNQVSLTCLVKGFYPSDIAV EWESNGQPENNYKTTPPVLDSDGSFFLYSKLTVDKSR WQQGNVFSCSVMHEALHNHYTQKSLSLSPGA | hIgG1(N297A) |
| 88 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APEFEGGPSVFLFPPKPKDTLMISRTPEVTCVVVDVSH EDPEVKFNWYVDGVEVHNAKTKPREEQYNSTYRVVS VLTVLHQDWLNGKEYKCKVSNKALPASIEKTISKAKG QPREPQVYTLPPCRDELTKNQVSLWCLVKGFYPSDIAV EWESNGQPENNYKTTPPVLDSDGSFFLYSKLTVDKSR WQQGNVFSCSVMHEALHNHYTQKSLSLSPGA | hIgG1_L234F_L235E_ P331S (knob)S354C/ T366W |
| 89 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APEFEGGPSVFLFPPKPKDTLMISRTPEVTCVVVDVSH EDPEVKFNWYVDGVEVHNAKTKPREEQYNSTYRVVS VLTVLHQDWLNGKEYKCKVSNKALPASIEKTISKAKG QPREPQVCTLPPSRDELTKNQVSLSCAVKGFYPSDIAV EWESNGQPENNYKTTPPVLDSDGSFFLVSKLTVDKSR WQQGNVFSCSVMHEALHNHYTQKSLSLSPGK | hIgG1_L234F_L235E_ P331S(hole) T366S/ L368A/Y407V |
| 90 | DIQMTQSPSSLSASVGDRVTITCKASQNVGAAVAWY QQKPGKAPKLLIYSVSDRYTGVPSRFSGSGSGTDFTL TISSLQPEDIATYYCQQYSNYPTFGQGTKLEIKRTVAA PSVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC | AM4B6-La.4-hKappa |
| 91 | QVQLVQSGAEVKKPGASVKVSCKASGYVFTDYYM NWVRQAPGQSLEWMGDINPNNAETLYNHKFKGRVT VTVDKSISTAYMELSRLRSDDTAVYYCVKWGDGPFA YWGQGTLVTVSSASTKGPSVFPLAPSSKSTSGGTAAL GCLVKDYFPEPVTVSWNSGALTSGVHTFPAVLQSSGL YSLSSVVTVPSSSLGTQTYICNVNHKPSNTKVDKKV EPKSCDKTHTCPPCPAPEFEGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPASIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALH NHYTQKSLSLSPGAGGGGSGGGGSGGGGSGGGGSG EEELQIIQPDKSVLVAAGETATLRCTITSLFPVGPIQWF RGAGPGRVLIYNQRQGPFPRVTTVSDTTKRNNMDFS IRIGNITPADAGTYYCIKFRKGSPDDVEFKSGAGTELS VRAKPS | AM4B6_Hg.3hIgG1_ L234F_L235E_P331S-(G4S)4G-SIRPa_CV1 |
| 92 | DIQMTQSPSSLSASVGDRVTITCRASQDVSTAVAWYQ QKPGKAPKLLIYSASFLYSGVPSRFSGSGSGTDFTLTI SSLQPEDFATYYCQQYLYHPATFGQGTKVEIKRTVAA PSVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC | 3280A-L-hKappa |
| 93 | EVQLVESGGGLVQPGGSLRLSCAASGFTFSDSWIHW VRQAPGKGLEWVAWISPYGGSTYYADSVKGRFTISA DTSKNTAYLQMNSLRAEDTAVYYCARRHWPGGFDY WGQGTLVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPELLGGPSVFLFPPKPKDTLMISR TPEVTCVVVDVSHEDPEVKFNWYVDGVEVHNAKT KPREEQYNSTYRVVSVLTVLHQDWLNGKEYKCKVS NKALPAPIEKTISKAKGQPREPQVYTLPPSREEMTKN QVSLTCLVKGFYPSDIAVEWESNGQPENNYKTTPPVL DSDGSFFLYSKLTVDKSRWQQGNVFSCSVMHEALH NHYTQKSLSLSPGAGGGGSGGGGSGGGGSGGGGSG EEELQIIQPDKSVLVAAGETATLRCTITSLFPVGPIQWF RGAGPGRVLIYNQRQGPFPRVTTVSDTTKRNNMDFS IRIGNITPADAGTYYCIKFRKGSPDDVEFKSGAGTELS VRAKPS | 3280A-H-hIgG1(N297A)-(G4S)4G- SIRPα_CV1 |
| 94 | QVQLVQSGAEVKKPGASVKVSCKASGYVFTDYYM NWVRQAPGQSLEWMGDINPNNAETLYNHKFKGRVT VTVDKSISTAYMELSRLRSDDTAVYYCVKWGDGPFA YWGQGTLVTVSSASTKGPSVFPLAPSSKSTSGGTAAL GCLVKDYFPEPVTVSWNSGALTSGVHTFPAVLQSSGL YSLSSVVTVPSSSLGTQTYICNVNHKPSNTKVDKKV EPKSCDKTHTCPPCPAPEFEGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPASIEKTISKAKGQPREPQVYTLPPCRDELTK NQVSLWCLVKGFYPSDIAVEWESNGQPENNYKTTPP VLDSDGSFFLYSKLTVDKSRWQQGNVFSCSVMHEAL HNHYTQKSLSLSPGAGGGGSGGGGSGGGGSGGGGS GEEELQIIQPDKSVLVAAGETATLRCTITSLFPVGPIQ WFRGAGPGRVLIYNQRQGPFPRVTTVSDTTKRNNM DFSIRIGNITPADAGTYYCIKFRKGSPDDVEFKSGAGT ELSVRAKPS | AM4B6_Hg.3_hIgG1_ L234F_L235E_P331S( knob) - SIRPa_CV1 |
| 95 | QVQLVQSGAEVKKPGASVKVSCKASGYVFTDYYM NWVRQAPGQSLEWMGDINPNNAETLYNHKFKGRVT VTVDKSISTAYMELSRLRSDDTAVYYCVKWGDGPFA YWGQGTLVTVSSASTKGPSVFPLAPSSKSTSGGTAAL GCLVKDYFPEPVTVSWNSGALTSGVHTFPAVLQSSGL YSLSSVVTVPSSSLGTQTYICNVNHKPSNTKVDKKV EPKSCDKTHTCPPCPAPEFEGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPASIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGK | AM4B6_Hg.3_hIgG1_ L234F_L235E_P331S( hole) |
| 96 | EVQLVESGGGLVQPGGSLRLSCAASGFTFSDSWIHW VRQAPGKGLEWVAWISPYGGSTYYADSVKGRFTISA DTSKNTAYLQMNSLRAEDTAVYYCARRHWPGGFDY WGQGTLVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPEFEGGPSVFLFPPKPKDTLMISR TPEVTCVVVDVSHEDPEVKFNWYVDGVEVHNAKT KPREEQYNSTYRVVSVLTVLHQDWLNGKEYKCKVS NKALPASIEKTISKAKGQPREPQVYTLPPCRDELTKN QVSLWCLVKGFYPSDIAVEWESNGQPENNYKTTPPV LDSDGSFFLYSKLTVDKSRWQQGNVFSCSVMHEALH NHYTQKSLSLSPGAGGGGSGGGGSGGGGSGGGGSG EEELQIIQPDKSVLVAAGETATLRCTITSLFPVGPIQWF RGAGPGRVLIYNQRQGPFPRVTTVSDTTKRNNMDFS IRIGNITPADAGTYYCIKFRKGSPDDVEFKSGAGTELS VRAKPS | 3280A_H_hIgG1_L234 F_L235E_P331S(knob) - SIRPa_CV1 |
| 97 | EVQLVESGGGLVQPGGSLRLSCAASGFTFSDSWIHW VRQAPGKGLEWVAWISPYGGSTYYADSVKGRFTISA DTSKNTAYLQMNSLRAEDTAVYYCARRHWPGGFDY WGQGTLVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPEFEGGPSVFLFPPKPKDTLMISR TPEVTCVVVDVSHEDPEVKFNWYVDGVEVHNAKT KPREEQYNSTYRVVSVLTVLHQDWLNGKEYKCKVS NKALPASIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NHYTQKSLSLSPGK | 3280A_H_hIgG1_L234 F_L235E_P331S(hole) |
| 98 | EEELQVIQPDKSVLVAAGETATLRCTVTSLIPVGPIQW FRGAGPGRELIYNQKEGHFPRVTTVSDSTKRNNMDF SIRIGNITPADAGTYYCVKFRKGSPDDVEFKSGAGTE LSVRAKPS | SIRPa_WT allele 1 |
| 99 | LQPGAEVPVVWAQEGAPAQLPCSPTIPLQDLSLLRRA GVTWQHQPDSGPPAAAPGHPLAPGHPAAPSSWGPR PRRYTVLSVGPGGLRSGRLPLQPRVQLDERGRQRGD FSLWLRPARRADAGEYRAAVHLRDRALSCRLRLRLG QASMT | LAG3 D1 |
| 100 | LQPGAEVPVVWAQEGAPAQLPCSPTIPLQDLSLLRRA GVTWQHQPDSGPPAAAPGHPLAPGHPAAPSSWGPR PRRYTVLSVGPGGLRSGRLPLQPRVQLDERGRQRGD FSLWLRPARRADAGEYRAAVHLRDRALSCRLRLRLG QASMTASPPGSLRASDWVILNCSFSRPDRPASVHWF RNRGQGRVPVRESPHHHLAESFLFLPQVSPMDSGPW GCILTYRDGFNVSIMYNLTVLGLEPPTPLTVYAGAGS RVGLPC | LAG3 D1+D2 |
| 101 | GPPAAAPGHPLAPGPHPAAPSSWGPRPRR | LAG3 extra-loop |
| 102 | | XOMA052 VH |
| 103 | | XOMA052 VL |
| 104 | TSGMGVG | XOMA052 HCDR1 |
| 105 | HIWWDGDESYNPSLKS | XOMA052 HCDR2 |
| 106 | NRYDPPWFVD | XOMA052 HCDR3 |
| 107 | RASQDISNYLS | XOMA052 LCDR1 |
| 108 | YTSKLHS | XOMA052 LCDR2 |
| 109 | LQGKMLPWT | XOMA052 LCDR3 |
| 110 | | ACZ885 VH |
| 111 | | ACZ885 VL |
| 112 | VYGMN | ACZ885 HCDR1 |
| 113 | IIWYDGDNQYYADSVKG | ACZ885 HCDR2 |
| 114 | DLRTGPFDY | ACZ885 HCDR3 |
| 115 | RASQSIGSSLH | ACZ885 LCDR1 |
| 116 | YASQSFS | ACZ885 LCDR2 |
| 117 | HQSSSLPFT | ACZ885 LCDR3 |
| 118 | QVQLQESGPGLVKPSQTLSLTCSFSGFSLSTSGMGVG WIRQPSGKGLEWLAHIWWDGDESYNPSLKSRLTISK DTSKNQVSLKITSVTAADTAVYFCARNRYDPPWFVD WGQGTLVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPEFEGGPSVFLFPPKPKDTLMISR TPEVTCVVVDVSHEDPEVKFNWYVDGVEVHNAKT KPREEQYNSTYRVVSVLTVLHQDWLNGKEYKCKVS NKALPASIEKTISKAKGQPREPQVYTLPPSRDELTKN QVSLTCLVKGFYPSDIAVEWESNGQPENNYKTTPPVL DSDGSFFLYSKLTVDKSRWQQGNVFSCSVMHEALH NHYTQKSLSLSPGAGGGGSGGGGSGGGGSGGGGSQ VQLVQSGAEVKKPGASVKVSCKASGYVFTDYYMN WVRQAPGQSLEWMGDINPNNAETLYNHKFKGRVTV TVDKSISTAYMELSRLRSDDTAVYYCVKWGDGPFAY WGQGTLVTVSSGGGGSGGGGSGGGGSGGGGSDIQM TQSPSSLSASVGDRVTITCKASQNVGAAVAWYQQKP GKAPKLLIYSVSDRYTGVPSRFSGSGSGTDFTLTISSL QPEDIATYYCQQYSNYPTFGQGTKLTVL | IgG-scFv-XOMA052-AM4B6 Heavy chain (HC) |
| 119 | DIQMTQSTSSLSASVGDRVTITCRASQDISNYLSWYQ QKPGKAVKLLIYYTSKLHSGVPSRFSGSGSGTDYTLT ISSLQQEDFATYFCLQGKMLPWTFGQGTKLEIKRTVA APSVFIFPPSDEQLKSGTASVVCLLNNFYPREAKVQW KVDNALQSGNSQESVTEQDSKDSTYSLSSTLTLSKA DYEKHKVYACEVTHQGLSSPVTKSFNRGEC | IgG-scFv-XOMA052-AM4B6 Light chain (LC) |
| 120 | QVQLVESGGGVVQPGRSLRLSCAASGFTFSVYGMN WVRQAPGKGLEWVAIIWYDGDNQYYADSVKGRFTI SRDNSKNTLYLQMNGLRAEDTAVYYCARDLRTGPF DYWGQGTLVTVSSASTKGPSVFPLAPSSKSTSGGTA ALGCLVKDYFPEPVTVSWNSGALTSGVHTFPAVLQS SGLYSLSSVVTVPSSSLGTQTYICNVNHKPSNTKVDK KVEPKSCDKTHTCPPCPAPEFEGGPSVFLFPPKPKDT LMISRTPEVTCVVVDVSHEDPEVKFNWYVDGVEVH NAKTKPREEQYNSTYRVVSVLTVLHQDWLNGKEYK CKVSNKALPASIEKTISKAKGQPREPQVYTLPPSRDE LTKNQVSLTCLVKGFYPSDIAVEWESNGQPENNYKT TPPVLDSDGSFFLYSKLTVDKSRWQQGNVFSCSVMH EALHNHYTQKSLSLSPGAGGGGSGGGGSGGGGSGG GGSQVQLVQSGAEVKKPGASVKVSCKASGYVFTDY YMNWVRQAPGQCLEWMGDINPNNAETLYNHKFKG RVTVTVDKSISTAYMELSRLRSDDTAVYYCVKWGDG PFAYWGQGTLVTVSSGGGGSGGGGSGGGGSGGGGS DIQMTQSPSSLSASVGDRVTITCKASQNVGAAVAWY QQKPGKAPKLLIYSVSDRYTGVPSRFSGSGSGTDFTL TISSLQPEDIATYYCQQYSNYPTFGCGTKLTVL | IgG-scFv-ACZ885-AM4B6 Heavy chain (HC) |
| 121 | EIVLTQSPDFQSVTPKEKVTITCRASQSIGSSLHWYQQ KPDQSPKLLIKYASQSFSGVPSRFSGSGSGTDFTLTIN SLEAEDAAAYYCHQSSSLPFTFGPGTKVDIKRTVAAP SVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC | IgG-scFv-ACZ885-AM4B6 Light chain (LC) |
| 122 | GGGGSGGGGSGGGGSGGGGS | G4S |

### Example B: formulations and effects thereof

The analytical methods, reagent-related information, and preparation methods of test samples involved in this example are as follows:
1. Dynamic light scattering DLS
   Protein particle size and distribution were determined in dynamic light scattering (DLS, Wyatt, model WP2-09) with the following parameters: the acquisition time was 5s, 20 acquisitions per measurement, and the measure temperature was 25°C.
2. Size exclusion chromatography SEC

Protein aggregation was determined by SEC method using Agilent 1260 system and TSKgel G3000SWXL column (300×7.8 mM, 5 µM). The mobile phase was 50 mM sodium phosphate buffer, 300 mM NaCl, pH 6.8±0.1. The flow rate was 1.0 mL/min. The sample was diluted to 10 mg/mL, and the detection volume was 10 µL, with detection wavelength at 280 nm.

High molecular weight polymer (at least dimer) HMW% in the sample was determined by SEC

### 3. Non-reducing capillary electrophoresis NR CE-SDS

Protein fragments were determined by the CE-SDS (NR) method. The standard or test sample was diluted to 4 mg/mL with a phosphate-citrate buffer, and then 25 µL of the sample was subjected to denaturation treatment by vortex mixing with 75 µL of SDS sample buffer and 5 µL of NEM (100 mM N-ethylmaleimide). The denatured samples were centrifuged, incubated at 70±2°C for 10±2 min, cooled at room temperature, and then centrifuged again. Separation was performed on PA800 plus using SDS separation gel kits and uncoated fused silica capillaries.

Small molecule fragments (the molecular weight is lower than monomer) LMW% in samples were determined by NR CE-SDS.

### 4. Binding activity

The coating antigen was TGFβ1, the detection antibody was Goat anti-Human IgG-Fc HRP conjugated, and the enzyme reaction substrate was TMB. The antigen was coated and adsorbed onto a 96-well high-adsorption plate, which was washed and blocked, and then the test sample was added to bind, incubated and washed. The detection antibody was added, incubated and washed to remove unbound detection antibody, and then the substrate was added to develop color. Finally, the reaction stop solution (1 M sulfuric acid) was added, and the absorbance was read on a plate reader at a detection wavelength of 450 nm and a reference wavelength of 650 nm.

### 5. Cell activity

The cells were plated in a 96-well detection plate, and the reference and test samples were diluted from 10 µg/mL as the first well to 0.0195 µg/mL with a 2-fold gradient, and then added to the 96-well detection plate. The cells with the gradient diluted samples were placed in an incubator (37.0°C±1.0°C, 5.0±1.0% CO₂) for 4-5 hours. The inhibition of the interaction between PD-1 and PD-L1 cells by the bi-functional protein molecules prepared in above examples, such as AM4B6-hIgG1-TGFβRII', was detected using a Bio-Lite^{™} chemiluminescence assay kit or equivalent reagents. The intensity of the chemiluminescence is linearly related to the degree of inhibition in each well. The dose-dependent curve was fitted with a four-parameter model, and the relative activity of the sample was calculated by the ratio of the half-effective concentration of the reference to the sample.

### 6. Flow injection HPLC fluorescence analysis method

The concentration of polysorbate 80 was determined by flow injection HPLC fluorescence analysis. Agilent 1260 system with fluorescence detector was used, excitation wavelength was 350 nm, emission wavelength was 420 nm, mobile phase was 0.15 M sodium chloride, 0.025 M sodium tetraborate, 5.0% acetonitrile, 5.0 µM naphthylaminobenzene, 2.5 ppm polysorbate 80.

### 7. The reagent-related information is as follows:

| Reagent name | Supplier | Grade | Catalog Number |
|---|---|---|---|
| Acetic acid | J.T. Baker | USP, EP, BP, JP | 9526-03 |
| Sodium acetate trihydrate | Merck | Ph Eur, BP, JP, USP | 1.37012.1000 |
| L-arginine hydrochloride | Merck | Ph Eur, JP, USP | 1.01544.1000 |
| Polysorbate 80 | NOF | JP, USP, EP, ChP | Polysorbate (HX2) |
| Sucrose | Pfanstiehl | Ph Eur, ChP, JP, NF | S-124-2-MC |
| Sorbitol | MERCK | EMPROVE^{®}exp, Ph Eur, BP, JP, NF | 1.11597.9028 |
| Sodium chloride | MERCK | EMPROVE^{®}exp, Ph Eur, BP, JP, USP | 1.16224.5000 |
| Disodium edetate | Titriplex III (EDTA·2Na) | Ph Eur,BP,JP,USP,ACS | 1.37004.1000 |

8. Unless otherwise specified, the test formulation samples in this example are prepared as follows:
(1). The buffer of the protein stock solution (i.e., PDL1/TGFβ protein (AM4B6-hIgG1-TGFβRII')) was replaced with the target buffer system using a dialysis method: a certain volume of the sample was placed in a Snake Skin^{®} dialysis bag, sealed, and placed in a target buffer with a volume of ≥100 times, stirred continuously to promote replacement. Dialysis was performed 3 times for 4 h, 4 h and overnight, respectively, at a stirring speed of 300 rpm.
(2). After dialysis, the required amount of stabilizer, surfactant mother liquor, and other excipients were added to the protein stock solution, which was diluted with the target buffer to the target protein concentration, and then sterile filtered and aseptically filled to obtain the test formulation solution. The specific parameters of the formulation are as described in the formula design table below, respectively .

### Example B-1: Study on the effects of pH, surfactants, and other excipients on formulation properties

### 1. Materials and methods

DOE (Design of Experiment) formula design was conducted using custom design of JMP software. The screened formulations include 20 mg/mL PDL1/TGFβ protein (AM4B6-hIgG1-TGFβRII'), 20 mM acetic acid - sodium acetate buffer solution with pH 4.5 to 5.5, 135 mM to 280 mM other excipients, and 0.025 to 0.1% (w/v, g/100 mL) of polysorbate 80. The specific formula design is shown in Table 23.

The test samples of all formulas were examined for particle size at the initial time T0, stability at 40°C for 4 weeks and under stirring at 25°C for 3 hours as shown in the following table.

**Table 23. Formula design**

| **Formula** | **pH** | **PS80 Concentration (w/v, %)** | **Buffer** | **Other Excipients** | **Stability conditions and test items** |
|---|---|---|---|---|---|
| F1 | 4.5 | 0.025 | 20 mM acetic acid - sodium acetate buffer | 150 mM arginine hydrochloride | 1. T0 test: dynamic light scattering DLS, size exclusion chromatography SEC, non-reducing capillary electrophoresis NR CE-SDS |
| F2 | 4.5 | 0.025 | 20 mM acetic acid - sodium acetate buffer | 260 mM sucrose | |
| F3 | 4.5 | 0.1 | 20 mM acetic acid - sodium acetate buffer | 135 mM sodium chloride | |
| F4 | 4.5 | 0.1 | 20 mM acetic acid - sodium acetate buffer | 280 mM sorbitol | |
| F5 | 5.0 | 0.025 | 20 mM acetic acid - sodium acetate buffer | 135 mM sodium chloride | |
| F6 | 5.0 | 0.025 | 20 mM acetic acid - sodium acetate buffer | 280 mM sorbitol | 2. 40°C (4W) test: SEC, NR CE-SDS |
| F7 | 5.0 | 0.1 | 20 mM acetic acid - sodium acetate buffer | 150 mM arginine hydrochloride | |
| F8 | 5.0 | 0.1 | 20 mM acetic acid - sodium acetate buffer | 260 mM sucrose | 3. Stirring (3hr) test: SEC, NR CE-SDS |
| F9 | 5.5 | 0.025 | 20 mM acetic acid - sodium acetate buffer | 150 mM arginine hydrochloride | |
| F10 | 5.5 | 0.025 | 20 mM acetic acid - sodium acetate buffer | 260 mM sucrose | |
| F11 | 5.5 | 0.1 | 20 mM acetic acid - sodium acetate buffer | 135 mM sodium chloride | |
| F12 | 5.5 | 0.1 | 20 mM acetic acid - sodium acetate buffer | 280 mM sorbitol | |

### 2. Experimental results and analysis

### 2.1 Particle size of each test sample at the initial time T0

The particle size of the samples was measured by a dynamic light scattering instrument. The detection conditions are as described above.

Particle size at TO (DLS) results are shown in Table 24. From the experimental results, it can be seen that the particle size of the formulation containing sucrose and sorbitol is obviously increased along with the increase of pH (F2, F4, F6, F8, F10 and F12, increased from ~7 nm to ~15 nm), and particularly, the particle size of the formula containing sucrose or sorbitol (F6, F8, F10 and F12) was obviously larger than that of the formula containing arginine hydrochloride or NaCl when the pH is 5.0 and 5.5. The experimental results show that the formulations containing arginine hydrochloride or NaCl do not have obvious difference in TO particle size at different pH levels. This indicates that the osmotic pressure regulator arginine hydrochloride or NaCl can also reduce the production of protein polymers, which is beneficial for protein stability.

**Table 24. DLS results**

| **Formula** | **Particle size** |
|---|---|
| F1 | 7.4 |
| F2 | 6.2 |
| F3 | 7.3 |
| F4 | 6.7 |
| F5 | 7.3 |
| F6 | 9.2 |
| F7 | 7.3 |
| F8 | 10.4 |
| F9 | 7.6 |
| F10 | 14.8 |
| F11 | 7.5 |
| F12 | 13.8 |

### 2.2 Stability of each test sample after 4 weeks at 40°C

Each formulation sample was left at 40°C for 4 weeks (T4w). SEC and NR CE-SDS were used for detection to obtain the high molecular weight polymer HMW% (measured by SEC) and small molecular fragment LMW% (measured by NR CE-SDS) data of the sample at TO (initial moment) and T4w. The changes in high molecular weight polymer HMW% and small molecular fragment LMW% (ΔHMW% and ΔLMW%) are the difference between T4w and T0.

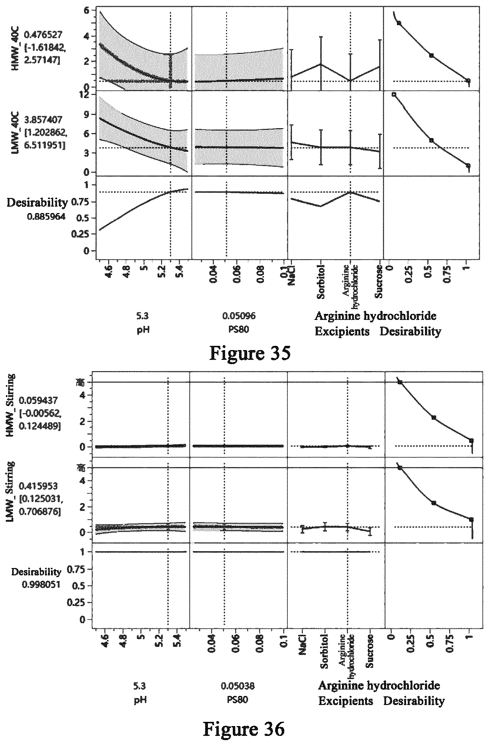

ΔHMW% and ΔLMW% were input as response values into JMP software, and the regression model was fitted using the least squares method. The model fitting R² for both HMW and LMW was 0.96, indicating a high degree of data fitting. Figure 35 shows the results of the data analysis by JMP software. Within the range of 0.025% to 0.1% (w/v), the concentration of PS80 has almost no effect on HMW and LMW. The type of excipient has an impact on HMW, where formulations containing sucrose and sorbitol show a greater increase in HMW; compared with formulations containing sucrose and sorbitol, formulations containing arginine hydrochloride and sodium chloride show a significantly reduced increase in HMW. This indicates that osmotic pressure regulators, such as arginine hydrochloride or NaCl, can also reduce the production of protein polymers, which is beneficial for protein stability. In addition, it can be seen from Figure 35 that within the pH range of 4.5 to 5.5, the higher the pH, the more favorable it is for the stability of PD-L1/TGF-β protein, and the slower the increase in HMW and LMW, i.e., the pH range of 5.0 to 5.5 is a superior pH range.

### 2.3 Stability under stirring conditions

Each formulation sample was stirred at 25°C for 3 hours (T3h). SEC and NR CE-SDS were used for detection to obtain the high molecular weight polymer HMW% (measured by SEC) and small molecular fragment LMW% (measured by NR CE-SDS) data of the sample at TO (initial time) and T3h. The changes in high molecular weight polymer HMW% and small molecular fragment LMW% (ΔHMW% and ΔLMW%) are the difference between T3h and T0.

ΔHMW% and ΔLMW% were input as response values into JMP software, and the regression model was fitted using the least squares method. The model fitting R² for HMW and LMW were 0.91 and 0.93, respectively, indicating a high degree of data fitting. Figure 36 shows the results of the data analysis by JMP software. It can be seen from Figure 36 that under the test conditions, pH, PS80 concentration, and the type of excipient have no effect on HMW and LMW for F1-F12.

In summary, TO particle size, stability at 40°C, and stirring experiments show that within the pH range of 5.0 to 5.5, the increase in HMW and LWM is relatively slow, and the pH has almost no effect on the change in HMW and LMW. Therefore, the preferred pH range is 5.0 to 5.5. Arginine hydrochloride or NaCl not only plays a role in osmotic pressure regulator but also reduces the production of protein polymers, which is beneficial for protein stability. Therefore, the preferred type of excipient is arginine hydrochloride or sodium chloride. The concentration of PS80 has almost no effect on HMW and LMW within the range of 0.025% to 0.1% (w/v).

### Example B-2: Comparative experiment of different buffer systems

### 1. Materials and methods

Under pH 5.5, the effects of acetate buffer system and histidine buffer systems on protein stability were compared. The specific formula design is shown in Table 25.

**Table 25. Comparing of formula design with different buffer systems**

| **Formula** | **Protein concentration (mg/mL)** | **pH** | **PS80 concentration (w/v, %)** | **Buffer** | **Excipient** | **Stability conditions and test items** |
|---|---|---|---|---|---|---|
| F11 | 20 | 5.5 | 0.1 | 20 mM acetic acid - sodium acetate buffer | 135 mM sodium chloride | **40°C** (2W, 4W): SEC, NR CE-SDS |
| F13 | 20 | 5.5 | 0.1 | 20 mM histidine buffer | 135 mM sodium chloride | **40°C** (2W, 4W): SEC, NR CE-SDS |

### 2. Results and Analysis of Different Buffer Systems

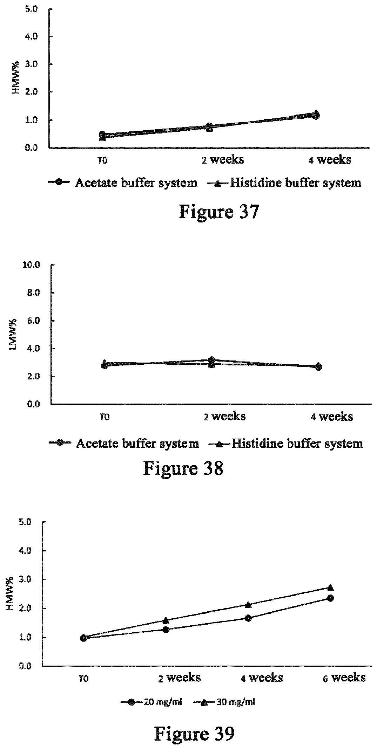

The two formulation samples were placed at 40°C for 4 weeks (T4w). SEC and NR CE-SDS were used for detection to obtain the high molecular weight polymer HMW% (measured by SEC) and small molecular fragment LMW% (measured by NR CE-SDS) data of the samples at TO (initial time), T2w, and T4w. As shown in Figures 37 and 38, in both acetate buffer system and histidine buffer system, the changes in high molecular weight polymer HMW and small molecular fragment LMW of PD-L1/TGF-β protein are small, indicating that the type of buffer salt has no effect on protein stability, i.e., both buffer systems can meet the protein stability requirements.

### Example B-3: Comparative experiment of different protein concentrations

### 1. Materials and methods

In this example, the protein concentration was screened. According to the results of Example B-1, Figure 35, the preferred pH range is 5.0 to 5.5. When the pH was 5.3, HMW was hardly increased under 40°C conditions, and LMW was only slightly increased, as shown in Figure 35. Therefore, the buffer solution with pH 5.3 was chosen as the buffer system in this example. Also according to the results in example B-1, Figure 35, the concentration of polysorbate 80 from 0.025% to 0.1% has no effect on protein stability, so an intermediate value of 0.05% (w/v) polysorbate 80 was chosen as the concentration of the surfactant in this experiment. The specific formula design and test conditions are shown in Table 26.

**Table 26. Formula design for different protein concentrations**

| **Formula** | **Protein concentration (mg/mL)** | **PS80 concentration (w/v, %)** | **Buffer** | **Excipient** | **Stability conditions and test items** |
|---|---|---|---|---|---|
| F14 | 20 | 0.05 | 20 mM acetic acid - sodium acetate buffer, pH5.3 | 150 mM arginine hydrochloride | 40°C (2W, 4W, 6W): SEC, NR CE-SDS |
| F15 | 30 | 0.05 | 20 mM acetic acid - sodium acetate buffer, pH5.3 | 150 mM arginine hydrochloride | |

### 2. Results and Analysis

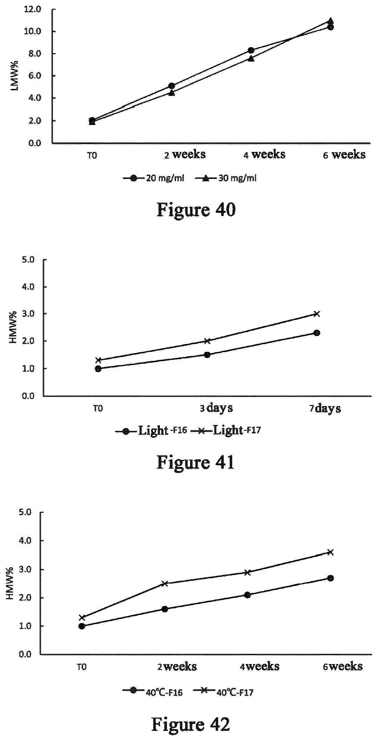

The two formulation samples were left at 40°C for 6 weeks (T6w). SEC and NR CE-SDS were used for detection to obtain the high molecular weight polymer HMW% (measured by SEC) and small molecular fragment LMW% (measured by NR CE-SDS) data of the samples at TO (initial moment), T2w, T4w, and T6w. As shown in Figures 39 and 40, the change trends of high molecular weight polymer HMW and small molecular fragment LMW of PD-L1/TGF-β protein are very close when the protein concentrations are 20 mg/mL and 30 mg/mL, indicating that the stability of PD-L1/TGF-β protein is similar within the range of 20 mg/mL to 30 mg/mL, meeting the stability requirements.

### Example B-4: Comparative experiment of different excipients

### 1. Materials and methods

In this example, the type of excipient was further screened. According to the results of Example B-1, a 20 mM acetic acid - sodium acetate buffer solution with pH 5.3 was chosen as the buffer system, 0.05% (w/v) polysorbate 80 as the surfactant, and arginine hydrochloride or sodium chloride as the stabilizer and osmotic pressure regulator. The specific formula design and test conditions are shown in Table 27.

**Table 27. Formula design of further screening excipients**

| **Formula** | **Protein concentration (mg/mL)** | **PS80 concentration (w/v, %)** | **Buffer** | **Excipient** | **Stability conditions** |
|---|---|---|---|---|---|
| F16 | 30 | 0.05 | 20 mM acetic acid - sodium acetate buffer, pH5.3 | 150 mM arginine hydrochloride | 5 Freeze-thaw cycles (-80°C freezing/25°C thawing): DLS, SEC, NR CE-SDS; Shaking for 7 days (speed: 200 rpm, temperature: 25°C): DLS, SEC, NR CE-SDS; |
| F17 | 30 | 0.05 | 20 mM acetic acid - sodium acetate buffer, pH5.3 | 135 mM sodium chloride | |
| | | | | | Light exposure for 7 days (light intensity: 7850 Lux): DLS, SEC, NR CE-SDS, binding activity, and cell activity; |
| | | | | | 40°C for 6 weeks: DLS, SEC, NR CE-SDS, binding activity, and cell activity; |
| | | | | | 25°C for 6 weeks: DLS, SEC, NR CE-SDS |

### 2. Results and analysis

### 2.1 Freeze-thaw and shaking results

Under freeze-thaw and shaking conditions, F16 and F17 showed no significant changes in appearance, protein particle size, HMW, and LMW, and there were also no significant changes in these aspects compared to T0. This indicates that F16 and F17 meet the stability requirements of the drug formulation under freeze-thaw and shaking conditions.

### 2.2 Light exposure results

As shown in Figure 41, under light exposure conditions, arginine hydrochloride has a significant advantage in inhibiting HMW compared with sodium chloride, showing that it is particularly beneficial for maintaining protein stability. In terms of appearance, protein particle size, LMW, and activity, F16 and F17 showed no significant changes, and there were also no significant changes in these aspects compared to T0. This indicates that F16 and F17 meet the stability requirements of the drug formulation under light exposure conditions, and F16 shows superior stability.

### 2.3 40°C results

As shown in Figure 42, arginine hydrochloride has a significant advantage in inhibiting HMW at 40°C compared with sodium chloride, showing that it is particularly beneficial for maintaining protein stability. In terms of appearance, protein particle size, and activity, F16 and F17 showed no significant changes; the increase in LMW of F17 was slightly less than that of F16, and the advantage was not significant; and there were also no significant changes in these aspects compared to T0. This indicates that F16 and F17 meet the stability requirements of the drug formulation at 40°C, and F16 shows superior stability.

### 2.4 25°C results

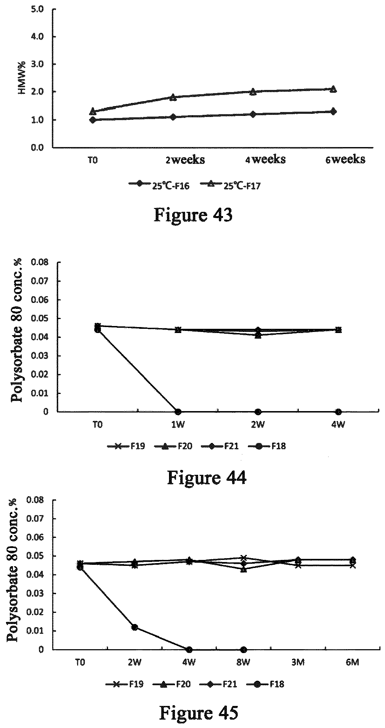

As shown in Figure 43, arginine hydrochloride has a significant advantage in inhibiting HMW at 25°C compared with sodium chloride. In terms of appearance, protein particle size, and LMW, F16 and F17 showed no significant changes, and there were also no significant changes in these aspects compared to T0. This indicates that F16 and F17 meet the stability requirements of the drug formulation under light exposure conditions, and F16 shows superior stability.

Considering the results under various conditions, arginine hydrochloride can better slow down the increase of HMW under light exposure, forced degradation at high temperature (40°C), and accelerated (25°C) conditions. In terms of LMW and activity, there is no significant difference in the effect of arginine hydrochloride and sodium chloride. Therefore, in this example, the formula with the best formulation stability is: 30 mg/mL PD-L1/TGF-β protein, 20 mM acetic acid - sodium acetate buffer solution with pH 5.3, 150 mM arginine hydrochloride, and 0.05% (w/v) polysorbate 80.

### Example B-5: Investigation of the protective effect of disodium edetate

### 1. Materials and methods

On the basis of the preferred formulation in Example B-4, further investigate the effect of adding disodium edetate on the stability of protein and polysorbate 80. The specific formula design and test conditions are shown in Table 28.

**Table 28. Formula Design**

| **Formula** | **Protein concentration (mg/mL)** | **Buffer** | **PS80 concentration (w/v, %)** | **Excipient** | **Disodium edetate concentration** | **Stability conditions** |
|---|---|---|---|---|---|---|
| F18 | 30 | 20 mM acetic acid - sodium acetate buffer, pH5.3 | 0.05 | 150 mM arginine hydrochloride | 0 | 40°C |
| | | | | | | 25°C |
| | | | | | | 5°C |
| F19 | 30 | 20 mM acetic acid - sodium acetate buffer, pH5.3 | 0.05 | 150 mM arginine hydrochloride | 75 µM | |
| F20 | 30 | 20 mM acetic acid - sodium acetate buffer, pH5.3 | 0.05 | 150 mM arginine hydrochloride | 150 µM | |
| F21 | 30 | 20 mM acetic acid - sodium acetate buffer, pH5.3 | 0.05 | 150 mM arginine hydrochloride | 300 µM | |

### 2. Results and analysis

As shown in Figures 44 and 45, within the tested range of 75 µM to 300 µM, disodium edetate can provide good protection for polysorbate 80 in the PD-L1/TGF-β protein formulation, and almost no degradation of polysorbate 80 occurred after storage at 40°C for one month and at 25°C for 6 months.

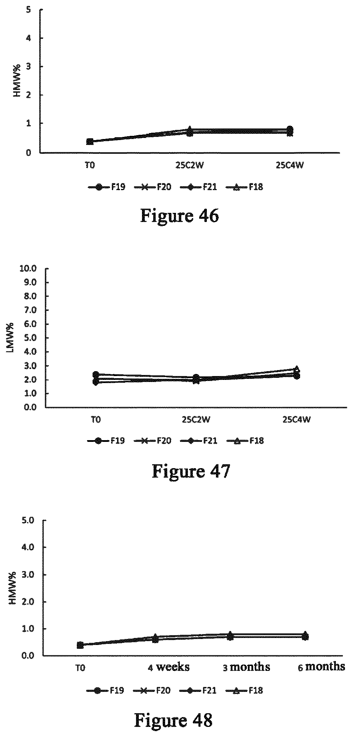

As shown in Figures 46, 47, 48, and 49, F19, F20, and F21 have similar effects to F18 in preventing the increase of HMW and LWM, indicating that the addition of disodium edetate does not affect the stability of the protein. The main effect of disodium edetate is to prevent the degradation of polysorbate 80, thereby improving the safety of the formulation.

### Example B-6: Investigation of the effect of adding disodium edetate in the formulation preparation process

### 1. Materials and methods

Based on Example B-5, investigate whether adding disodium edetate in the process can still prevent the degradation of polysorbate 80. The process is described as follows:
To a 40 mg/mL protein solution (i.e., PDL1/TGFβ protein (AM4B6-hIgG1-TGFβRII')) in a 20 mM acetic acid - sodium acetate buffer with pH of 5.3 was added disodium edetate mother liquid to achieve final concentrations of 0.5 mM and 5 mM, and the resulting solutions were left at room temperature overnight.

The protein solution obtained in the above steps was placed in a Thermo dialysis bag (product number: 88245) and dialyzed three times with 20 mM acetic acid - sodium acetate buffer solution with pH of 5.3, wherein each dialysis time being ≥4 hours, to remove disodium edetate.

The formulation solution (30 mg/mL PD-L1/TGF-β protein, 20 mM acetic acid - sodium acetate buffer solution pH 5.3, 150 mM arginine hydrochloride, 0.05% (w/v) polysorbate 80) was prepared from the dialyzed protein solution according to the general method in Example B, Section (2), and the degradation of polysorbate 80 under 40°C conditions was examined.

At the same time, a control solution was also prepared by a similar method, except that the protein solution was not treated with disodium edetate.

### 2. Results and Analysis

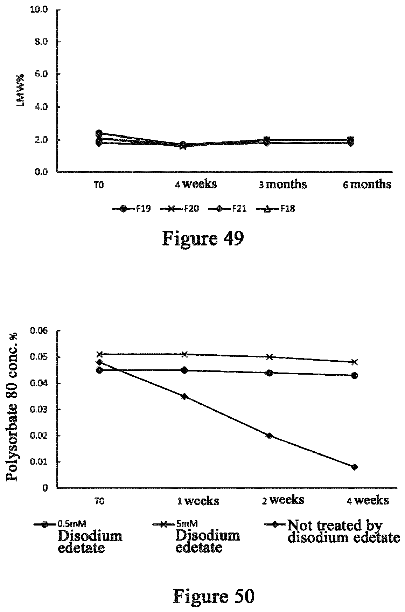

As shown in Figure 50, compared with the formulation not treated with disodium edetate, the formulations treated with 0.5 mM or 5 mM disodium edetate can prevent the degradation of polysorbate 80 at 40°C, reduce the production of degradation by-products, and thus improve the safety of the formulation.

## Claims

1. A protein formulation, comprising:
(1) a bi-functional molecule, comprising:
a first moiety that binds to an immune checkpoint molecule, the first moiety preferably is an antibody against PD-L1 or an antigen-binding fragment thereof, and
a second moiety that i) blocks activity of an immunosuppressive cytokine or ii) stimulates immunity, the second moiety preferably is a TGFβ-binding moiety, an IL-1-binding moiety, a LAG-3-binding moiety or a Sirpa-binding moiety, wherein the TGFβ-binding moiety preferably is a soluble TGFβ receptor (TGFβR) or a TGFβ-binding fragment or variant thereof, or an antibody against TGFβ and an antigen-binding fragment thereof;
(2) a buffer; and
(3) a surfactant.

2. The protein formulation according to claim 1, comprising:
(1) a bi-functional molecule comprising a first moiety that binds to PD-L1 and a second moiety that a) blocks activity of an immunosuppressive cytokine or b) stimulates immunity, wherein the first moiety comprises an antibody against PD-L1 or an antigen-binding fragment thereof comprising a heavy chain variable (VH) region and/or a light chain variable (VL) region, wherein the heavy chain variable region comprises:
a) a HCDR1 comprising **DYYMN** (SEQ ID NO: 1) or a homologous sequence of at least 80% sequence identity thereof,
b) a HCDR2 comprising **DINPNNX₁X₂TX₃YNHKFKG** (SEQ ID NO: 19) or a homologous sequence of at least 80% sequence identity thereof, and
c) a HCDR3 comprising **WGDGPFAY** (SEQ ID NO: 3) or a homologous sequence of at least 80% sequence identity thereof, and/or
wherein the light chain variable region comprises:
d) a LCDR1 comprising a sequence selected from the group consisting of **KASQNVX₄X₅X₆VA** (SEQ ID NO: 20) or a homologous sequence of at least 80% sequence identity thereof,
e) a LCDR2 comprising a sequence selected from the group consisting of **SX₇SX₈RYT** (SEQ ID NO: 21) or a homologous sequence of at least 80% sequence identity thereof, and
f) a LCDR3 comprising a sequence selected from the group consisting of **QQYSNYPT** (SEQ ID NO: 6) or a homologous sequence of at least 80% sequence identity thereof;
wherein X₁ is G or A, X₂ is G or D or Q or E or L, X₃ is S or M or Q or L or V, X₄ is G or P or K, X₅ is A or G, X₆ is A or I, X₇ is A or N or R or V, and X₈ is N or H or V or D;
(2) a buffer; and
(3) a surfactant.

3. The protein formulation according to any of the preceding claims, wherein the first moiety of the bi-functional molecule comprises the antibody against PD-L1 or an antigen-binding fragment thereof, and the second moiety comprises TGFβ-binding moiety,
wherein the antibody against PD-L1 or an antigen-binding fragment thereof comprises a pair of heavy chain variable region and light chain variable region sequences selected from the group consisting of: SEQ ID NO: 49/54, 50/54, 51/54, 52/54, 49/55, 50/55, 51/55, 52/55, 58/62, 58/63, 58/64, 58/65, 59/62, 59/63, 59/64, 59/65, 60/62, 60/63, 60/64 and 60/65 and a homologous sequence thereof having at least 80% sequence identity thereof; and/or
wherein the TGFβ-binding moiety comprises a soluble TGFβ receptor (TGFβR) or a TGFβ-binding fragment or variant thereof, wherein the soluble TGFβR comprises an extracellular domain (ECD) of the TGFβR or a TGFβ-binding fragment or variant thereof, and the ECD of TGFβR comprises an amino acid sequence of SEQ ID NO: 66, 79, 78, 77 or a sequence having at least 80% sequence identity thereof yet retains specific binding specificity and/or affinity to TGF-β.

4. The protein formulation according to any of the preceding claims, wherein the concentration of the bi-functional molecule is about 1-150 mg/mL, for example about 10-50 mg/mL or about 20-30 mg/mL.

5. The protein formulation according to any of the preceding claims, wherein the buffer is acetate, histidine, citrate, succinate, malate, glutamate, phosphate, or lactate buffer, for example acetic acid-sodium acetate buffer or histidine-hydrochloric acid buffer.

6. The protein formulation according to any of the preceding claims, wherein the concentration of the buffer is about 5-100 mM, for example about 10-50 mM, about 15-30 mM or about 20 mM.

7. The protein formulation according to any of the preceding claims, wherein the pH of said formulation is in the range of about 4.5-6.0, for example about 5.0-5.6, about 5.0-5.5 or about 5.3.

8. The protein formulation according to any of the preceding claims, wherein the surfactant is nonionic surfactant, preferably polysorbate, for example polysorbate 80 or polysorbate 20.

9. The protein formulation according to any of the preceding claims, wherein the concentration of the surfactant is about 0.01-0.1% (w/v), for example about 0.025%-0.1% (w/v) or about 0.05% (w/v).

10. The protein formulation according to any of the preceding claims, wherein the formulation comprises a stabilizer, for example, selected from one or more of an amino acid, an inorganic salt, a sugar, a polyol and a chelating agent, and preferably is arginine hydrochloride or NaCl.

11. The protein formulation according to any of the preceding claims, wherein the formulation comprises a stabilizer, the concentration of which is about 10-300 mM, for example about 100-300 mM, about 120-300 mM, about 100-200 mM, about 130-170 mM or about 150 mM.

12. The protein formulation according to any of the preceding claims, wherein the formulation comprises arginine hydrochloride, the concentration of which preferably is about 100-200 mM, about 130-170 mM or about 150 mM.

13. The protein formulation according to any of the preceding claims, wherein the formulation optionally comprises a chelating agent, for example EDTA•2Na, the concentrationg of which preferably is about 30-350 µM.

14. The protein formulation according to any of the preceding claims, comprising:
10-50 mg/mL of a bi-functional molecule;
10-50 mM of an acetate buffer or histidine buffer;
0.025%-0.1% (w/v) of polysorbate 80 or polysorbate 20;
100-200 mM of arginine hydrochloride; and
optionally 30-350 µM of EDTA•2Na;
the pH is 5.0-5.6;
or comprising:
20-30 mg/mL of a bi-functional molecule;
20-30 mM of an acetate buffer or histidine buffer;
0.025%-0.1% (w/v) of polysorbate 80; and
140-160 mM of arginine hydrochloride;
the pH is 5.3±5%.

15. The protein formulation according to any of the preceding claims, wherein the bi-functional molecule is treated with a chelating agent, for example EDTA•2Na (e.g., about 0.3-10 mM of EDTA•2Na) during the preparation process, and then the chelating agent is removed.

16. The protein formulation according to any of the preceding claims, which is in a liquid form, a lyophilized form or a liquid form reconstituted from a lyophilized form, for example an injection, for example an intravenous injection, an intramuscular injection or a subcutaneous injection.

17. The protein formulation according to any of claims 1-16, which is used for treating, preventing, or alleviating PD-L1-related diseases in a subject, for example said disease is an immune-related disease or condition, cancer, or an infectious disease, for example said cancer is selected from the group consisting of: lung cancer (e.g., non-small cell lung cancer), liver cancer, pancreatic cancer, breast cancer, bronchial cancer, bone cancer, liver and bile duct cancer, ovarian cancer, testicle cancer, kidney cancer, bladder cancer, head and neck cancer, spine cancer, brain cancer, cervix cancer, uterine cancer, endometrial cancer, colon cancer, colorectal cancer, prostate cancer, gastric-esophageal cancer, rectal cancer, anal cancer, gastrointestinal cancer, skin cancer, pituitary cancer, stomach cancer, vagina cancer, thyroid cancer, glioblastoma, astrocytoma, melanoma, myelodysplastic syndrome, sarcoma, teratoma, glioma, and adenocarcinoma, for example said subject has been identified as having a PD-L1-expressing cancer cell, for example wherein the PD-L1-related disease is resistant to PD-L1/PD-1 mono therapy.

18. The protein formulation according to any of claims 1-16, which is used for treating, preventing, or alleviating a disease or condition that would benefit from suppression of an immunosuppressive cytokine, from induction of sustained immune responses, or from stimulation of anti-tumor immunity;
wherein, for example, the immunosuppressive cytokine is TGFβ; for example, said disease or condition is a TGFβ-related disease or condition; for example, said TGFβ-related disease is cancer, fibrotic disease, or kidney disease;
wherein, for example, the immunosuppressive cytokine is IL-1; for example, said disease or condition is an IL-1-related disease or condition;
wherein, for example, said disease or condition would benefit from induction of sustained immune responses by stimulating MHCII signaling with an immunostimulatory polypeptide; for example, said immunostimulatory polypeptide is a soluble LAG-3; or
wherein, for example, said disease or condition would benefit from stimulation of anti-tumor immunity by inhibiting an immunoinhibitory receptor signaling; for example, said immunoinhibitory receptor is SIRPα.
